# EUROPÄISCHE PATENTSCHRIFT

(11) **EP 4 519 383 B1**
(45) Veröffentlichungstag und Bekanntmachung des Hinweises auf die Patenterteilung: **14.01.2026**
(21) Anmeldenummer: 23724726.7
(22) Anmeldetag: 03.05.2023
(51) Int. Cl.: C09K 11/06, C07D 253/00, C07D 247/00, H01L 21/00, C07C 211/56, C07C 211/60, C07C 211/61, C07D 209/56, C07D 209/86, C07D 235/08, C07D 235/16, C07D 251/24, C07D 333/76, C07D 403/04, C07D 403/10, C07D 403/12, C07D 403/14, C07D 405/04, C07D 405/12

(54) **CYCLISCHE VERBINDUNGEN FÜR ORGANISCHE ELEKTROLUMINESZENZVORRICHTUNGEN**
CYCLIC COMPOUNDS FOR ORGANIC ELECTROLUMINESCENT DEVICES
COMPOSÉS CYCLIQUES POUR DISPOSITIFS ÉLECTROLUMINESCENTS ORGANIQUES

(30) Priorität: 06.05.2022 EP 22172121
(43) Veröffentlichungstag der Anmeldung: 12.03.2025
(73) Patentinhaber: Merck Patent GmbH, 64293 Darmstadt (DE)
(72) Erfinder: STOESSEL, Philipp, 64293 Darmstadt (DE)
(74) Vertreter: Merck Patent Association
(86) Internationale Anmeldenummer: PCT/EP2023/061596
(87) Internationale Veröffentlichungsnummer: WO 2023/213837

(56) Entgegenhaltungen:
- EP-A1- 3 363 782
- WO-A1-2021/129072
- WO-A1-2022/012439
- WO-A1-2022/042267
- US-A1- 2023 217 825

## Beschreibung

Die vorliegende Erfindung betrifft cyclische Verbindungen für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese Materialien.

In organischen Elektrolumineszenzvorrichtungen werden als emittierende Materialien häufig phosphoreszierende metallorganische Komplexe eingesetzt. Aus quantenmechanischen Gründen ist unter Verwendung metallorganischer Verbindungen als Phosphoreszenzemitter eine bis zu vierfache Energie- und Leistungseffizienz möglich. Generell gibt es bei Elektrolumineszenzvorrichtungen, insbesondere auch bei Elektrolumineszenzvorrichtungen, die Triplettemission (Phosphoreszenz) zeigen, immer noch Verbesserungsbedarf. Die Eigenschaften phosphoreszierender Elektrolumineszenzvorrichtungen werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu deutlichen Verbesserungen der Eigenschaften der Elektrolumineszenzvorrichtungen führen.

Unter anderem werden die zuvor dargelegten Elektrolumineszenz-vorrichtungen in Dokument WO 2014/015938 A1 beschrieben. Im Patent EP3363782A1 sind Verbindungen offenbart, die denen der vorliegenden Anmeldung entfernt ähneln und in einem elektronischen Gerät verwendet werden.

Generell besteht bei diesen Materialien, beispielsweise für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Darüber hinaus ist es die Aufgabe der vorliegenden Erfindung Verbindungen bereitzustellen, die sich durch einen niedrigen Brechungsindex (Refractive Index RI) auszeichnen.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Matrixmaterial. Insbesondere ist es eine Aufgabe der vorliegenden Erfindung, Matrixmaterialien bereitzustellen, welche sich für rot und gelb phosphoreszierende Elektrolumineszenzvorrichtungen, insbesondere für rot phosphoreszierende Elektrolumineszenzvorrichtungen und gegebenenfalls auch für blau phosphoreszierende Elektrolumineszenzvorrichtungen eignen.

Weiterhin sollten die Verbindungen, insbesondere bei ihrem Einsatz als Matrixmaterialien, als Lochtransportmaterialien oder als Elektronenblockiermaterialien in organischen Elektrolumineszenzvorrichtung zu Vorrichtungen führen, die eine ausgezeichnete Farbreinheit aufweisen.

Eine weitere Aufgabe kann darin gesehen werden, elektronische Vorrichtungen mit einer ausgezeichneten Leistungsfähigkeit möglichst kostengünstig und in konstanter Qualität bereitzustellen Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, unten näher beschriebene Verbindungen diese Aufgabe lösen, sich gut für die Verwendung in Elektrolumineszenzvorrichtungen eignen und zu organischen Elektrolumineszenzvorrichtungen führen, die insbesondere in Bezug auf die Lebensdauer, der Farbreinheit, der Effizienz, der Betriebsspannung und dem Brechungsindex sehr gute Eigenschaften vorweisen. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung umfassend mindestens eine Struktur der Formel (I), vorzugsweise Verbindung gemäß der Formel (I), 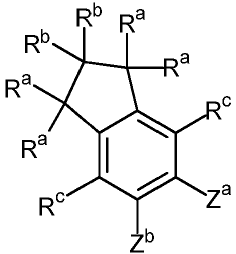 wobei für die Symbole gilt:
- Z^{a}: steht bei jedem Auftreten gleich oder verschieden für Ar^{a}, N(Ar^{c})₂ oder (Ar)N(Ar^{c})₂;
- Z^{b}: steht bei jedem Auftreten gleich oder verschieden für Ar^{b}, N(Ar^{d})₂ oder (Ar)N(Ar^{d})₂;
- R^{a}: ist bei jedem Auftreten gleich oder verschieden eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten R² substituiert sein kann, vorzugsweise eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, dabei können zwei oder mehrere, vorzugsweise benachbarte Substituenten R^{a} miteinander ein Ringsystem bilden;
- R^{b}: ist bei jedem Auftreten gleich oder verschieden eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten R² substituiert sein kann, vorzugsweise eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, dabei können zwei benachbarte Substituenten R^{b} miteinander ein Ringsystem bilden;
- R^{c}: ist bei jedem Auftreten gleich oder verschieden H, D, eine geradkettige Alkyl-, Alkoxy- oder Thioalkoxygruppe mit 1 bis 10 C-Atomen, vorzugsweise 1 bis 6 C-Atomen oder eine verzweigte oder cyclische Alkyl-, Alkoxy- oder Thioalkoxygruppe mit 3 bis 10 C-Atomen, die jeweils mit einem oder mehreren Resten R² substituiert sein kann, vorzugsweise H, D, eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, bevorzugt H, D, eine geradkettige Alkylgruppe mit 1 bis 10 C-Atomen, vorzugsweise 1 bis 6 C-Atomen, besonders bevorzugt H, D;
- Ar^{a}, Ar^{b}, Ar^{c}, Ar^{d}: ist bei jedem Auftreten gleich oder verschieden ein aromatisches oder heteroaromatisches Ringsystem mit 6 bis 60 aromatischen Ringatomen, das mit einem oder mehreren Resten R substituiert sein kann, dabei können zwei Reste Ar^{c}, Ar^{d}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein, vorzugsweise steht Ar^{a}, Ar^{b}, Ar^{c}, Ar^{d} bei jedem Auftreten gleich oder verschieden für eine Aryl- oder Heteroarylgruppe mit 6 bis 40 aromatischen Ringatomen, die mit einem oder mehreren Resten R substituiert sein kann, dabei können zwei Reste Ar, Ar^{c}, Ar^{d}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein,
- Ar: ist bei jedem Auftreten gleich oder verschieden ein verbindendes aromatisches oder heteroaromatisches Ringsystem mit 5 bis 60 aromatischen Ringatomen, das mit einem oder mehreren Resten R substituiert sein kann, dabei ein Rest Ar mit einem oder beiden der Reste Ar^{c}, Ar^{d}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein, vorzugsweise steht Ar bei jedem Auftreten gleich oder verschieden für eine Arylen- oder Heteroarylengruppe mit 5 bis 40 aromatischen Ringatomen, die mit einem oder mehreren Resten R substituiert sein kann, dabei ein Rest Ar mit einem oder beiden der Reste Ar^{c}, Ar^{d}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein;
- R: ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R auch miteinander oder einer weiteren Gruppe ein Ringsystem bilden;
- Ar': ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R', miteinander verbrückt sein;
- R¹: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden, dabei können einer oder mehrere Reste R¹ mit einem weiteren Teil der Verbindung ein Ringsystem bilden;
- Ar": ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C- Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden;
wobei die Gruppen Z^{a} und Z^{b} kein Ringsystem bilden.

Die Gruppen Z^{a} und Z^{b} bilden kein Ringsystem. Dies bedeutet, dass die beiden Gruppen nur durch die in Formel (I) dargelegte Grundstruktur miteinander verbunden sind, nicht jedoch über Reste Ar, Ar^{a}, Ar^{b}, Ar^{c}, Ar^{d}, über die die Gruppen Z^{a} und Z^{b} definiert sind. Dies schließt Reste R, R¹ und R² ein, durch die die Reste Ar, Ar^{a}, Ar^{b}, Ar^{c}, Ar^{d}, substituiert sein können.

Ferner kann vorgesehen sein, dass die Verbindung in Bezug auf die Gruppen Z^{a} und Z^{b} symmetrisch ist.

Weiterhin kann vorgesehen sein, dass die Verbindung in Bezug auf die Gruppen Z^{a} und Z^{b} unsymmetrisch ist.

Eine Arylgruppe im Sinne dieser Erfindung enthält 6 bis 40 C-Atome; eine Heteroarylgruppe im Sinne dieser Erfindung enthält 3 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem. Ein heteroaromatisches Ringsystem im Sinne dieser Erfindung enthält 3 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

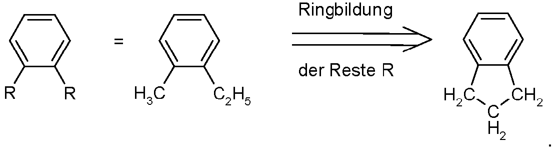

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

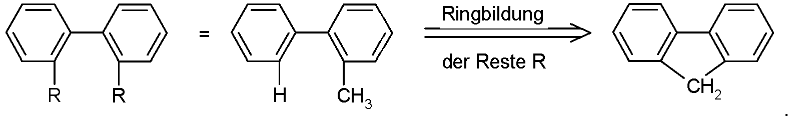

In einer bevorzugten Ausgestaltung können die erfindungsgemäßen Verbindungen vorzugsweise mindestens eine Struktur der Formeln (I-1) bis (1-6), und sind besonders bevorzugt ausgewählt aus den Verbindungen der Formeln (I-1) bis (I-6), 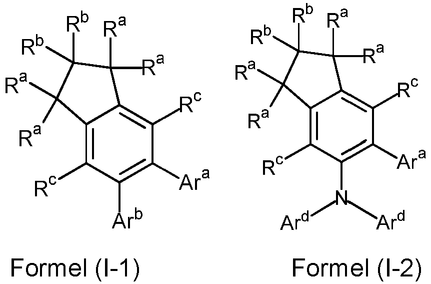 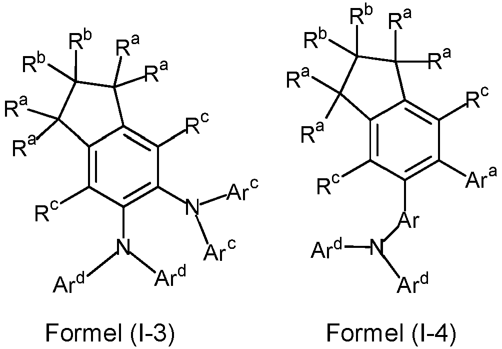 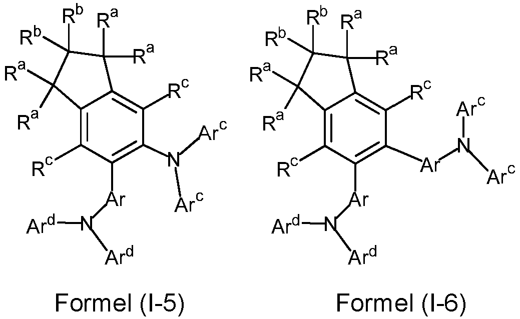 wobei die Symbole Ar, Ar^{a}, Ar^{b}, Ar^{c}, Ar^{d}, R^{a}, R^{b} und R^{c} die zuvor, insbesondere für Formel (I) genannten Bedeutungen aufweisen.

Ferner kann vorgesehen sein, dass die Gruppe Ar, Ar^{a}, Ar^{b}, Ar^{c} und/oder Ar^{d} gleich oder verschieden bei jedem Auftreten ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Anthracen, Pyren, Perylen, Chrysen, Phenanthren oder Triphenylen, welche jeweils mit einem oder mehreren Resten R substituiert sein können, vorzugsweise Phenyl, Biphenyl, Fluoren, Dibenzofuran, Triphenylen, Carbazol, Indolocarbazol.

Vorzugsweise kann vorgesehen sein, dass die Gruppe Ar^{a}, Ar^{b}, Ar^{c} und/oder Ar^{d} gleich oder verschieden bei jedem Auftreten ausgewählt ist aus Strukturen der Formeln (Ar^{a}-1) bis (Ar^{a}-29), 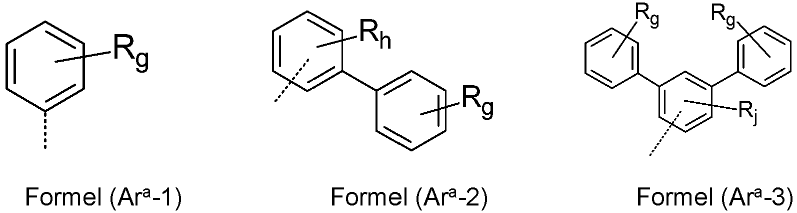 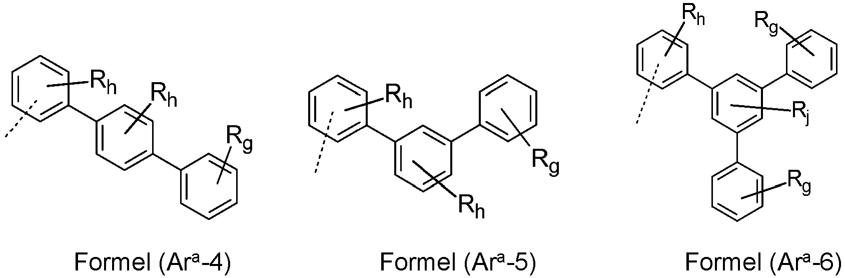 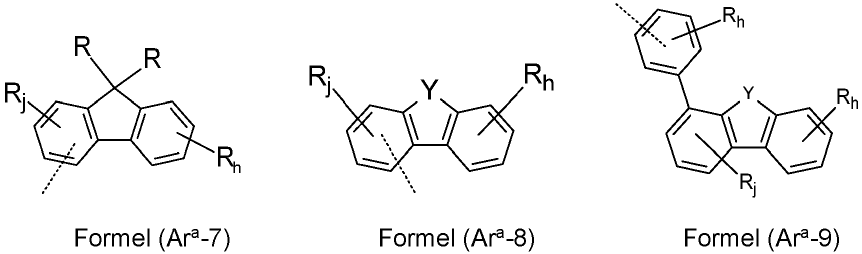 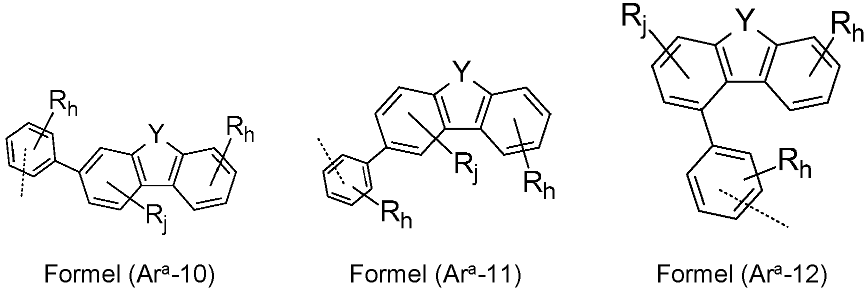 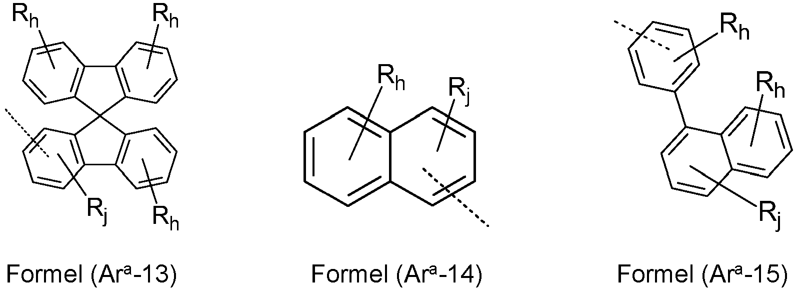 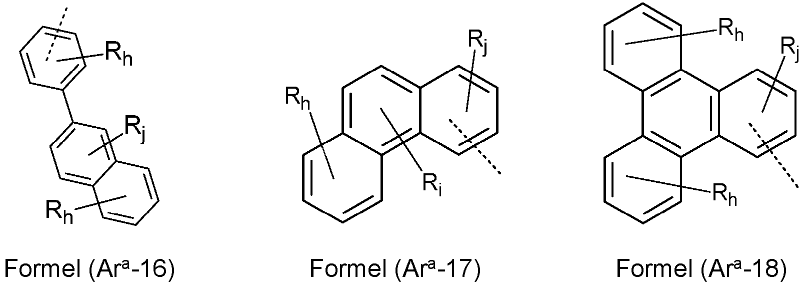 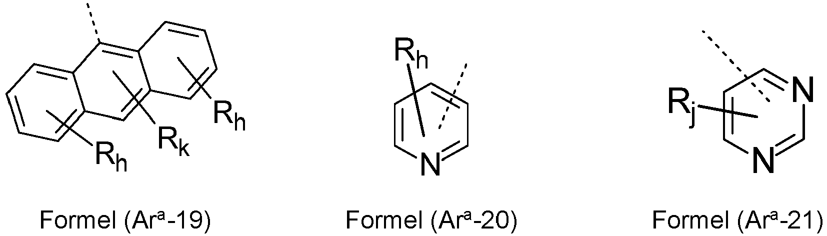 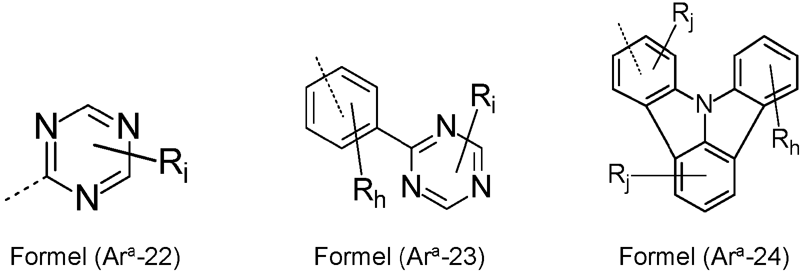 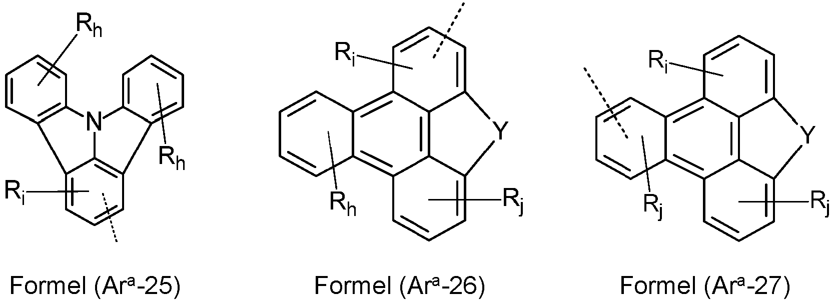 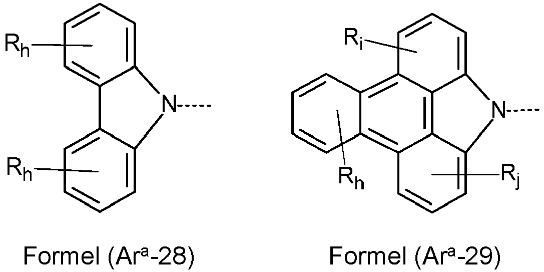 wobei für die verwendeten Symbole gilt:
- Y: ist O, S oder NR, vorzugsweise O oder NR;
- k: ist bei jedem Auftreten unabhängig 0 oder 1;
- i: ist bei jedem Auftreten unabhängig 0, 1 oder 2;
- j: ist bei jedem Auftreten unabhängig 0, 1, 2 oder 3;
- h: ist bei jedem Auftreten unabhängig 0, 1, 2, 3 oder 4;
- g: ist bei jedem Auftreten unabhängig 0, 1, 2, 3, 4 oder 5;
- R: weist die zuvor, insbesondere für Formel (I) genannte Bedeutung auf und die gestrichelte Bindung markiert die Anbindungsposition.

Vorzugsweise kann vorgesehen sein, dass die Gruppe Ar^{a} und/oder Ar^{b} für eine Struktur der Formel -Ar^{e}-Q steht, wobei Ar^{e} bei jedem Auftreten gleich oder verschieden ein aromatisches oder heteroaromatisches Ringsystem mit 5 bis 40 aromatischen Ringatomen ist, das mit einem oder mehreren Resten R substituiert sein kann, vorzugsweise steht Ar^{e} bei jedem Auftreten gleich oder verschieden für eine Aryl- oder Heteroarylgruppe mit 5 bis 30 aromatischen Ringatomen, die mit einem oder mehreren Resten R substituiert sein kann, vorzugsweise ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Anthracen, Pyren, Perylen, Chrysen, Phenanthren oder Triphenylen, welche jeweils mit einem oder mehreren Resten R substituiert sein können, vorzugsweise Phenyl, Biphenyl, Fluoren, Dibenzofuran, Triphenylen, Carbazol, Indolocarbazol; und Q für eine Elektronentransportgruppe, vorzugsweise für eine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe steht, die mit einem oder mehreren Resten R substituiert sein kann.

Bevorzugte aromatische bzw. heteroaromatische Ringsysteme Ar und/oder Ar^{e} sind ausgewählt aus Phenylen, Biphenylen,, insbesondere ortho-, meta- oder para- Biphenylen, Terphenylen, insbesondere ortho-, meta-, para- oder verzweigtem Terphenylen, Quaterphenylen, insbesondere ortho-, meta-, para- oder verzweigtem Quaterphenylen, Fluorenylen, welches über die 1-, 2-, 3- oder 4-Position verknüpft sein kann, Spirobifluorenylen, welches über die 1-, 2-, 3- oder 4-Position verknüpft sein kann, Naphthylen, insbesondere 1- oder 2-verknüpftem Naphthylen, Indolylen, Benzofuranylen, Benzothiophenylen, Carbazolylen, welches über die 1-, 2-, 3-, 4- oder 9-Position verknüpft sein kann, Dibenzofuranylen, welches über die 1-, 2-, 3- oder 4-Position verknüpft sein kann, Benzothiophenylen, welches über die 1-, 2-, 3- oder 4-Position verknüpft sein kann, Carbazolylen, Indenocarbazolylen, Indolocarbazolylen, Pyridinylen, Pyrimidinylen, Pyrazinylen, Pyridazinylen, Triazinylen, Chinolinylen, Isochinolinylen, Chinazolinylen, Chinoxalinylen, Phenanthrenylen oder Triphenylenylen, welche jeweils mit einem oder mehreren Resten R substituiert sein können.

In einer weiterhin bevorzugten Ausgestaltung kann vorgesehen sein, dass die Gruppe Ar und/oder Ar^{e} gleich oder verschieden bei jedem Auftreten ausgewählt ist aus Strukturen der Formeln (Ar^{e}-1) bis (Ar^{a}-9), 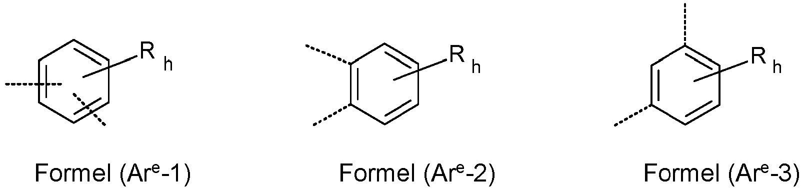 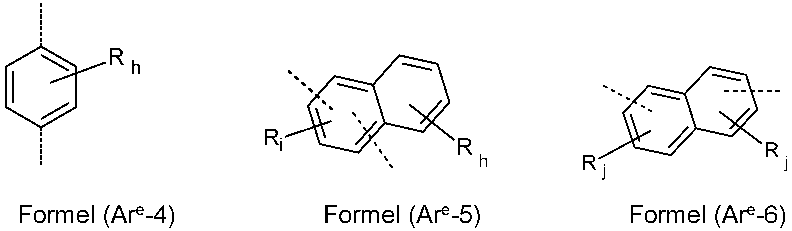 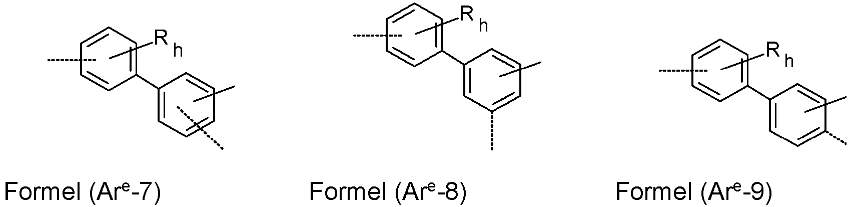 wobei für die verwendeten Symbole gilt:
- j: ist bei jedem Auftreten unabhängig 0, 1, 2 oder 3;
- h: ist bei jedem Auftreten unabhängig 0, 1, 2, 3 oder 4;
- R: weist die zuvor, insbesondere für Formel (I) genannte Bedeutung auf und die gestrichelte Bindung markiert die Anbindungsposition.

In einer bevorzugten Ausführungsform kann vorgesehen sein, dass die Gruppe Q gleich oder verschieden bei jedem Auftreten ausgewählt ist aus Strukturen der Formeln (Q-1) bis (Q-8), 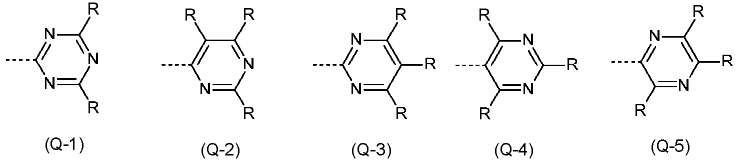 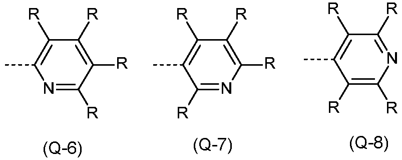 wobei R die zuvor, insbesondere für Formel (I) genannte Bedeutung aufweist und die gestrichelte Bindung die Anbindungsposition markiert.

In einer speziell bevorzugten Ausgestaltung kann vorgesehen sein, dass die Gruppe Q gleich oder verschieden bei jedem Auftreten ausgewählt ist aus Strukturen der Formeln (Q-1a), (Q-1b), (Q-1c), (Q-1d), (Q-1e), (Q-1f), (Q-1g), (Q-1h), (Q-1i) und/oder (Q-1j), 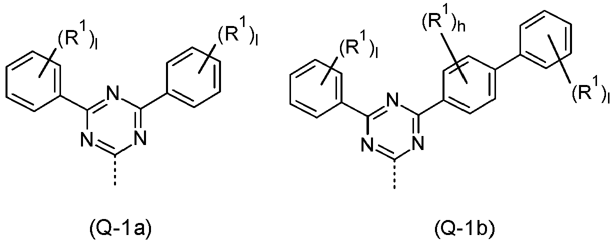 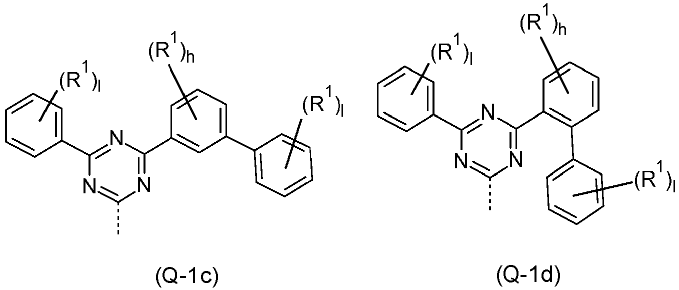 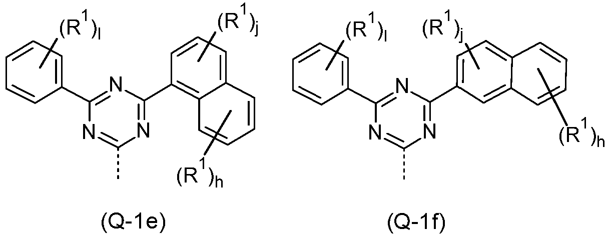 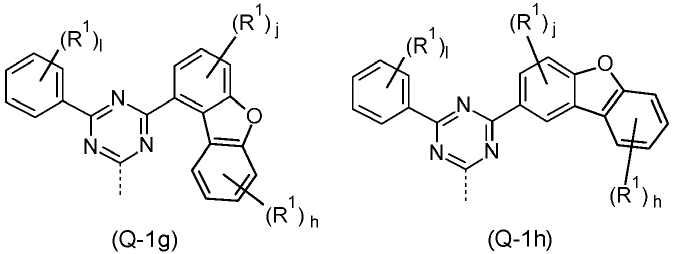 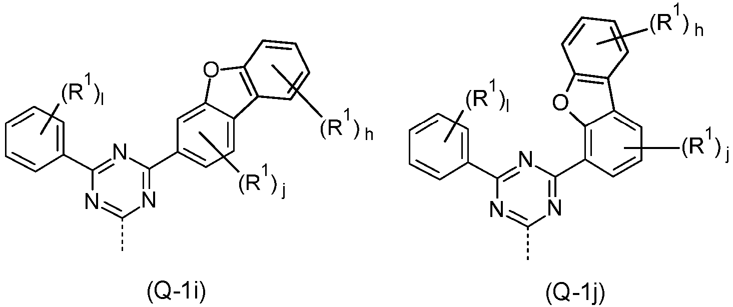 wobei R¹ die zuvor, insbesondere für Formel (I) genannte Bedeutung aufweist, die gestrichelten Bindung die Anbindungsposition markiert, der Index I ist bei jedem Auftreten gleich oder verschieden 0, 1, 2, 3, 4 oder 5, vorzugsweise 0, 1, 2 oder 3, besonders bevorzugt 0 oder 1; der Index h ist bei jedem Auftreten gleich oder verschieden 0, 1, 2, 3 oder 4, vorzugsweise 0, 1 oder 2, besonders bevorzugt 0 oder 1; der Index j ist bei jedem Auftreten gleich oder verschieden 0, 1, 2 oder 3, vorzugsweise 0, 1 oder 2, besonders bevorzugt 0 oder 1. Hierbei sind Strukturen der Formel (Q 1a) bevorzugt.

In einer Ausführungsform kann vorgesehen sein, dass die Verbindung mindestens eine Struktur gemäß Formel (I-2) umfasst, wobei die Gruppe Ar^{a} ausgewählt ist aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2) und (Ar^{a}-7) bis (Ar^{a}-18). Hierbei sind in einer bevorzugten Ausgestaltung die Gruppen Ar^{d} vorzugsweise durch eine Einfachbindung verbunden, wobei diese Gruppen Ar^{d} besonders bevorzugt einen Carbazol-Rest bilden.

In einer Ausführungsform kann vorgesehen sein, dass die Verbindung mindestens eine Struktur gemäß Formel (I-4) umfasst, wobei die Gruppe Ar ausgewählt ist aus Strukturen der Formeln (Ar^{a}-1) bis (Ar^{e}-4), die Gruppe Ar^{a}, ausgewählt ist aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist, und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18). Hierbei sind in einer bevorzugten Ausgestaltung die Gruppen Ar^{d} oder mindestens eine der Gruppen Ar^{d} mit der Gruppe Ar durch eine Einfachbindung verbunden, wobei diese Gruppen Ar^{d} oder mindestens eine der Gruppen Ar^{d} mit der Gruppe Ar besonders bevorzugt einen Carbazol-Rest bilden.

In einer Ausführungsform kann vorgesehen sein, dass die Verbindung mindestens eine Struktur gemäß Formel (I-3) umfasst, wobei die Gruppen Ar^{c} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, vorzugsweise sind die Gruppen Ar^{c} bei jedem Auftreten gleich oder verschieden ausgewählt aus Strukturen der Formeln (Ar^{a}-1), (Ar^{a}-2), (Ar^{a}-7) bis (Ar^{a}-18); und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18). Hierbei sind in einer bevorzugten Ausgestaltung die Gruppen Ar^{c} vorzugsweise durch eine Einfachbindung verbunden, wobei diese Gruppen Ar^{c} besonders bevorzugt einen Carbazol-Rest bilden. In einer speziell bevorzugten Ausgestaltung sind jeweils die Gruppen Ar^{c} und jeweils die Gruppen Ar^{d} durch eine Einfachbindung verbunden, wobei diese Gruppen Ar^{c} und Ar^{d} besonders bevorzugt jeweils einen Carbazol-Rest bilden.

In einer Ausführungsform kann vorgesehen sein, dass die Verbindung mindestens eine Struktur gemäß Formel (I-5) und/oder Formel (I-6) umfasst, wobei die Gruppe Ar bei jedem Auftreten gleich oder verschieden ausgewählt ist aus Strukturen der Formeln (Ar^{e}-1) bis (Ar^{e}-4), die Gruppen Ar^{c} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, vorzugsweise sind die Gruppen Ar^{c} bei jedem Auftreten gleich oder verschieden ausgewählt aus Strukturen der Formeln (Ar^{a}-1), (Ar^{a}-2), (Ar^{a}-7) bis (Ar^{a}-18); und die Gruppen Ar^{d} bei jedem Auftreten gleich oder verschieden ausgewählt sind aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18). Hierbei sind in einer bevorzugten Ausgestaltung die Gruppen Ar^{c} oder mindestens eine der Gruppen Ar^{c} mit der Gruppe Ar vorzugsweise durch eine Einfachbindung verbunden, wobei diese Gruppen Ar^{c} oder mindestens eine der Gruppen Ar^{c} mit der Gruppe Ar besonders bevorzugt einen Carbazol-Rest bilden. In einer weiteren bevorzugten Ausgestaltung sind die Gruppen Ar^{d} oder mindestens eine der Gruppen Ar^{d} mit der Gruppe Ar vorzugsweise durch eine Einfachbindung verbunden, wobei diese Gruppen Ar^{d} oder mindestens eine der Gruppen Ar^{d} mit der Gruppe Ar besonders bevorzugt einen Carbazol-Rest bilden. In einer speziell bevorzugten Ausgestaltung sind die Gruppen Ar^{c} oder mindestens eine der Gruppen Ar^{c} mit der Gruppe Ar durch eine Einfachbindung verbunden, wobei diese Gruppen vorzugsweise einen Carbazol-Rest bilden, und die Gruppen Ar^{d} oder mindestens eine der Gruppen Ar^{d} mit der Gruppe Ar sind durch eine Einfachbindung verbunden, wobei diese Gruppen vorzugsweise einen Carbazol-Rest bilden. Diese speziell bevorzugten Verbindungen weisen demgemäß zwei Carbazol-Reste auf, wobei ein Carbazol-Rest eine Gruppe Ar^{c} umfasst und ein Carbazol-Rest eine Gruppe Ar^{d} umfasst.

Vorzugsweise kann vorgesehen sein, dass die Verbindung mindestens eine Struktur gemäß Formel (I-2) umfasst, wobei die Gruppe Ar^{a} für eine Struktur der Formel Ar^{e}-Q steht, wobei die Symbole Ar^{e} und Q die zuvor genannte Bedeutung aufweisen und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18). Hierbei sind in einer bevorzugten Ausgestaltung die Gruppen Ar^{d} vorzugsweise durch eine Einfachbindung verbunden, wobei diese Gruppen Ar^{d} besonders bevorzugt einen Carbazol-Rest bilden.

Darüber hinaus kann vorgesehen sein, dass die Verbindung mindestens eine Struktur gemäß Formel (I-4) umfasst, wobei die Gruppe Ar ausgewählt ist aus Strukturen der Formeln (Ar^{e}-1) bis (Ar^{e}-4), die Gruppe Ar^{a} für eine Struktur der Formel Ar^{e}-Q steht, wobei die Symbole Ar^{e} und Q die zuvor genannte Bedeutung aufweisen und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18). Hierbei sind in einer bevorzugten Ausgestaltung die Gruppen Ar^{d} oder mindestens eine der Gruppen Ar^{d} mit der Gruppe Ar durch eine Einfachbindung verbunden, wobei diese Gruppen Ar^{d} oder mindestens eine der Gruppen Ar^{d} mit der Gruppe Ar besonders bevorzugt einen Carbazol-Rest bilden.

In einer weiteren Ausgestaltung kann vorgesehen sein, dass die Verbindung mindestens eine Struktur gemäß Formel (I-1) und/oder Formel (I-2) umfasst, wobei die Gruppe Ar^{a} eine Elektronentransportgruppe umfasst oder für eine Elektronentransportgruppe steht, wobei Gruppe Ar^{a} vorzugsweise eine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe umfasst oder für eine der genannten Gruppen steht, wobei diese durch einen oder mehrere Reste R substituiert sein können, wobei Triazin-Gruppen besonders bevorzugt sind.

Vorzugsweise kann vorgesehen sein, dass die Verbindung mindestens eine Struktur gemäß Formel (I-1) umfasst, wobei die Gruppe Ar^{b} eine Elektronentransportgruppe umfasst oder für eine Elektronentransportgruppe steht, wobei Gruppe Ar^{b} vorzugsweise eine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe umfasst oder für eine der genannten Gruppen steht, wobei diese durch einen oder mehrere Reste R substituiert sein können, wobei Triazin-Gruppen besonders bevorzugt sind.

Besonders bevorzugt kann vorgesehen sein, dass die Verbindung mindestens eine Struktur gemäß Formel (I-1) umfasst, wobei die Gruppe Ar^{a} für eine Struktur der Formel Ar^{e}-Q steht und die Gruppe Ar^{b} für eine Struktur der Formel Ar^{e}-Q steht, wobei die Symbole Ar^{e} und Q die zuvor genannte Bedeutung aufweisen.

In einer weiteren Ausgestaltung kann vorgesehen sein, dass die Verbindung mindestens eine Struktur gemäß Formel (I-1) umfasst, wobei die Gruppe Ar^{a} eine Elektronentransportgruppe umfasst oder für eine Elektronentransportgruppe steht, wobei Gruppe Ar^{a} vorzugsweise eine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe umfasst oder für eine der genannten Gruppen steht, wobei diese durch einen oder mehrere Reste R substituiert sein können, wobei Triazin-Gruppen besonders bevorzugt sind und die Gruppe Ar^{b} ausgewählt ist aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist; wobei die Gruppe Ar^{a} vorzugsweise für eine Struktur der Formel Ar^{e}-Q steht, wobei die Symbole Ar^{e} und Q die zuvor genannte Bedeutung aufweisen.

Die vorliegenden Verbindungen eignen sich insbesondere als Hostmaterial für Emitter, vorzugsweise als Hostmaterial für Singulett-, Triplett- und TADF Emitter, Elektronentransportmaterial, Elektroneninjektionsmaterial, Lochleitermaterial, Loch-injektionsmaterial, Elektronenblockiermaterial, Lochblockiermaterial in einer elektronischen Vorrichtung. Die spezifischen Eigenschaften der Verbindungen sind dabei abhängig von der Art und Anzahl der jeweiligen funktionalen Gruppen. Verbindungen, die ein, zwei oder mehr Elektronentransportgruppen, jedoch keine Lochtransportgruppe umfassen, eignen sich insbesondere als Hostmaterial, Elektronentransportmaterial, Elektroneninjektionsmaterial und/oder Lochblockiermaterial. Verbindungen, die ein, zwei oder mehr Lochtransportgruppen, jedoch keine Elektronentransportgruppe umfassen, eignen sich insbesondere als Hostmaterial, Lochleitermaterial, Loch-injektionsmaterial und/oder Elektronenblockiermaterial. Verbindungen, die ein, zwei oder mehr Lochtransportgruppen und ein, zwei oder mehr Elektronentransportgruppen umfassen, eignen sich insbesondere als Hostmaterial.

Elektronentransportgruppen sind in der Fachwelt weithin bekannt und fördern die Fähigkeit von Verbindungen Elektronen zu transportieren und/oder zu leiten. Beispiele für Elektronentransportgruppen sind Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppen.

Lochtransportgruppen sind in der Fachwelt ebenfalls bekannt, wobei diese vorzugsweise Triarylamin- oder Carbazolgruppen umfassen.

Ferner kann vorgesehen sein, dass die Gruppen Ar, Ar^{b}, Ar^{c}, Ar^{d} keine Triazin-Gruppe, vorzugsweise keine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe und besonders bevorzugt keine Elektronentransportgruppe umfassen.

Weiterhin kann vorgesehen sein, dass die Gruppen Ar, Ar^{a}, Ar^{b}, Ar^{c}, Ar^{d} keine Triazin-Gruppe, vorzugsweise keine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe und besonders bevorzugt keine Elektronentransportgruppe umfassen.

Darüber hinaus kann vorgesehen sein, dass die Gruppen Ar, Ar^{a}, Ar^{c}, Ar^{d} keine Carbazol-Gruppe, vorzugsweise keine Carbazol-Gruppe und/oder keine Substituenten der Formel N(Ar')₂, N(R¹)₂, und besonders bevorzugt keine Lochtransportgruppe umfassen.

Des Weiteren kann vorgesehen sein, dass die Gruppen Ar, Ar^{b}, Ar^{c}, Ar^{d} keine Carbazol-Gruppe, vorzugsweise keine Carbazol-Gruppe und/oder keine Substituenten der Formel N(Ar')₂, N(R¹)₂, und besonders bevorzugt keine Lochtransportgruppe umfassen.

Ferner kann vorgesehen sein, dass die Gruppen Ar, Ar^{a}, Ar^{b}, Ar^{c}, Ar^{d} keine Carbazol-Gruppe, vorzugsweise keine Carbazol-Gruppe und/oder keine Substituenten der Formel N(Ar')₂, N(R¹)₂, und besonders bevorzugt keine Lochtransportgruppe umfassen.

Weiterhin kann vorgesehen sein, dass die Gruppe Ar^{e} keine Carbazol-Gruppe, vorzugsweise keine Carbazol-Gruppe und/oder keine Substituenten der Formel N(Ar')₂, N(R¹)₂, und besonders bevorzugt keine Lochtransportgruppe umfassen.

Darüber hinaus kann vorgesehen sein, dass die Gruppe Q keine Carbazol-Gruppe, vorzugsweise keine Carbazol-Gruppe und/oder keine Substituenten der Formel N(Ar')₂, N(R¹)₂, und besonders bevorzugt keine Lochtransportgruppe umfassen.

In einer weiterhin bevorzugten Ausgestaltung kann vorgesehen sein, dass die erfindungsgemäßen Verbindungen eine Struktur der Formeln (II-1) bis (II-154) umfassen, wobei die erfindungsgemäßen Verbindungen besonders bevorzugt ausgewählt sein können aus den Verbindungen der Formeln (II-1) bis (II-154), 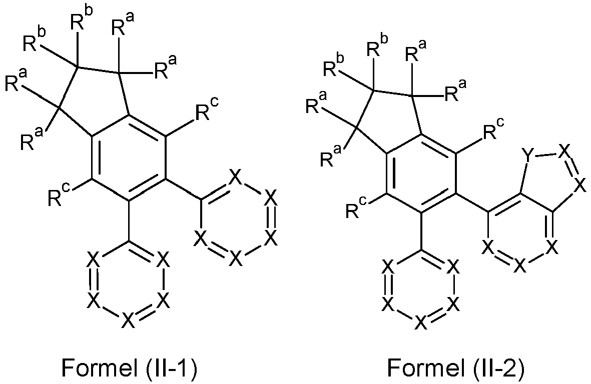 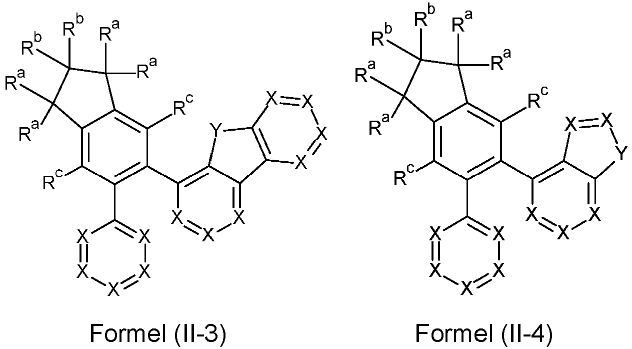 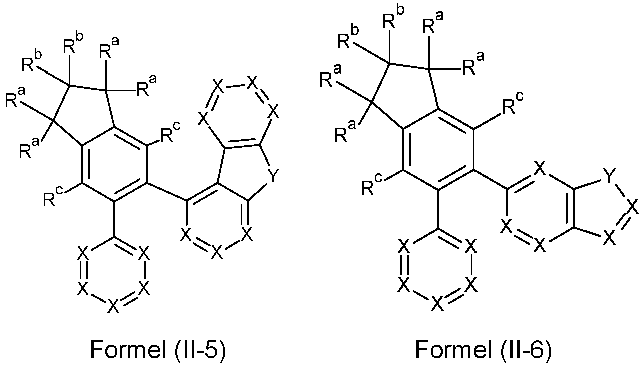 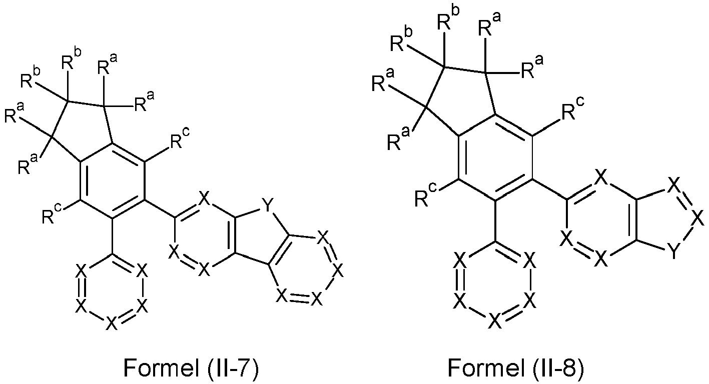 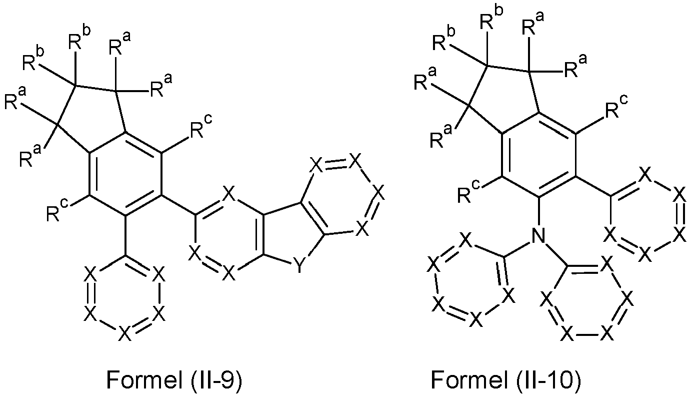 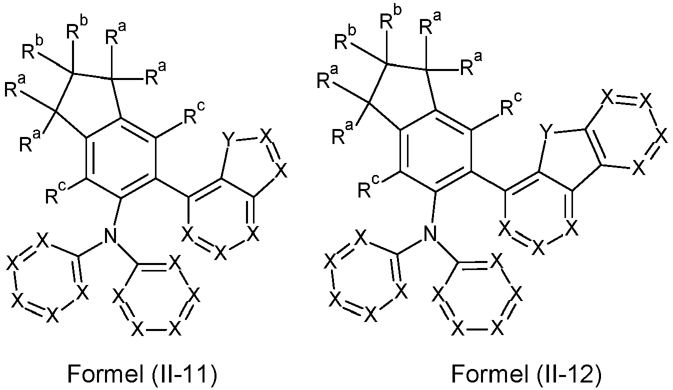 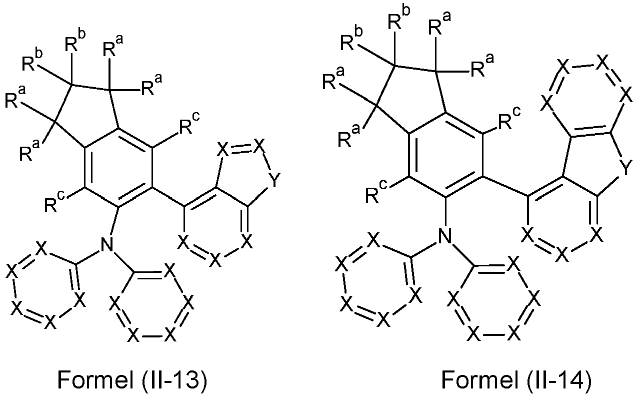 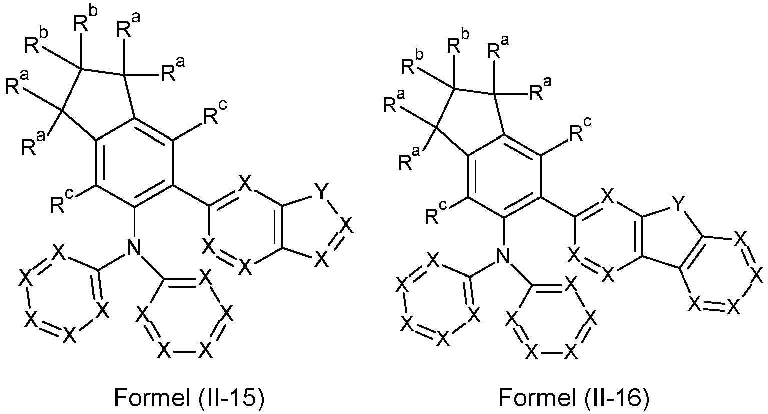 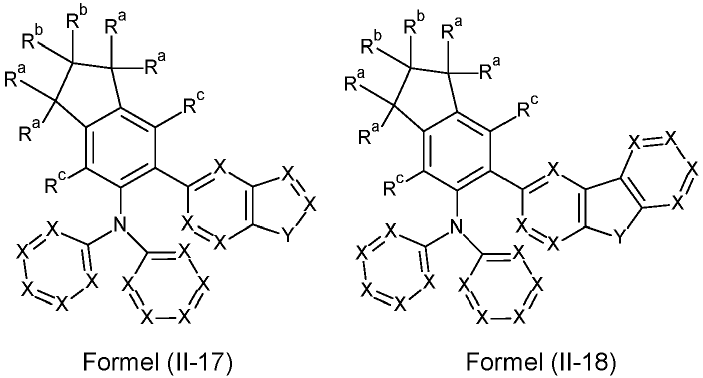 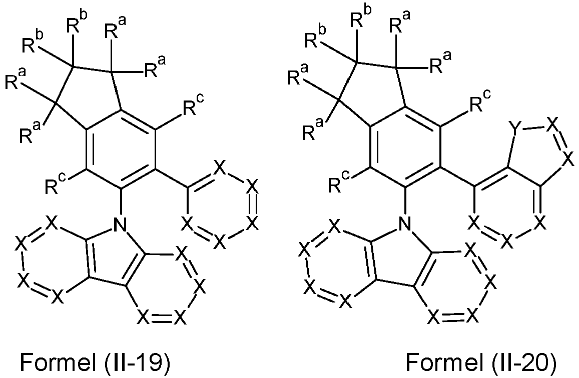 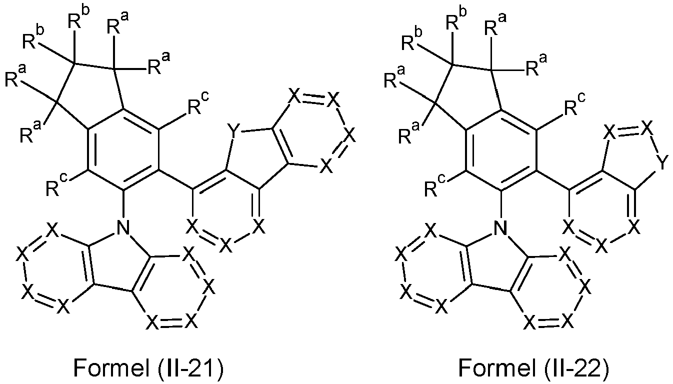 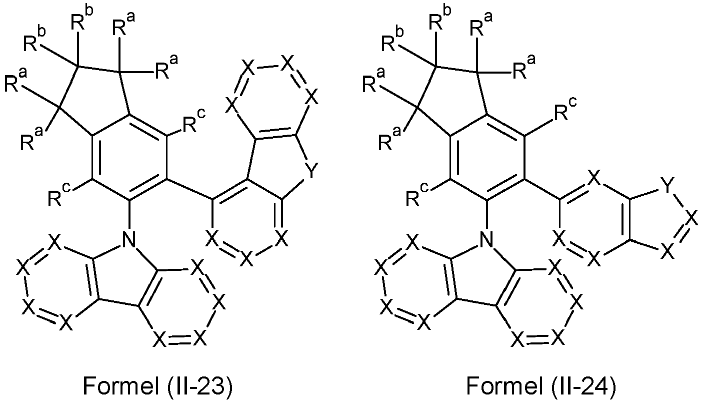 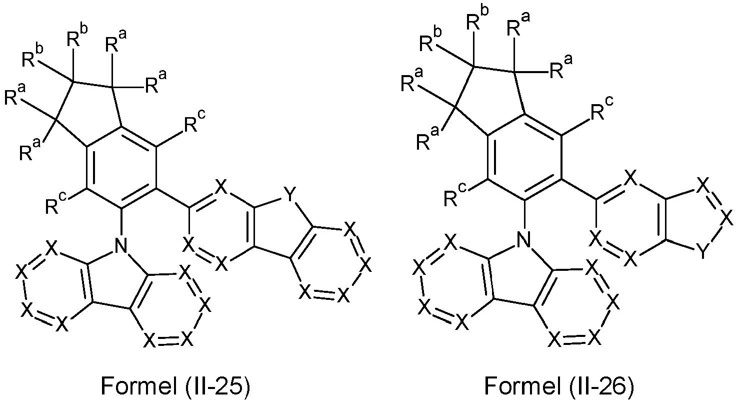 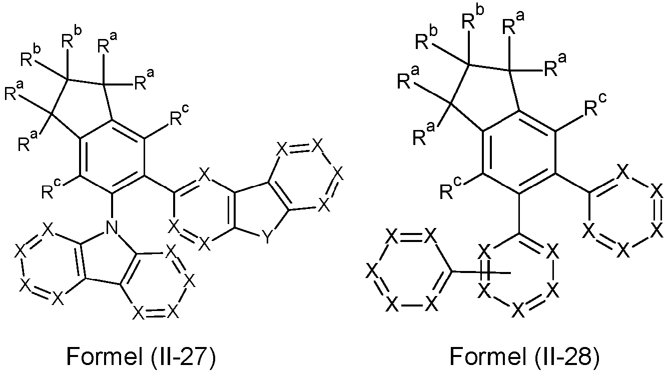 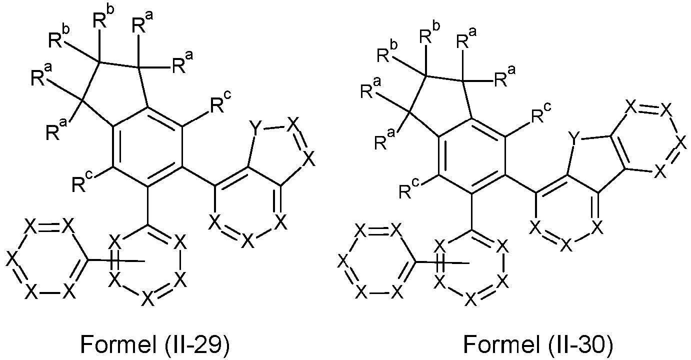 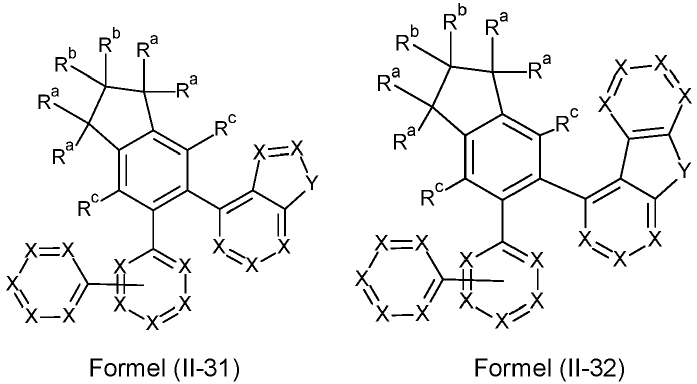 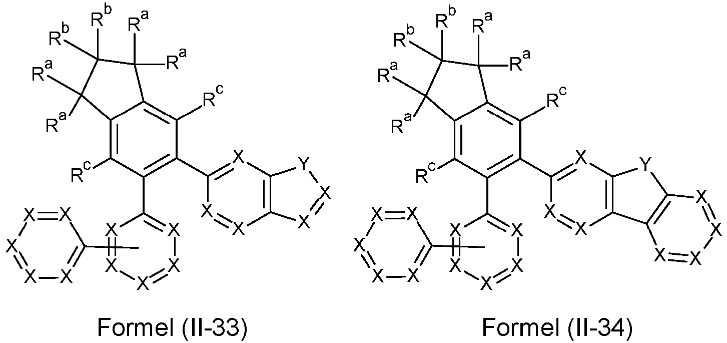 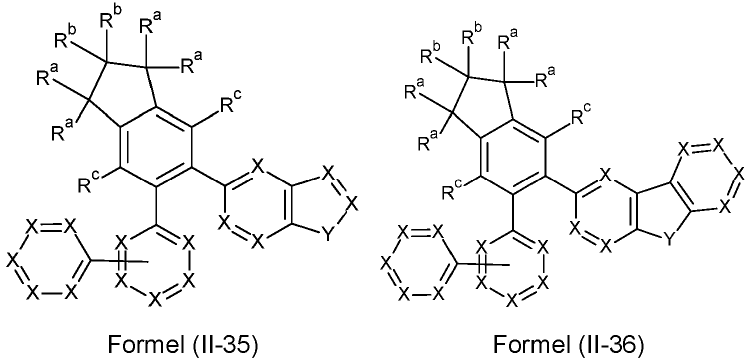 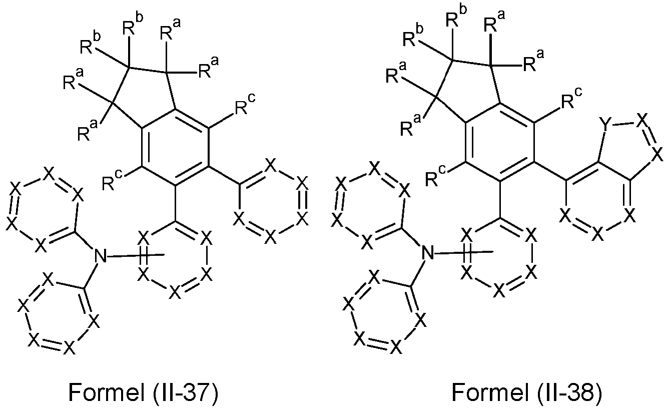 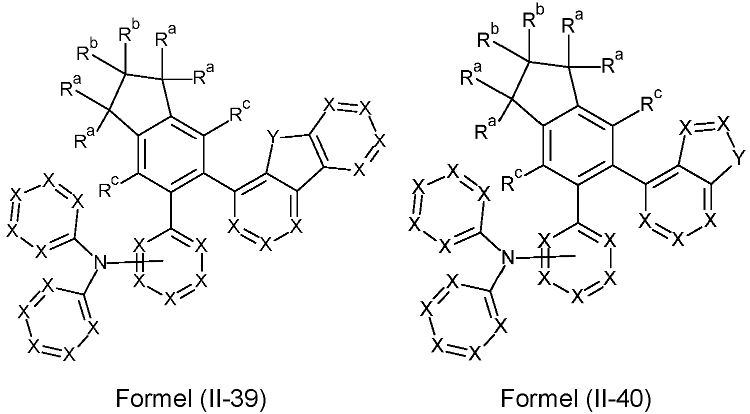 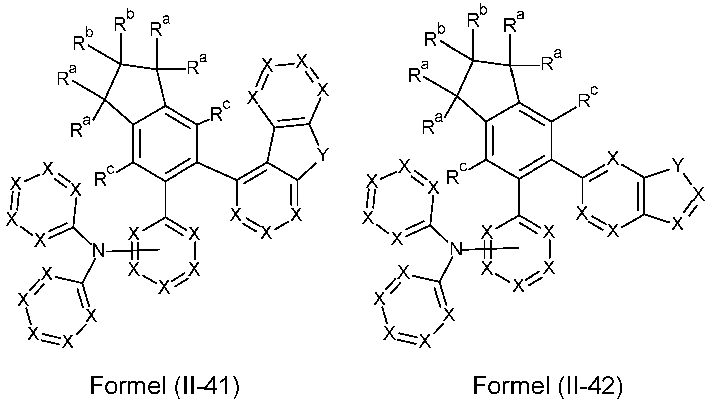 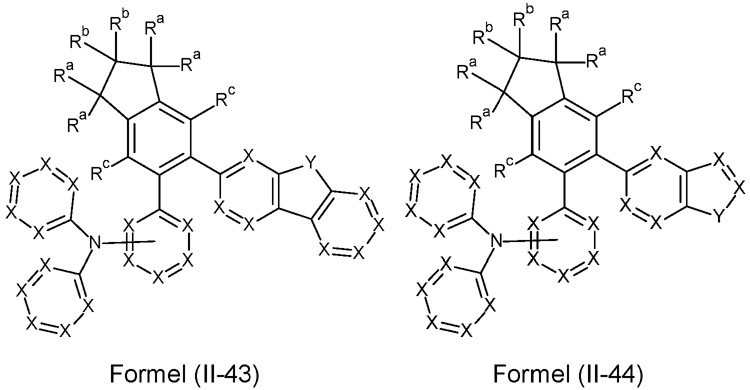 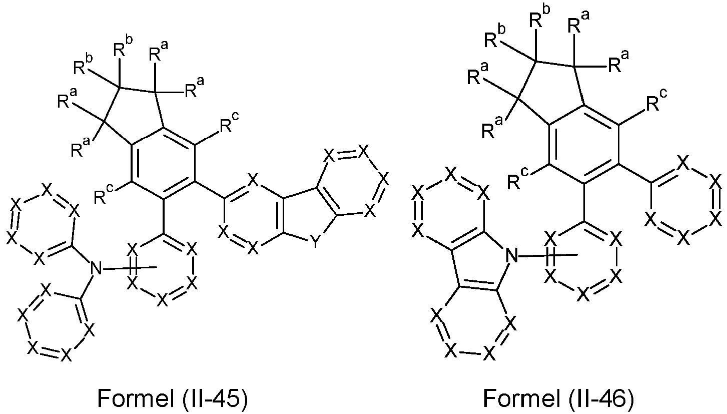 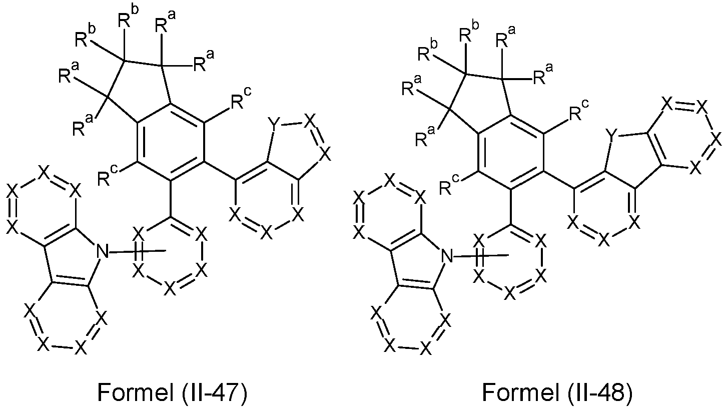 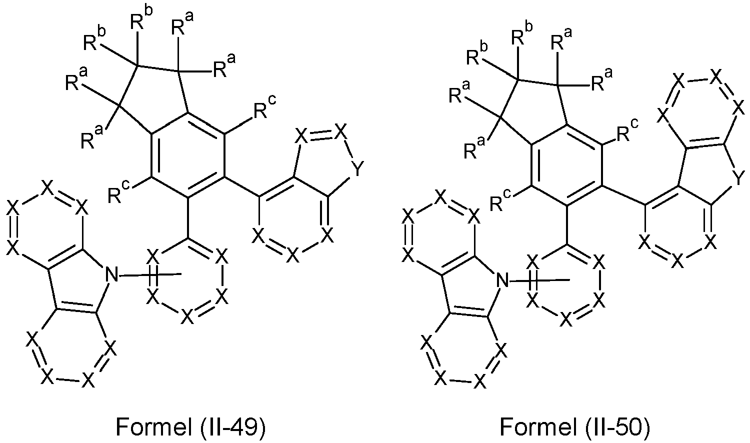 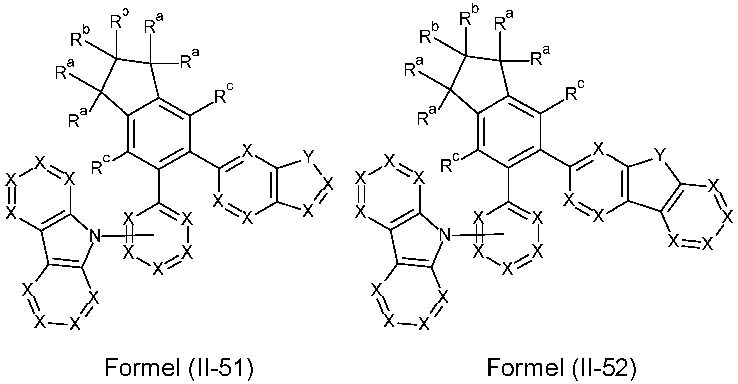 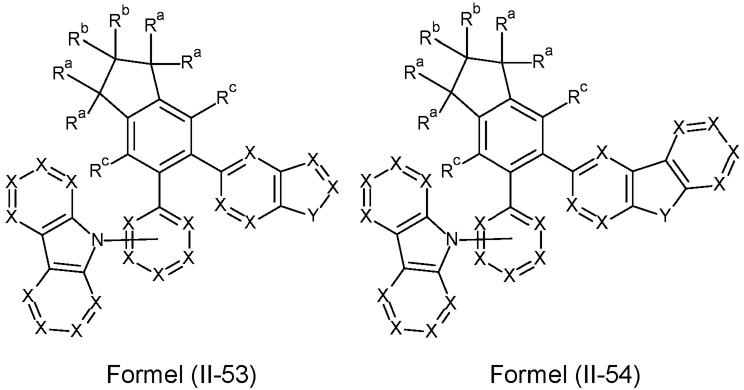 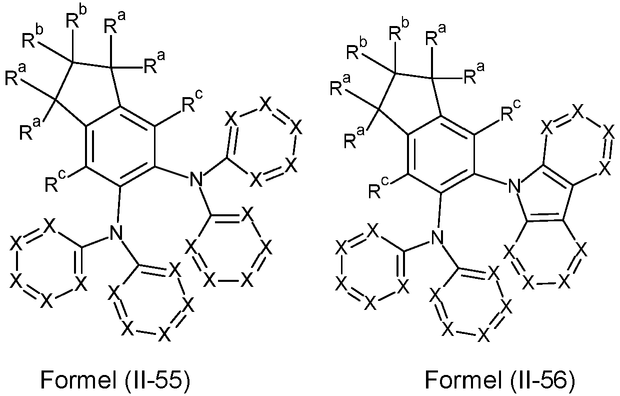 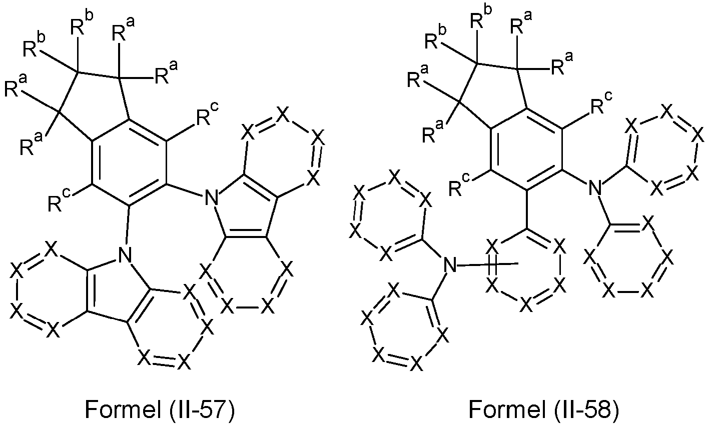 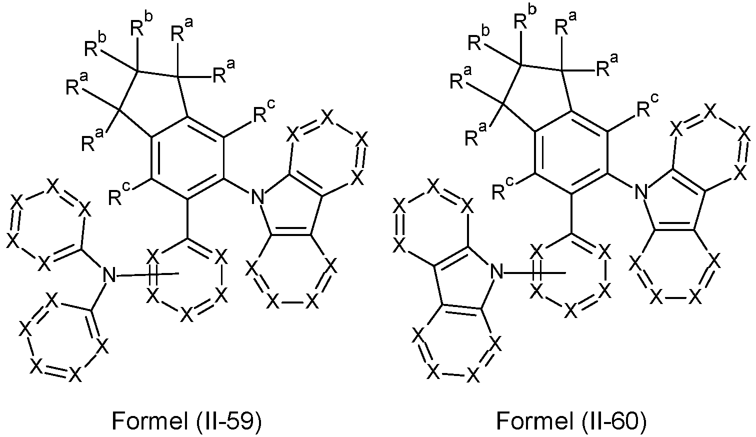 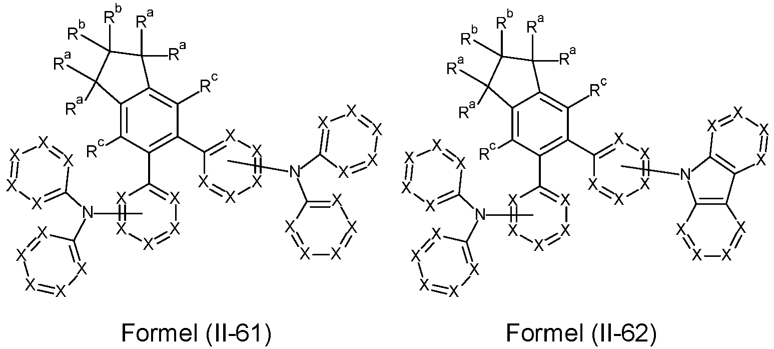 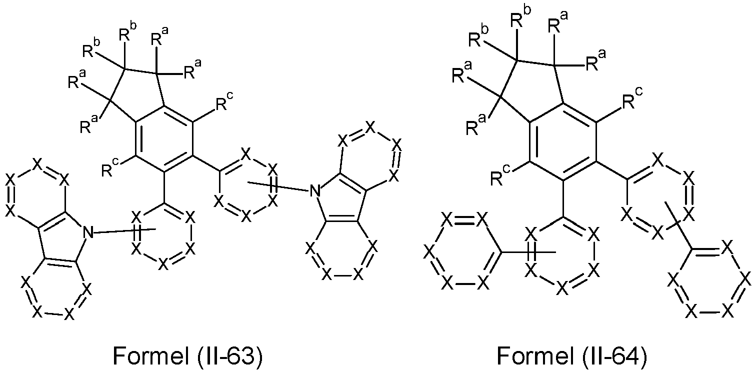 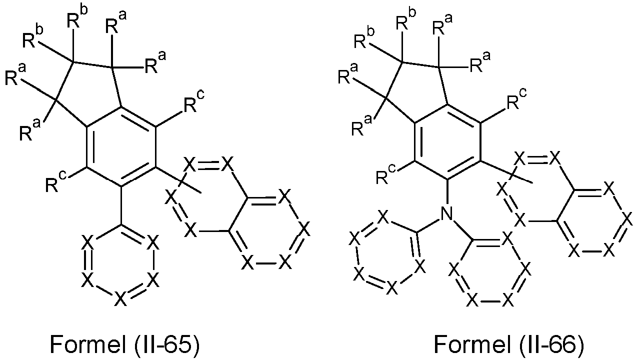 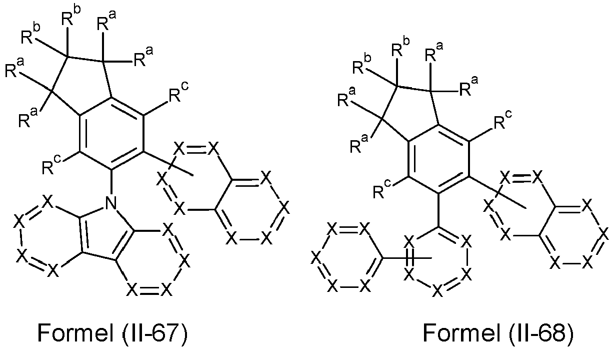 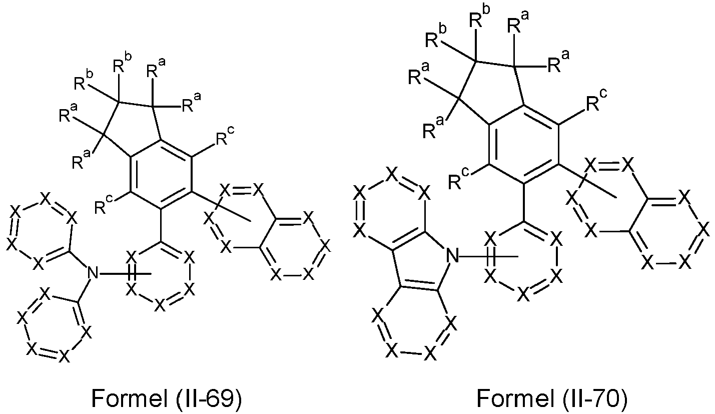 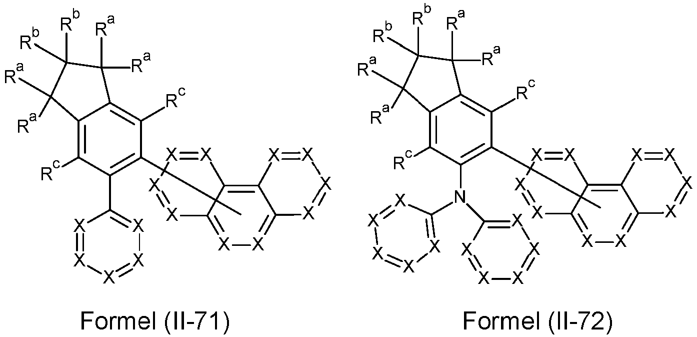 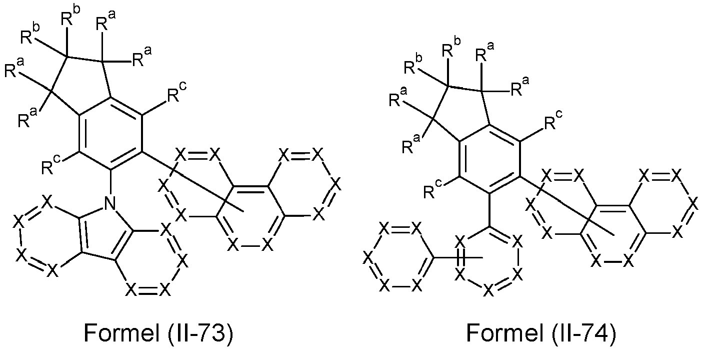 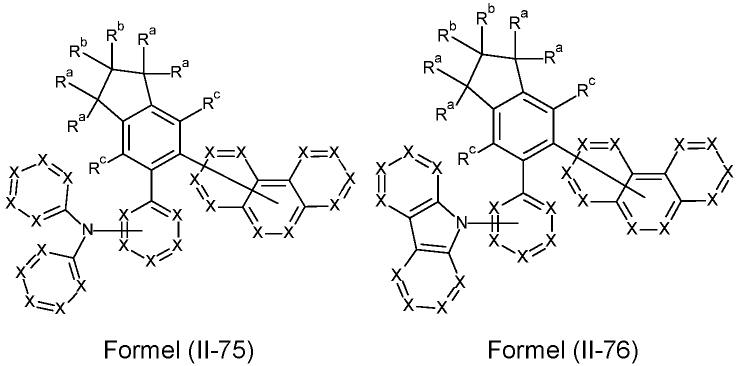 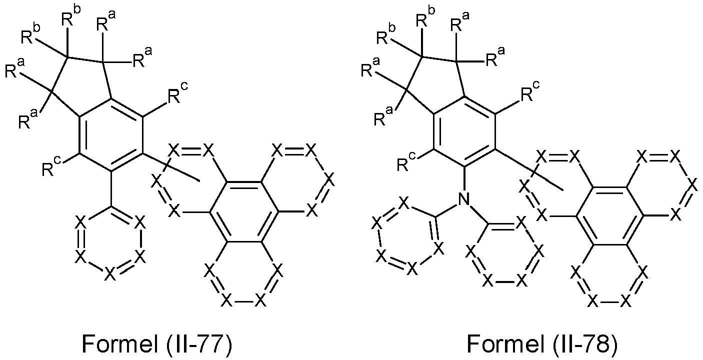 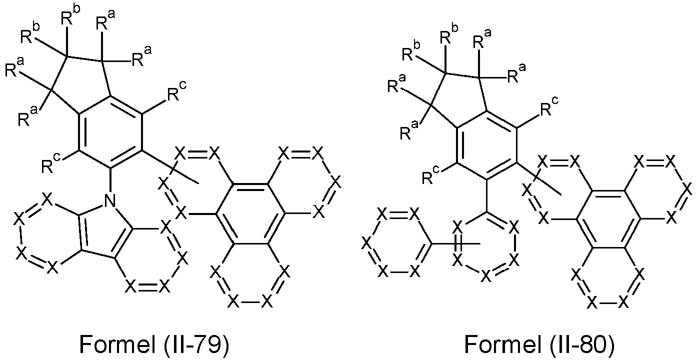 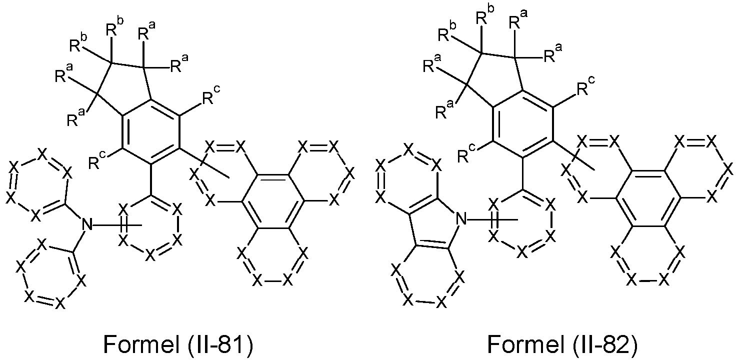 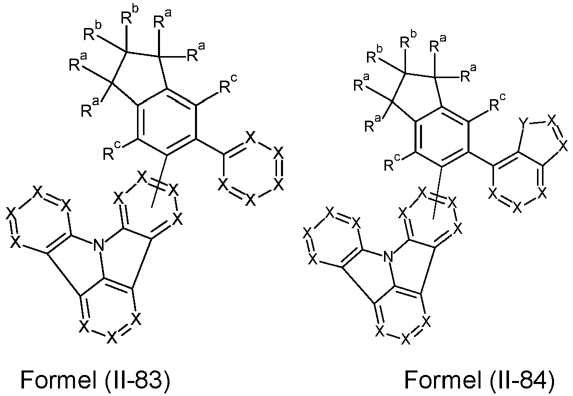 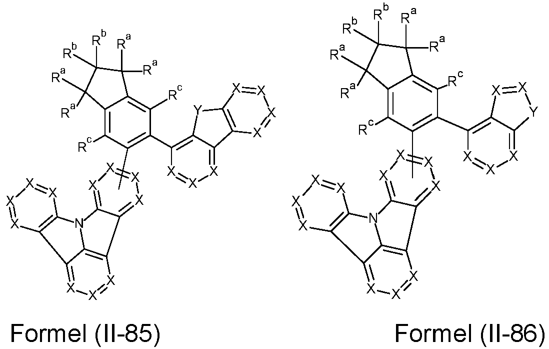 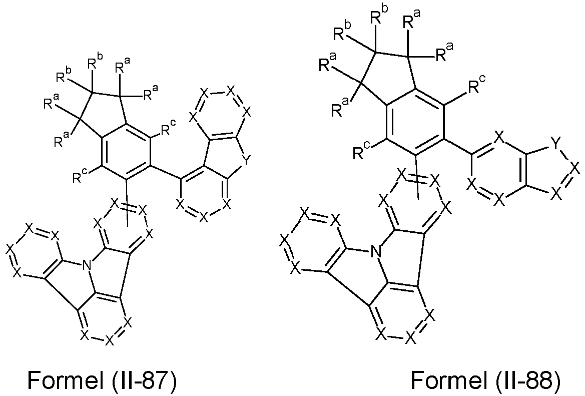 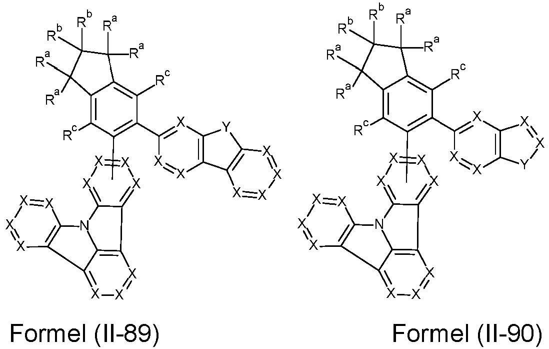 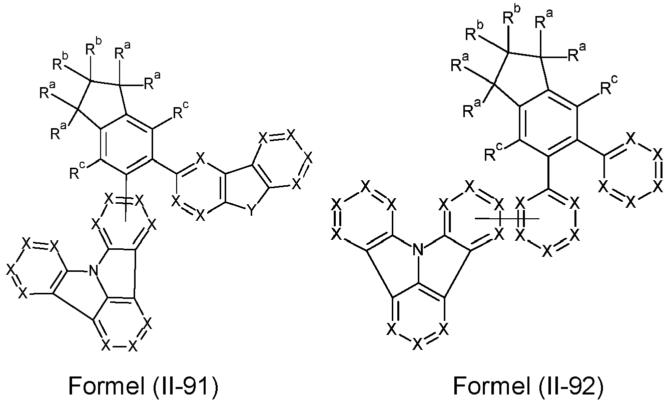 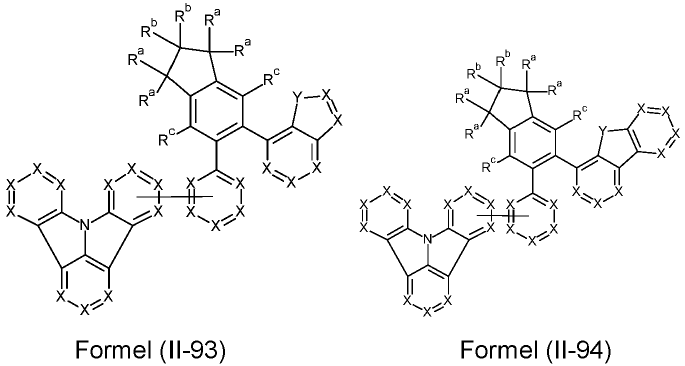 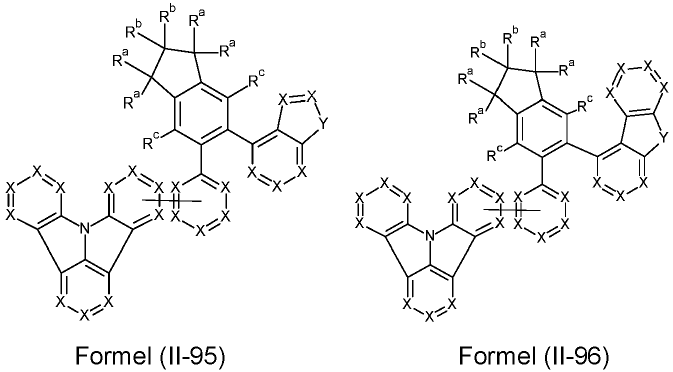 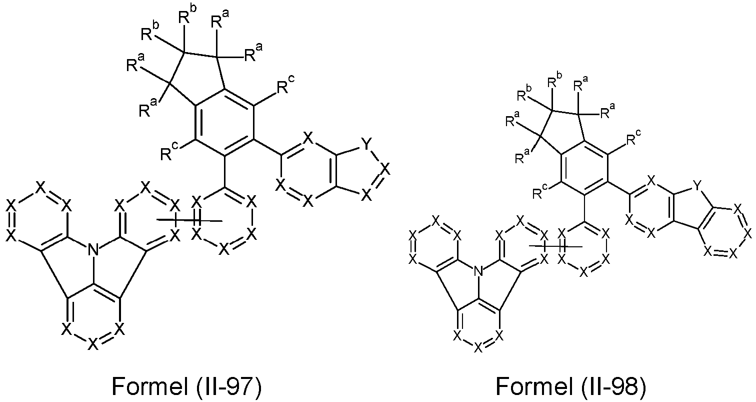 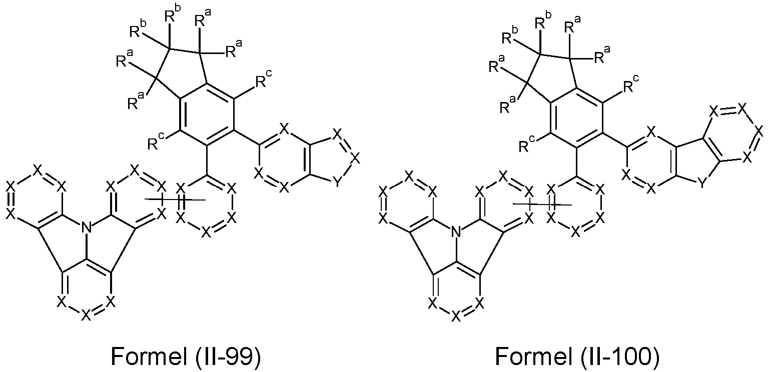 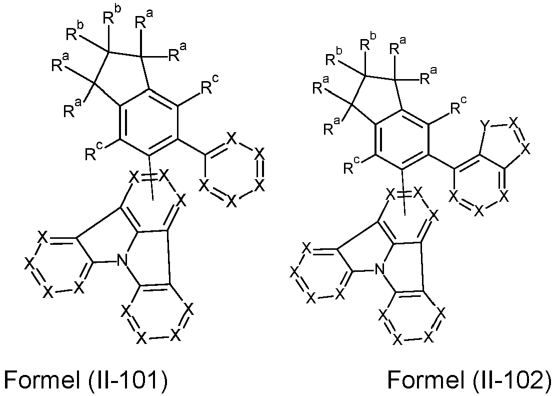 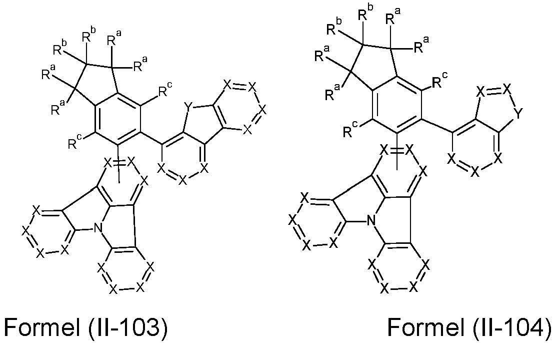 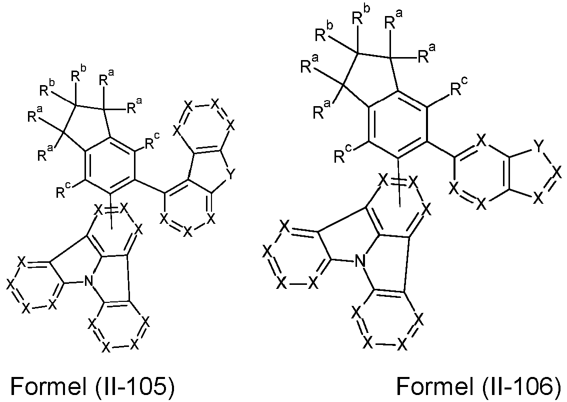 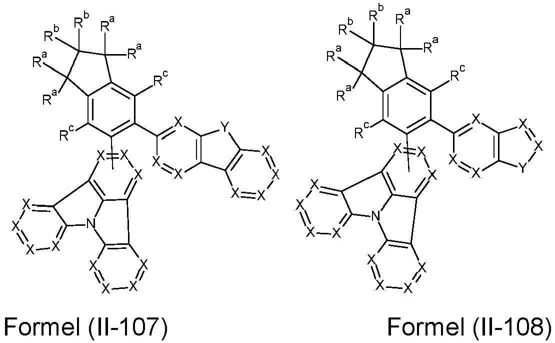 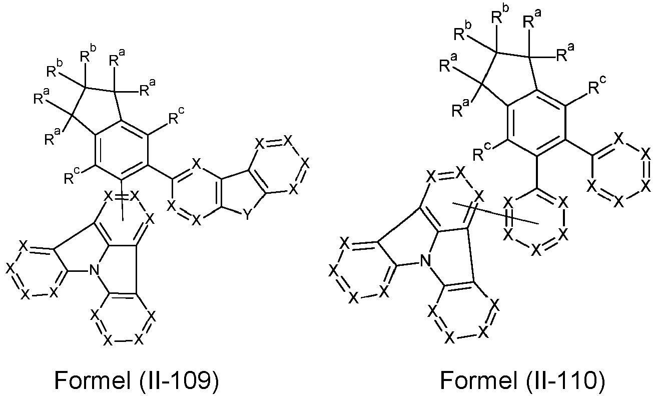 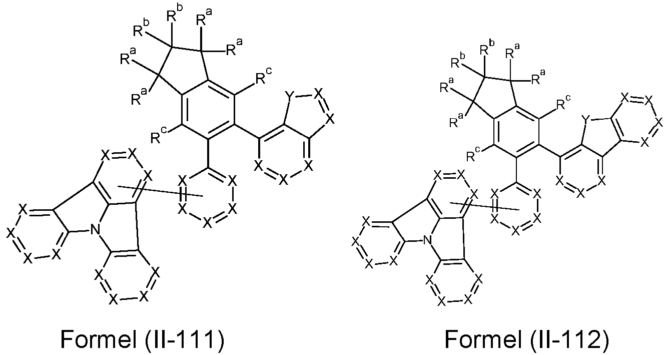 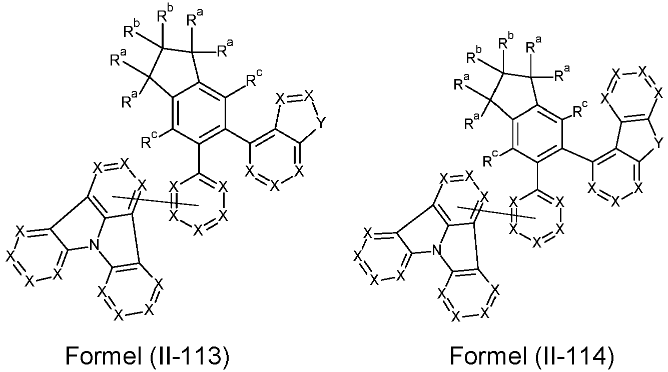 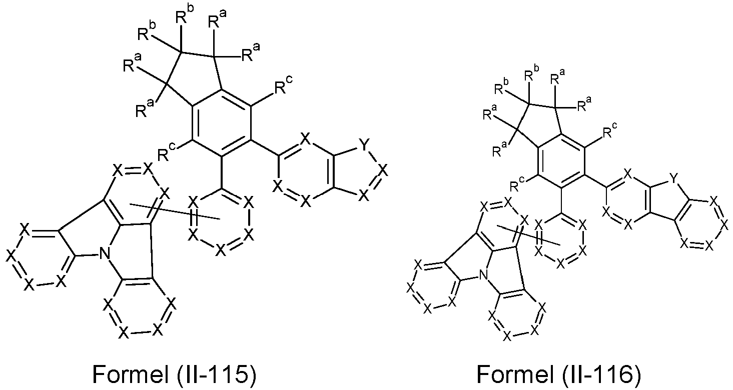 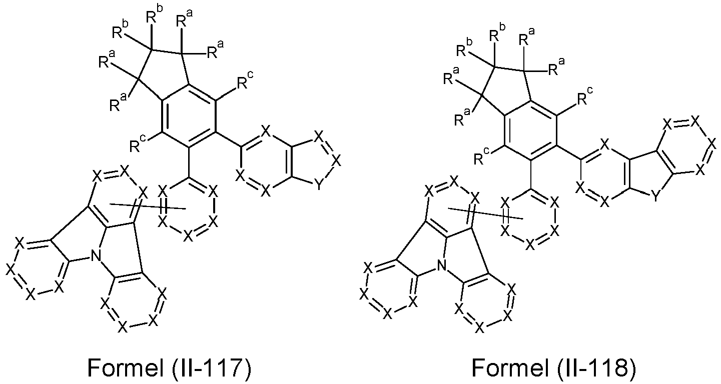 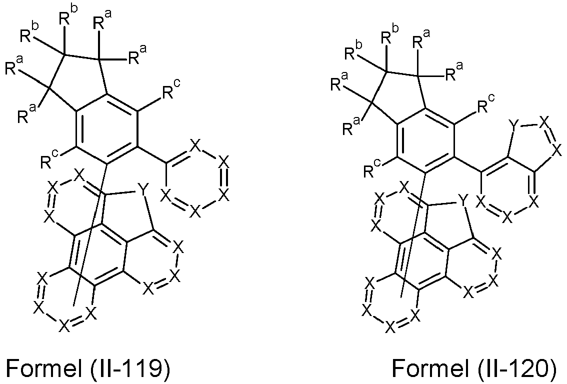 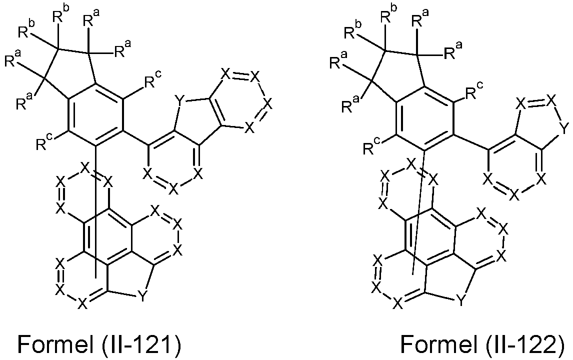 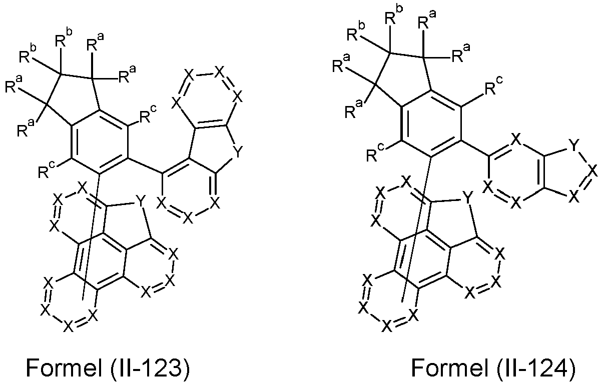 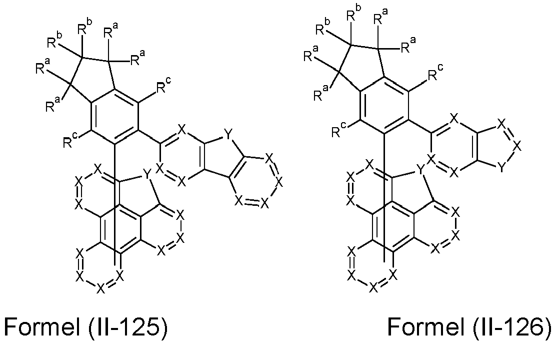 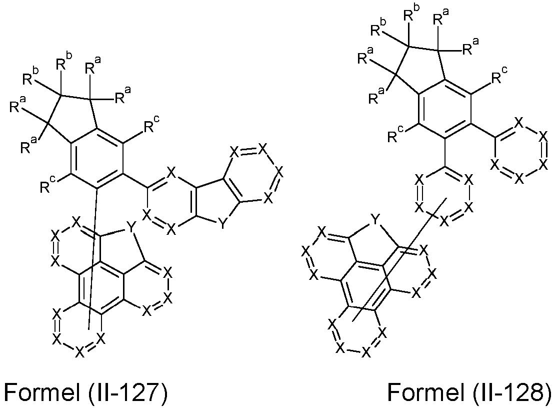 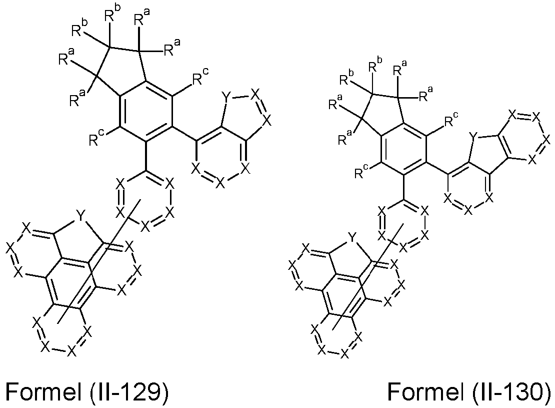 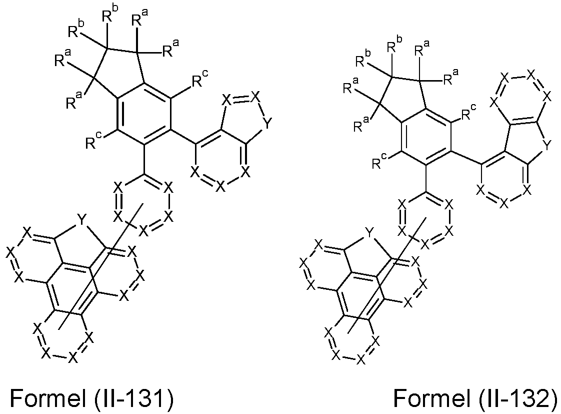 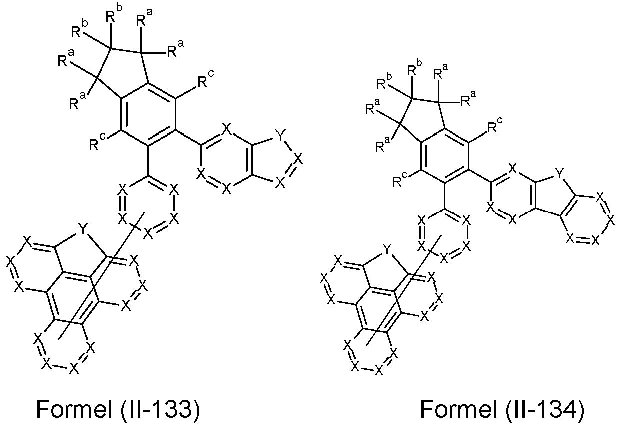 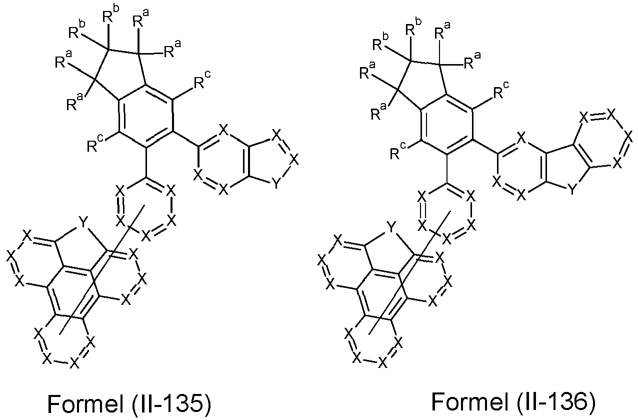 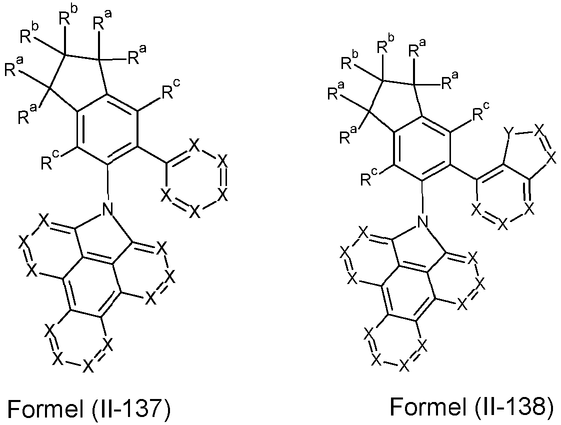 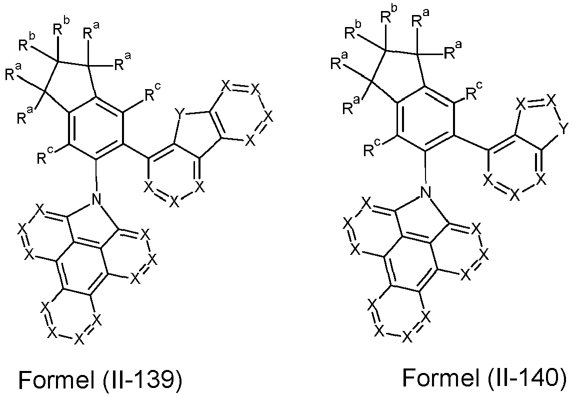 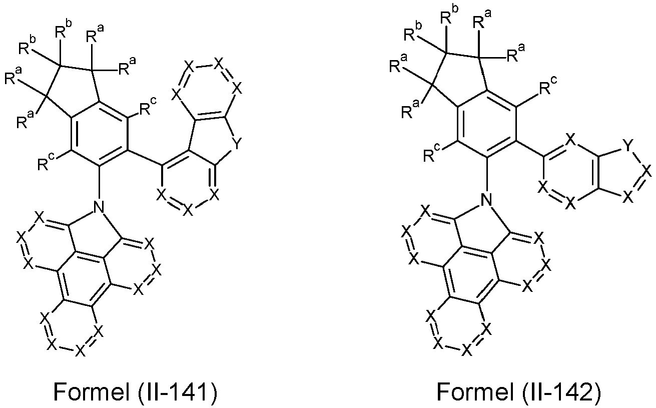 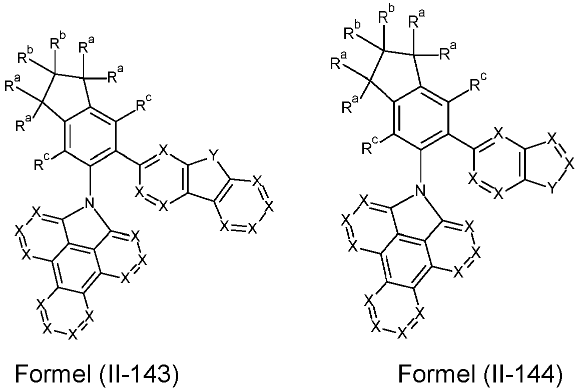 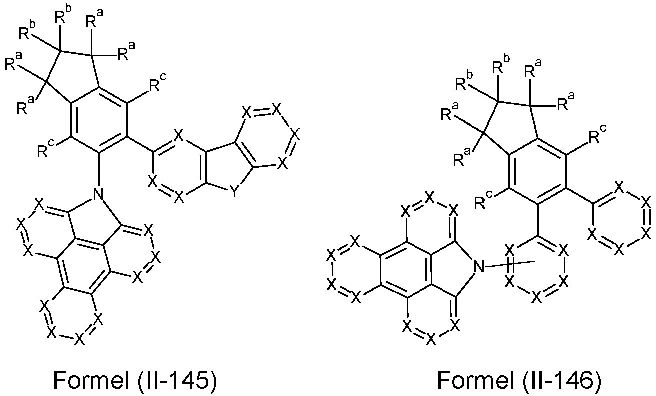 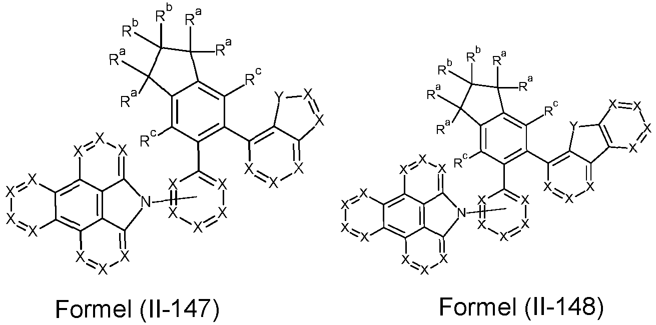 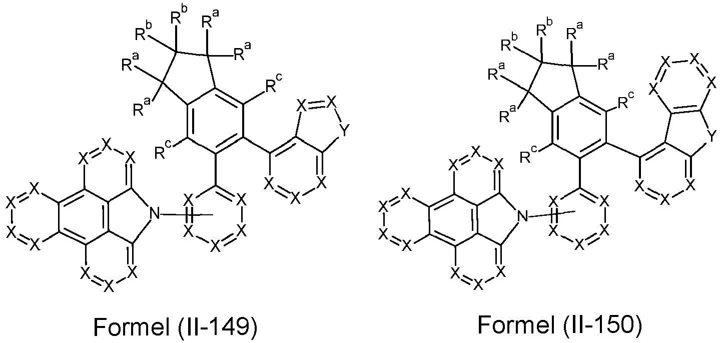 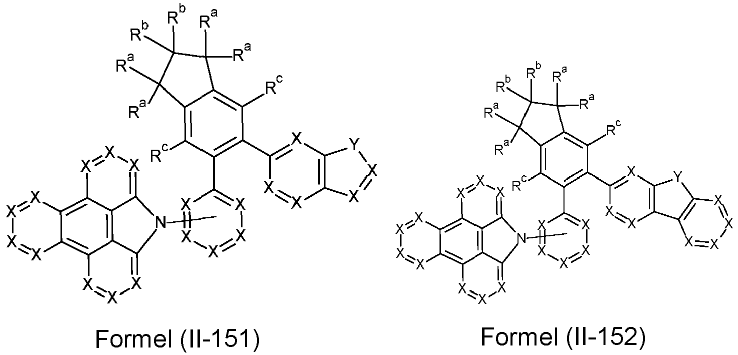 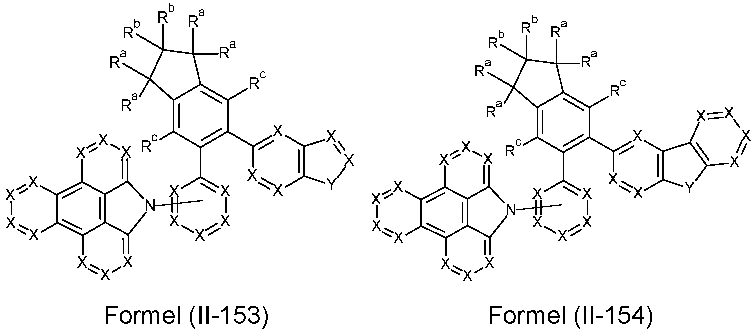 wobei die Symbole R, R^{a}, R^{b} und R^{c} die zuvor, insbesondere für Formel (I) genannten Bedeutungen aufweisen und für die weiteren Symbole gilt:
- X: steht bei jedem Auftreten gleich oder verschieden für N, CR oder C, für den Fall, dass eine Gruppe an die Struktur bindet; und
- Y: steht für O, S, NR oder C(R)₂, vorzugsweise O, NR oder C(R)₂.

Vorzugsweise kann vorgesehen sein, dass in Strukturen/Verbindungen der Formeln (II-1) bis (II-154) höchstens zwei Gruppen X pro Ring für N stehen, bevorzugt alle X für CR stehen, bevorzugt mindestens eine, besonders bevorzugt mindestens zwei der Gruppen X pro Ring ausgewählt sind aus C-H und C-D.

Ferner kann vorgesehen sein, dass in Strukturen/Verbindungen der Formeln (II-1) bis (II-154) mindestens zwei Gruppen X pro Ring für N stehen, die nicht benachbart sind, die Gruppe Y für NR steht und mindestens eine Gruppe X in einem Ring, der zum Ring mit der Gruppe Y kondensiert ist, für N steht und/oder in einem Ring die Gruppe Y für NR und in diesem Ring eine Gruppe X, die nicht zur Gruppe Y benachbart ist, für N steht. Diese Strukturen/Verbindungen umfassen vorzugsweise Elektronentransportgruppen und sind daher als Elektronentransportmaterialien und/oder Matrixmaterialien besonders geeignet.

In einer weiteren Ausführungsform kann vorgesehen sein, dass in Strukturen/Verbindungen der Formeln (II-1) bis (II-154) nicht mehr als vier, vorzugsweise nicht mehr als zwei Gruppen X für N stehen, besonders bevorzugt alle Gruppen X für CR stehen, wobei vorzugsweise höchstens 4, besonders bevorzugt höchstens 3 und speziell bevorzugt höchstens 2 der Gruppen CR, für die X steht, ungleich der Gruppe CH ist. Diese Strukturen/Verbindungen umfassen vorzugsweise keine Elektronentransportgruppen und sind daher als Lochtransportmaterialien und/oder Matrixmaterialien besonders geeignet.

In einer weiterhin bevorzugten Ausgestaltung kann vorgesehen sein, dass die erfindungsgemäßen Verbindungen eine Struktur der Formeln (III-1) bis (III-60) umfassen, wobei die erfindungsgemäßen Verbindungen besonders bevorzugt ausgewählt sein können aus den Verbindungen der Formeln (III-1) bis (III-60), 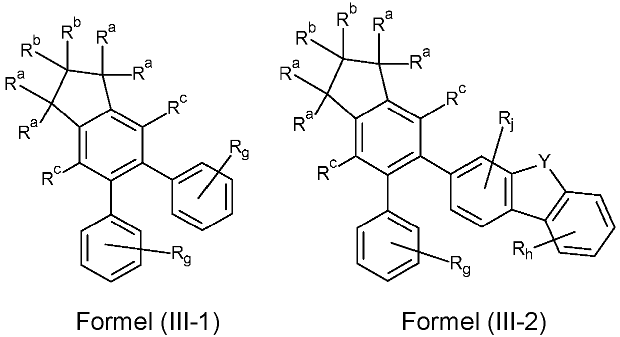 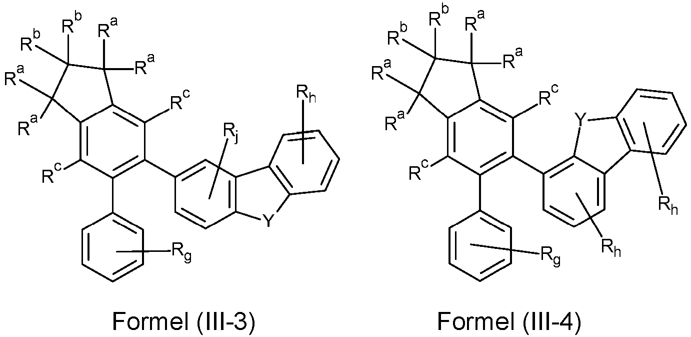 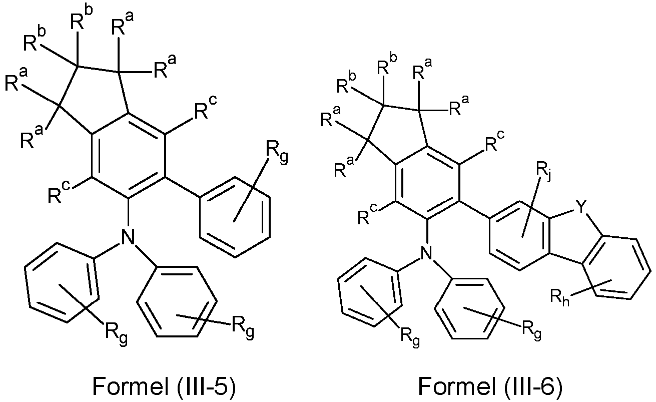 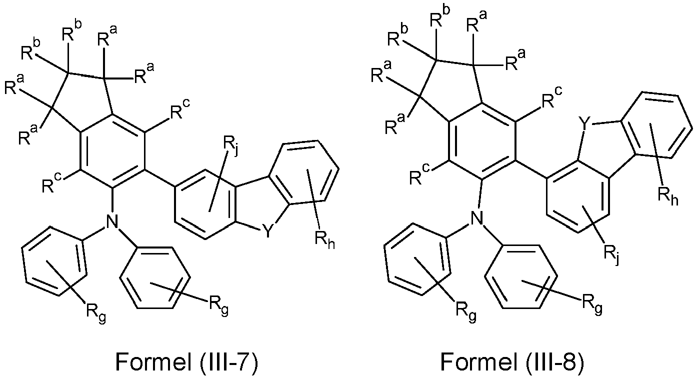 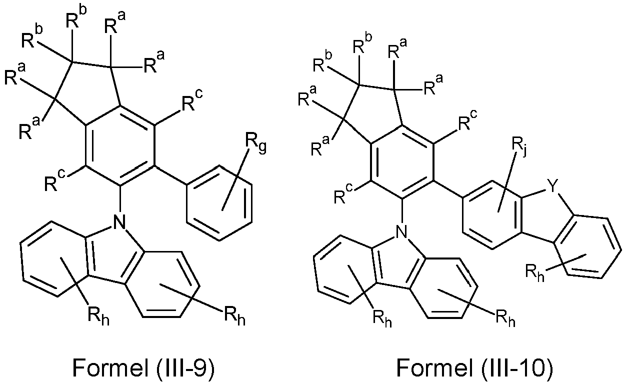 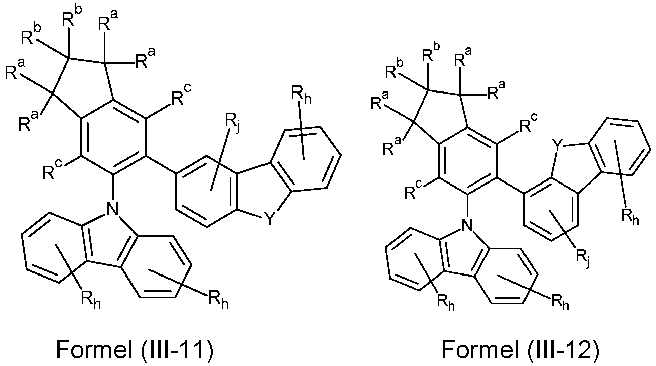 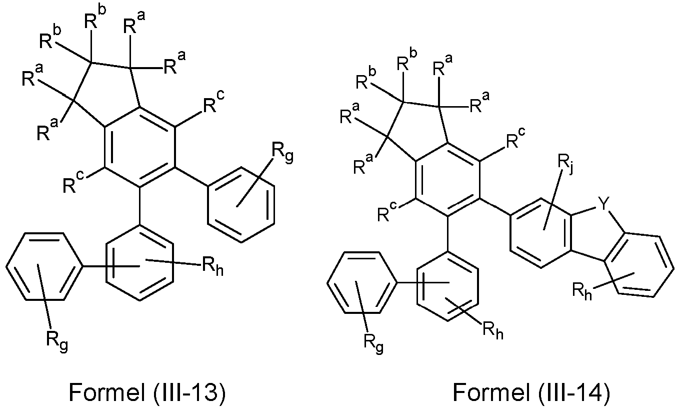 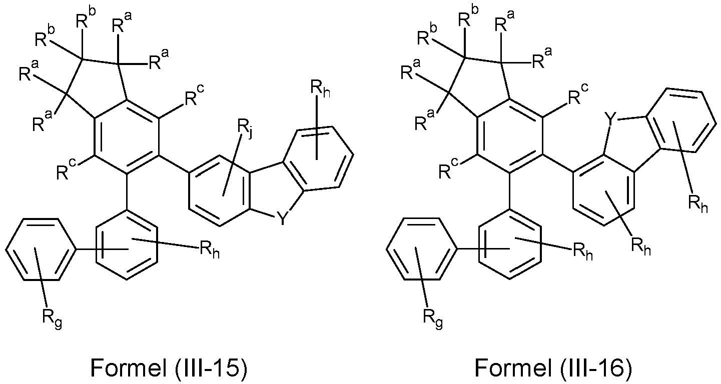 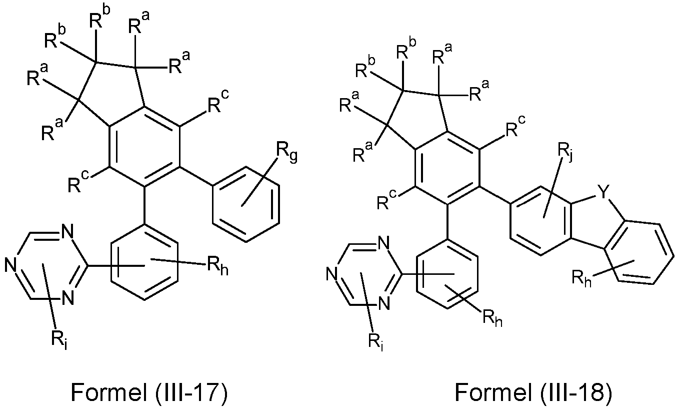 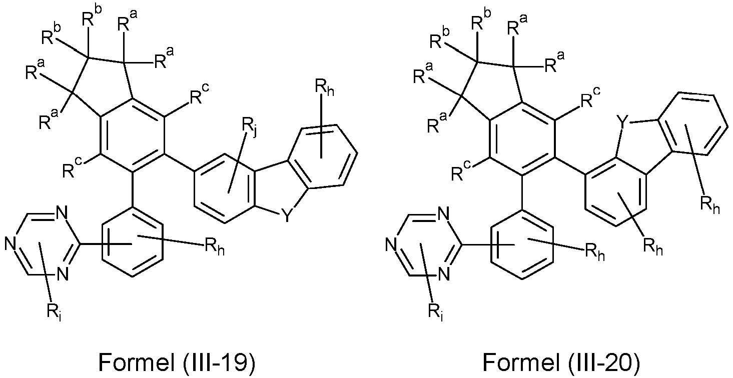 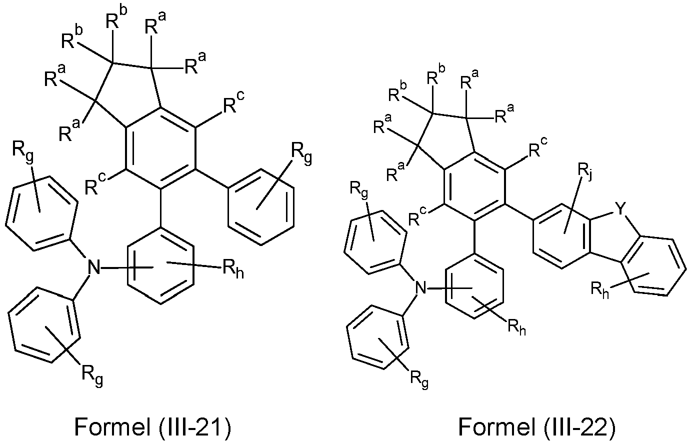 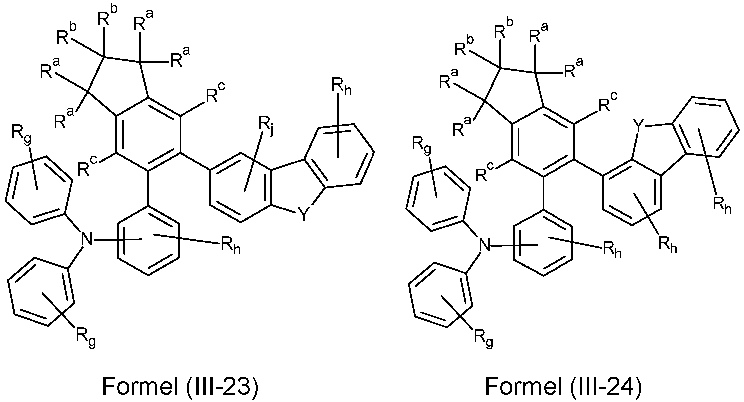 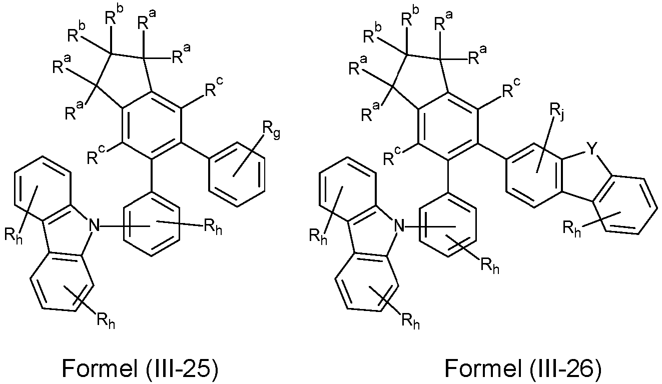 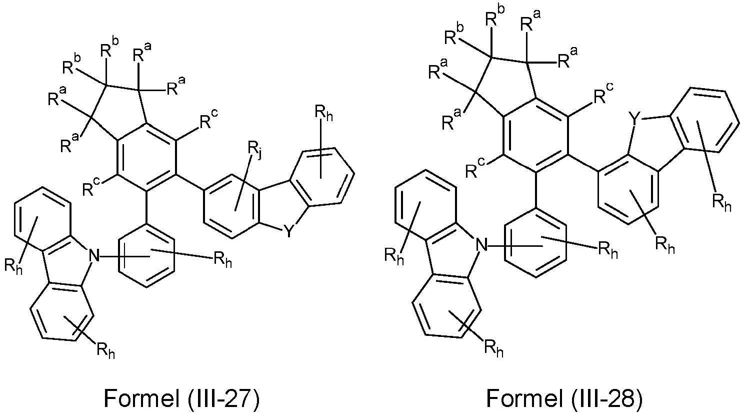 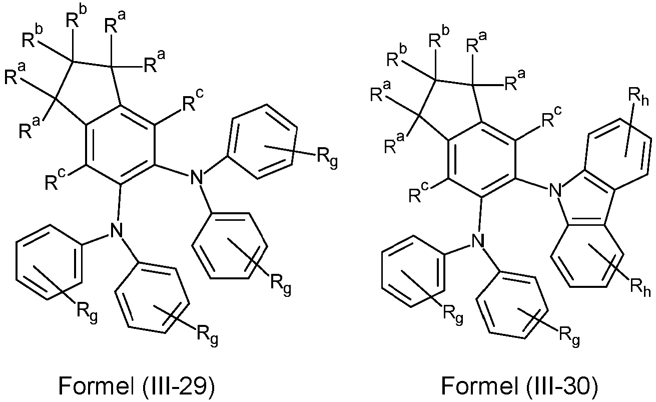 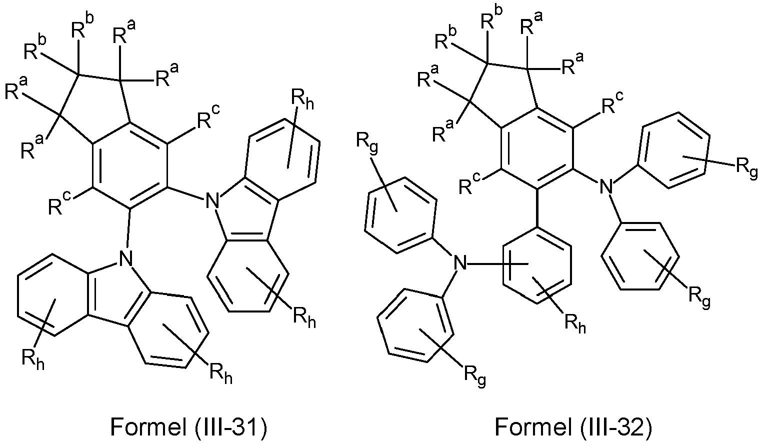 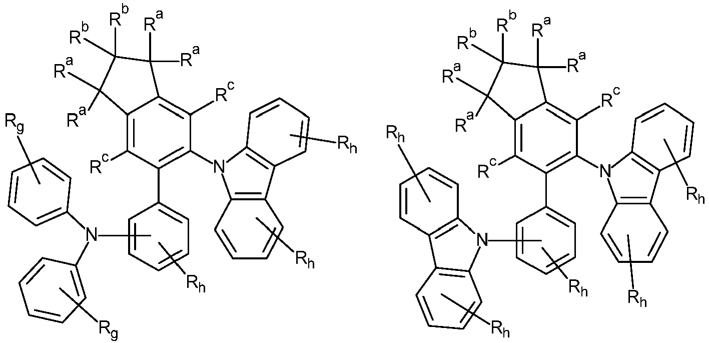 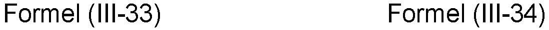 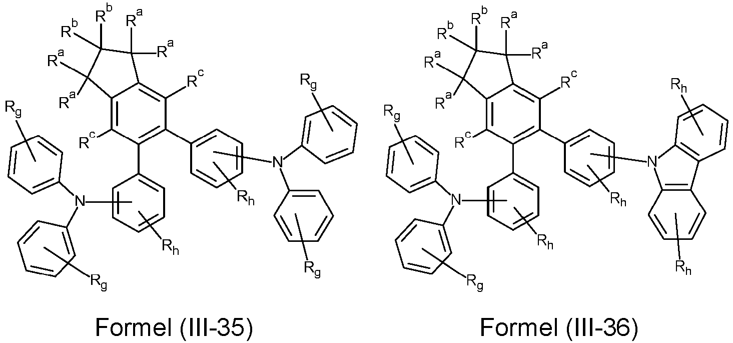 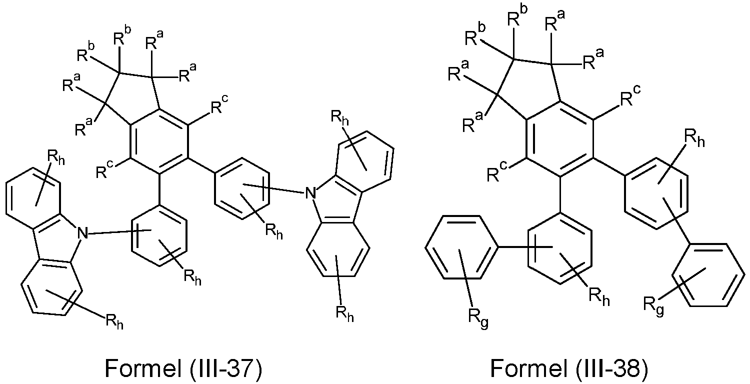 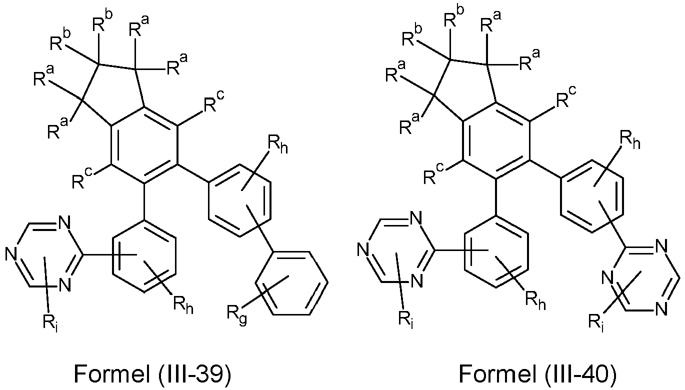 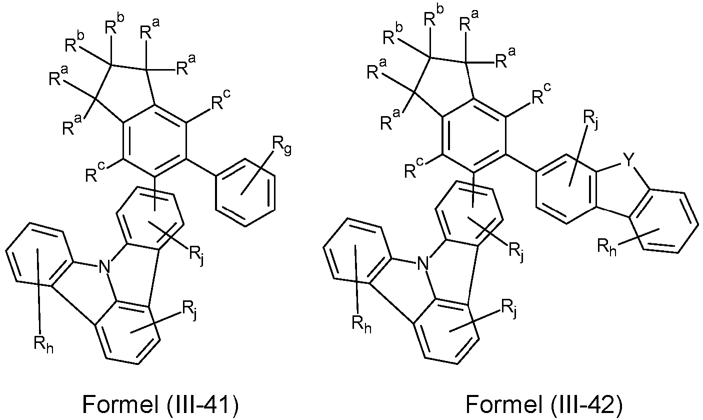 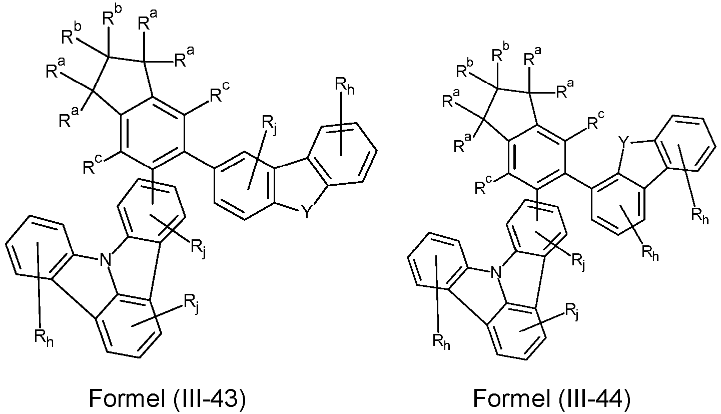 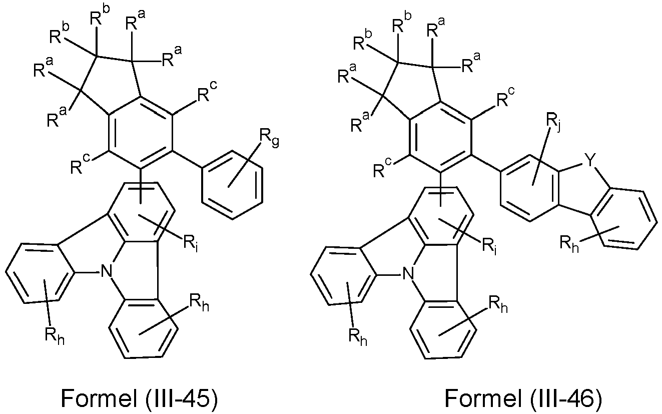 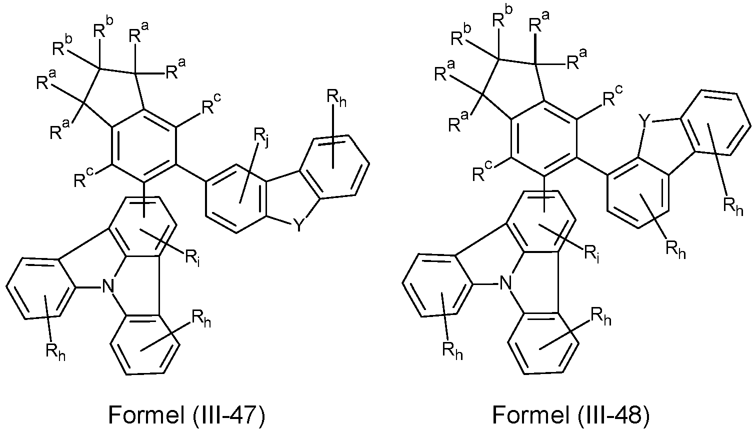 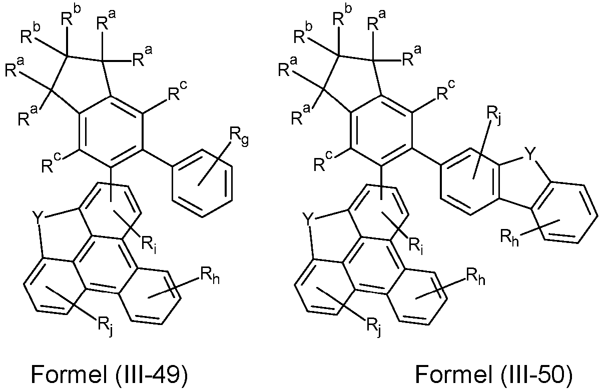 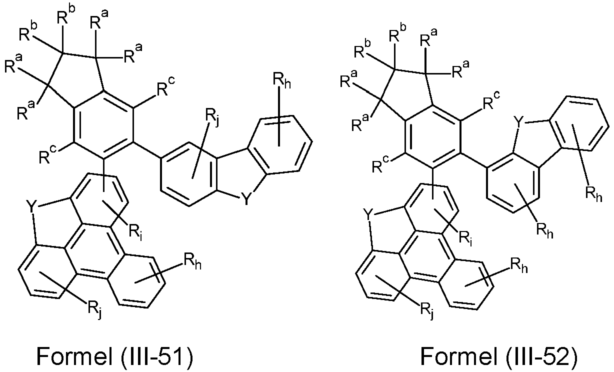 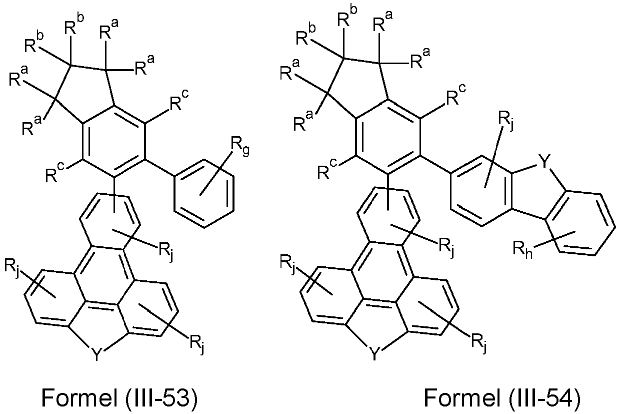 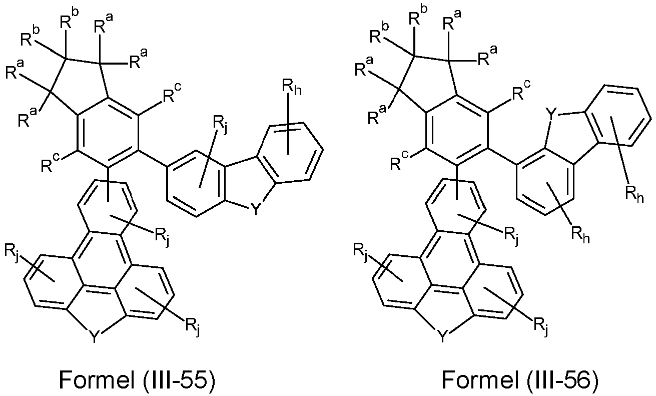 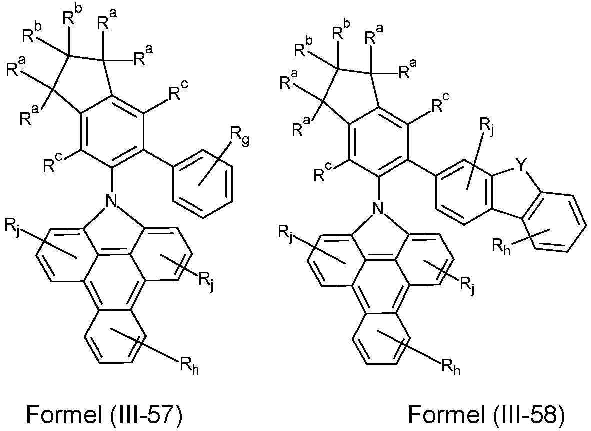 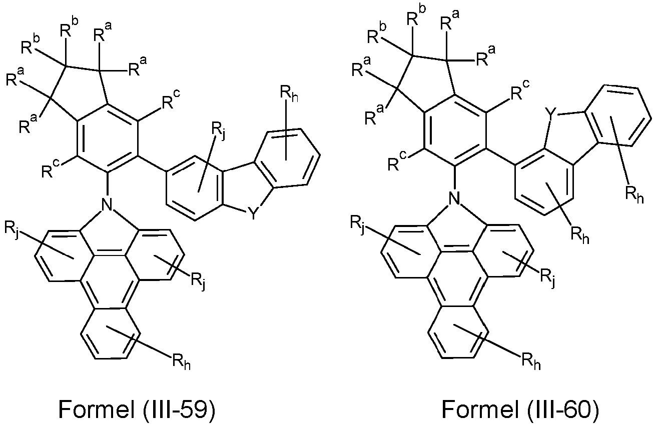 wobei die Symbole R, R^{a}, R^{b} und R^{c} die zuvor, insbesondere für Formel (I) genannten Bedeutungen aufweisen und für die weiteren Symbole gilt:
- Y: ist O, S, NR oder C(R)₂, vorzugsweise O, NR oder C(R)₂;
- i: ist bei jedem Auftreten unabhängig 0, 1 oder 2;
- j: ist bei jedem Auftreten unabhängig 0, 1, 2 oder 3;
- h: ist bei jedem Auftreten unabhängig 0, 1, 2, 3 oder 4;
- g: ist bei jedem Auftreten unabhängig 0, 1, 2, 3, 4 oder 5.

Die Summe der Indices i, j, h und g in Strukturen/Verbindungen der Formeln (III-1) bis (III-60) beträgt vorzugsweise höchstens 6, insbesondere bevorzugt höchstens 4 und besonders bevorzugt höchstens 2.

In einer bevorzugten Weiterbildung der vorliegenden Erfindung kann vorgesehen sein, dass mindestens zwei, vorzugsweise benachbarte Reste R mit den weiteren Gruppen, an die die zwei Reste R binden, einen kondensierten Ring bilden, wobei die zwei Reste R mindestens eine Struktur der Formeln (RA-1) bis (RA-13) formen 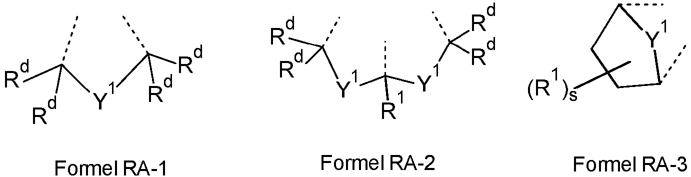 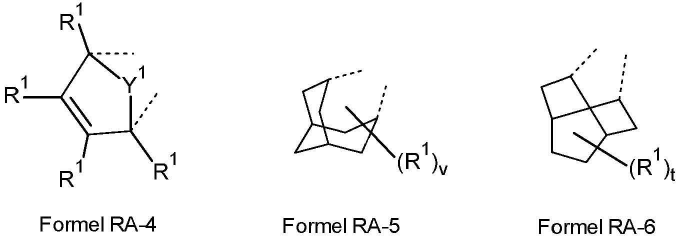 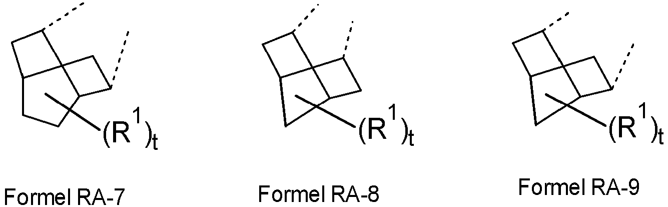 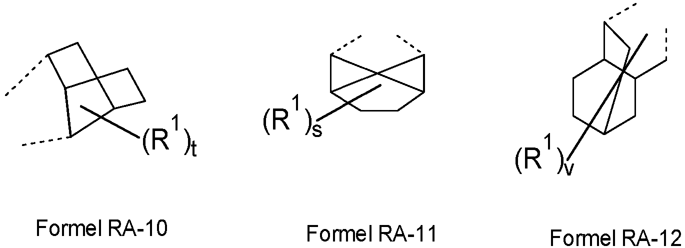 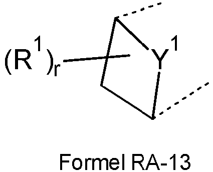 wobei R¹ die zuvor dargelegte Bedeutung hat, die gestrichelten Bindungen die Anbindungsstellen an die Atome der Gruppen, an die die zwei Reste R binden, darstellen, und die weiteren Symbole die folgende Bedeutung aufweisen:
- Y¹: ist bei jedem Auftreten gleich oder verschieden C(R¹)₂, (R¹)₂C-C(R¹)₂, (R¹)C=C(R¹), NR¹, NAr', O oder S, vorzugsweise C(R¹)₂, (R¹)₂C-C(R¹)₂, (R¹)C=C(R¹), O oder S;
- R^{d}: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, C=S, C=Se, C=NR², -C(=O)O-, -C(=O)NR²-, NR², P(=O)(R²), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{d} auch miteinander oder ein Rest R^{d} mit einem Rest R¹ oder mit einer weiteren Gruppe ein Ringsystem bilden, wobei R² die zuvor, insbesondere für Formel (I) genannte Bedeutung aufweist;
- r: ist 0, 1, 2, 3 oder 4, vorzugsweise 0, 1, oder 2, besonders bevorzugt 0 oder 1;
- s: ist 0, 1, 2, 3, 4, 5 oder 6, vorzugsweise 0, 1, 2, 3, oder 4, besonders bevorzugt 0, 1 oder 2;
- t: ist 0, 1, 2, 3, 4, 5, 6, 7 oder 8, vorzugsweise 0, 1, 2, 3, oder 4, besonders bevorzugt 0, 1 oder 2;
- v: ist 0, 1, 2, 3, 4, 5, 6, 7, 8 oder 9, vorzugsweise 0, 1, 2, 3, oder 4, besonders bevorzugt 0, 1 oder 2.

Hierbei sind Strukturen der Formeln RA-1, RA-3, RA-4 und RA-5 bevorzugt und Strukturen der Formeln RA-4 und RA-5 besonders bevorzugt.

In einer bevorzugten Ausführungsform der Erfindung bilden bevorzugt mindestens zwei, vorzugsweise benachbarte Reste R mit den weiteren Gruppen, an die die zwei Reste R binden, einen kondensierten Ring, wobei die zwei Reste R Strukturen der Formeln (RA-1a) bis (RA-4f) formen 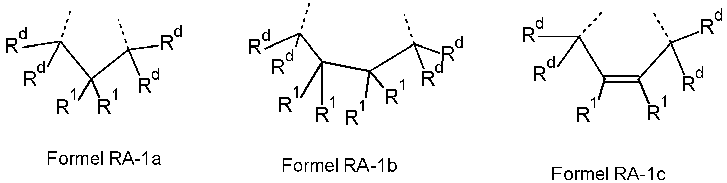 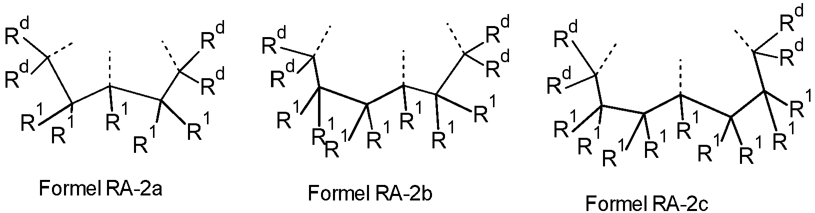 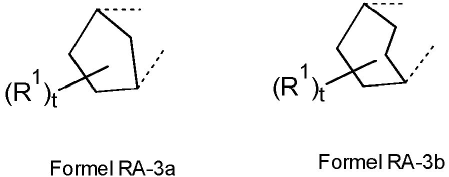 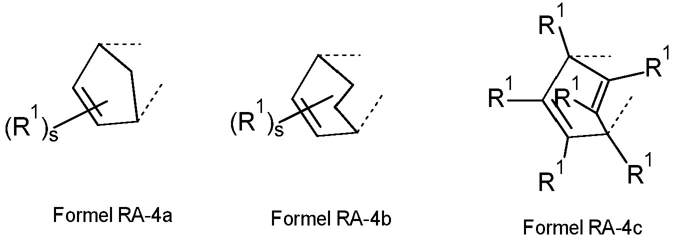 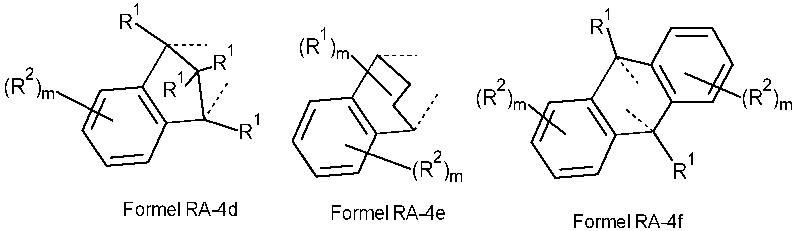 wobei die gestrichelten Bindungen die Anbindungsstellen an die Atome der Gruppen, an die die zwei Reste R binden, darstellen, der Index m 0, 1, 2, 3 oder 4, vorzugsweise 0, 1 oder 2 ist und die Symbole R¹, R², R^{d} und die Indices s, und t die zuvor, insbesondere für Formel (I) und/oder Formeln (RA-1) bis (RA-13) dargelegte Bedeutung haben.

Hierbei sind Strukturen der Formeln RA-4f bevorzugt.

Ferner kann vorgesehen sein, dass die mindestens zwei Reste R, die Strukturen der Formeln (RA-1) bis (RA-12) und/oder (RA-1a) bis (RA-4f) formen und einen kondensierten Ring bilden, Reste R aus benachbarten Gruppen X darstellen oder Reste R darstellen, die jeweils an benachbarte C-Atome binden, wobei diese C-Atome vorzugsweise über eine Bindung verbunden sind.

In einer weiterhin bevorzugten Ausgestaltung bilden bevorzugt mindestens zwei, vorzugsweise benachbarte Reste R mit den weiteren Gruppen, an die die zwei Reste R binden, einen kondensierten Ring, wobei die zwei Reste R Strukturen der Formel (RB) formen 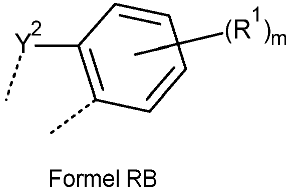 wobei R¹ die zuvor, insbesondere für Formel (I) genannte Bedeutung aufweist, die gestrichelten Bindungen die Anbindungsstellen darstellen, über die die zwei Reste R binden, der Index m 0, 1, 2, 3 oder 4, vorzugsweise 0, 1 oder 2 ist, und Y² C(R¹)₂, NR¹, NAr', BR¹, BAr', O oder S ist, vorzugsweise C(R¹)₂, NAr' oder O, besonders bevorzugt C(R¹)₂ oder O, wobei Ar' die zuvor, insbesondere für Formel (I) genannte Bedeutung aufweist.

Ferner kann vorgesehen sein, dass die mindestens zwei Reste R, die Strukturen der Formel (RB) formen und einen kondensierten Ring bilden, Reste R aus benachbarten Gruppen X darstellen oder Reste R darstellen, die jeweils an benachbarte C-Atome binden, wobei diese C-Atome vorzugsweise über eine Bindung verbunden sind.

Insbesondere kann vorgesehen sein, dass in bevorzugten Strukturen/Verbindungen die Summe der Indices r, s, t, v, m und n vorzugsweise 0, 1, 2 oder 3, besonders bevorzugt 1 oder 2 ist.

Besonders bevorzugt umfassen die Verbindungen mindestens eine Struktur der Formeln (IV-1) bis (IV-12), besonders bevorzugt sind die Verbindungen ausgewählt aus Verbindungen der Formeln (IV-1) bis (IV-12), wobei die Verbindungen mindestens einen kondensierten Ring aufweisen, 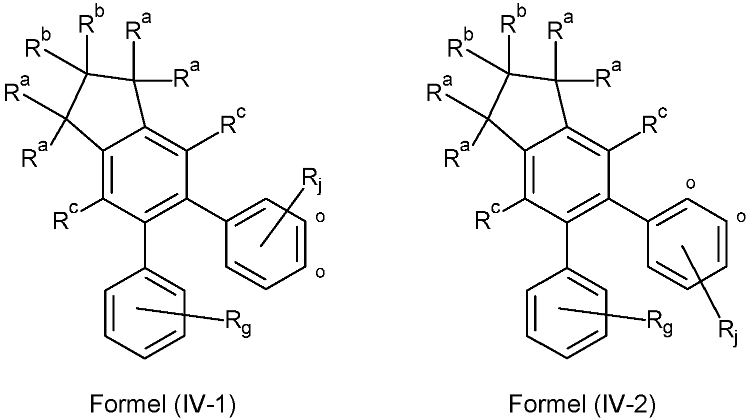 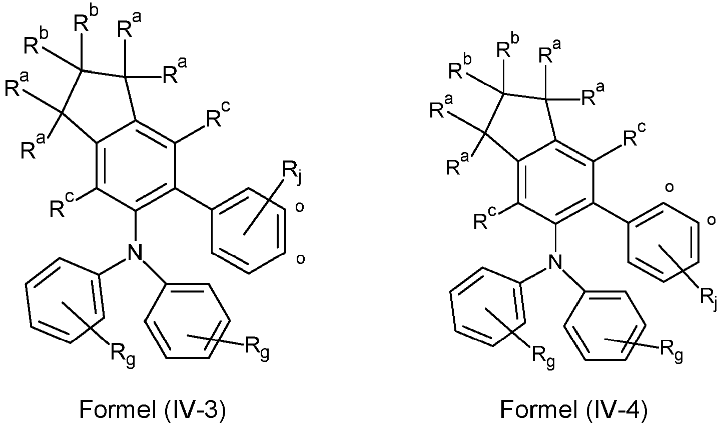 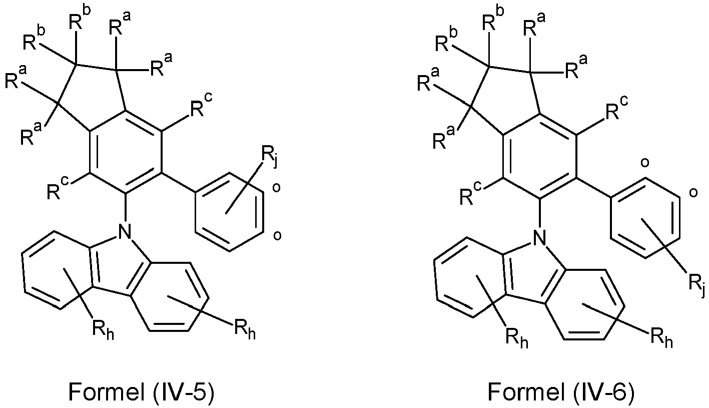 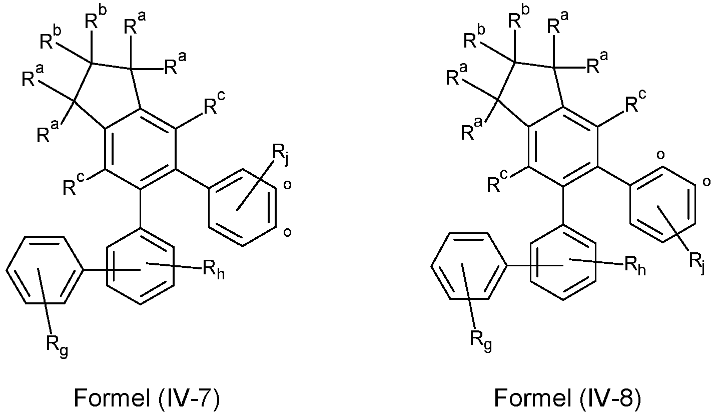 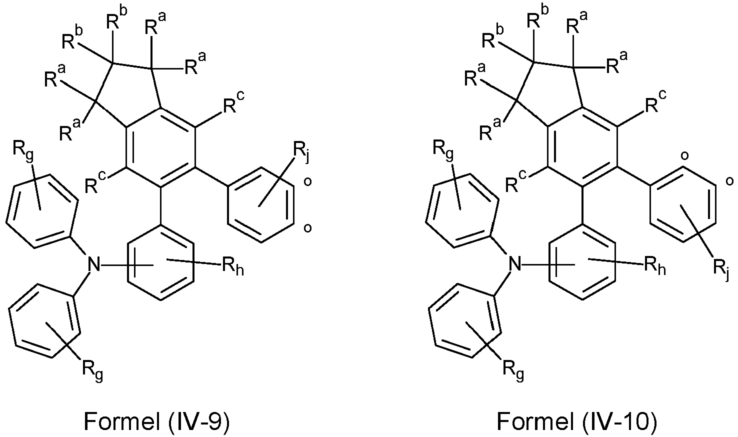 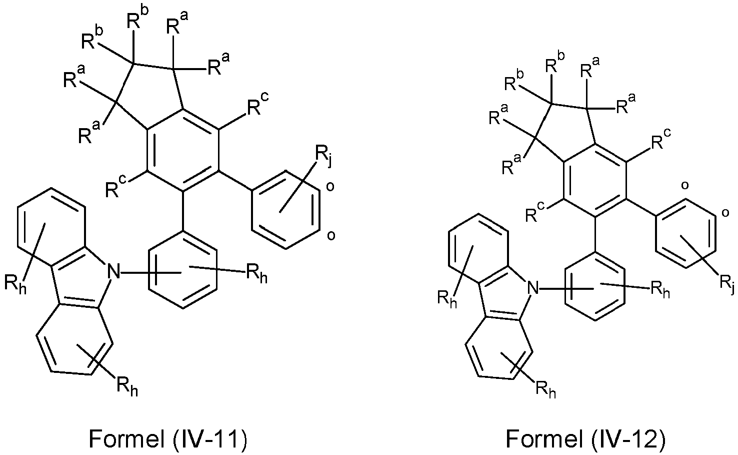 wobei die Symbole R, R^{a}, R^{b} und R^{c} die zuvor, insbesondere für Formel (I) genannten Bedeutungen aufweisen, das Symbol o für die Kondensationsstellen des mindestens einen kondensierten Rings steht und für die weiteren verwendeten Indices gilt:
- j: ist bei jedem Auftreten unabhängig 0, 1, 2 oder 3;
- h: ist bei jedem Auftreten unabhängig 0, 1, 2, 3 oder 4;
- g: ist bei jedem Auftreten unabhängig 0, 1, 2, 3, 4 oder 5.

Ferner kann insbesondere für Strukturen/Verbindungen der Formeln (IV-1) bis (IV-12) vorgesehen sein, dass der kondensierte Ring durch Strukturen der Formeln (RA-1) bis (RA-13), (RA-1a) bis (RA-4f) und/oder (RB) gebildet ist, wie diese zuvor dargestellt sind, vorzugsweise durch Strukturen der Formeln (RA-1) bis (RA-13) und/oder (RA-1a) bis (RA-4f) gebildet ist.

Bevorzugt kann vorgesehen sein, dass die Verbindungen mindestens zwei kondensierte Ringe aufweisen, wobei mindestens ein kondensierter Ring durch Strukturen der Formeln (RA-1) bis (RA-13) und/oder (RA-1a) bis (RA-4f) gebildet ist und ein weiterer Ring durch Strukturen der Formeln (RA-1) bis (RA-13), (RA-1a) bis (RA-4f) oder (RB) gebildet ist.

Weiterhin kann vorgesehen sein, dass die Substituenten R, R^{d} und R¹ gemäß obigen Formeln mit den Ringatomen des Ringsystems, an das die Substituenten R, R^{d} und R¹ binden, kein kondensiertes aromatisches oder heteroaromatisches Ringsystem bilden. Dies schließt die Bildung eines kondensierten aromatischen oder heteroaromatischen Ringsystems mit möglichen Substituenten R^{d}, R¹ und R² ein, die an die Substituenten R, R^{d} und R¹ gebunden sein können.

Die Reste R^{a}, R^{b}, R^{c}, bilden mit weiteren Gruppen vorzugsweise kein Ringsystem aus.

Wenn die erfindungsgemäße Verbindung mit aromatischen oder heteroaromatischen Gruppen R, R^{d}, R¹ beziehungsweise R² substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Ferner kann vorgesehen sein, dass der Reste R kein aromatisches oder heteroaromatisches Ringsystem umfasst, welches drei linear kondensierte aromatische 6 Ringe aufweist, wobei vorzugsweise keiner der Reste R ein aromatisches oder heteroaromatisches Ringsystem umfasst, welches drei linear kondensierte aromatische 6-Ringe aufweist.

Bevorzugt kann die Gruppe Z^{a}, Z^{b} mit der Gruppe, an die die Gruppe Z^{a}, Z^{b} gemäß Formel (I) oder den bevorzugten Ausführungsformen dieser Formel gebunden ist, eine durchgängige Konjugation ausbilden. Eine durchgängige Konjugation der aromatischen beziehungsweise heteroaromatischen Systeme wird ausgebildet, sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht.

Weiterhin kann vorgesehen sein, dass die Substituenten R und R¹ gemäß obigen Formeln mit den Ringatomen des Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R¹ und R² ein, die an die Reste R, R¹ gebunden sein können.

Wenn zwei Reste, die insbesondere ausgewählt sein können aus R, R¹ und/oder R², miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. Weiterhin können die mit den Substituenten R, R¹ und/oder R² versehenen Ringsysteme auch über eine Bindung miteinander verbunden sein, so dass hierdurch ein Ringschluss bewirkt werden kann. In diesem Fall ist jede der entsprechenden Bindungsstellen vorzugsweise mit einem Substituenten R, R¹ und/oder R²versehen.

Ferner kann vorgesehen sein, dass mindestens ein Reste R gleich oder verschieden bei jedem Auftreten ausgewählt ist aus der Gruppe bestehend aus einer verzweigten oder cyclischen Alkyl-, Alkoxy- oder Thioalkoxygruppe mit 3 bis 20 C-Atomen oder einem aromatischen oder heteroaromatischen Ringsystem ausgewählt aus den Gruppen der folgenden Formeln Ar-1 bis Ar-75, vorzugsweise die Substituenten R entweder einen kondensierten Ring, vorzugsweise gemäß den Strukturen der Formeln (RA-1) bis (RA-13) oder (RB) bilden oder der Substituent R gleich oder verschieden bei jedem Auftreten ausgewählt ist aus der Gruppe bestehend aus einem aromatischen oder heteroaromatischen Ringsystem ausgewählt aus den Gruppen der folgenden Formeln Ar-1 bis Ar-75, und/oder die Gruppe Ar' gleich oder verschieden bei jedem Auftreten ausgewählt ist aus den Gruppen der folgenden Formeln Ar-1 bis Ar-75 und/oder die Gruppe Ar^{a}, Ar^{b}, Ar^{c}, Ar^{d} und/oder Ar' gleich oder verschieden bei jedem Auftreten ausgewählt ist aus den Gruppen der folgenden Formeln Ar-1 bis Ar-75, 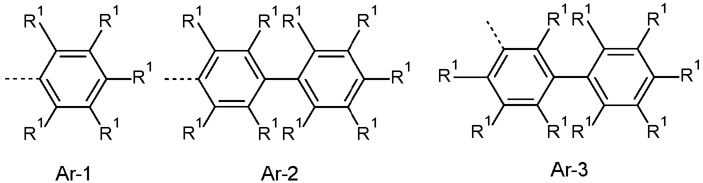 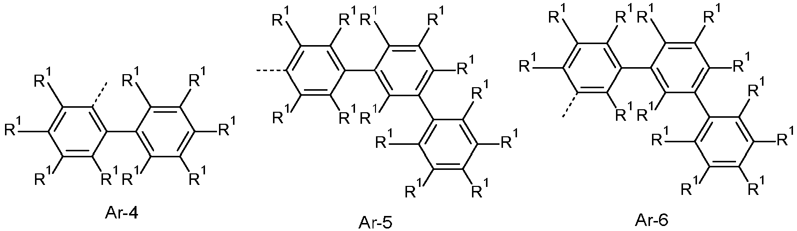 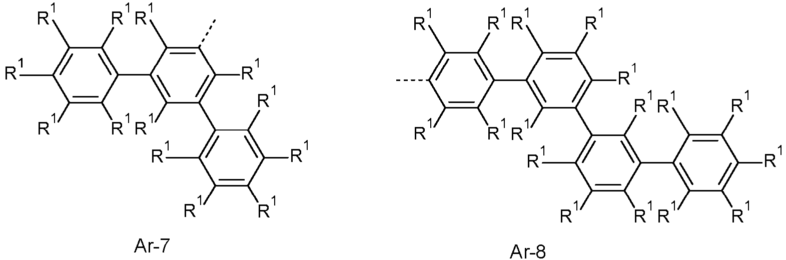 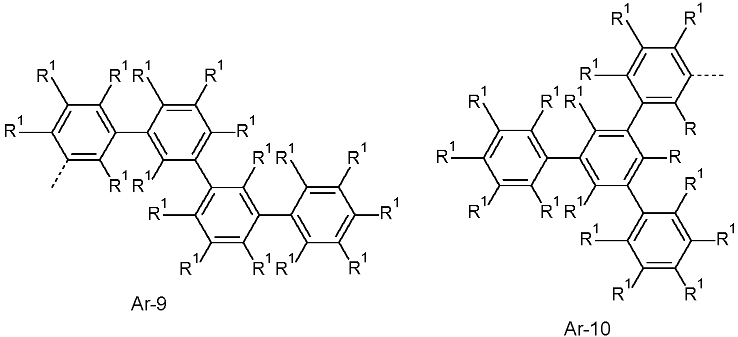 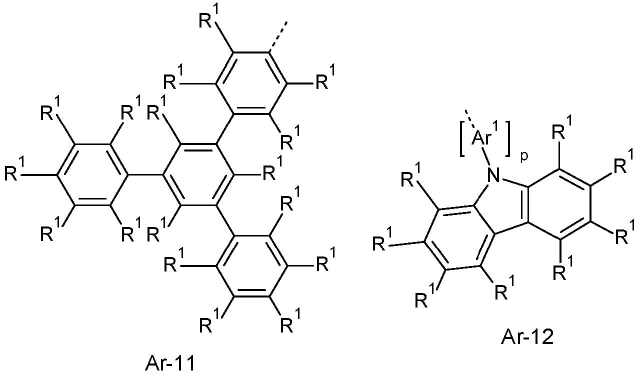 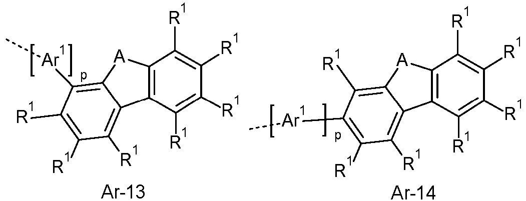 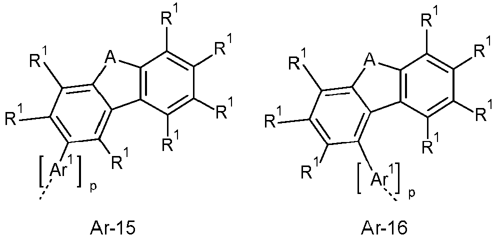 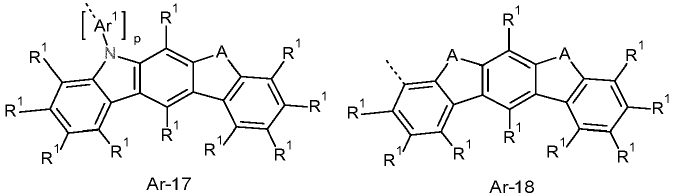 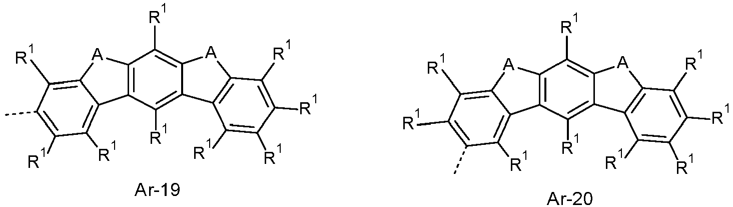 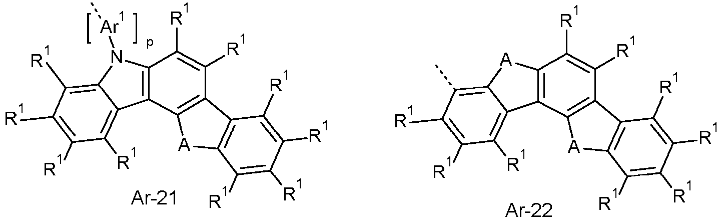 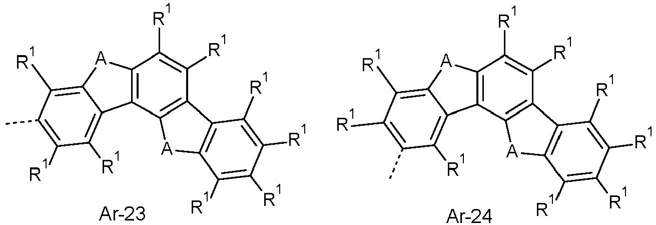 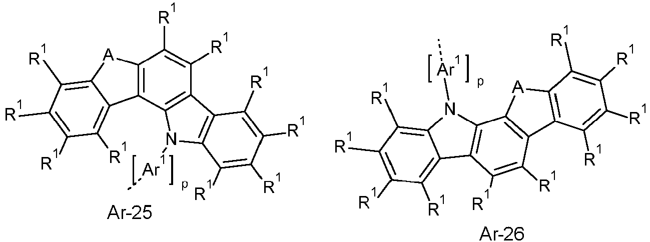 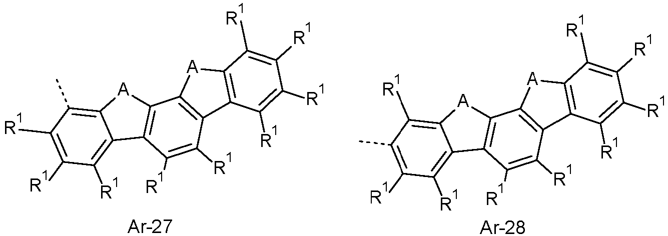 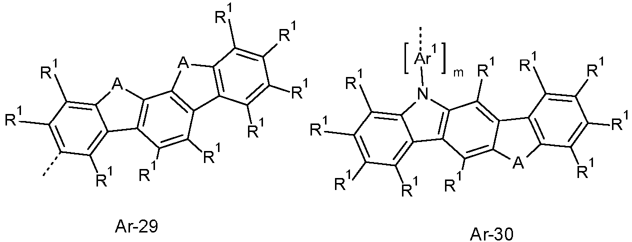 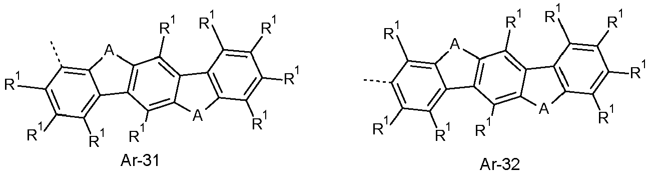 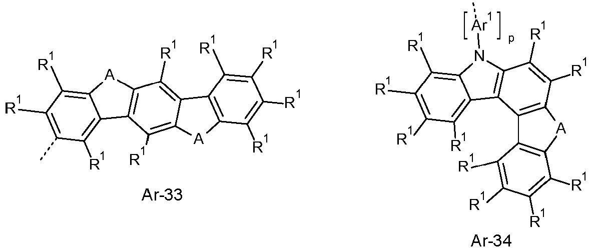 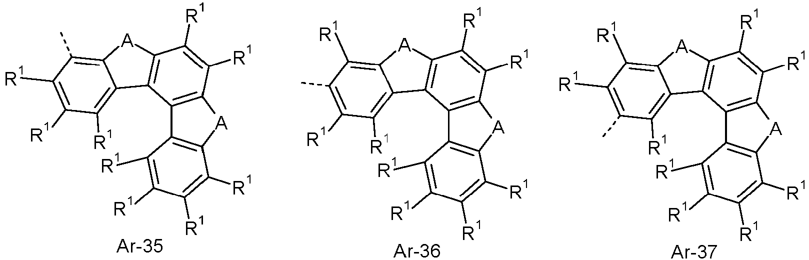 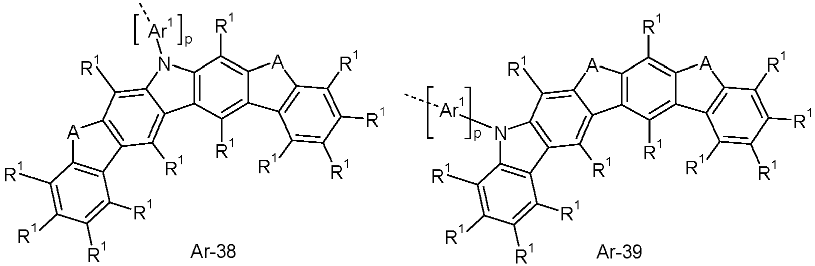 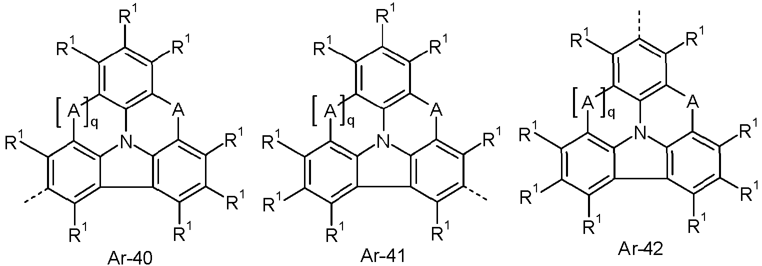 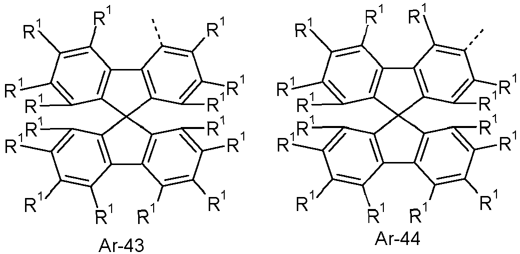 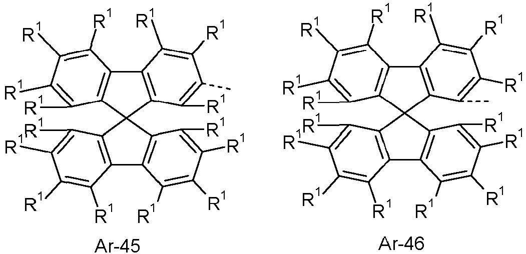 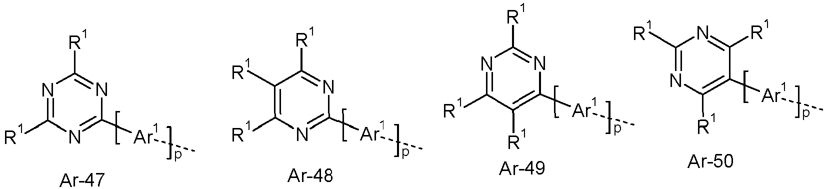 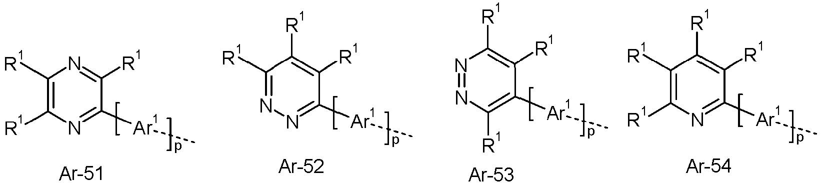 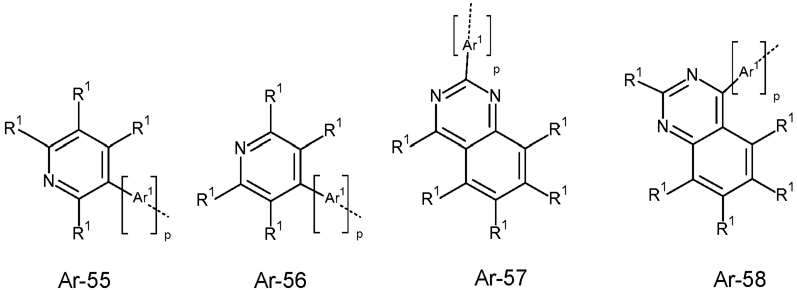 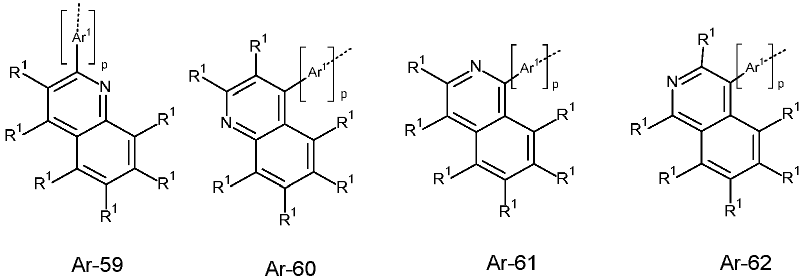 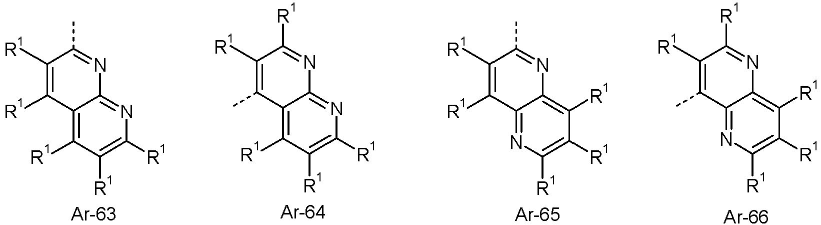 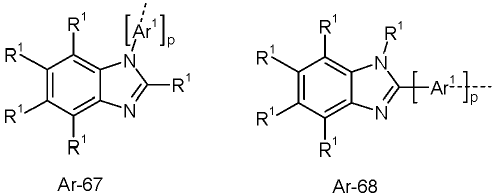 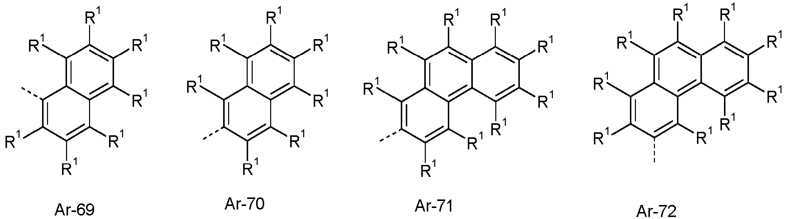 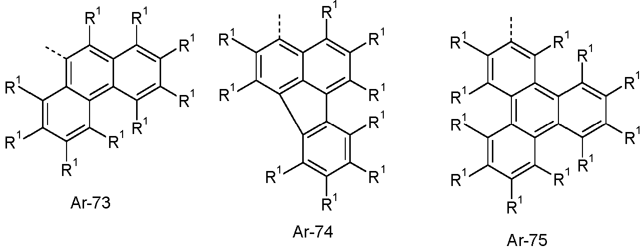 wobei R¹ die oben genannten Bedeutungen aufweist, die gestrichelte Bindung die Bindung an die entsprechende Gruppe darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Die zuvor dargelegten Strukturen der Formeln (Ar-1) bis (Ar-75) stellen bevorzugte Ausgestaltungen des Reste Ar^{a}, Ar^{b}, Ar^{c}, Ar^{d} dar, wie diese beispielsweise in Strukturen der Formel (I) definiert sind, wobei in diesem Fall die Substituenten R¹ in Formeln (Ar-1) bis (Ar-75) durch R zu ersetzen sind, wobei R die zuvor, insbesondere für Formel (I) dargelegte Bedeutung aufweist.

Die zuvor dargelegten Strukturen der Formeln (Ar-1) bis (Ar-75) stellen bevorzugte Ausgestaltungen der Reste Ar und Ar^{e} dar, wie diese beispielsweise für Strukturen der Formel (I) definiert sind, wobei in diesem Fall die Substituenten R¹ in Formeln (Ar-1) bis (Ar-75) durch R zu ersetzen sind, wobei R die zuvor, insbesondere für Formel (I) dargelegte Bedeutung aufweist. Ferner umfassen die Reste Ar und Ar^{e} eine weitere Anbindungsstelle.

Hierbei sind Strukturen der Formeln (Ar-1), (Ar-2), (Ar-3), (Ar-12), (Ar-13), (Ar-14), (Ar-15), (Ar-16), (Ar-40), (Ar-41), (Ar-42), (Ar-43), (Ar-44), (Ar-45), (Ar-46), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt.

Wenn die oben genannten Gruppen für Strukturen der Formeln (Ar-1) bis (Ar-75) mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Im Folgenden werden bevorzugte Substituenten R, R^{a}, R^{b}, R^{c} und R^{d} beschrieben.

In einer bevorzugten Ausführungsform der Erfindung ist R gleich oder verschieden bei jedem Auftreten ausgewählt aus der Gruppe bestehend aus H, D, F, CN, NO₂, Si(R¹)₃, B(OR¹)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer weiterhin bevorzugten Ausführungsform der Erfindung ist Substituent R gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

Ferner kann vorgesehen sein, dass mindestens ein Rest R, vorzugsweise ein Substituent R gleich oder verschieden bei jedem Auftreten ausgewählt ist aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂, besonders bevorzugt ist mindestens ein Substituent R gleich oder verschieden bei jedem Auftreten ausgewählt ist aus der Gruppe bestehend aus einem aromatischen oder heteroaromatischen Ringsystem mit 6 bis 30 aromatischen Ringatomen, welches mit einem oder mehreren Resten R¹ substituiert sein kann. In einer weiterhin bevorzugten Ausführungsform der Erfindung bilden die Substituenten R entweder einen Ring gemäß den Strukturen der Formeln (RA-1) bis (RA-12), (RA-1a) bis (RA-4f) oder (RB) oder der Substituent R ist gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. Besonders bevorzugt ist der Rest R, vorzugsweise der Substituent R gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

Ferner kann vorgesehen sein, dass mindestens ein Rest R ein aromatisches oder heteroaromatisches Ringsystem mit 5 bis 13 aromatischen Ringatomen darstellt, das mit einem oder mehreren Resten R¹ substituiert sein kann.

Bevorzugt kann vorgesehen sein, dass mindestens ein Rest, vorzugsweise ein Substituent R ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können. Hierbei bedeutet der Ausdruck Substituent insbesondere, dass R ungleich H sind. Ferner können die Substituenten R gleich oder verschieden sein, falls zwei oder mehr Substituenten vorhanden sind, die aus den genannten aromatischen oder heteroaromatischen Gruppe ausgewählt sind.

Vorzugsweise kann vorgesehen sein, dass die Gruppe R^{a} für Methyl, Ethyl, Propyl steht oder zwei Gruppen R^{a}, die an das gleiche C-Atom binden einen Cycloalkylrest mit 5 oder 6, vorzugsweise 5 Kohlenstoffatome bilden, wobei die Gruppe R^{a} vorzugsweise für Methyl steht, wobei diese Gruppen deuteriert sein können.

Vorzugsweise kann vorgesehen sein, dass die Gruppe R^{b} für Methyl, Ethyl, Propyl steht oder zwei Gruppen R^{b}, die an das gleiche C-Atom binden einen Cycloalkylrest mit 5 oder 6, vorzugsweise 5 Kohlenstoffatome bilden, wobei die Gruppe R^{b} vorzugsweise für Methyl steht, wobei diese Gruppen deuteriert sein können.

Vorzugsweise kann vorgesehen sein, dass die Gruppe R^{c} für H, D, Methyl, Ethyl, Propyl steht, wobei diese Gruppen deuteriert sein können, wobei die Gruppe R^{c} vorzugsweise für H oder D steht.

In einer bevorzugten Ausführungsform der Erfindung ist R^{d} gleich oder verschieden bei jedem Auftreten ausgewählt aus der Gruppe bestehend aus einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R² substituiert sein kann.

In einer weiterhin bevorzugten Ausführungsform der Erfindung ist R^{d} gleich oder verschieden bei jedem Auftreten ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, einem aromatischen oder heteroaromatischen Ringsystem mit 6 bis 30 aromatischen Ringatomen, welches mit einem oder mehreren Resten R² substituiert sein kann. Besonders bevorzugt ist R^{d} gleich oder verschieden bei jedem Auftreten ausgewählt aus der Gruppe bestehend aus einer geradkettigen Alkylgruppe mit 1 bis 5 C-Atomen oder einer verzweigten oder cyclischen Alkylgruppe mit 3 bis 5 C-Atomen, wobei die Alkylgruppe jeweils mit einem oder mehreren Resten R² substituiert sein kann oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R² substituiert sein kann.

In einer bevorzugten Ausführungsform der Erfindung ist R^{d} bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann; dabei können zwei Reste R^{d} auch miteinander ein Ringsystem bilden. Besonders bevorzugt ist R^{d} bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R² substituiert sein kann, bevorzugt aber unsubstituiert ist, oder einem aromatischen Ringsystem mit 6 bis 12 aromatischen Ringatomen, insbesondere mit 6 aromatischen Ringatomen, das jeweils durch einen oder mehrere, bevorzugt nicht-aromatische Reste R² substituiert sein kann, bevorzugt aber unsubstituiert ist; dabei können zwei Reste R^{d} miteinander ein Ringsystem bilden. Ganz besonders bevorzugt ist R^{d} bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen. Ganz besonders bevorzugt steht R^{d} für eine Methylgruppe oder für eine Phenylgruppe, wobei zwei Phenylgruppen zusammen ein Ringsystem bilden können, wobei eine Methylgruppe gegenüber einer Phenylgruppe bevorzugt ist.

Bevorzugte aromatische bzw. heteroaromatische Ringsysteme, für die Substituent R, R^{d} beziehungsweise Ar^{a}, Ar^{b}, Ar^{c}, Ar^{d} oder Ar' stehen, sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Anthracen, Pyren, Perylen, Chrysen, Phenanthren oder Triphenylen, welche jeweils mit einem oder mehreren Resten R, R¹ beziehungsweise R² substituiert sein können. Besonders bevorzugt sind die oben aufgeführten Strukturen Ar-1 bis Ar-75, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75) bevorzugt und Strukturen der Formeln (Ar-1), (Ar-2), (Ar-3), (Ar-12), (Ar-13), (Ar-14), (Ar-15), (Ar-16) besonders bevorzugt sind. Hinsichtlich der Strukturen Ar-1 bis Ar-75 ist festzuhalten, dass diese mit einem Substituenten R¹ dargestellt sind. Im Falle der Ringsysteme Ar^{a}, Ar^{b}, Ar^{c}, Ar^{d} sind diese Substituenten R¹ durch R und im Falle R^{d} sind diese Substituenten R¹ durch R² zu ersetzen.

Weitere geeignete Gruppen R sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R⁵ substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (I) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Ferner kann vorgesehen sein, dass die Verbindung genau zwei oder genau drei Strukturen gemäß Formel (I), (1-1) bis (I-6) (II-1) bis (II-154), (III-1) bis (III-60) und/oder (IV-1) bis (IV-12) umfasst.

**In** einer bevorzugten Ausgestaltung sind die Verbindungen ausgewählt aus Verbindungen der Formel (D-1), 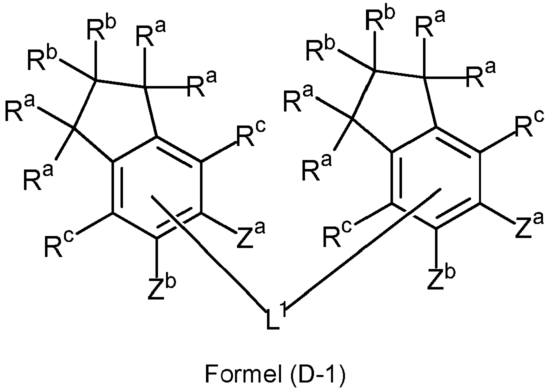 wobei die Gruppe L¹ eine Verbindungsgruppe, vorzugsweise eine Bindung oder ein aromatisches oder heteroaromatisches Ringsystem mit 5 bis 40, bevorzugt 5 bis 30 aromatischen Ringatomen darstellt, welches durch einen oder mehrere Reste R substituiert sein kann, und die weiteren verwendeten Symbole die zuvor, insbesondere für Formel (I) genannten Bedeutungen aufweisen, wobei die Gruppe L¹ an Stelle eines Wasserstoffatoms oder eines Substituenten eine Bindung an die Grundstruktur bildet, vorzugsweise die Gruppe L¹ an die Reste Z^{a}, Z^{b} bindet.

In einer weiteren bevorzugten Ausführungsform der Erfindung steht L¹ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste R substituiert sein kann, bevorzugt aber unsubstituiert ist, wobei R die zuvor, insbesondere für Formel (I) genannte Bedeutung aufweisen kann. Besonders bevorzugt steht L¹ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R¹ substituiert sein kann, bevorzugt aber unsubstituiert ist, wobei R¹ die zuvor, insbesondere für Formel (I) genannte Bedeutung aufweisen kann.

Weiterhin bevorzugt steht das unter anderem in Formel (D1) dargelegte Symbol L¹ gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (D1) dargelegte Gruppe L¹ ein aromatisches Ringsystem mit höchstens vier, bevorzugt höchstens drei, besonders bevorzugt höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L¹ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste R¹ substituiert sein können, bevorzugt aber unsubstituiert sind.

Gemäß einer bevorzugten Ausgestaltung ist eine erfindungsgemäße Verbindung durch mindestens eine der Strukturen gemäß Formeln (I), (1-1) bis (I-6) (II-1) bis (II-154), (III-1) bis (III-60) und/oder (IV-1) bis (IV-12) darstellbar. Vorzugsweise weisen erfindungsgemäße Verbindungen, bevorzugt umfassend Strukturen gemäß Formeln (I), (1-1) bis (I-6) (II-1) bis (II-154), (III-1) bis (III-60) und/oder (IV-1) bis (IV-12) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol, spezieller bevorzugt kleiner oder gleich 1200 g/mol und ganz besonders bevorzugt kleiner oder gleich 900 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Ferner kann vorgesehen sein, dass die Verbindung umfassend Strukturen gemäß Formel (I), vorzugsweise die Verbindung gemäß Formel (I) oder eine bevorzugte Ausführungsform dieser Struktur/Verbindung nicht in unmittelbaren Kontakt mit einem Metallatom steht, vorzugsweise kein Ligand für einen Metallkomplex darstellt.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

| | |
|---|---|
| 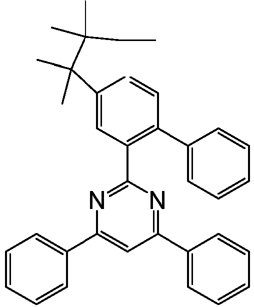 | 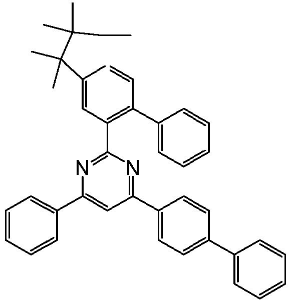 |
| 1 | 2 |
| 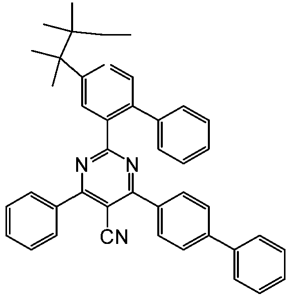 | 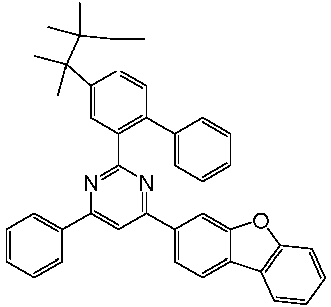 |
| 3 | 4 |
| 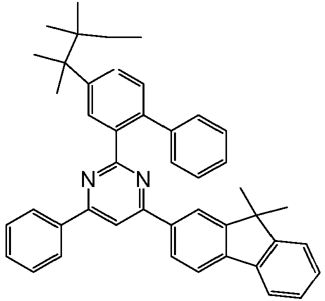 | 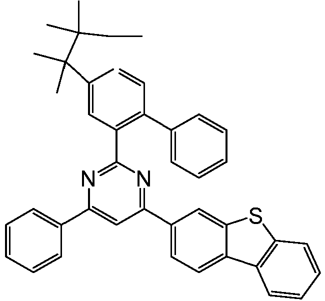 |
| 5 | 6 |
| 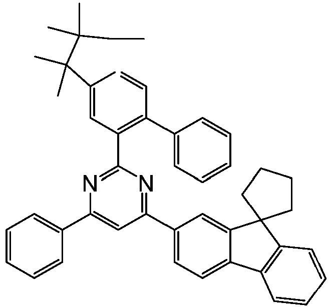 | 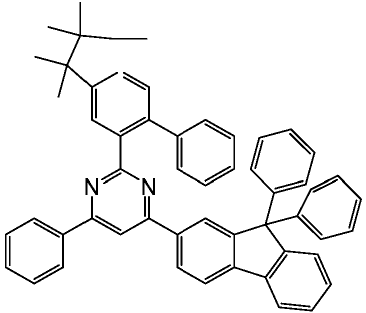 |
| 7 | 8 |
| 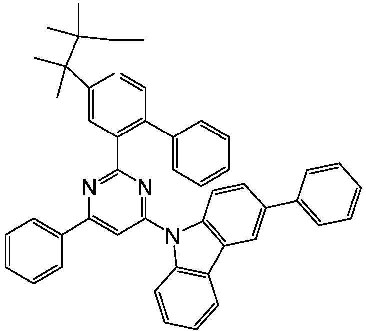 | 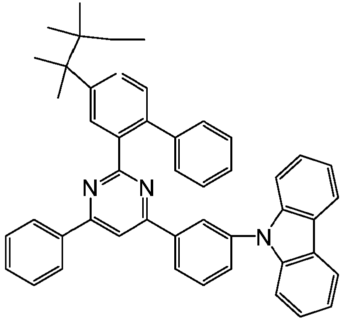 |
| 9 | 10 |
| 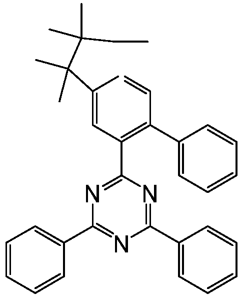 | 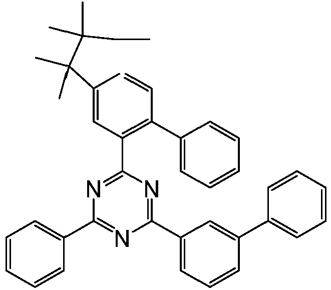 |
| 11 | 12 |
| 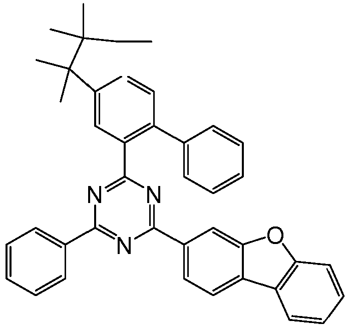 | 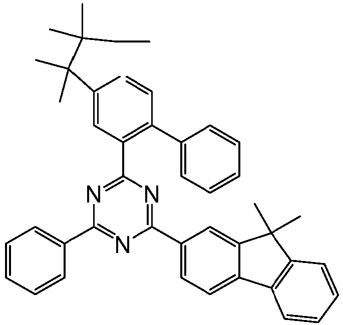 |
| 13 | 14 |
| 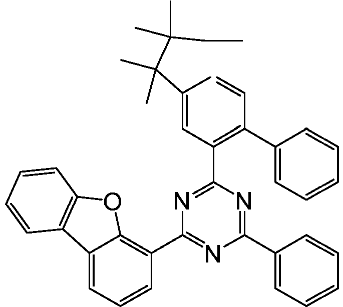 | 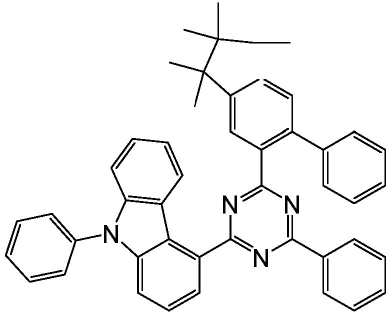 |
| 15 | 16 |
| 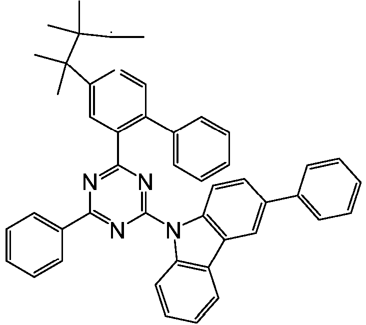 | 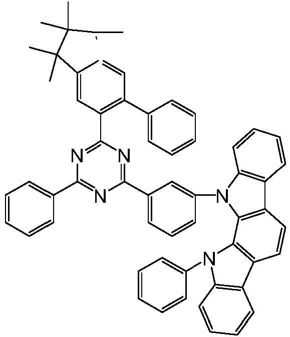 |
| 17 | 18 |
| 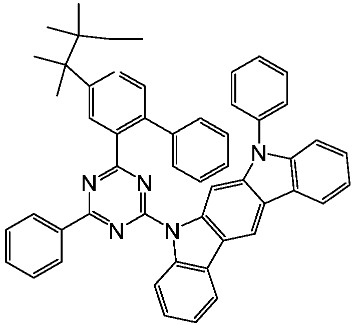 | 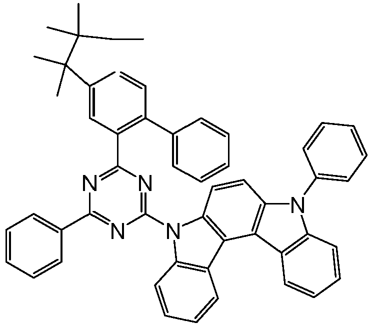 |
| 19 | 20 |
| 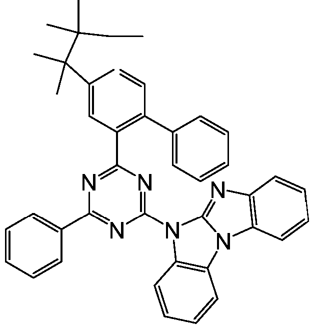 | 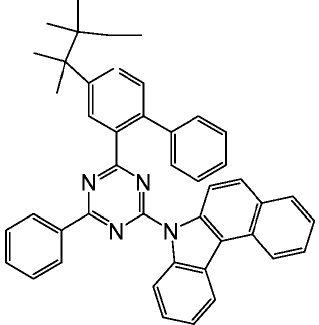 |
| 21 | 22 |
| 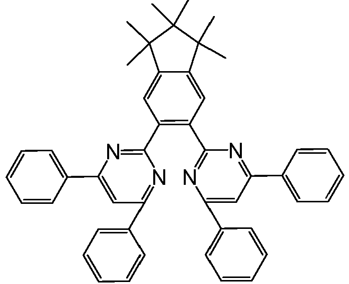 | 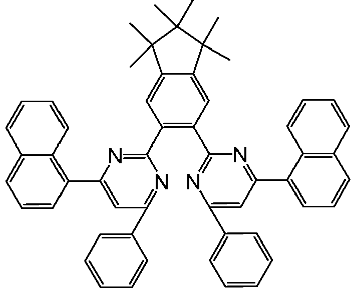 |
| 23 | 24 |
| 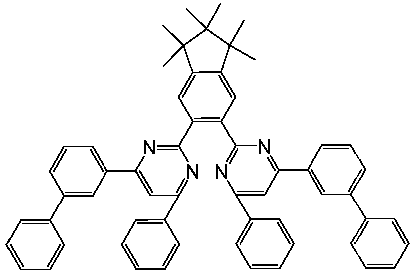 | 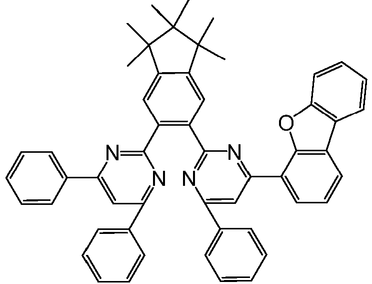 |
| 25 | 26 |
| 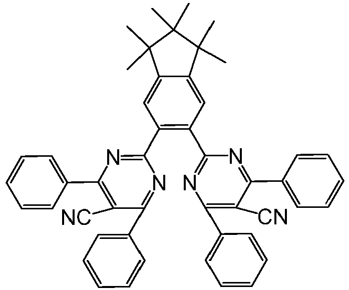 | 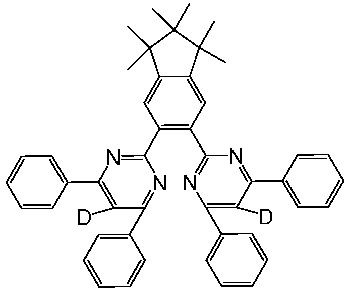 |
| 27 | 28 |
| 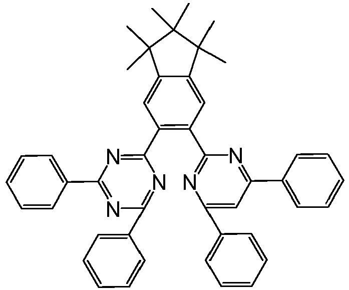 | 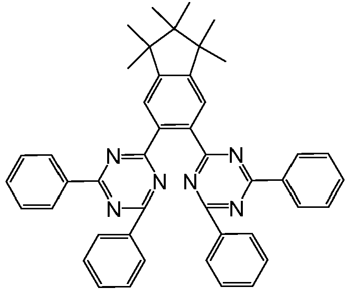 |
| 29 | 30 |
| 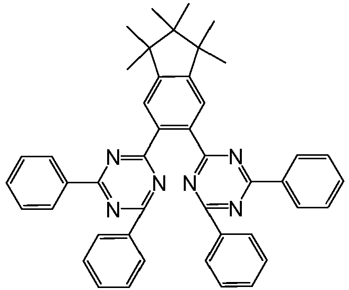 | 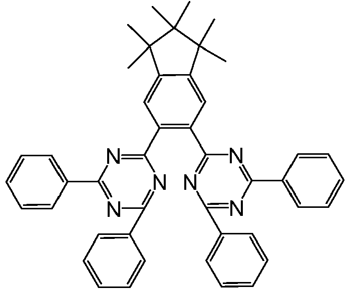 |
| 31 | 30 |
| 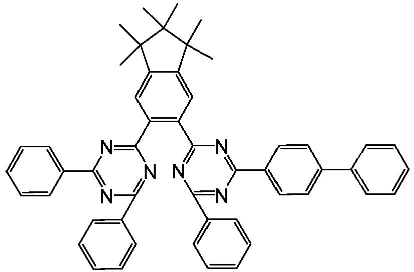 | 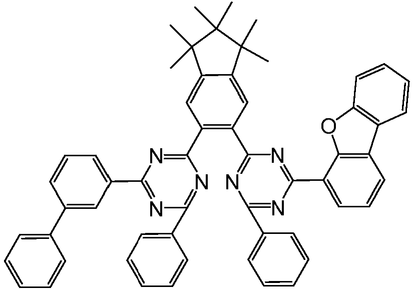 |
| 32 | 33 |
| 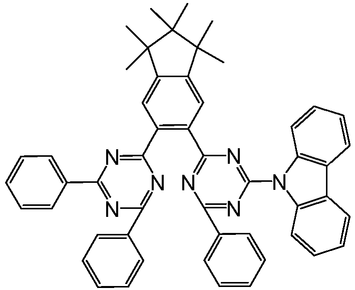 | 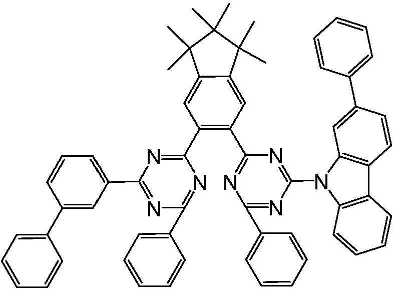 |
| 34 | 35 |
| 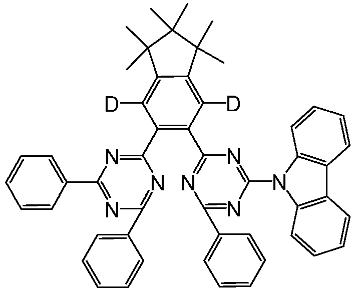 | 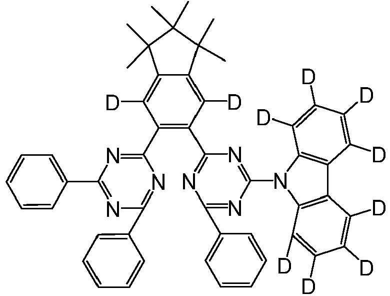 |
| 36 | 37 |
| 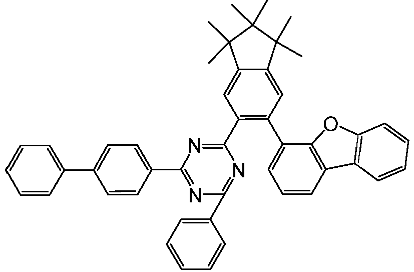 | 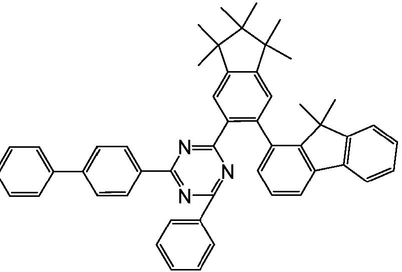 |
| 38 | 39 |
| 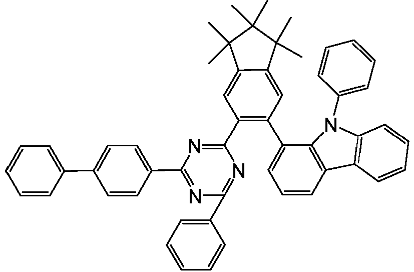 | 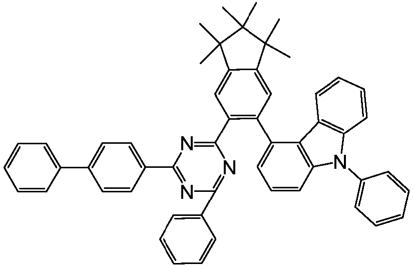 |
| 40 | 41 |
| 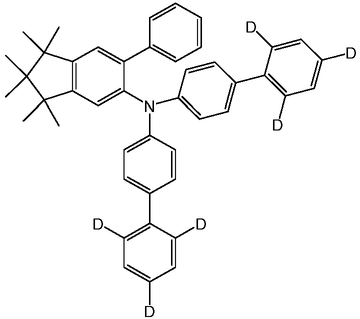 | 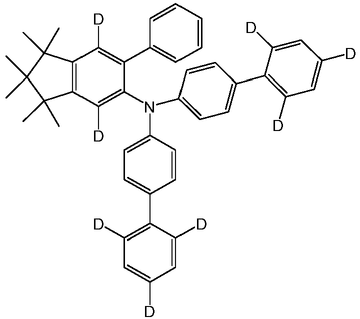 |
| 42 | 43 |
| 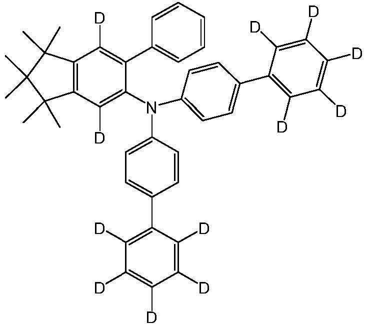 | 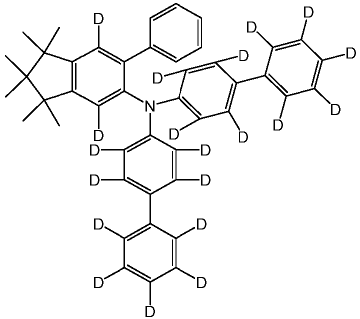 |
| 44 | 45 |
| 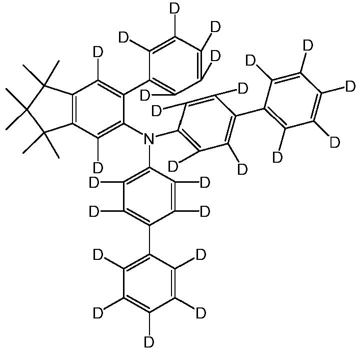 | 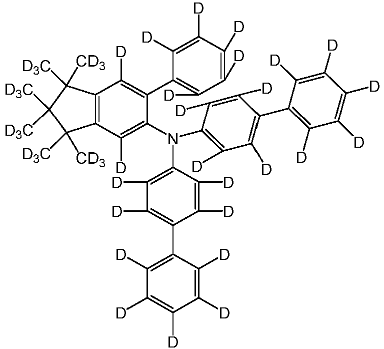 |
| 46 | 47 |
| 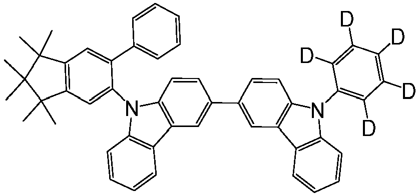 | 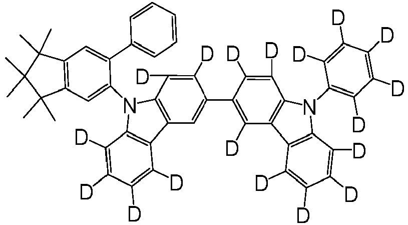 |
| 48 | 49 |
| 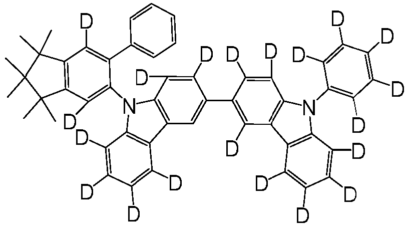 | 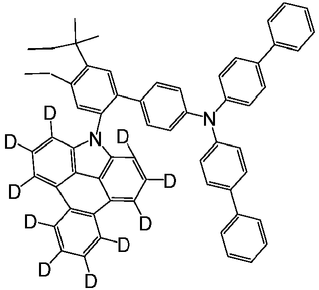 |
| 50 | 51 |
| 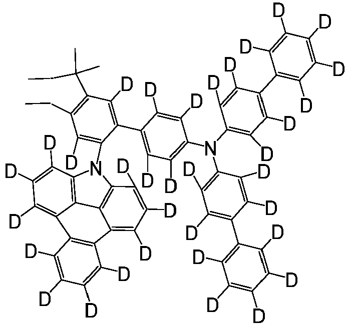 | 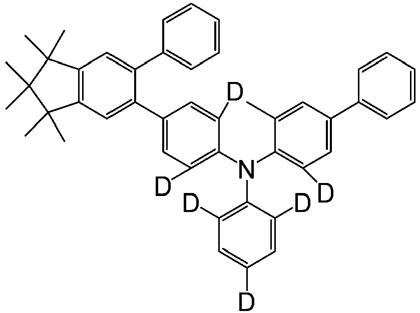 |
| 52 | 53 |
| 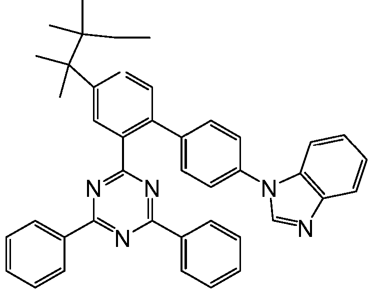 | 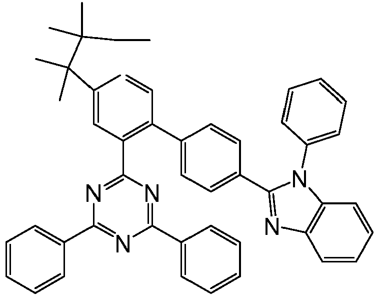 |
| 54 | 55 |
| 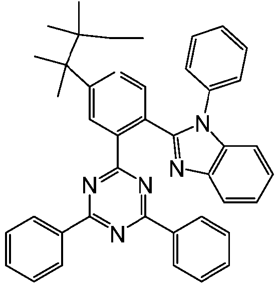 | 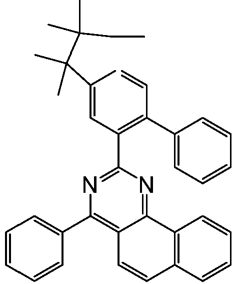 |
| 56 | 57 |
| 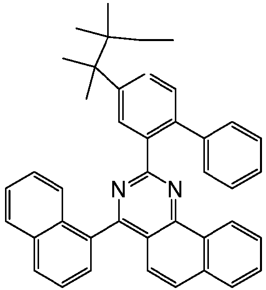 | 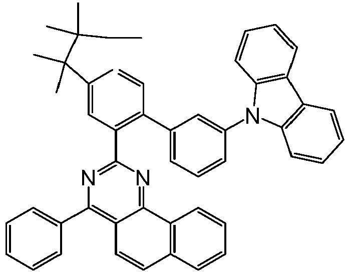 |
| 58 | 59 |
| 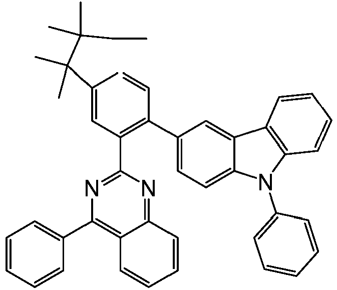 | 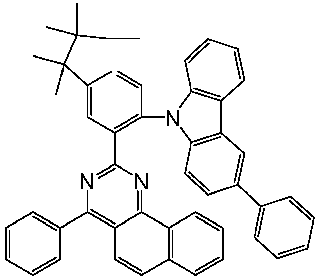 |
| 60 | 61 |
| 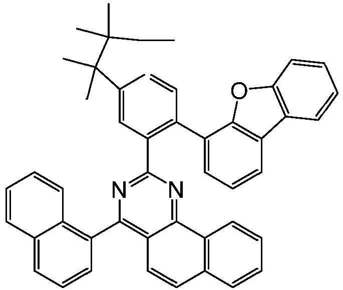 | 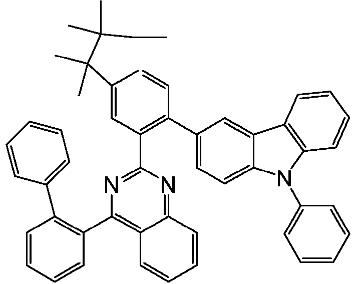 |
| 62 | 63 |
| 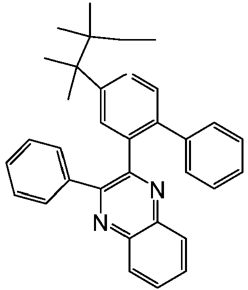 | 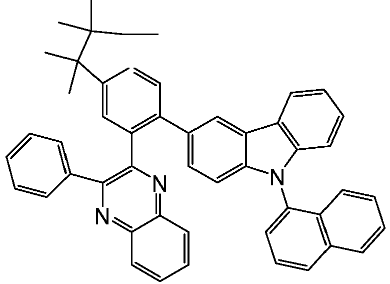 |
| 64 | 65 |
| 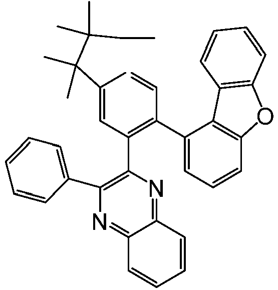 | 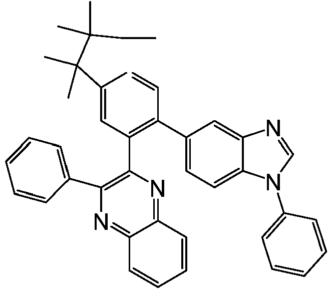 |
| 66 | 67 |

Die Grundstruktur der erfindungsgemäßen Verbindungen kann nach den in den nachfolgenden Schemata skizzierten Wegen dargestellt werden. Dabei sind die einzelnen Syntheseschritte, wie beispielsweise Kupplungsreaktionen, die zu C-C-Verknüpfungen und/oder C-N-Verknüpfungen führen dem Fachmann prinzipiell bekannt. Hierzu gehören unter anderem Reaktionen gemäß BUCHWALD, SUZUKI, YAMAMOTO, STILLE, HECK, NEGISHI, SONOGASHIRA und HIYAMA. Weitere Informationen zur Synthese der erfindungsgemäßen Verbindungen können den Synthesebeispielen entnommen werden.

Die nachfolgenden Schemata beschreiben die Darstellung der erfindungsgemäßen Verbindungen durch die Verwendung von 5,6-Dibromo-2,3-dihydro-1,1,2,2,3,3-hexamethyl-1**H**-indene [1541101-19-2] bzw. dessen teil oder vollständig deuterierten Varianten. Diese Verwendung ist beispielhaft zu verstehen, so dass weitere erfindungsgemäße Verbindungen über ähnliche Synthesewege erhalten werden können, die von anderen Grundstrukturen ausgehen.

Die Synthese von Phenylverbindungen, an die eine Cyclopentylgruppe kondensiert ist, ist in der Fachwellt weithin bekannt. Viele dieser Verbindungen, insbesondere 5,6-Dibromo-2,3-dihydro-1,1,2,2,3,3-hexamethyl-1**H**-indene [1541101-19-2] bzw. dessen teil- oder vollständig deuterierten Varianten, sind kommerziell erhältlich. Hierzu gehören beispielsweise 5,6-Dibromo-2,3-dihydro-1,1,2,2,3,3-hexamethyl-1*H*-inden-*4,7-d2* [1541101-31-8] und 5,6-Dibromo-2,3-dihydro-1,1,2,2,3,3-hexa(methyl-*d*₃)-1*H*-inden [1541101-25-0].

Die erfindungsgemäßen Verbindungen mit Amin-Gruppen, insbesondere Verbindungen umfassend Strukturen gemäß Formeln (I-2) bis (I-6) können ausgehend von 5,6-Dibromo-2,3-dihydro-1,1,2,2,3,3-hexamethyl-1**H-**indene [1541101-19-2] bzw. dessen teil oder vollständig deuterierten Varianten durch folgende Synthesewege erhalten werden:
1) Suzuki-Kupplungen, mit einer Aryl-/Heteroaryl-Boronsäure bzw. -ester ((HO)₂B-Ar), gefolgt von einer Buchwald-Hartwig- oder Ullmann-Kupplung unter Einführung eines Diaryl-Amin-Restes NAr¹Ar²: 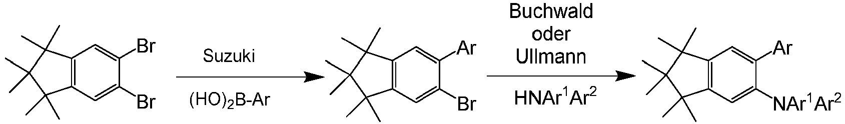
2) eine erste Suzuki-Kupplung 1, mit einer Aryl-/Heteroaryl-Boronsäure bzw. -ester ((HO)₂B-Ar¹), gefolgt von einer zweiten Suzuki-Kupplung 2, mit einer Aryl-/Heteroaryl-Boronsäure bzw. -ester ((HO)₂B-Ar²), wobei wenigstens eine der beiden Aryl-/Heteroaryl-Boronsäure bzw. -ester eine Amin- oder Carbazol-Gruppe umfasst: 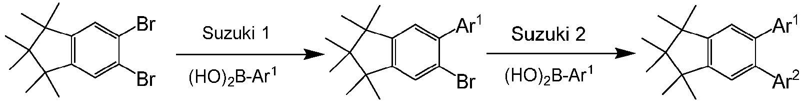
3) eine erste Buchwald-Hartwig- oder Ullmann-Kupplung 1, mit einem Diaryl-Amin (HNAr¹Ar²), gefolgt von einer zweiten Buchwald-Hartwig- oder Ullmann-Kupplung 2, mit einem Diaryl-Amin (HNAr³Ar⁴): 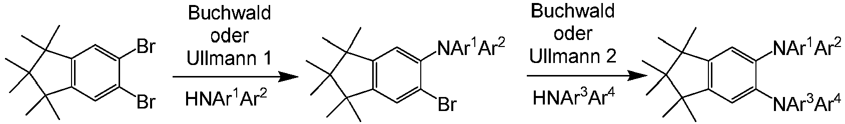 dargestellt werden.

Die erfindungsgemäßen Verbindungen tragend elektronenarme Heterocyclen, insbesondere Pyrimidine und Triazine, beispielsweise Verbindungen umfassend Strukturen gemäß Formel (I-1) können ausgehend von 5,6-Dibromo-2,3-dihydro-1,1,2,2,3,3-hexamethyl-1**H-**indene [1541101-19-2] bzw. dessen teil oder vollständig deuterierten Varianten durch folgende Synthesewege erhalten werden:
1) Suzuki-Kupplungen, mit einer Aryl-/Heteroaryl-Boronsäure bzw. -ester ((HO)₂B-Ar), gefolgt von einer Metallierung (Lithiierung oder Grignard-Bildung) und Umsetzung mit einem Chlor-di-aryl-pyrimidin oder -triazin: 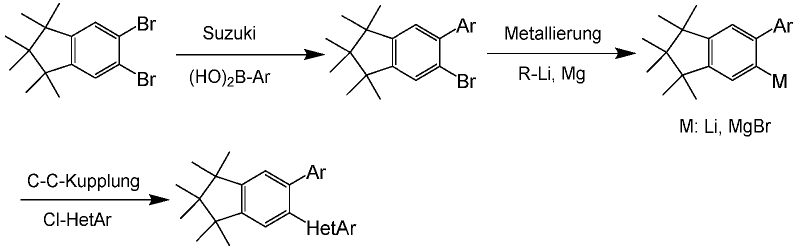
2) eine erste Suzuki-Kupplung 1, mit einer Aryl-/Heteroaryl-Boronsäure bzw. -ester ((HO)₂B-Ar¹), gefolgt von einer zweiten Suzuki-Kupplung 2, mit einer Aryl-/Heteroaryl-Boronsäure bzw. -ester ((HO)₂B-Ar²), wobei wenigstens eine der beiden Aryl-/Heteroaryl-Boronsäure bzw. -ester eine erfindungsgemäße Heteroaryl-Gruppe umfasst: 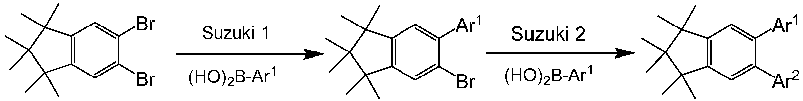
3) eine Bisborylierung und nachfolgend eine erste Suzuki-Kupplung 1, mit einem Halogen-aromaten bzw. -heteroaromaten (Hal-Ar¹), gefolgt von einer zweiten Suzuki-Kupplung 2, mit einem Halogen-aromaten bzw. -heteroaromaten (Hal-Ar²), wobei wenigstens einer der beiden Halogen-aromaten bzw. -heteroaromaten (Hal-Ar¹) eine erfindungsgemäße Heteroaryl-Gruppe umfasst, wobei so bevorzugt Pyrimidine bzw. Triazine direkt an das 2,3-dihydro-1,1,2,2,3,3-hexamethyl-1**H**-inden angeknüpft werden können: 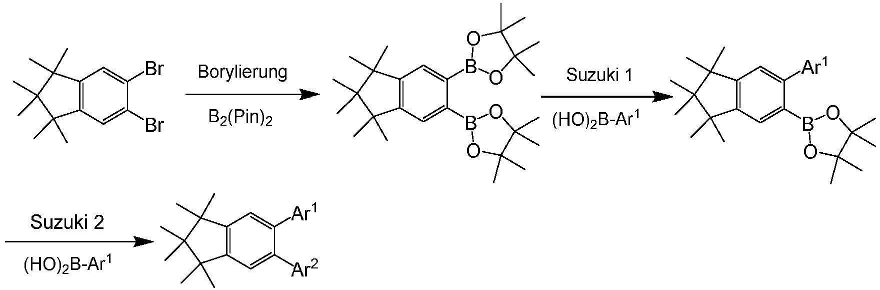 dargestellt werden.

Die Bedeutung der in den zuvor dargelegten Schemata verwendeten Symbole entspricht im Wesentlichen denen, die für Formel (I) definiert wurde, wobei aus Gründen der Übersichtlichkeit auf eine Nummerierung sowie auf eine vollständige Darstellung aller Symbole verzichtet wurde.

Ein weiterer Gegenstand der vorliegenden Erfindung ist daher ein Verfahren zur Herstellung einer erfindungsgemäßen Verbindung, wobei eine Phenylverbindung, an die eine Cyclopentylgruppe kondensiert ist, synthetisiert wird und mindestens ein aromatischer oder heteroaromatischer Rest eingeführt wird, vorzugsweise mittels einer nukleophilen aromatischen Substitutionsreaktion oder einer Kupplungsreaktion.

Durch diese Verfahren, gegebenenfalls gefolgt von Aufreinigung, wie z. B. Umkristallisation oder Sublimation, lassen sich die erfindungsgemäßen Verbindungen in hoher Reinheit, bevorzugt mehr als 99 % (bestimmt mittels ¹H-NMR und/oder HPLC) erhalten.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) und bevorzugten Ausführungsformen dieser Formel oder erfindungsgemäße Verbindungen, wobei ein oder mehrere Bindungen der erfindungsgemäßen Verbindungen oder der Strukturen der Formel (I) und bevorzugten Ausführungsformen dieser Formel zum Polymer, Oligomer oder Dendrimer vorhanden sind. Je nach Verknüpfung der Strukturen der Formel (I) und bevorzugten Ausführungsformen dieser Formel bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, umfassend Strukturen gemäß den Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Vorzugsweise kann vorgesehen sein, dass mindestens eine weitere Verbindung ausgewählt ist aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Loch-injektionsmaterialien, Elektronenblockiermaterialien und Loch-blockiermaterialien, vorzugsweise Hostmaterialien.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung. Vorzugsweise kann vorgesehen sein, dass die erfindungsgemäßen Verbindung in einer elektronischen Vorrichtung als Hostmaterial, Elektronentransportmaterial, Elektroneninjektionsmaterial, Lochleitermaterial, Loch-injektionsmaterial, Elektronenblockiermaterial, Lochblockiermaterial eingesetzt wird.

Ein nochmals weiterer Gegenstand der vorliegenden Erfindung ist eine elektronische Vorrichtung enthaltend mindestens eine erfindungsgemäße Verbindung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Besonders bevorzugt ist elektronische Vorrichtung ausgewählt aus der Gruppe bestehend aus organischen Elektrolumineszenzvorrichtungen (OLEDs, sOLED, PLEDs, LECs, etc.),vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem- Elektrolumineszenzvorrichtung handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Verbindung gemäß Formel (I) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. Besonders bevorzugt wird die erfindungsgemäße Verbindung als Matrixmaterial für phosphoreszierende Emitter, insbesondere für rot, orange, grün oder gelb phosphoreszierende Emitter, in einer emittierenden Schicht, als Elektronentransport- bzw. Lochblockiermaterial in einer Elektronentransport- bzw. Lochblockierschicht oder als Lochtransport- bzw. Elektronenblockiermaterial in einer Lochtransport- bzw. Elektronenblockierschicht eingesetzt.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

In einer Ausführungsform der Erfindung wird die erfindungsgemäße Verbindung dabei als einziges Matrixmaterial ("single host") für den phosphoreszierenden Emitter eingesetzt.

Eine weitere Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Ebenso kann ein weiterer phosphoreszierender Emitter, welcher kürzerwellig als der eigentliche Emitter emittiert, als Co-Host in der Mischung vorhanden sein. Besonders gute Ergebnisse werden erzielt, wenn als Emitter ein rot phoshoreszierender Emitter eingesetzt wird und als Co-Host in Kombination mit der erfindungsgemäßen Verbindung ein gelb phosphoreszierender Emitter verwendet wird.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680. In diesem Zusammenhang ist festzuhalten, dass erfindungsgemäße Verbindungen ohne spezielle funktionale Gruppen, beispielsweise Lochtransportgruppen und/oder Elektronentransportgruppen vorteilhafte Eigenschaften aufweisen.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und WO 2018/011186 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind in der folgenden Tabelle aufgeführt.

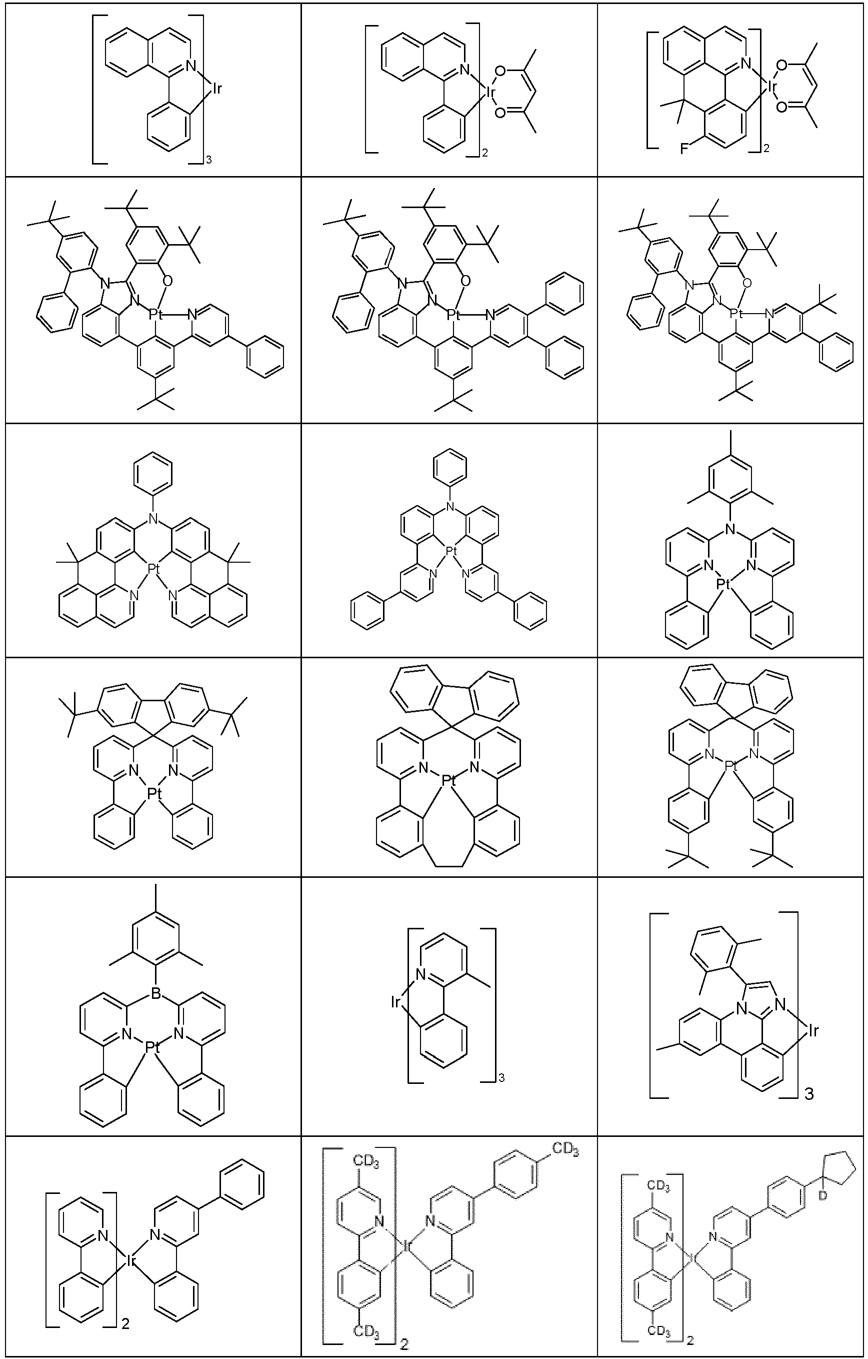

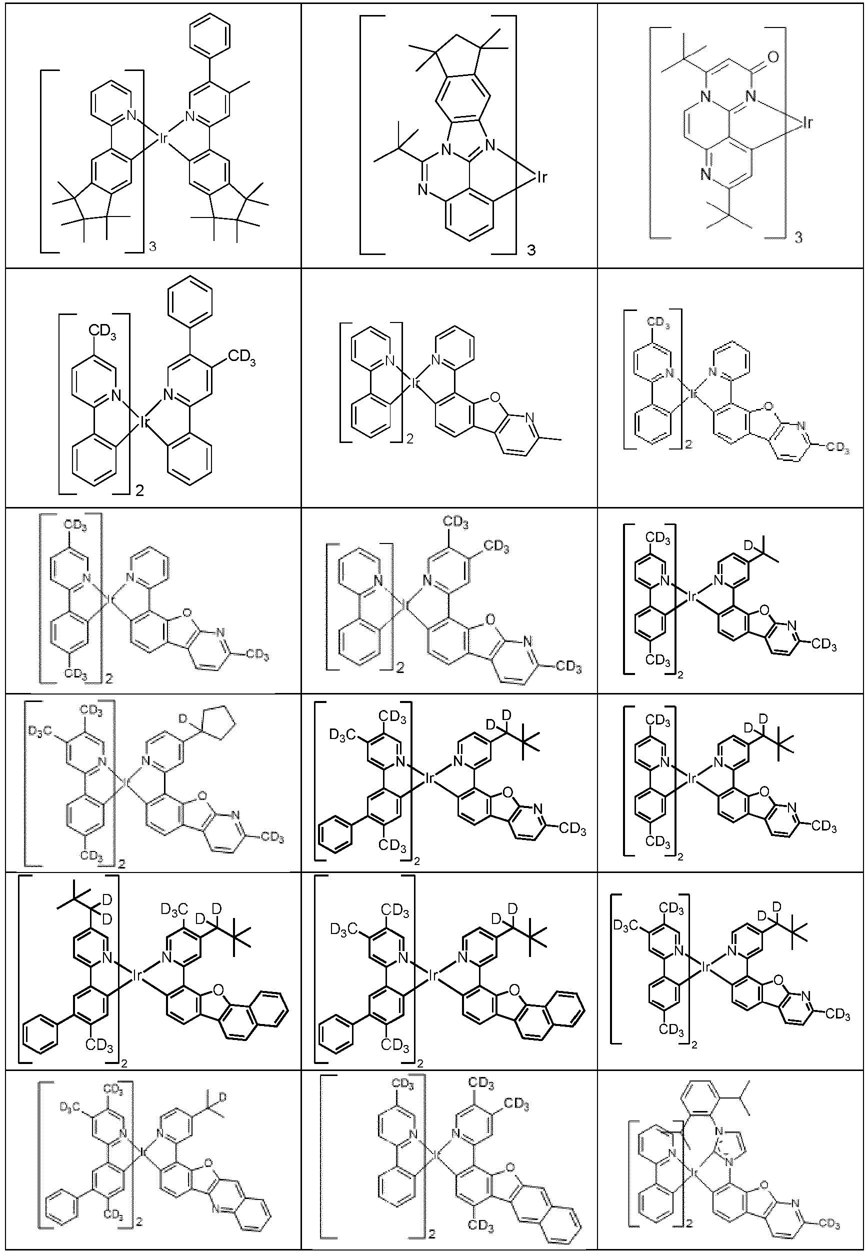

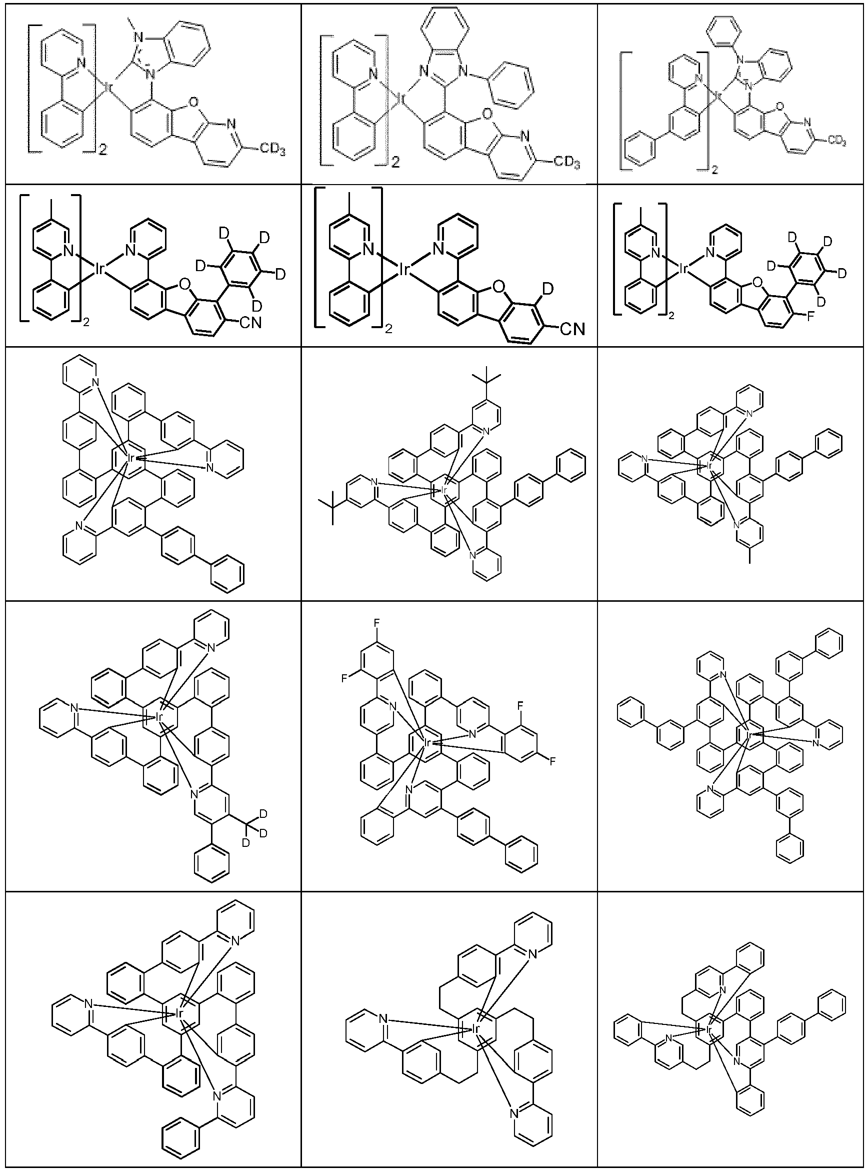

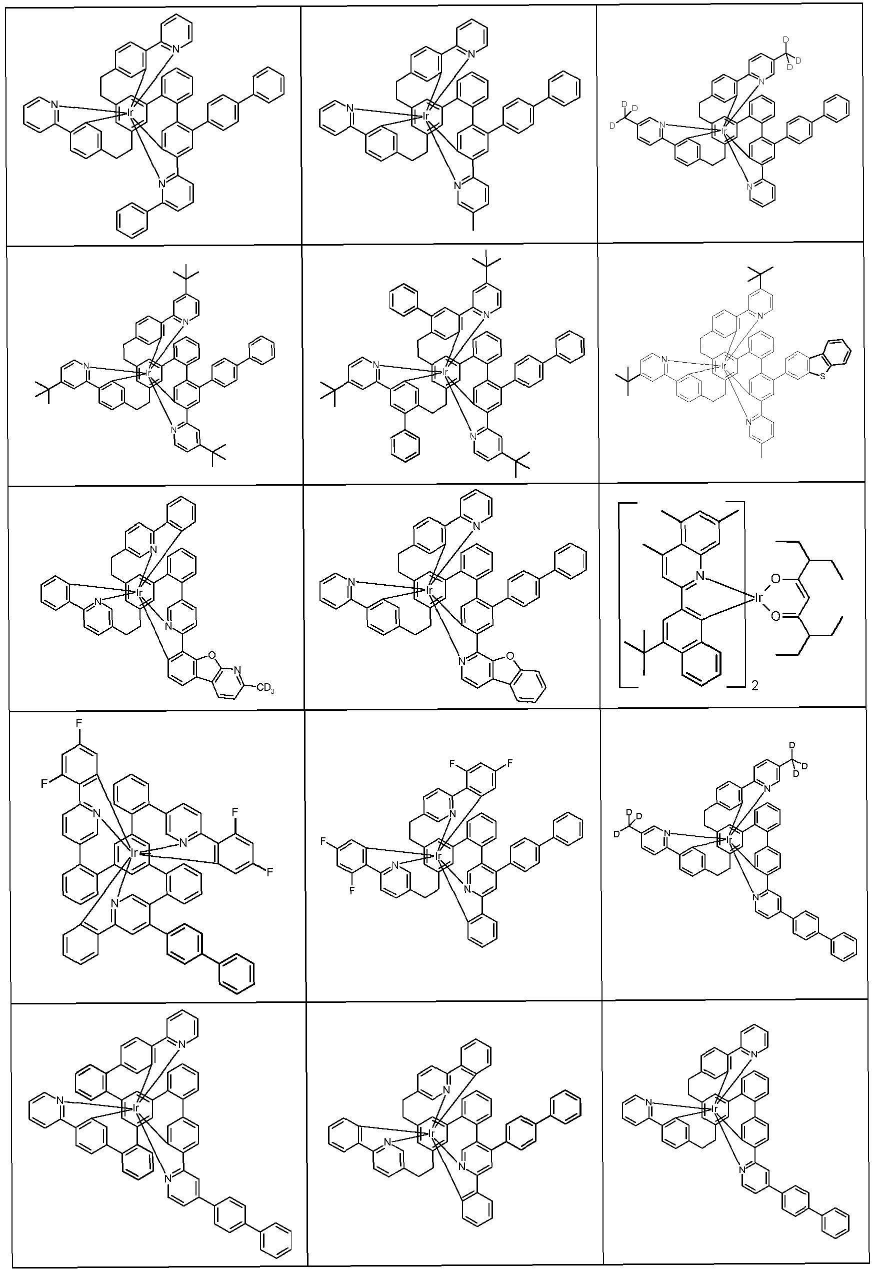

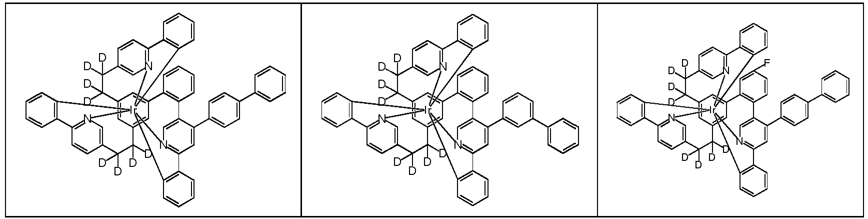

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer Verbindung gemäß Formel (I) oder deren oder deren zuvor dargelegten bevorzugten Ausführungsformen sind neu Ein weiterer Gegenstand der vorliegenden Erfindung ist daher eine Formulierungen, enthaltend mindestens ein Lösungsmittel und eine Verbindung gemäß Formel (I) oder deren zuvor dargelegten bevorzugten Ausführungsformen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich gegenüber dem Stand der Technik insbesondere durch einen niedrigen Brechungsindex (Refractive Index RI) aus. Weiterhin zeigen diese Verbindungen und die hieraus erhältlichen organischen Elektrolumineszenzvorrichtungen eine verbesserte Lebensdauer aus. Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer aus.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen insbesondere als Matrixmaterial, als elektronenleitende Materialien oder als lochleitende Materialien weisen eine hervorragende Effizienz auf. Hierbei bewirken erfindungsgemäße Verbindungen gemäß Formel (I) bzw. die zuvor und nachfolgend ausgeführten bevorzugten Ausführungsformen eine geringe Betriebsspannung bei Verwendung in elektronischen Vorrichtungen.
2. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen, insbesondere als Matrixmaterial, als elektronenleitende Materialien oder als lochleitende Materialien, weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.
3. Die erfindungsgemäßen Verbindungen gemäß Formel (I) bzw. die zuvor und nachfolgend ausgeführten bevorzugten Ausführungsformen zeigen eine sehr hohe Stabilität und Lebensdauer.
4. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen, insbesondere als Matrixmaterial, als elektronenleitende Materialien oder als lochleitende Materialien, weisen eine sehr niedrige Brechungsindices auf.
5. Mit Verbindungen gemäß Formel (I) bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.
6. Verbindungen gemäß Formel (I) bzw. die zuvor und nachfolgend ausgeführten bevorzugten Ausführungsformen weisen eine ausgezeichnete Glasfilmbildung auf.
7. Verbindungen gemäß Formel (I) bzw. die zuvor und nachfolgend ausgeführten bevorzugten Ausführungsformen bilden aus Lösungen sehr gute Filme.

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbinden die mehrere enantiomere, diastereomere oder tautomere Formen aufweisen können wird eine Form stellvertretend gezeigt.

### Literaturbekannte Synthone LS:

| | | | |
|---|---|---|---|
| LS1 | 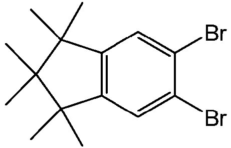 1541101-19-2 | LS2 | 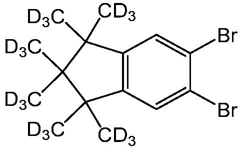 1541101-25-0 |
| LS3 | 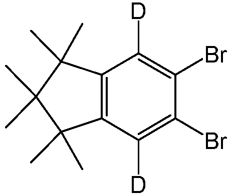 1541101-31-8 | | |

### A) Synthese von Synthonen S:

### Beispiel S1:

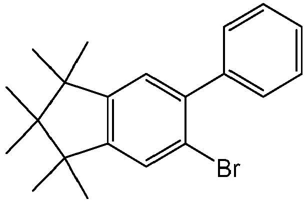

Durchführung analog C.-G-Dong et al, Synlett, 2009, No. 7, 1081, Table 2, Entry 3. Ansatz: 36.0 g (100 mmol) LS1, 12.0 g (100 mmol) Phenylboronsäure, 3% Pd(PPh₃)₄ + 9% Ph₃P, Toluol 80 °C, 16 h. Ausbeute: 28.7 g (80 mmol) 80 %; Reinheit: ca. 97 % ig n. ¹H-NMR.

Analog können folgende Verbindungen dargestellt werden:

| Bsp. | Edukte | Produkt | Ausbeute |
|---|---|---|---|
| S2 | 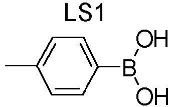 5720-05-8 | 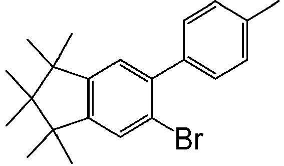 | 77 % |
| S3 | 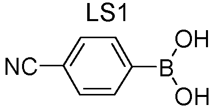 126747-14-6 | 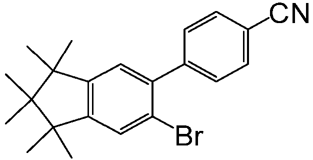 | 82 % |
| S4 | 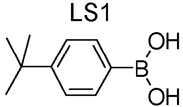 123324-71-0 | 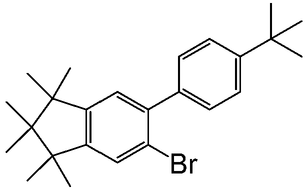 | 80 % |
| S5 | 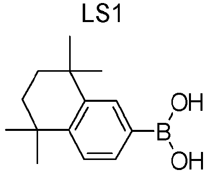 169126-63-0 | 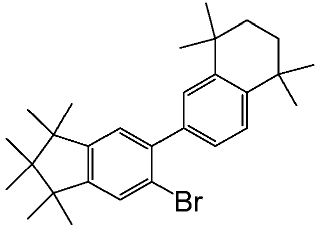 | 75 % |
| S6 | 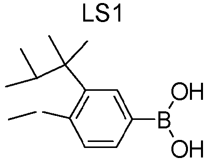 1562418-16-9 | 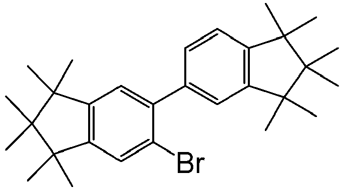 | 73 % |
| S7 | 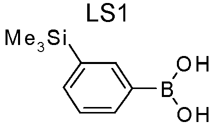 177171-16-3 | 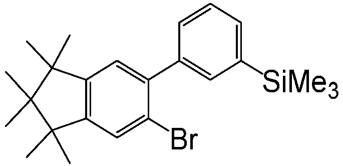 | 48 % |
| S8 | 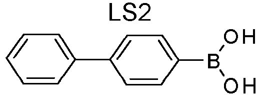 5122-94-1 | 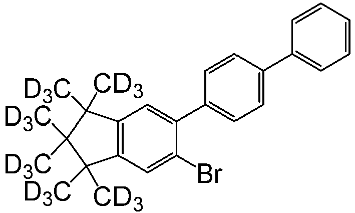 | 83 % |
| S9 | 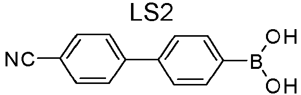 406482-73-3 | 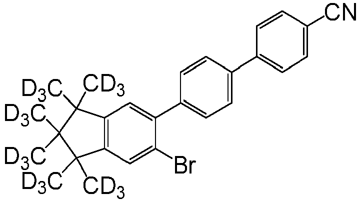 | 80 % |
| S10 | 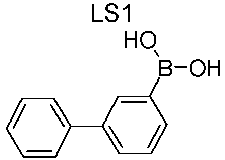 5122-95-2 | 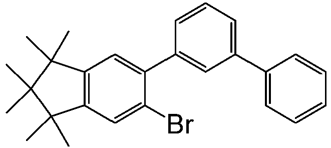 | 79 % |
| S11 | 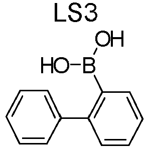 914675-52-8 | 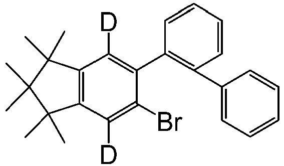 | 76 % |
| S12 | 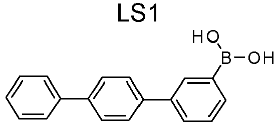 881911-81-5 | 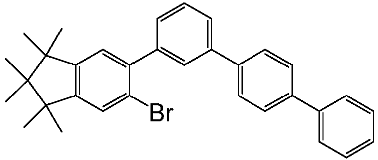 | 82 % |
| S13 | 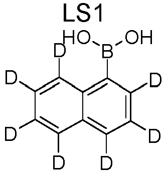 1280709-91-2 | 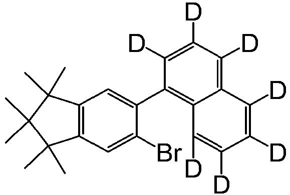 | 78 % |
| S14 | 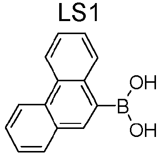 68572-87-2 | 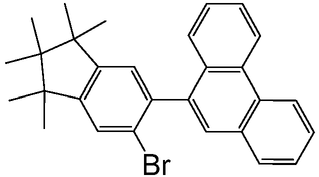 | 76 % |
| S15 | 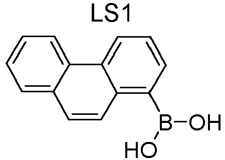 146746-63-6 | 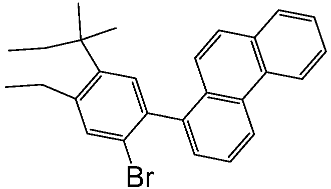 | 72 % |
| S16 | 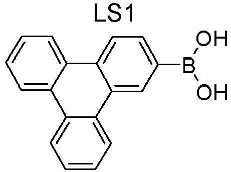 654664-63-8 | 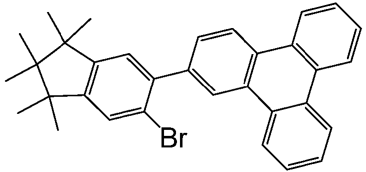 | 84 % |
| S17 | 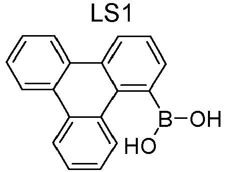 1430392-46-3 | 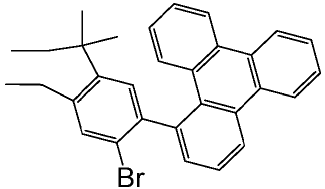 | 69 % |
| S18 | 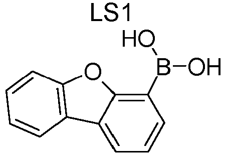 100124-06-9 | 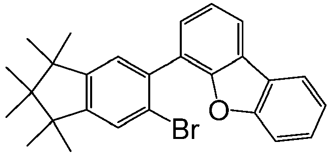 | 76 % |
| S19 | 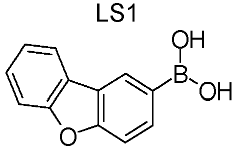 402936-15-6 | 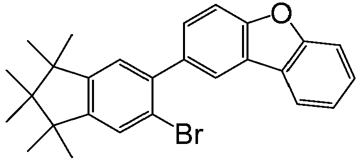 | 81 % |
| S20 | 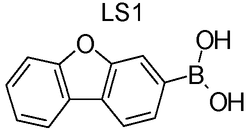 395087-89-5 | 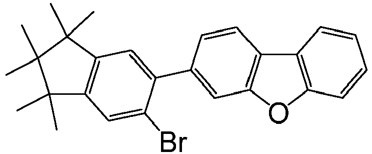 | 85 % |
| S21 | 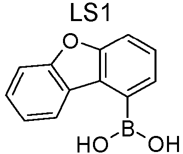 162607-19-4 | 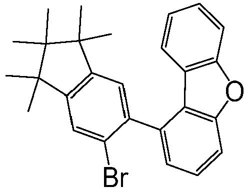 | 68 % |
| S22 | 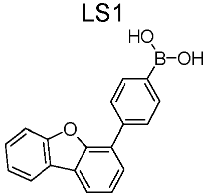 796071-96-0 | 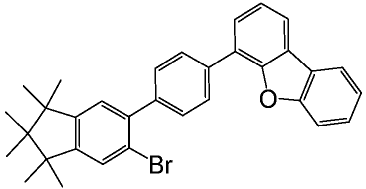 | 84 % |
| S23 | 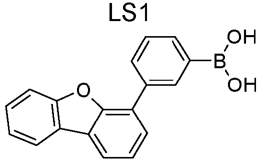 1271726-52-3 | 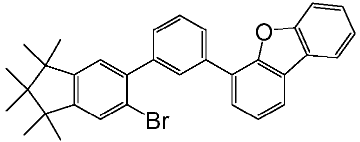 | 80 % |
| S24 | 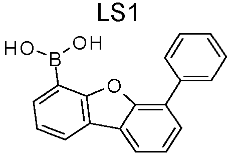 1010068-85-5 | 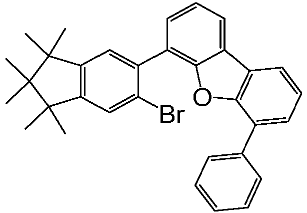 | 75 % |
| S25 | 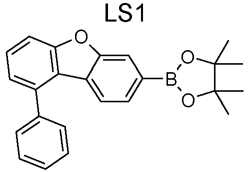 2360830-98-2 | 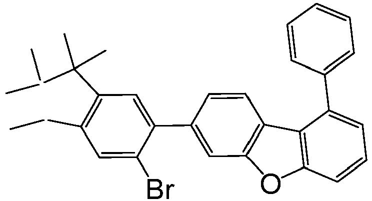 | 79 % |
| S26 | 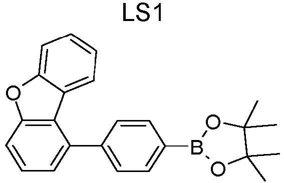 2186731-24-6 | 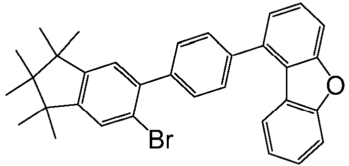 | 83 % |
| S27 | 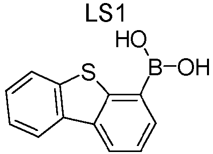 108847-20-7 | 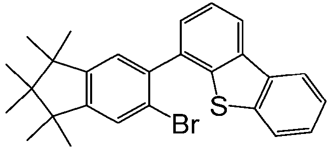 | 70 % |
| S28 | 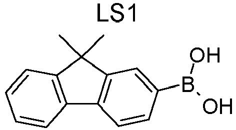 333432-28-3 | 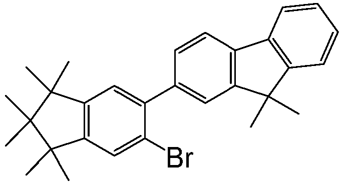 | 79 % |
| S29 | 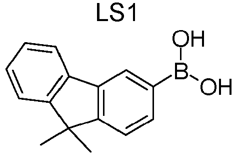 1251773-34-8 | 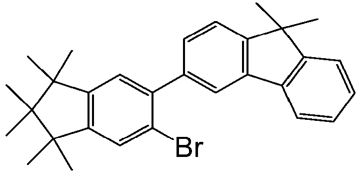 | 80 % |
| S30 | 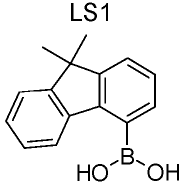 1246022-50-3 | 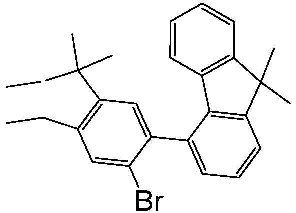 | 73 % |
| S31 | 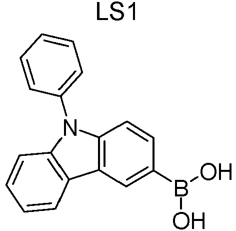 854952-58-2 | 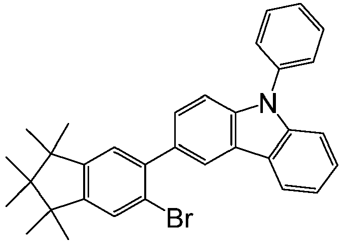 | 79 % |
| S32 | 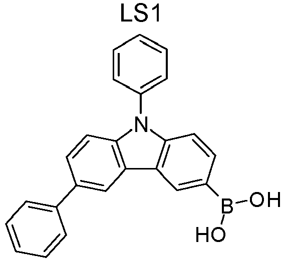 81359833-28-5 | 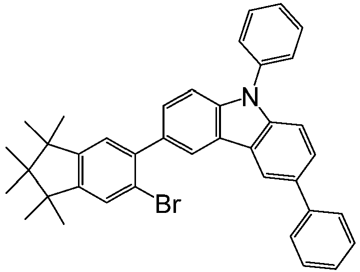 | 84 % |
| S33 | 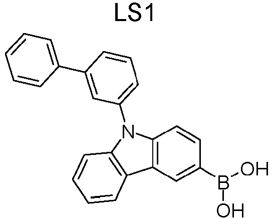 1416814-68-0 | 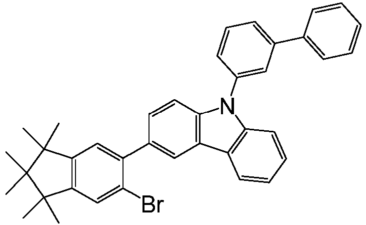 | 80 % |
| S34 | 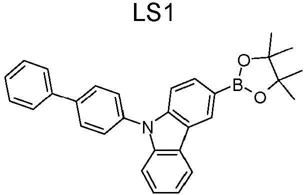 1391729-66-0 | 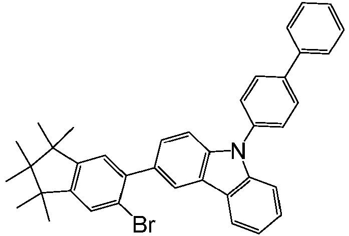 | 83 % |
| S35 | 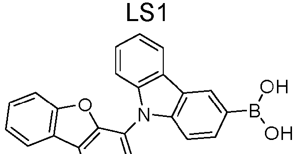 1547397-15-8 | 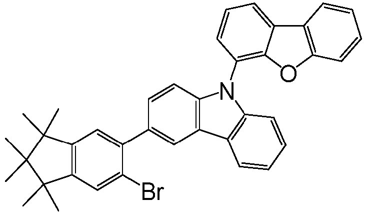 | 79 % |
| S36 | 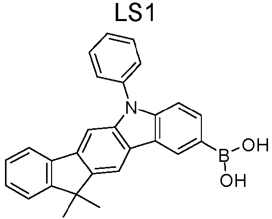 1373359-70-6 | 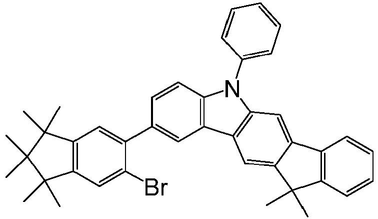 | 79 % |
| S37 | 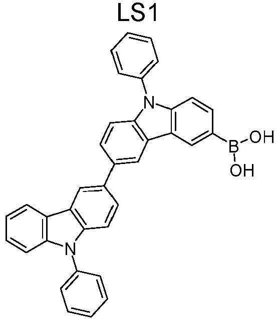 918137-86-7 | 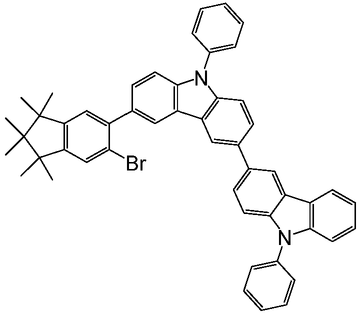 | 82 % |
| S38 | 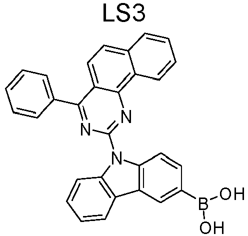 1813537-15-3 | 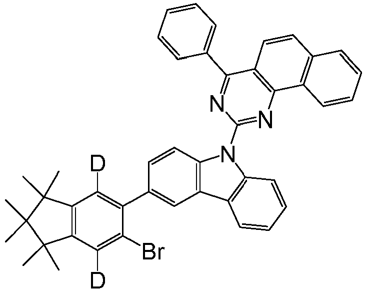 | 80 % |
| S39 | 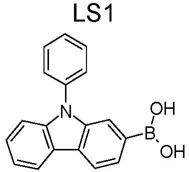 1001911-63-2 | 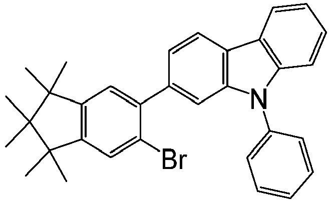 | 82 % |
| S40 | 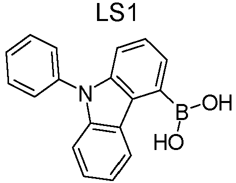 1370555-65-9 | 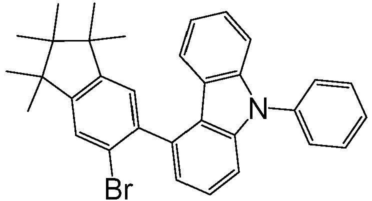 | 71% |
| S41 | 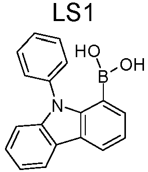 1333002-41-7 | 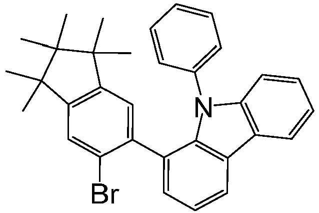 | 55 % |
| S42 | 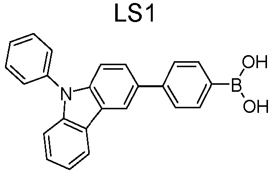 1240963-55-6 | 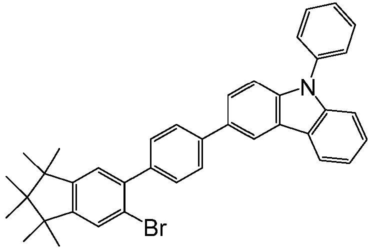 | 81% |
| S43 | 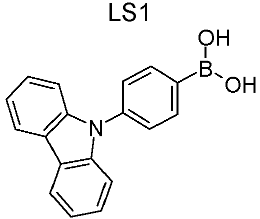 419536-33-7 | 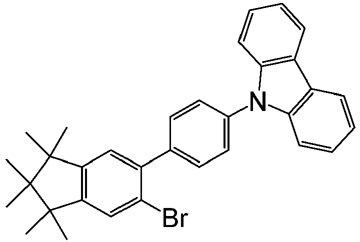 | 80 % |
| S44 | 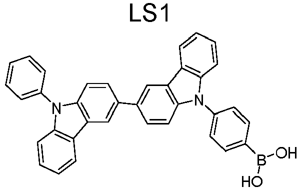 1398394-82-5 | 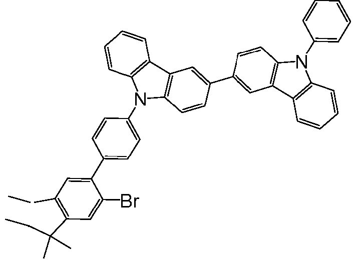 | 77 % |
| S45 | 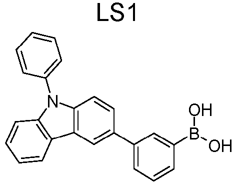 854952-60-6 | 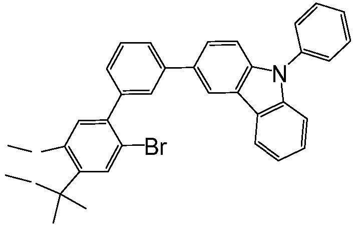 | 78 % |
| S46 | 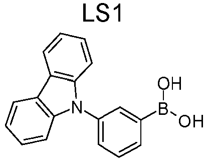 864377-33-3 | 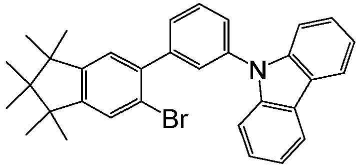 | 78 % |
| S47 | 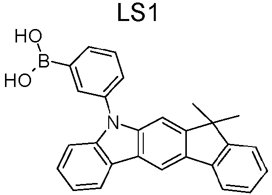 1369587-64-3 | 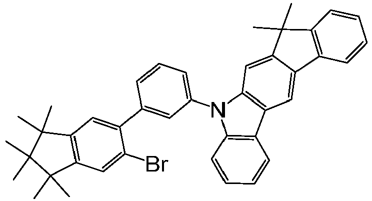 | 79 % |
| S48 | 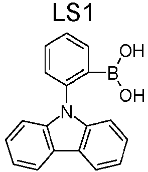 1189047-28-6 | 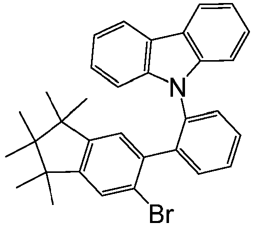 | 68 % |
| S49 | 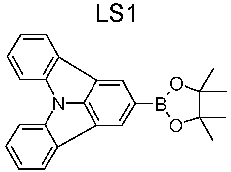 1369369-44-7 | 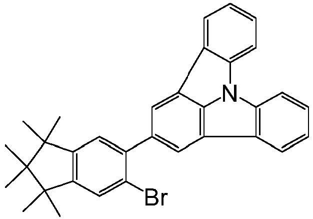 | 84 % |
| S50 | 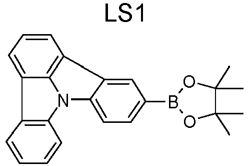 1454807-26-1 | 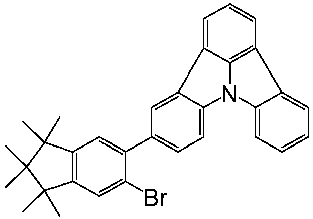 | 81 % |
| S51 | 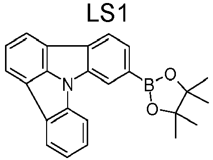 2068731-68-8 | 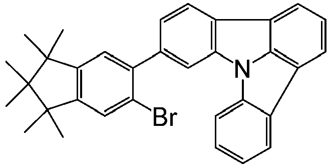 | 83 % |
| S52 | 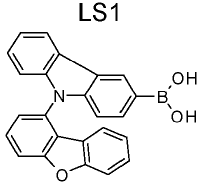 2413352-33-5 | 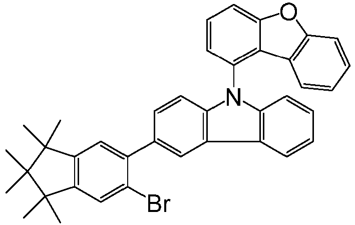 | 79 % |
| S53 | 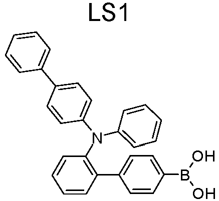 1776937-60-0 | 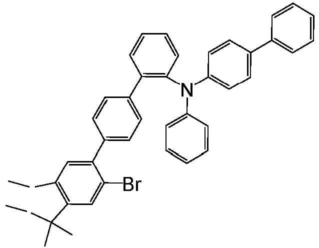 | 74 % |
| S54 | 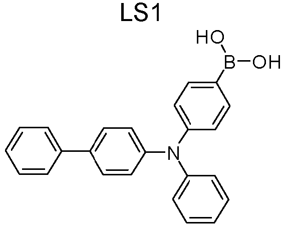 1084334-86-0 | 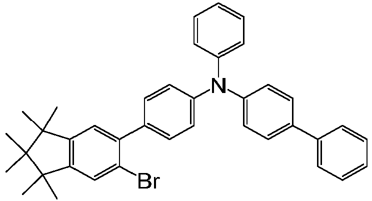 | 81% |
| S55 | 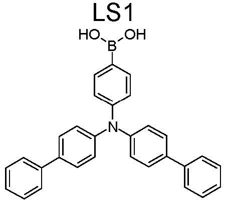 943836-24-6 | 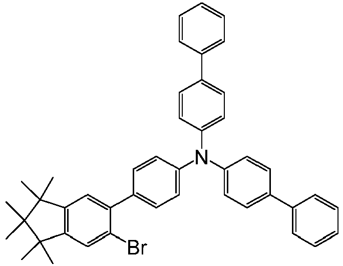 | 86 % |
| S56 | 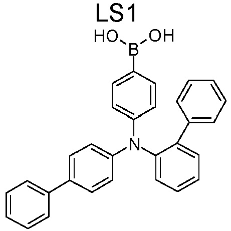 1608462-54-9 | 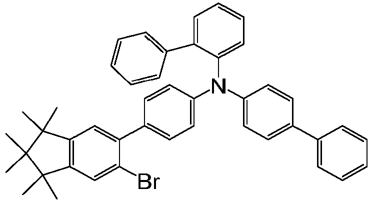 | 81% |
| S57 | 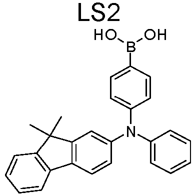 950986-07-9 | 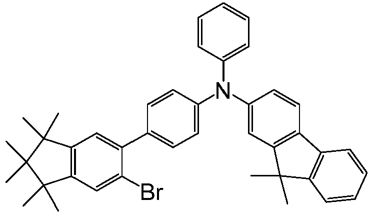 | 82 % |
| S58 | 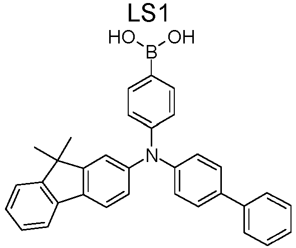 1265177-27-2 | 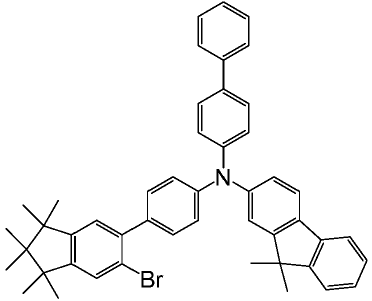 | 85 % |
| S59 | 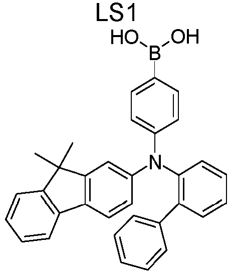 1959599-90-6 | 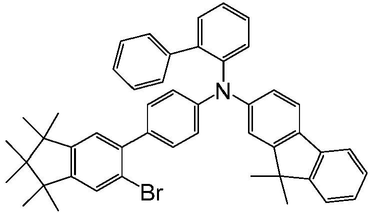 | 80 % |
| S60 | 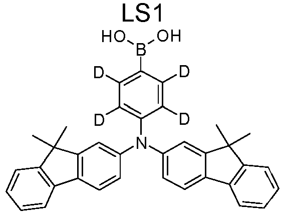 1648570-88-0 | 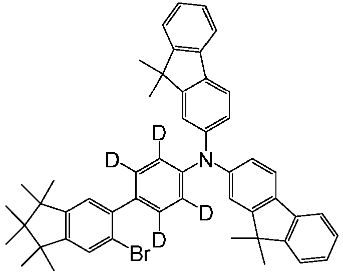 | 80 % |
| S61 | 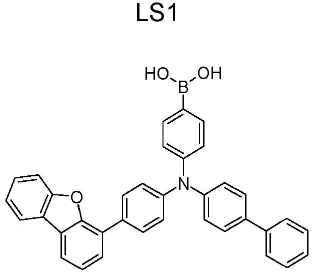 2126887-02-1 | 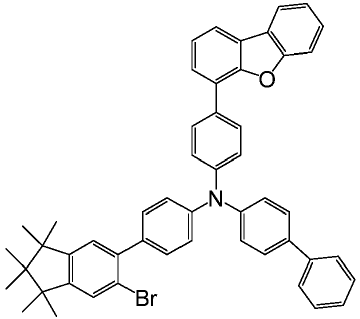 | 83 % |
| S62 | 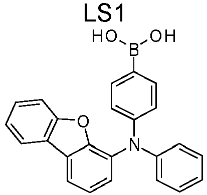 1428329-78-5 | 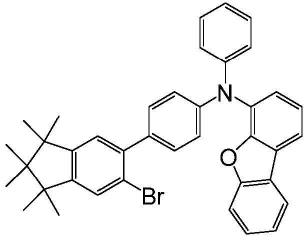 | 80 % |
| S63 | 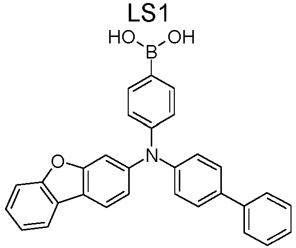 1960443-69-9 | 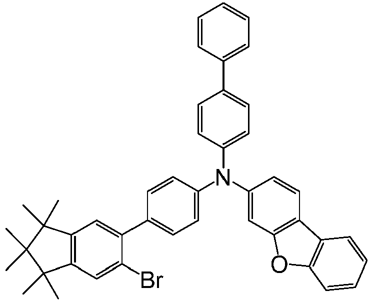 | 84 % |
| S64 | 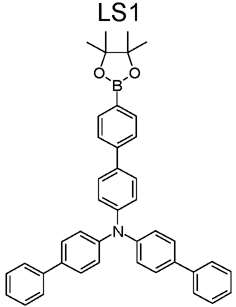 1421701-43-0 | 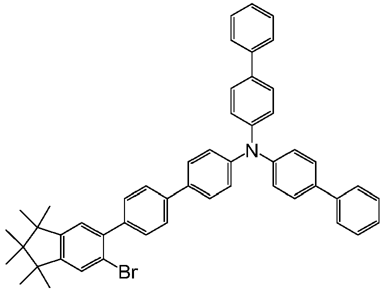 | 86 % |
| S65 | 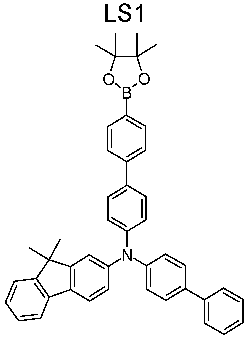 1825336-99-9 | 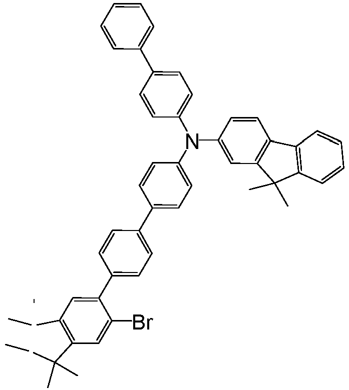 | 83 % |
| S66 | 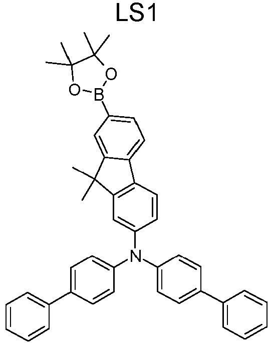 1620895-07-9 | 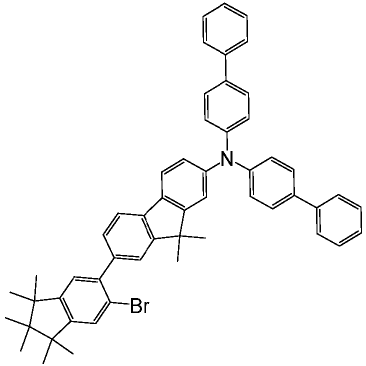 | 81 % |
| S67 | 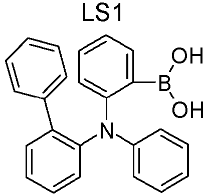 1776935-49-9 | 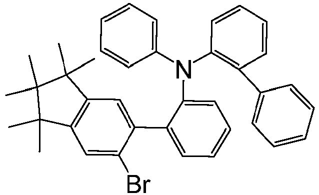 | 64 % |
| S68 | 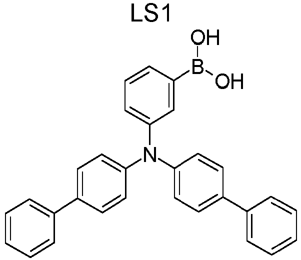 1776936-65-2 | 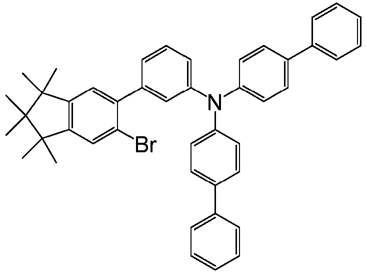 | 80 % |
| S69 | 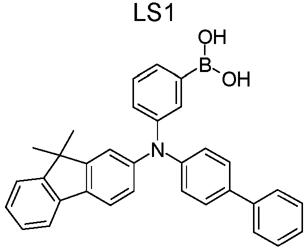 1610950-84-9 | 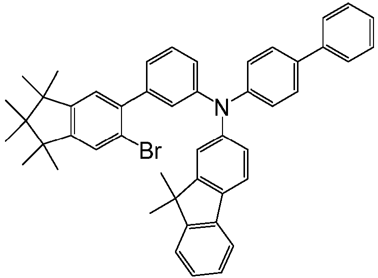 | 79 % |
| S70 | 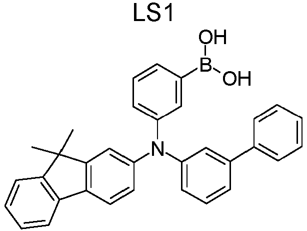 2334467-29-5 | 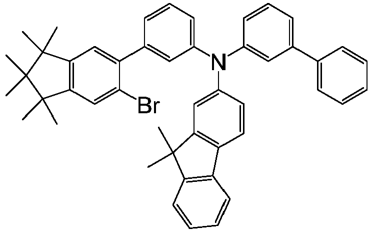 | 75 % |
| S71 | 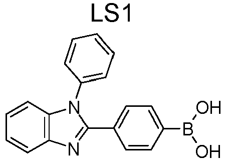 952514-79-3 | 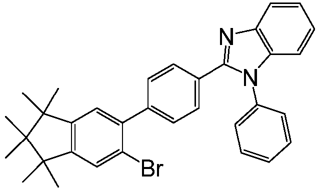 | 80 % |
| S72 | 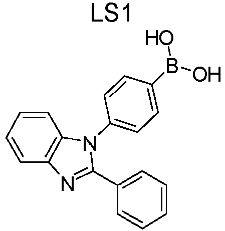 867044-33-5 | 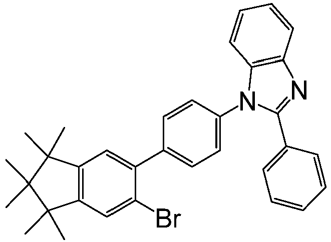 | 83 % |
| S73 | 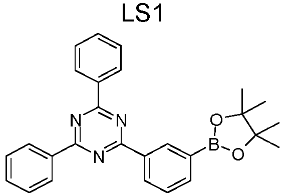 1269508-31-7 | 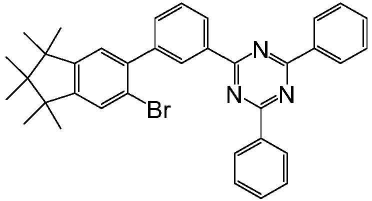 | 77 % |
| S74 | 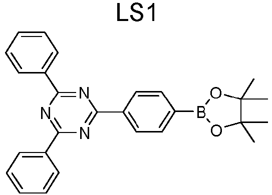 1219956-23-6 | 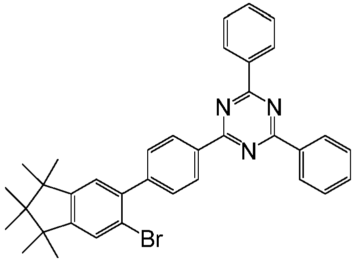 | 74 % |
| S75 | 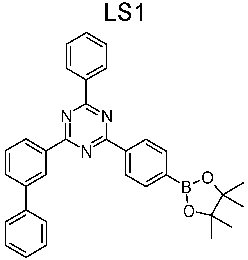 2168567-62-0 | 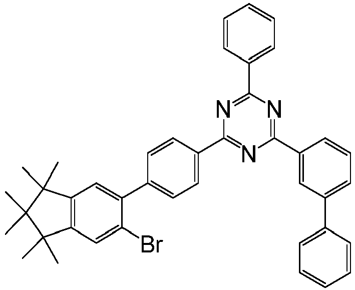 | 76 % |
| S76 | 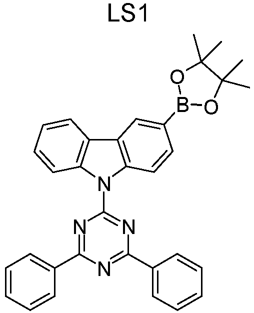 1361094-91-8 | 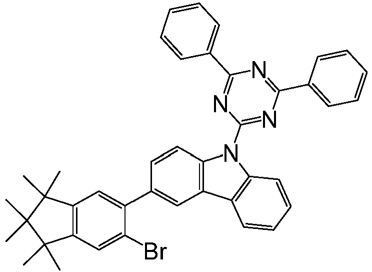 | 79 % |
| S77 | 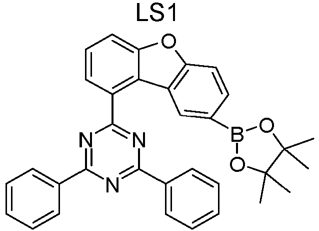 2138490-96-5 | 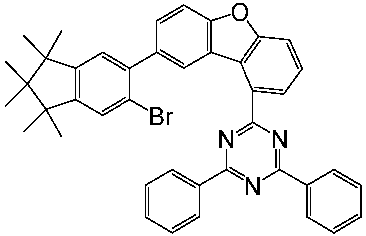 | 63 % |

### B) Synthese der erfindungsgemäßen Verbindungen

### Beispiel A1:

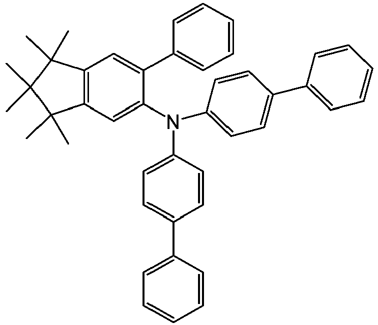

Eine Lösung von 35.7 g (100 mmol) S1 und 38.6 g (120 mmol) Bis-p-biphenylamin [102113-98-4] in 500 ml Toluol wird mit 4.0 ml (4.0 mmol) einer Tri-*tert*-butylphosphin-Lösung 1.0 M in Toluol, 449 mg (2 mmol) Palladiumacetat und 15.4 g Natrium-*tert*-butanolat (160 mmol) versetzt und 16 h unter Rückfluss erhitzt. Das Reaktionsgemisch wird auf Raumtemperatur abgekühlt, mit Toluol erweitert und über Celite-Bett filtriert. Das Filtrat wird im Vakuum eingeengt und der Rückstand aus Essigsäureethylester/n-Heptan kristallisiert. Das Rohprodukt wird durch Heißextraktionskristallisation (typische organische Lösungsmittel, bevorzugt Acetonitril bzw. Acetonitril/Dichlormethan Gemische 4:1 bis 1:4 vv) oder Chromatographie (Torrent-Säulenautomat der Fa. A. Semrau) und durch zweimalige Zonensublimation im Vakuum (p ~ 10⁻⁵ mbar, T ~ 280 °C) gereinigt. Ausbeute: 44.8 g (75 mmol) 75 %. Reinheit n. HPLC >99.9 %.

Analog können folgende Verbindungen dargestellt werden:

| Bsp. | Edukte | Produkt | Ausbeute |
|---|---|---|---|
| A2 | 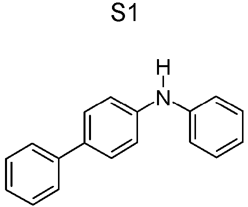 32228-99-2 | 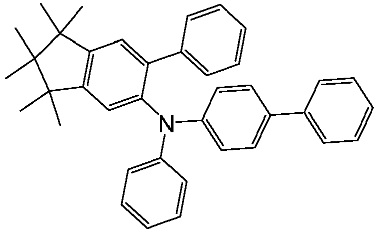 | 70 % |
| A3 | 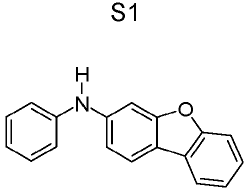 406488-21-9 | 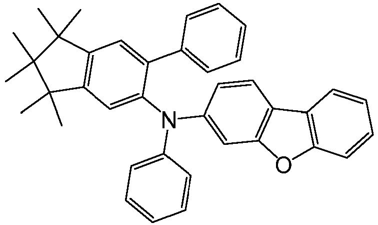 | 72 % |
| A4 | 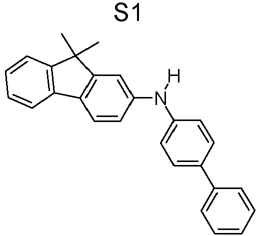 897671-69-1 | 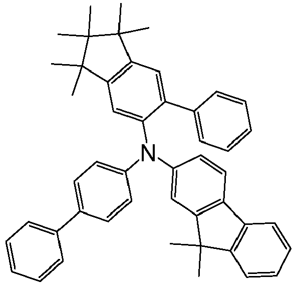 | 69 % |
| A5 | 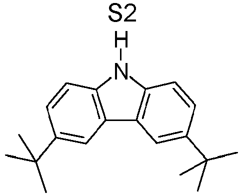 37500-95-1 | 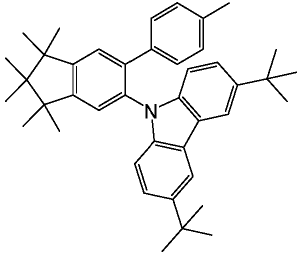 | 63 % |
| A6 | 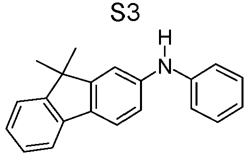 355832-04-1 | 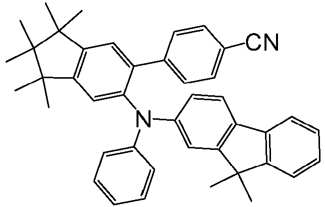 | 76 % |
| A7 | 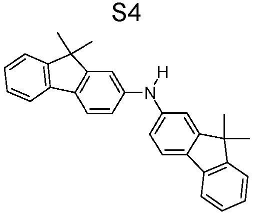 500717-23-7 | 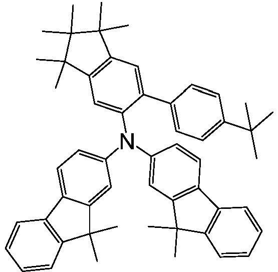 | 74 % |
| A8 | 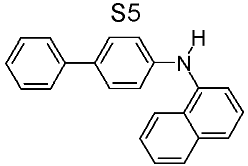 446242-37-1 | 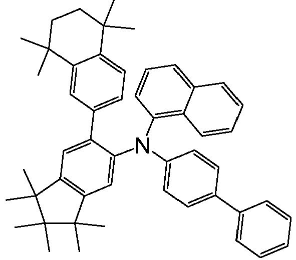 | 75 % |
| A9 | 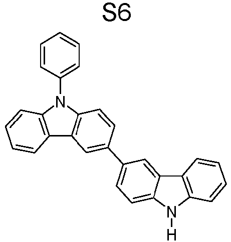 1060735-14-9 | 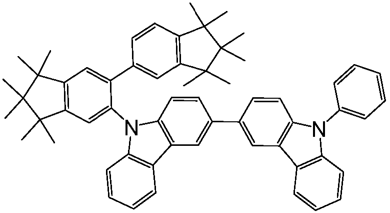 | 73 % |
| A10 | 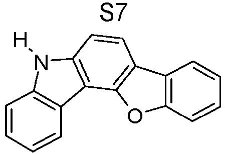 1199616-66-4 | 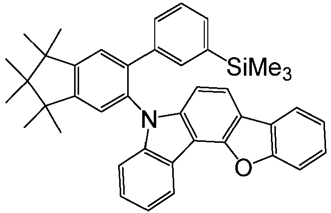 | 37 % |
| A11 | 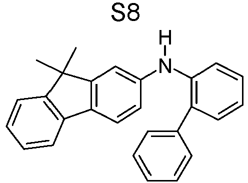 1198395-24-2 | 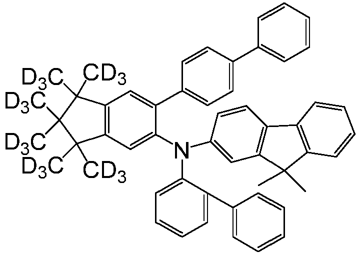 | 70 % |
| A12 | 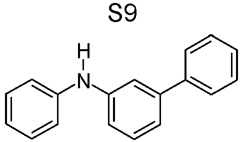 198275-79-5 | 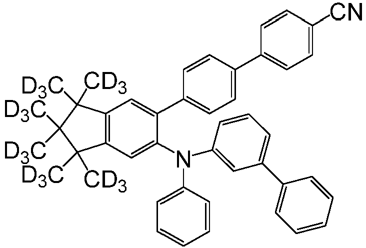 | 66 % |
| A13 | 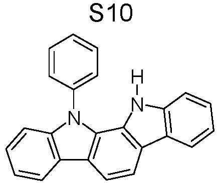 1024598-06-8 | 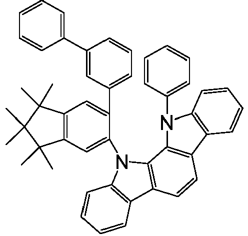 | 69 % |
| A14 | 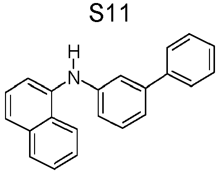 59994-77-3 | 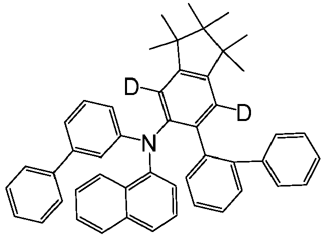 | 70 % |
| A15 | 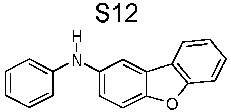 861317-95-5 | 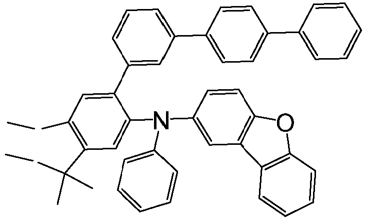 | 74 % |
| A16 | 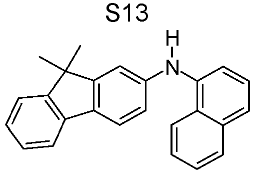 672289-02-0 | 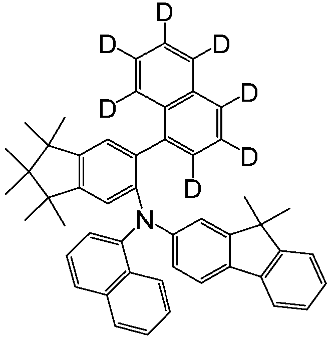 | 73 % |
| A17 | 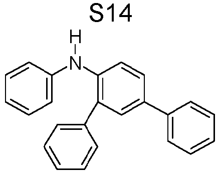 1417334-01-0 | 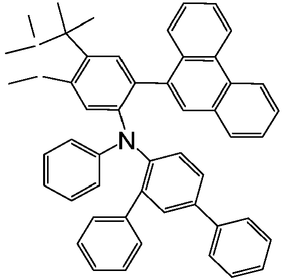 | 70 % |
| A18 | 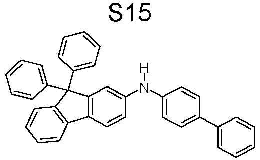 1268520-04-2 | 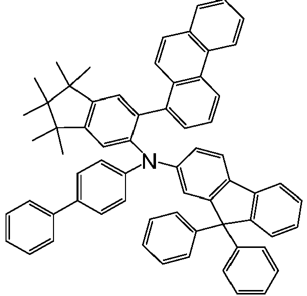 | 68 % |
| A19 | 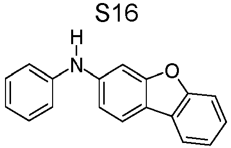 406488-21-9 | 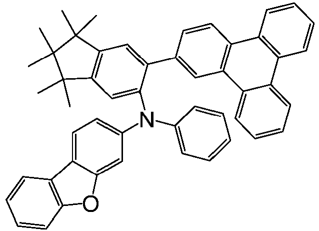 | 78 % |
| A20 | 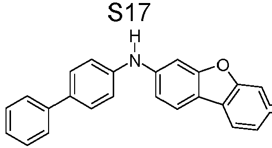 1290039-85-8 | 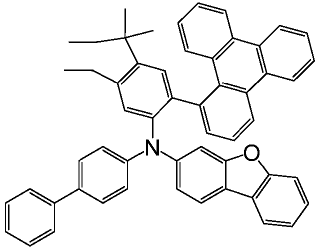 | 71 % |
| A21 | 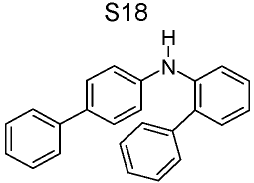 1372775-52-4 | 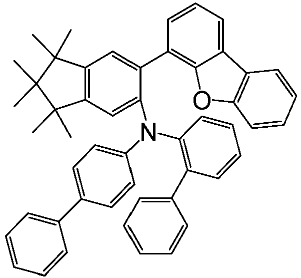 | 69 % |
| A22 | 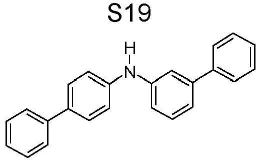 570391-47-8 | 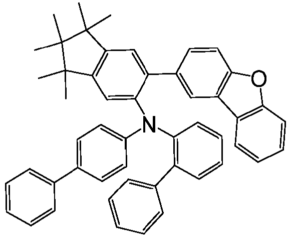 | 74 % |
| A23 | 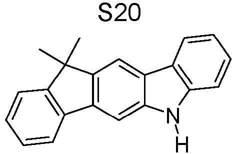 1260228-95-2 | 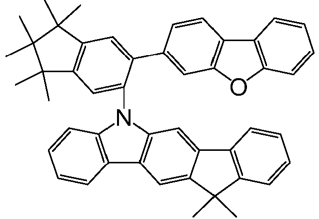 | 74 % |
| A24 | 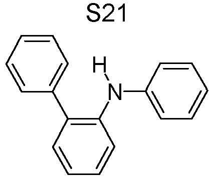 35887-50-4 | 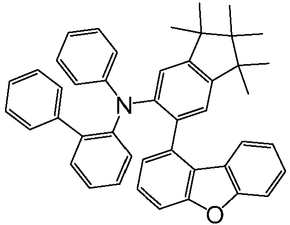 | 69 % |
| A25 | 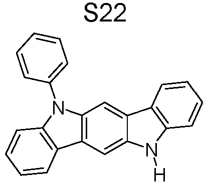 1316311-27-9 | 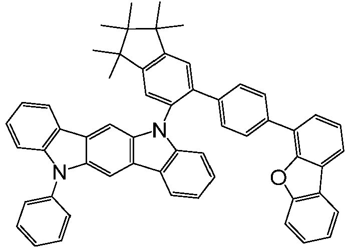 | 73 % |
| A26 | 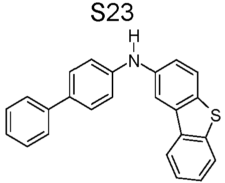 1300028-93-6 | 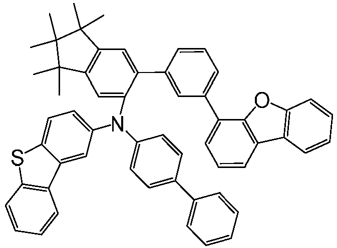 | 67 % |
| A27 | 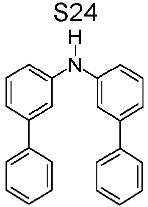 169224-65-1 | 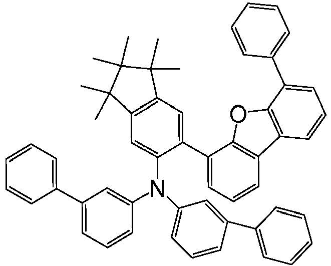 | 69 % |
| A28 | 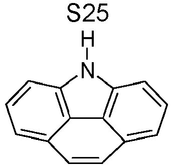 203-65-6 | 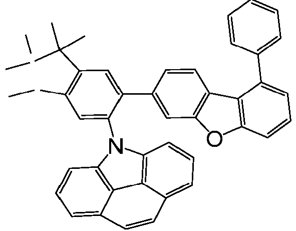 | 76 % |
| A29 | 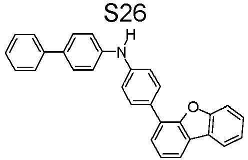 955959-89-4 | 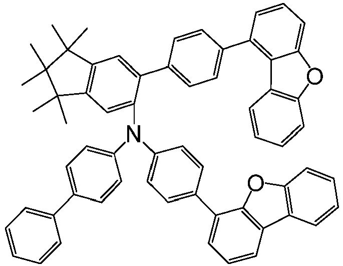 | 70 % |
| A30 | 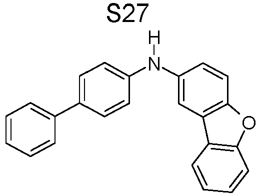 1300028-94-7 | 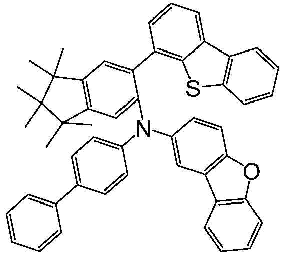 | 67 % |
| A31 | 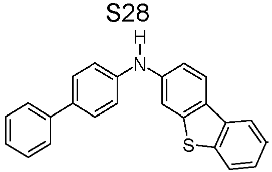 1290039-87-0 | 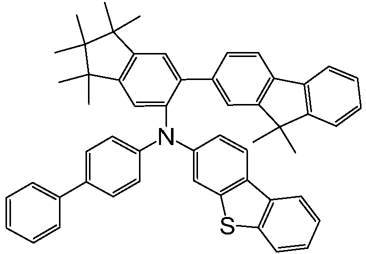 | 63 % |
| A32 | 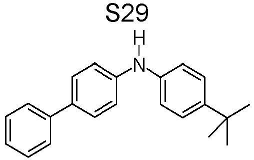 950917-84-7 | 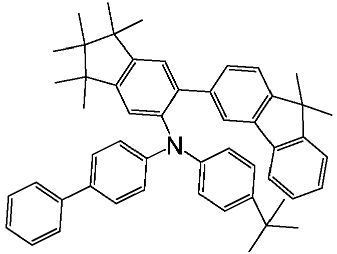 | 69 % |
| A33 | 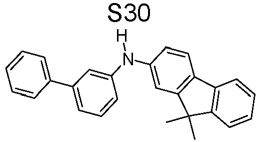 1372778-66-9 | 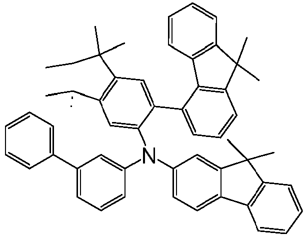 | 68 % |
| A34 | 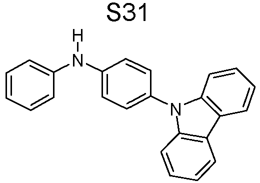 858641-06-2 | 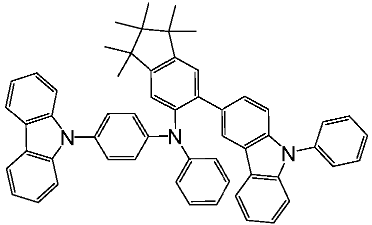 | 56 % |
| A35 | 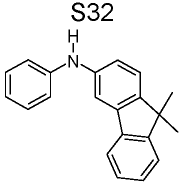 850181-65-6 | 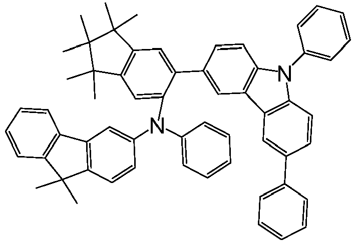 | 72 % |
| A36 | 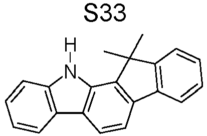 1329054-41-2 | 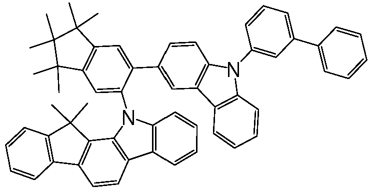 | 76 % |
| A37 | 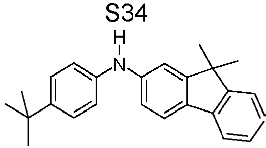 944418-46-6 | 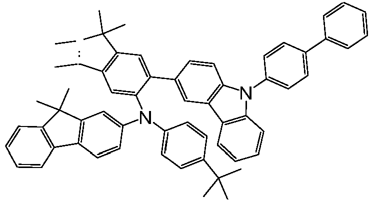 | 75 % |
| A38 | 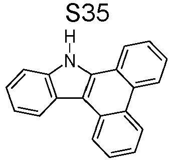 201-67-2 | 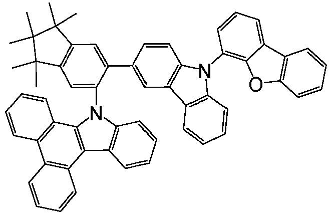 | 77 % |
| A39 | 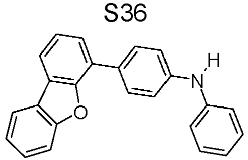 955959-87-2 | 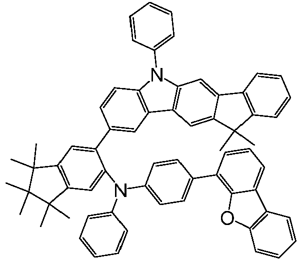 | 69 % |
| A40 | 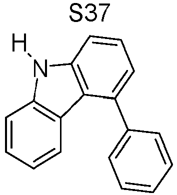 1201561-34-3 | 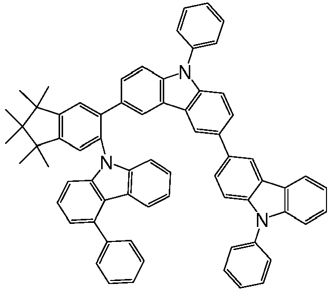 | 80 % |
| A41 | 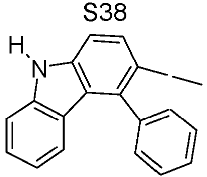 1623813-70-6 | 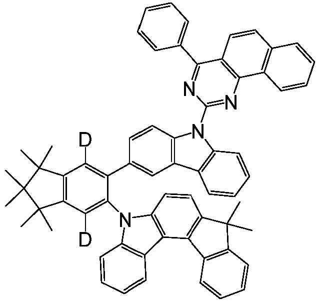 | 78 % |
| A42 | 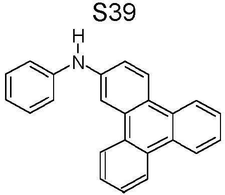 1203922-52-4 | 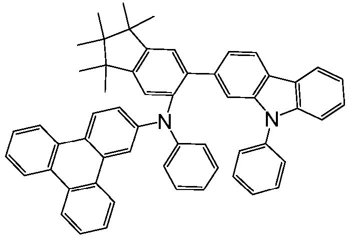 | 74 % |
| A43 | 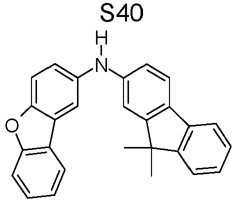 1359833-89-8 | 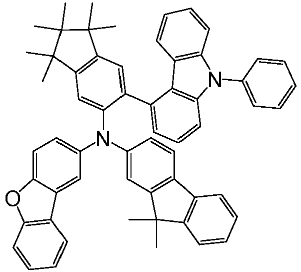 | 70 % |
| A44 | 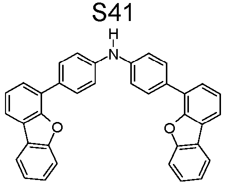 955959-91-8 | 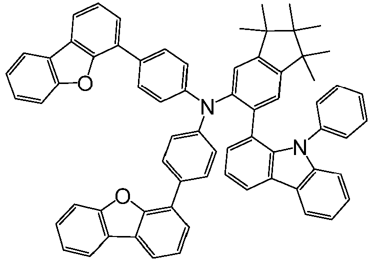 | 68 % |
| A45 | 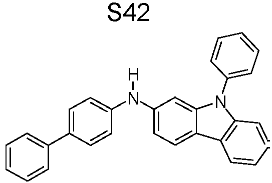 1427316-58-2 | 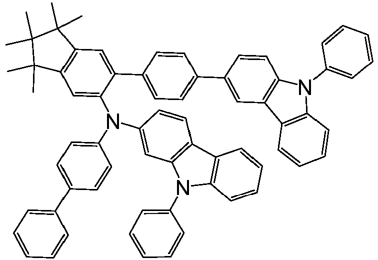 | 74 % |
| A46 | 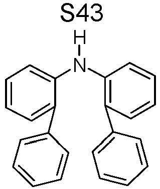 91923-32-9 | 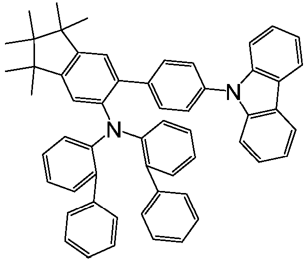 | 67 % |
| A47 | 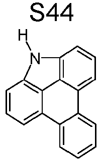 109606-75-9 | 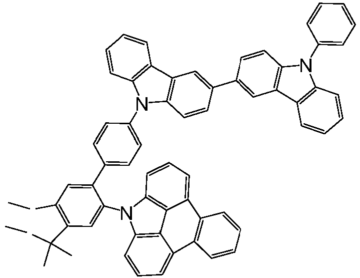 | 78 % |
| A48 | 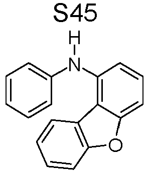 1325195-27-4 | 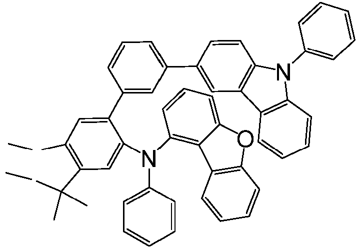 | 67 % |
| A49 | 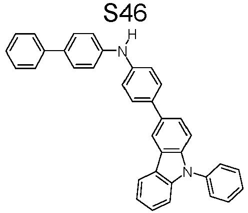 1160294-96-1 | 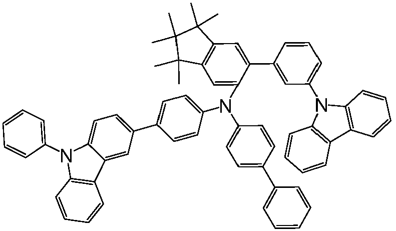 | 70 % |
| A50 | 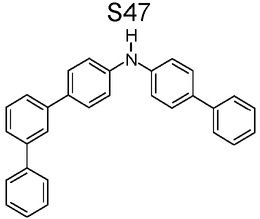 1222633-96-6 | 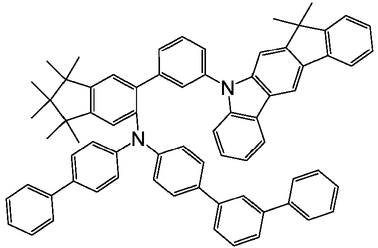 | 75 % |
| A51 | 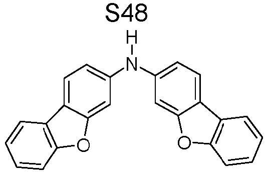 1705595-86-3 | 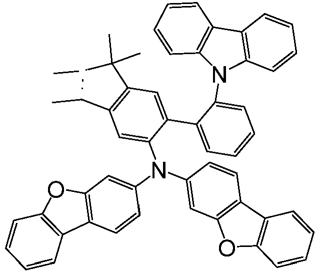 | 72 % |
| A52 | 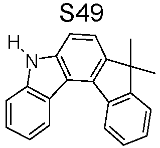 1623813-70-6 | 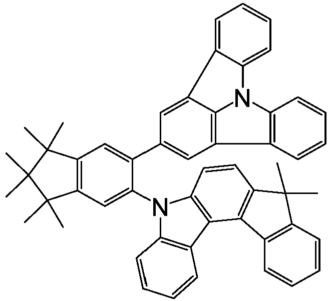 | 76 % |
| A53 | 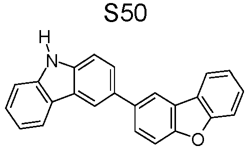 953805-18-0 | 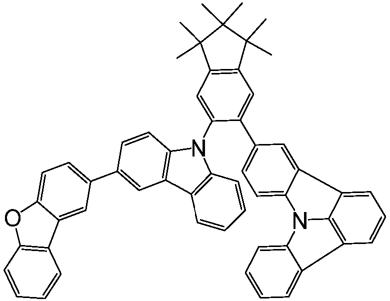 | 75 % |
| A54 | 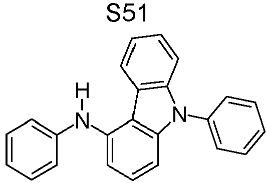 1607445-46-4 | 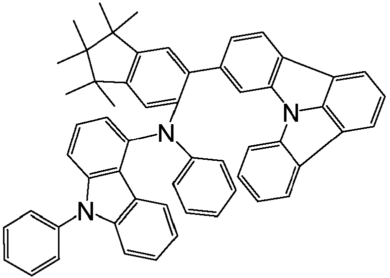 | 68 % |
| A55 | 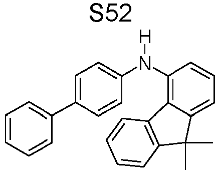 1421789-16-3 | 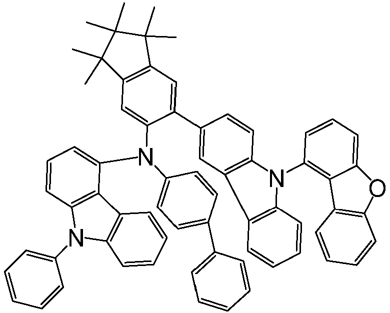 | 66 % |
| A56 | 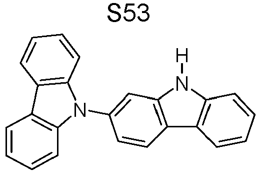 1226810-15-6 | 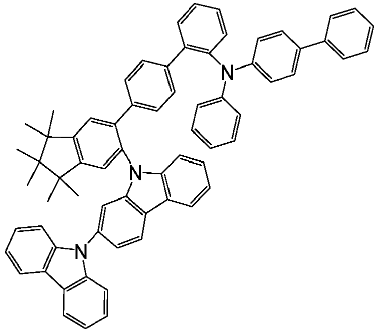 | 68 % |
| A57 | 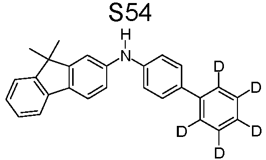 1359833-90-1 | 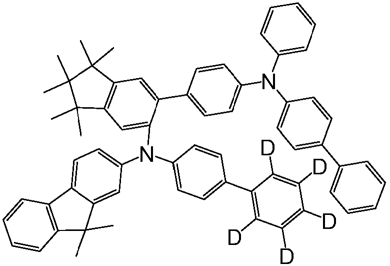 | 70 % |
| A58 | 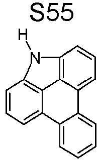 109606-75-9 | 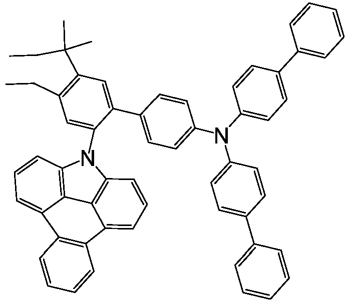 | 74 % |
| A59 | 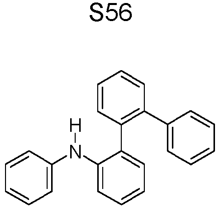 1776936-11-8 | 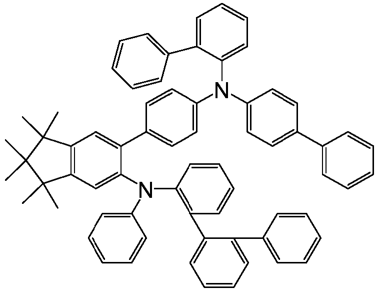 | 68 % |
| A60 | 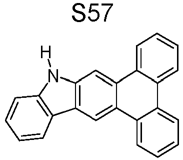 1374446-05-5 | 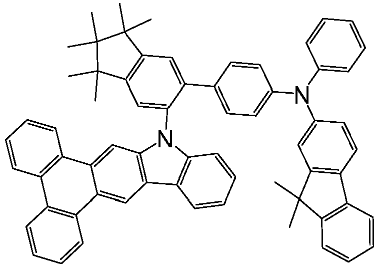 | 71 % |
| A61 | 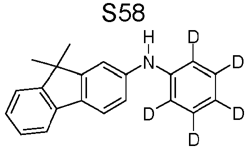 1359833-31-0 | 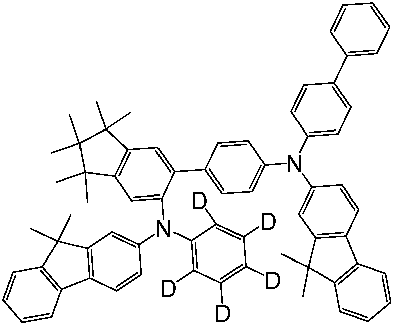 | 69 % |
| A62 | 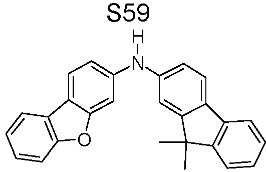 1427556-50-0 | 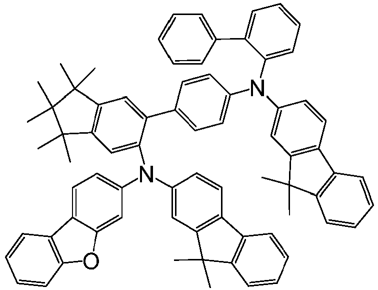 | 69 % |
| A63 | 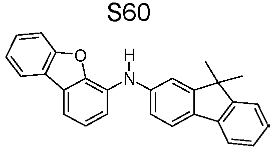 1426933-82-5 | 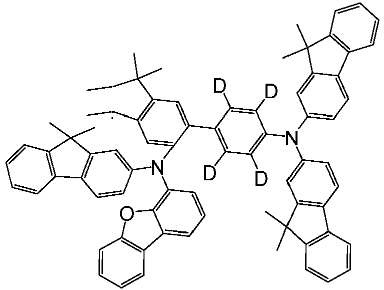 | 70 % |
| A64 | 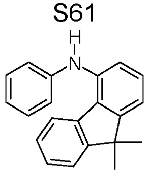 1776969-70-0 | 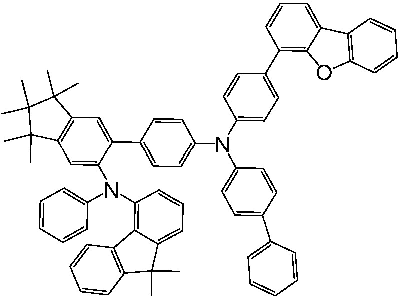 | 72 % |
| A65 | 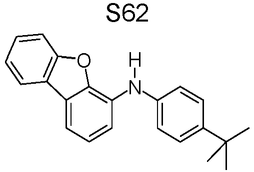 1438401-13-8 | 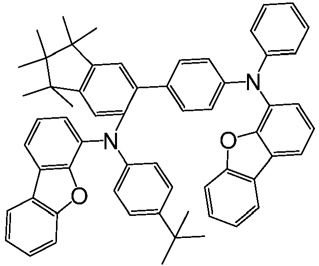 | 66 % |
| A66 | 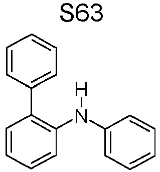 35887-50-4 | 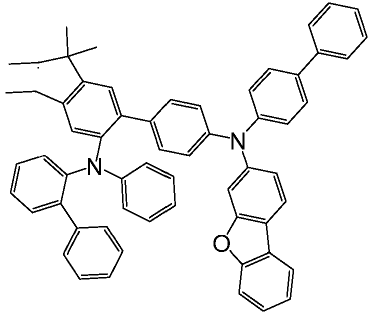 | 72 % |
| A67 | 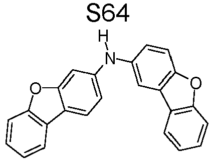 1923735-83-4 | 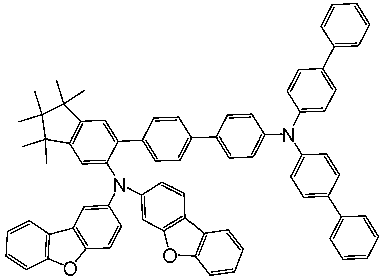 | 69 % |
| A68 | 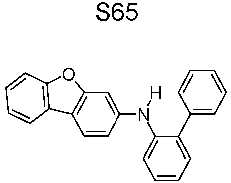 1427556-44-2 | 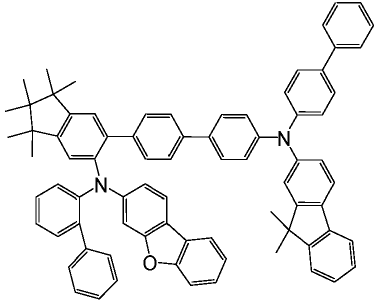 | 70 % |
| A69 | 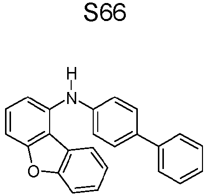 1776057-10-3 | 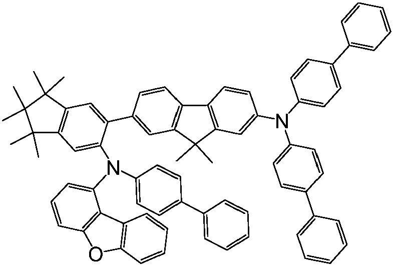 | 68 % |
| A70 | 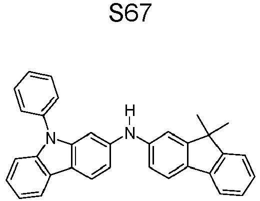 1456702-57-0 | 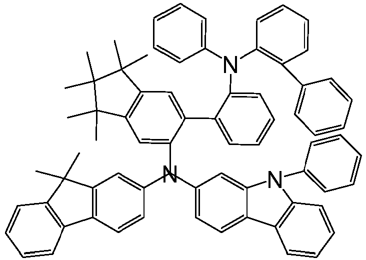 | 67 & |
| A71 | 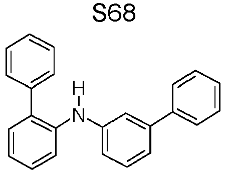 1258515-01-3 | 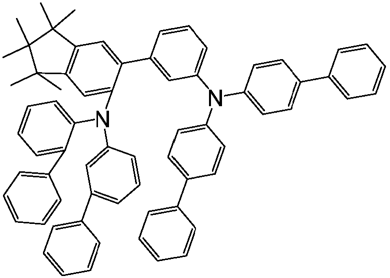 | 68 % |
| A72 | 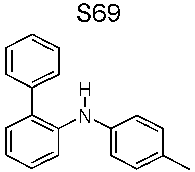 118987-69-2 | 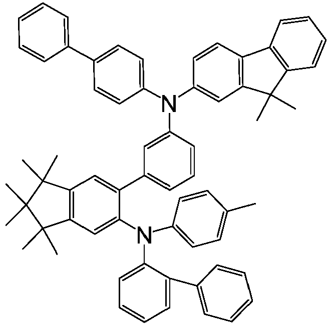 | 70 % |
| A73 | 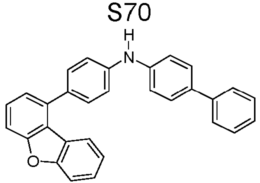 1922919-50-3 | 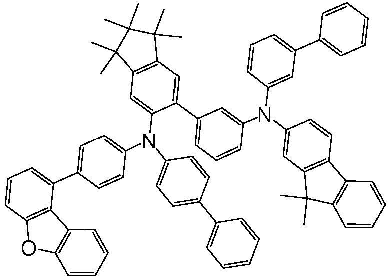 | 70 % |
| A74 | 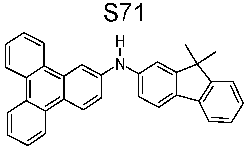 1430393-63-7 | 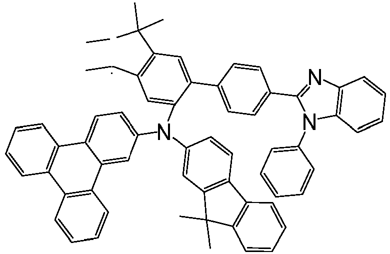 | 74 % |
| A75 | 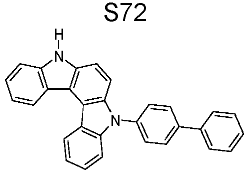 2071630-78-7 | 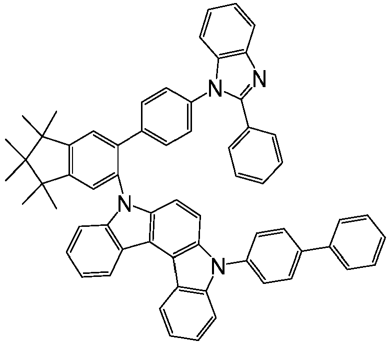 | 75 % |
| A76 | 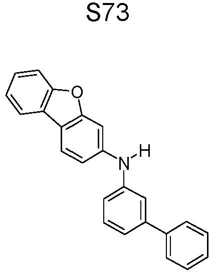 1427556-45-3 | 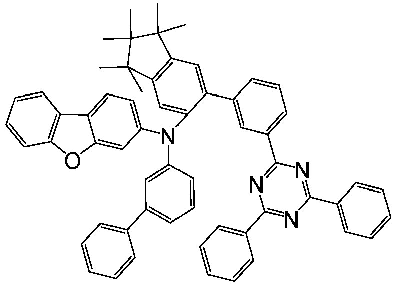 | 70 % |
| A77 | 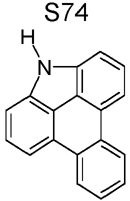 109606-75-9 | 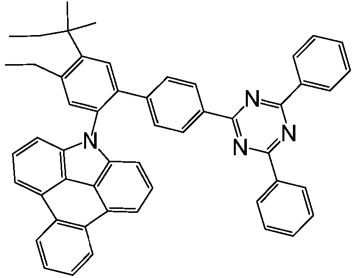 | 78 % |
| A78 | 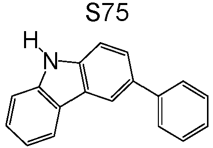 103012-26-6 | 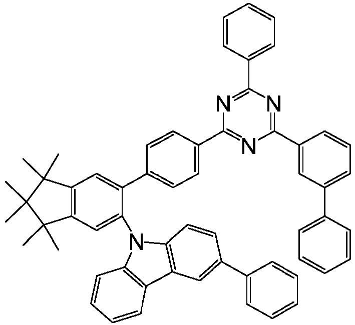 | 75 % |
| A79 | 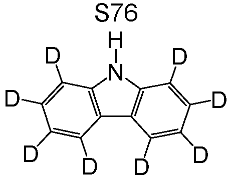 | 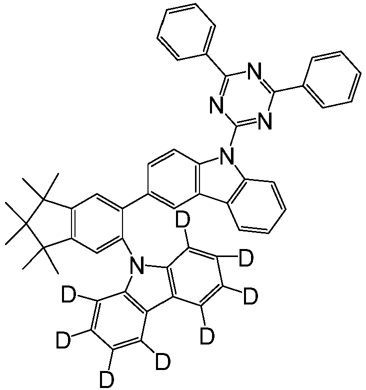 | 68 % |
| A80 | 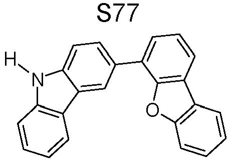 1346669-46-2 | 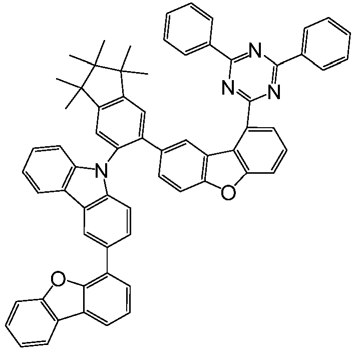 | 65 % |

### Beispiel A100:

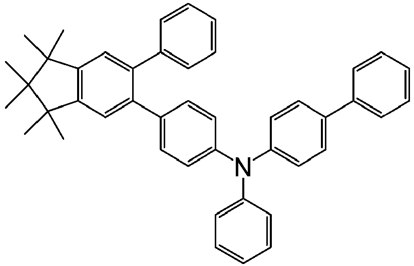

Durchführung analog J. L. Bolliger et al., Chem. Eur. J. 2010, 16, 4075, Tabelle 1, Entry 28. Ansatz: 36.0 g (100 mmol) LS1, 40.2 g (110 mmol) **B-**[4-([1,1'-Biphenyl]-4-ylphenylamino)phenyl]boronsäure [1084334-86-0]. Die Reinigung des Rohprodukts erfolgt durch Chromatographie und wiederholte Heißextraktionskristallisation (übliche org. Lösungsmittel bzw. deren Kombinationen, bevorzugt Acetonitril-DCM, 1:3 bis 3:1 vv) und fraktionierte Sublimation bzw. Tempern im Hochvakuum. Ausbeute: 45.6 g (76 mmol) 76 %; Reinheit: ca. 99.9 % ig n. HPLC.

Analog können folgende Verbindungen dargestellt werden:

| Bsp. | Edukte | Produkt | Ausbeute |
|---|---|---|---|
| A101 | 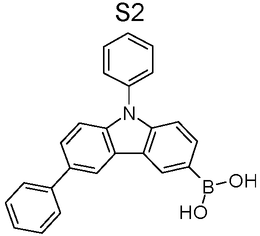 81359833-28-5 | 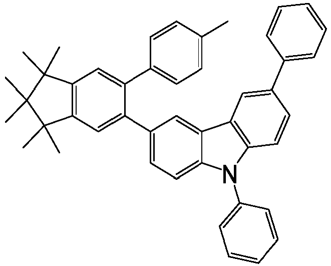 | 75 % |
| A102 | 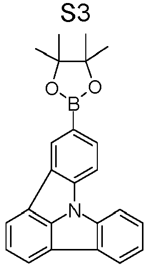 1454807-26-1 | 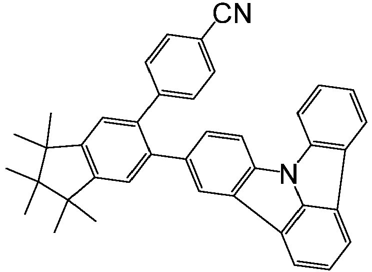 | 81 % |
| A103 | 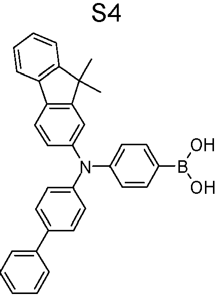 1265177-27-2 | 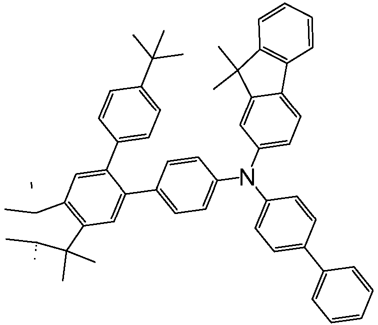 | 79 % |
| A104 | 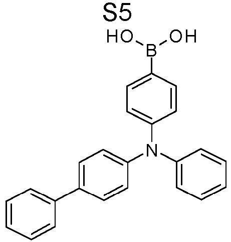 1084334-86-0 | 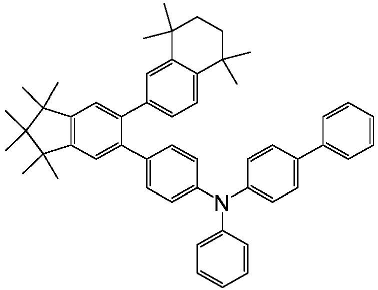 | 77 % |
| A105 | 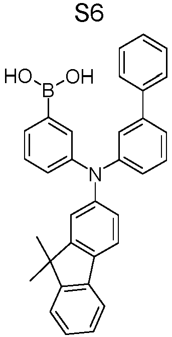 2334467-29-5 | 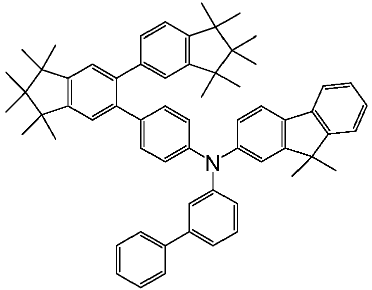 | 73 % |
| A106 | 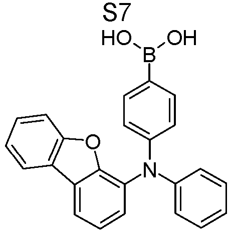 1428329-78-5 | 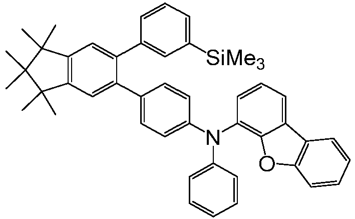 | 55 % |
| A107 | 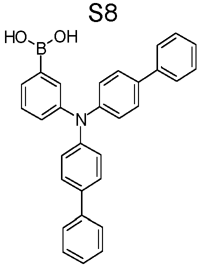 1776936-65-2 | 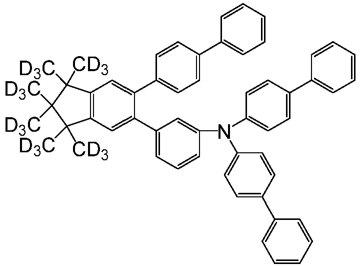 | 76 % |
| A108 | 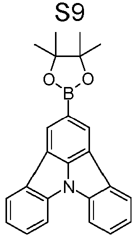 1369369-44-7 | 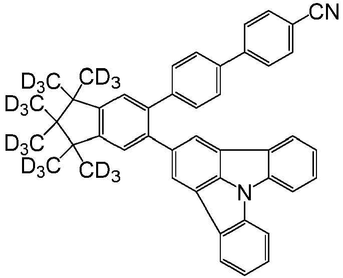 | 79 % |
| A109 | 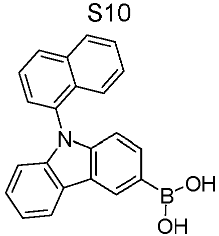 1133057-97-2 | 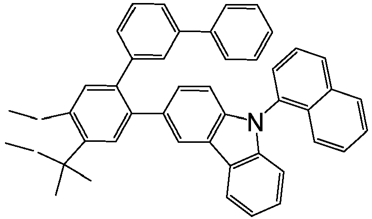 | 69 % |
| A110 | 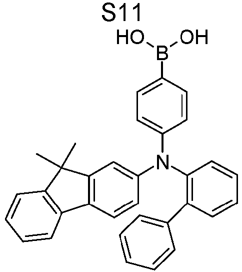 1959599-90-6 | 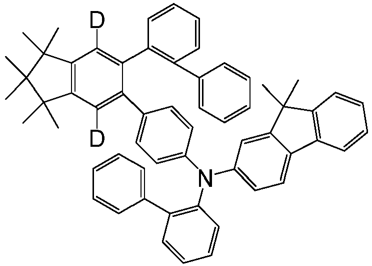 | 67 % |
| A111 | 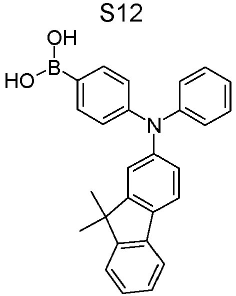 950986-07-9 | 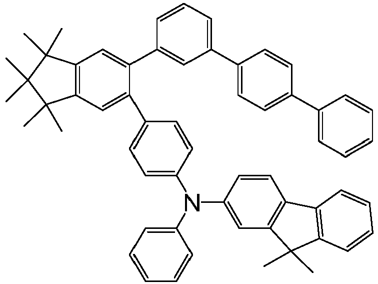 | 69 % |
| A112 | 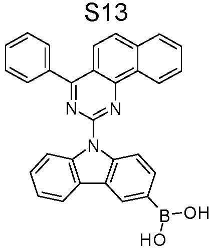 1813537-15-3 | 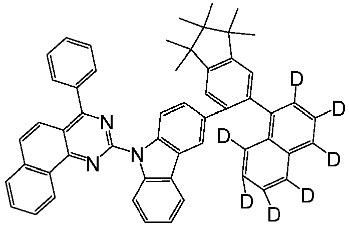 | 64 % |
| A113 | 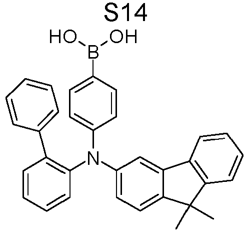 2609773-06-8 | 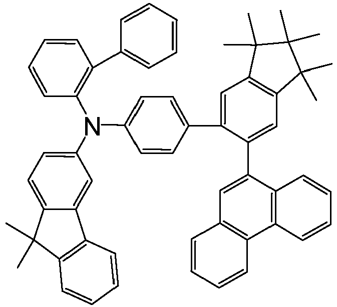 | 68 % |
| A114 | 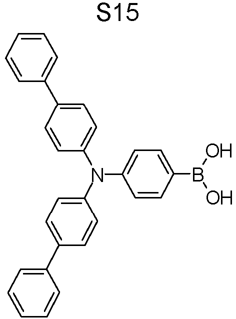 943836-24-6 | 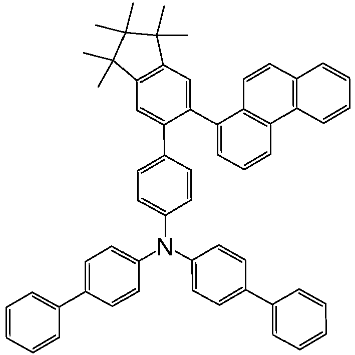 | 81 % |
| A115 | 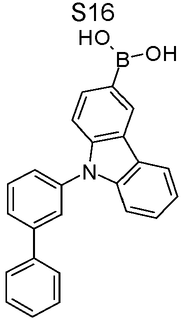 1416814-68-0 | 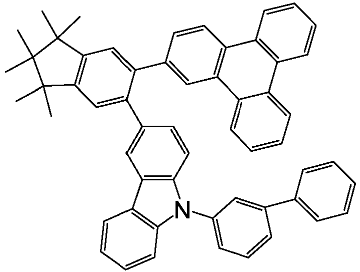 | 78 % |
| A116 | 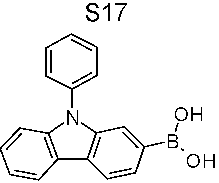 1001911-63-2 | 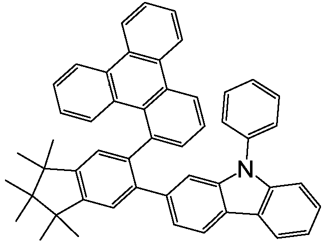 | 71 % |
| A117 | 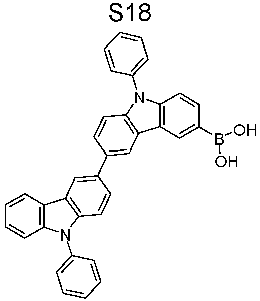 918137-86-7 | 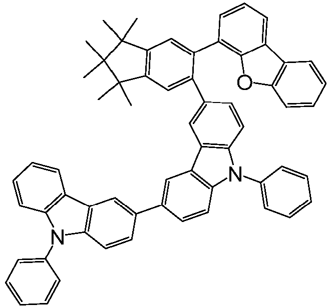 | 67 % |
| A118 | 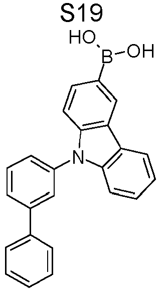 1416814-68-0 | 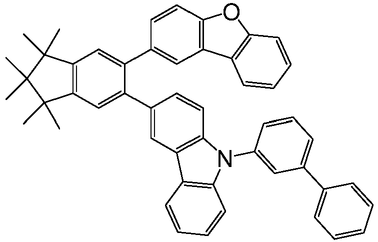 | 70 % |
| A119 | 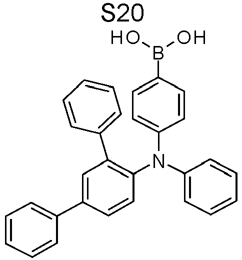 1417334-02-1 | 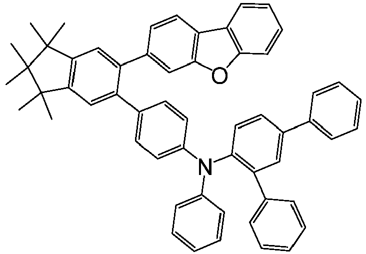 | 76 % |
| A120 | 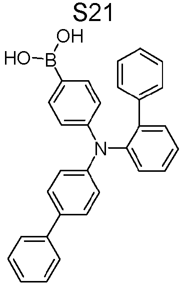 1608462-54-9 | 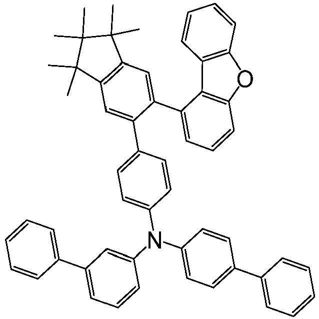 | 70 % |
| A121 | 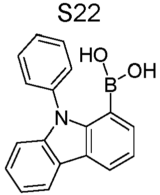 1333002-41-7 | 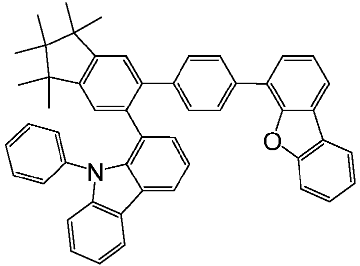 | 65 % |
| A122 | 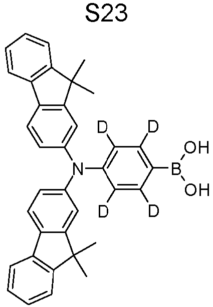 1648570-88-0 | 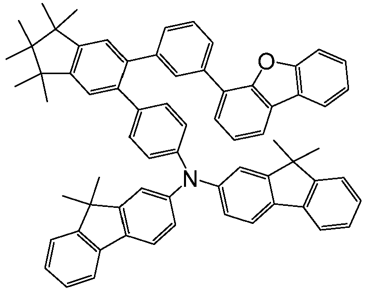 | 68 % |
| A123 | 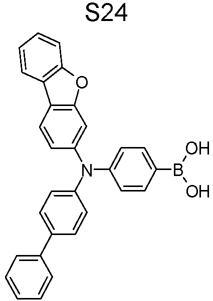 1960443-69-9 | 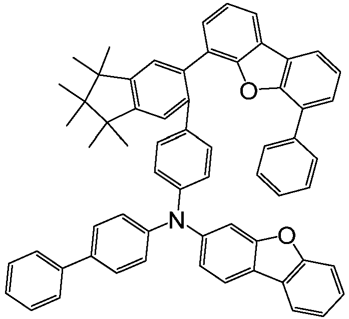 | 70 % |
| A124 | 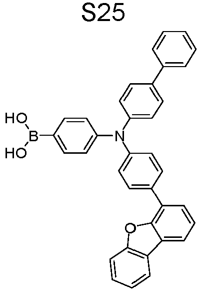 2126887-02- | 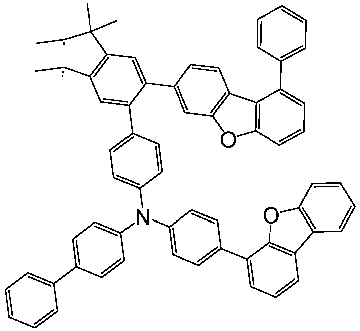 | 69 % |
| A125 | 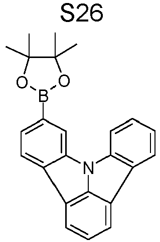 2068731-68-8 | 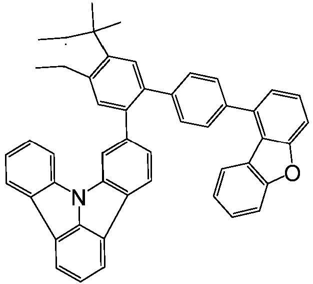 | 79 % |
| A126 | 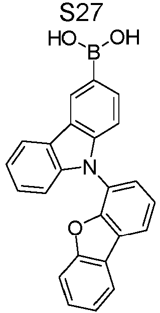 1547397-15-8 | 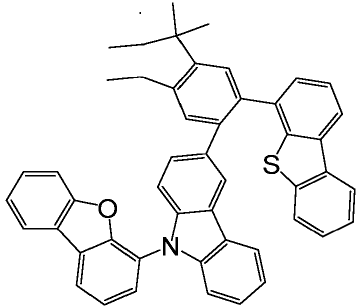 | 76 % |
| A127 | 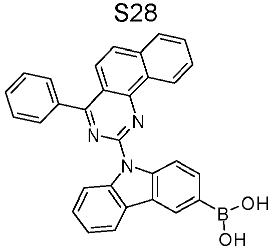 1813537-15-3 | 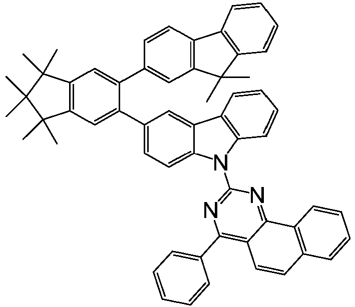 | 77 % |
| A128 | 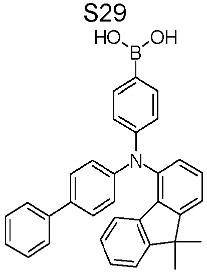 2410401-87-3 | 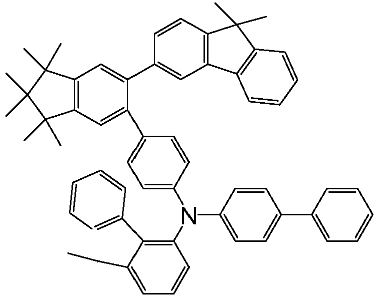 | 69 % |
| A129 | 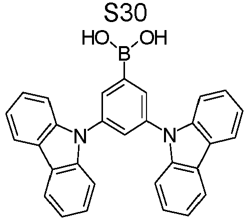 854952-51-5 | 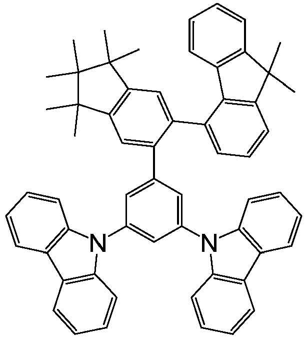 | 73 % |
| A130 | 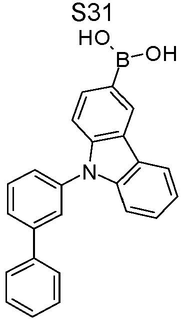 1416814-68-0 | 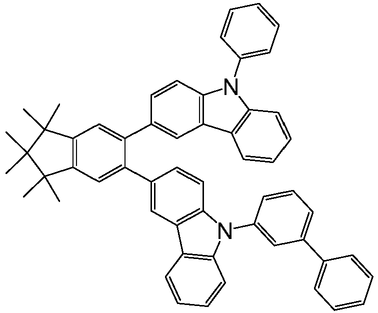 | 70 % |
| A131 | 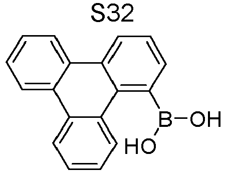 1430392-46-3 | 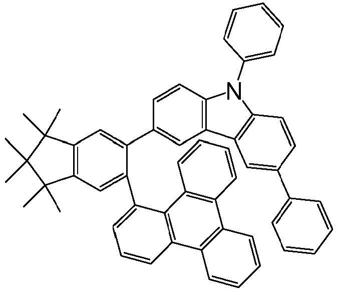 | 66 % |
| A132 | 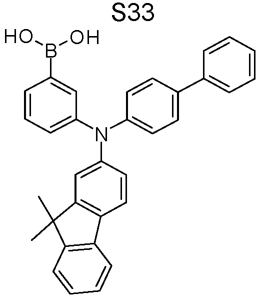 1610950-84-9 | 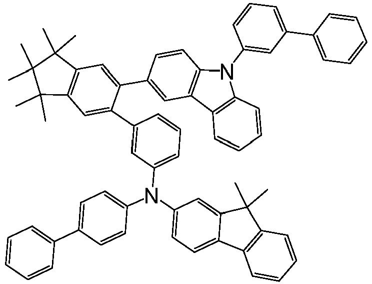 | 68 % |
| A133 | 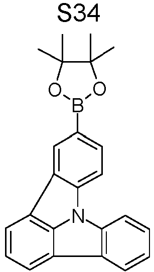 1454807-26-1 | 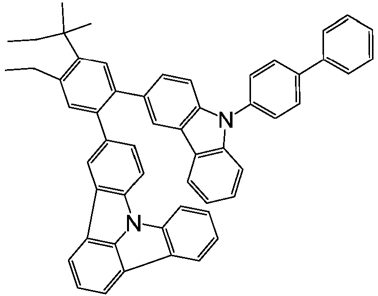 | 74 % |
| A134 | 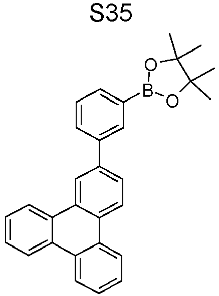 1115639-92-3 | 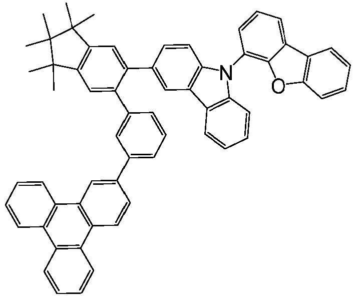 | 74 % |
| A135 | 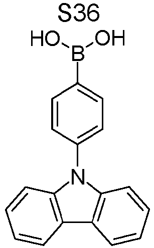 419536-33-7 | 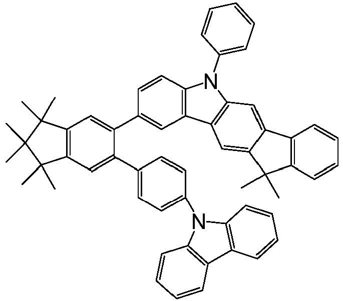 | 70 % |
| A136 | 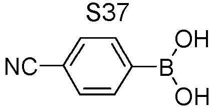 126747-14-6 | 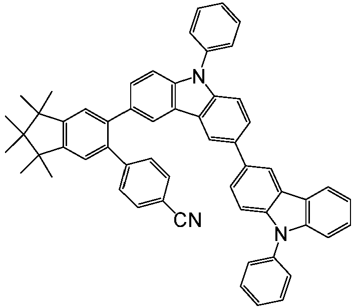 | 72 % |
| A137 | 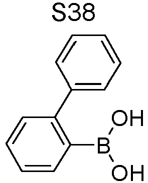 914675-52-8 | 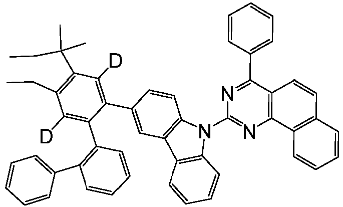 | 75 % |
| A138 | 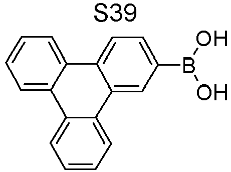 654664-63-8 | 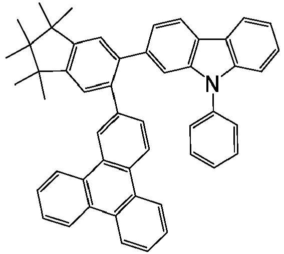 | 78 % |
| A139 | 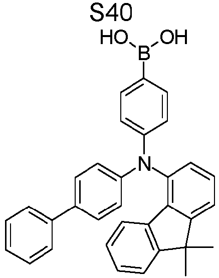 2410401-87-3 | 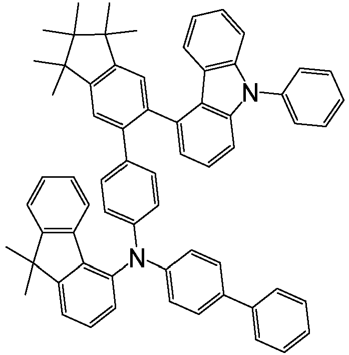 | 71 % |
| A140 | 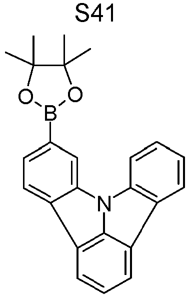 2068731-68-8 | 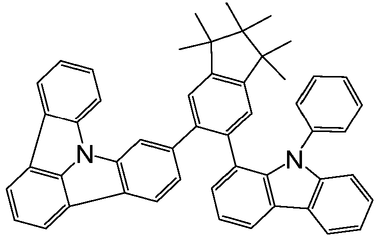 | 74 % |
| A141 | 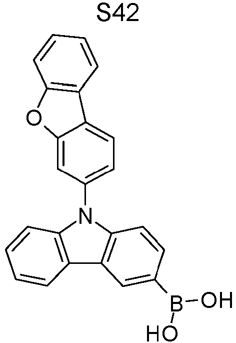 1637323-05-7 | 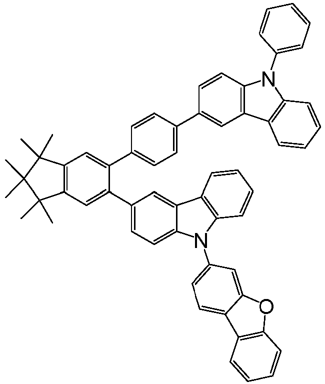 | 70 % |
| A142 | 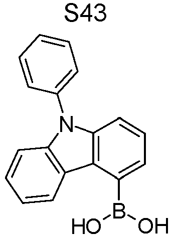 1370555-65-9 | 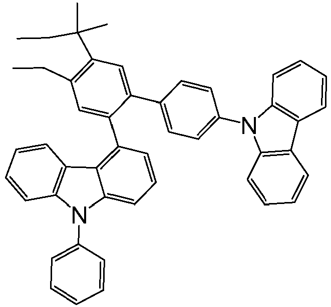 | 78 % |
| A143 | 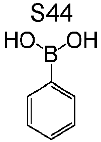 98-80-60 | 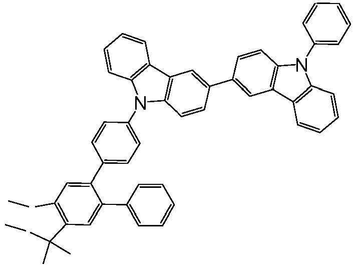 | 77 % |
| A144 | 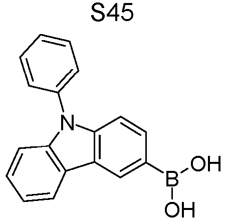 854952-58-2 | 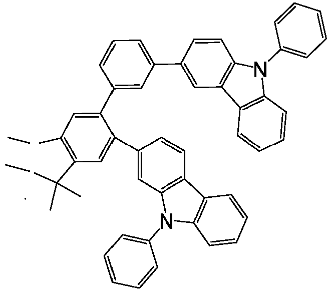 | 78 % |
| A145 | 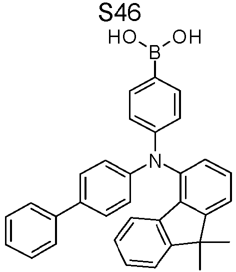 2410401-87-3 | 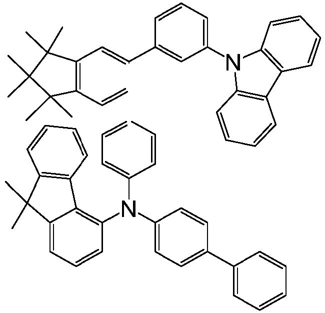 | 73 % |
| A146 | 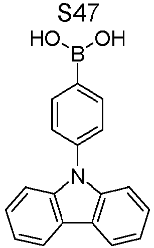 419536-33-7 | 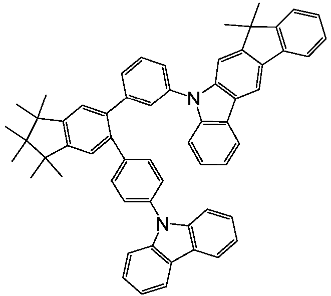 | 71 % |
| A147 | 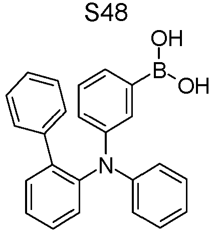 1776936-42-5 | 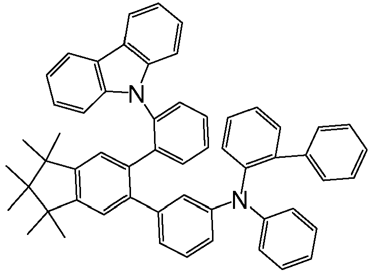 | 67 % |
| A148 | 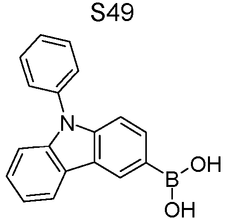 854952-58-2 | 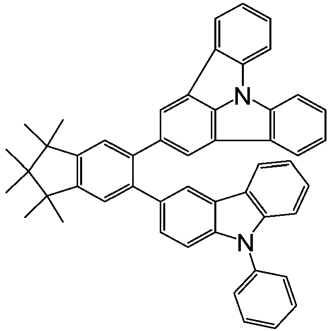 | 79 % |
| A149 | 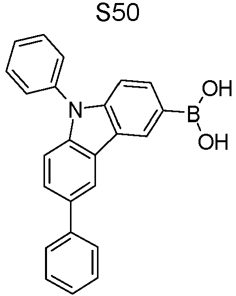 81359833-28-5 | 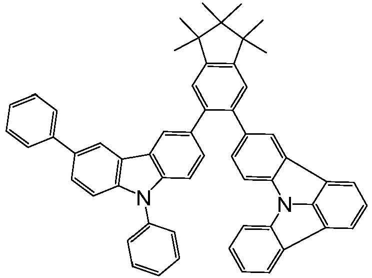 | 80 % |
| A150 | 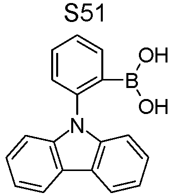 1189047-28-6 | 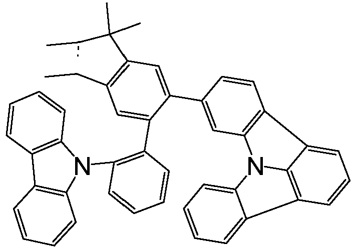 | 70 % |
| A151 | 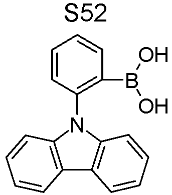 1189047-28-6 | 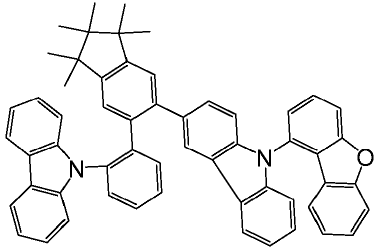 | 68 % |
| A152 | 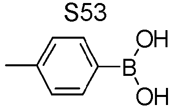 5720-05-8 | 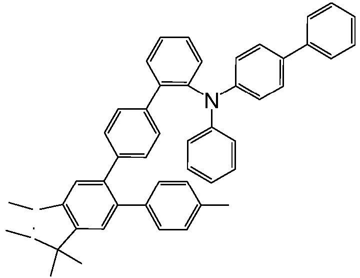 | 74 % |
| A153 | 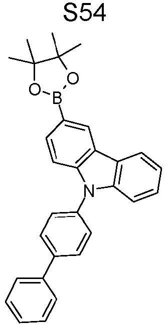 1391729-66-0 | 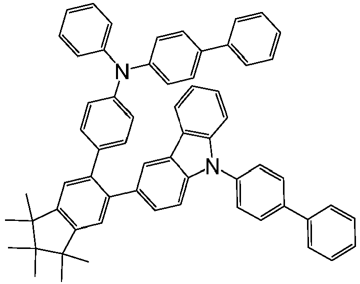 | 76 % |
| A154 | 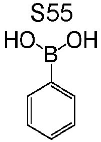 98-80-6 | 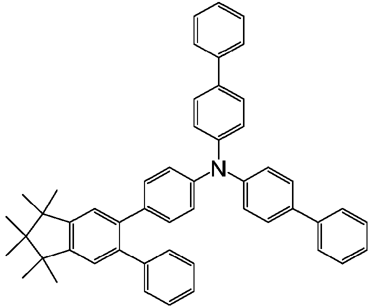 | 79 % |
| A155 | 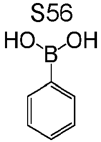 98-80-6 | 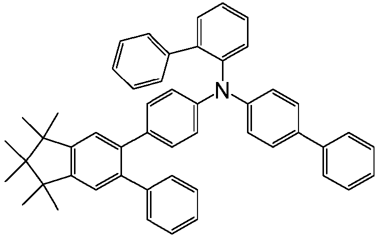 | 70 % |
| A156 | 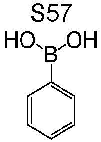 98-80-6 | 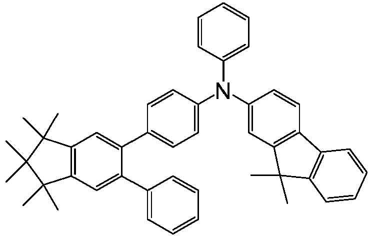 | 75 % |
| A157 | 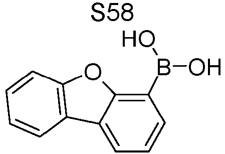 100124-06-9 | 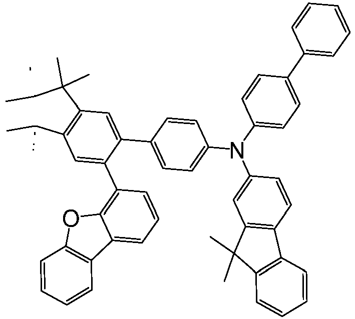 | 77 % |
| A158 | 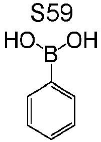 98-80-6 | 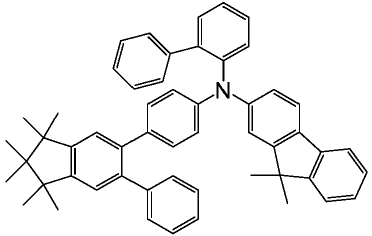 | 70 % |
| A159 | 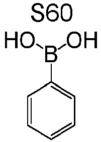 98-80-6 | 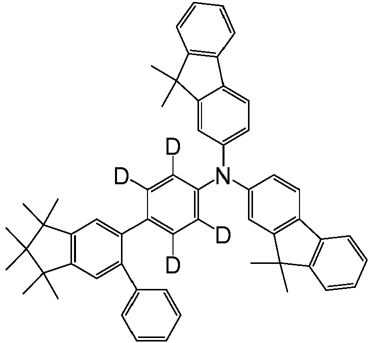 | 73 % |
| A160 | 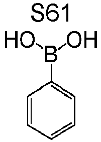 98-80-6 | 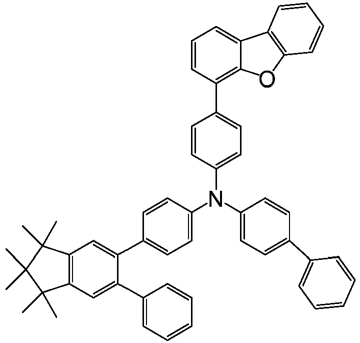 | 72 % |
| A161 | 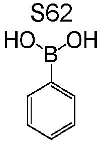 98-80-6 | 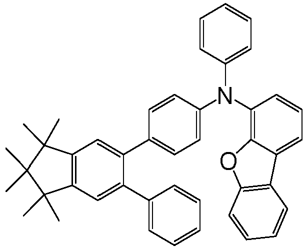 | 76 % |
| A162 | 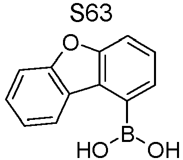 162607-19-4 | 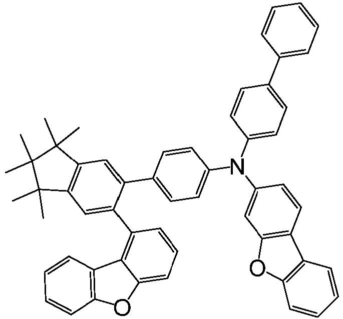 | 77 % |
| A163 | 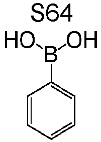 98-80-6 | 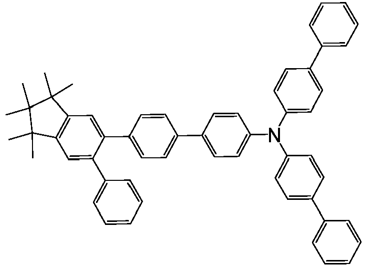 | 79 % |
| A164 | 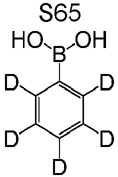 215527-70-1 | 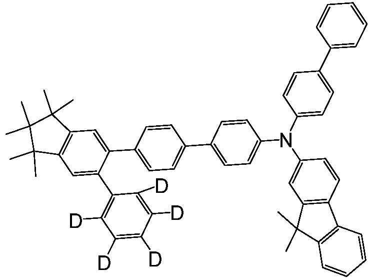 | 76 % |
| A165 | 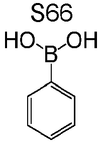 98-80-6 | 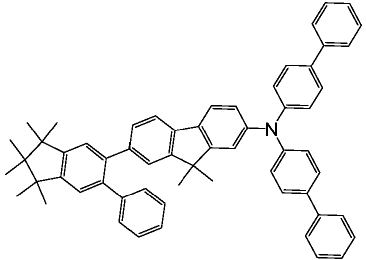 | 75 % |
| A166 | 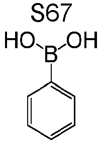 98-80-6 | 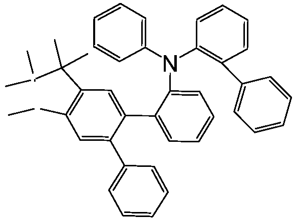 | 64 % |
| A167 | 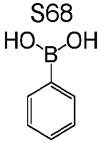 98-80-6 | 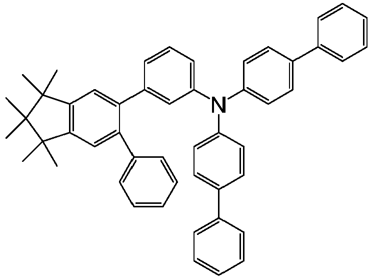 | 68 % |
| A168 | 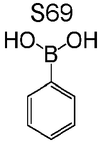 98-80-6 | 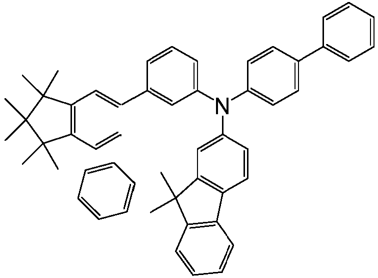 | 69 % |
| A169 | 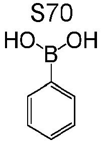 98-80-6 | 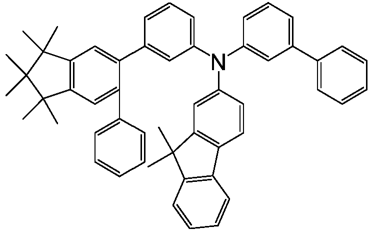 | 67 % |
| A170 | 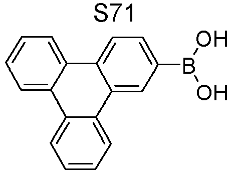 654664-63-8 | 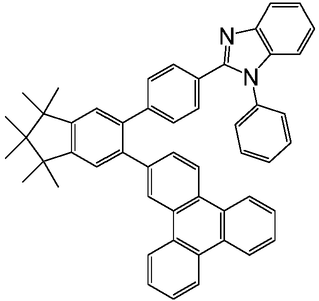 | 82 % |
| A171 | 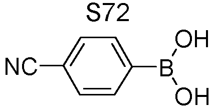 126747-14-6 | 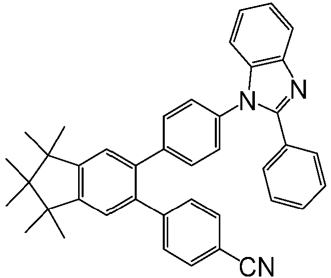 | 78 % |
| A172 | 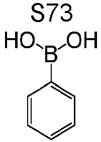 98-80-6 | 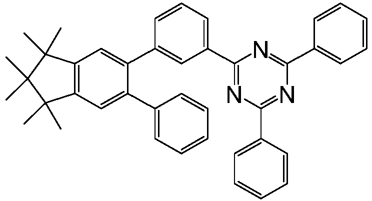 | 76 % |
| A173 | 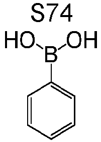 98-80-6 | 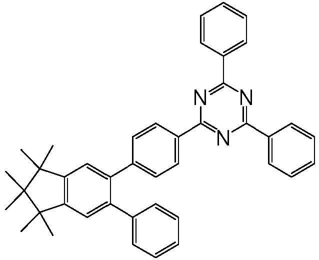 | 78 % |
| A174 | 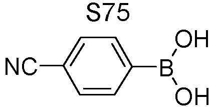 126747-14-6 | 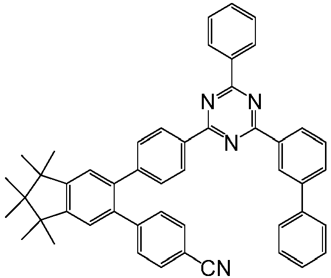 | 79 % |
| A175 | 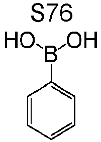 98-80-6 | 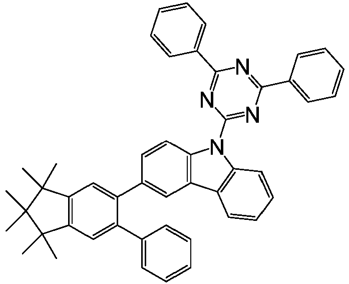 | 75 % |
| A176 | 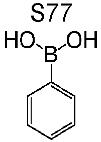 98-80-6 | 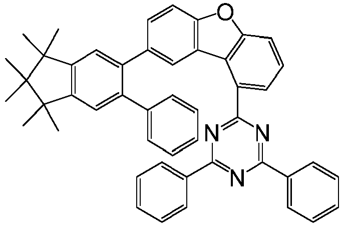 | 64 % |
| A177 | 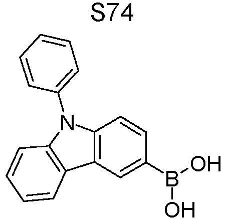 854952-58-2 | 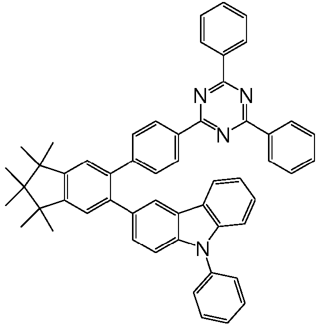 | 78 % |
| A178 | 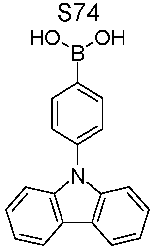 419536-33-7 | 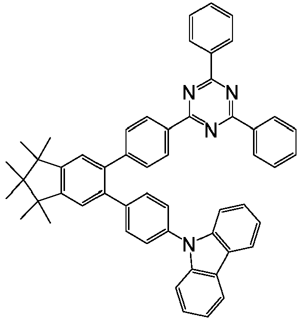 | 78 % |
| A179 | 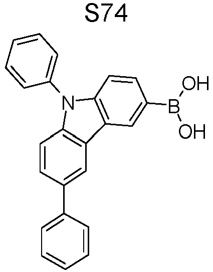 81359833-28-5 | 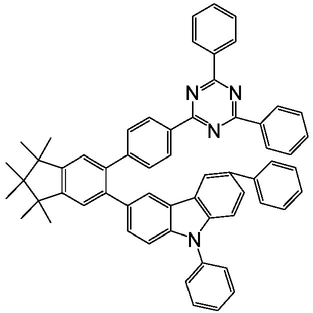 | 67 % |
| A180 | 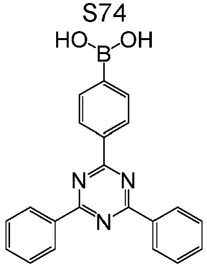 1313018-07-3 | 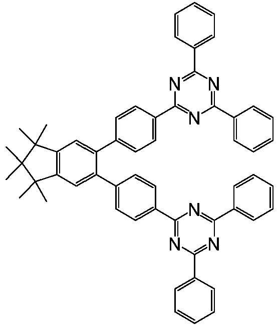 | 80 % |
| A181 | 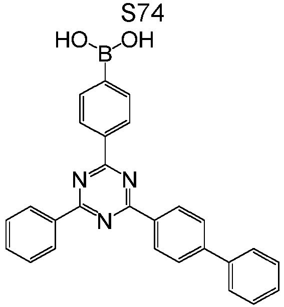 1987895-22-6 | 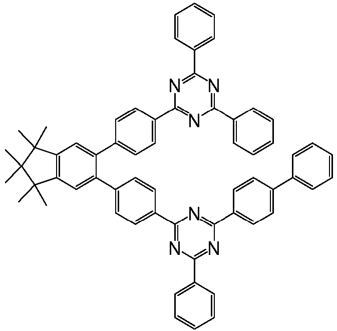 | 79 % |
| A182 | 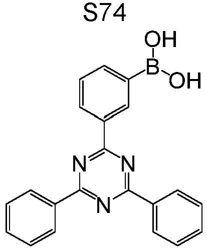 1269508-31-7 | 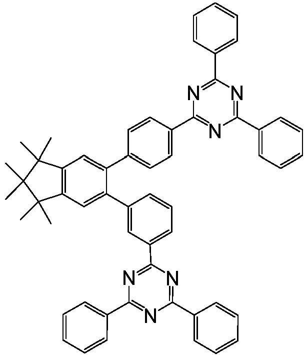 | 76 % |
| A183 | 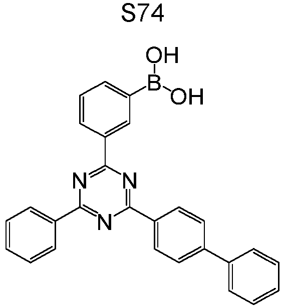 1696425-30-5 | 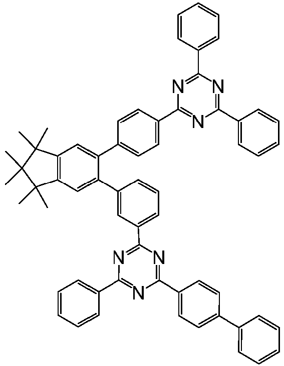 | 73 % |
| A184 | 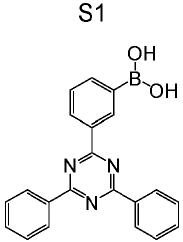 1269508-31-7 | 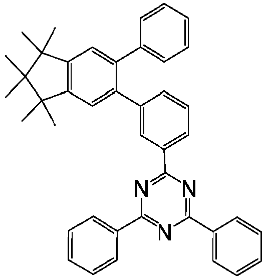 | 70 % |
| A185 | 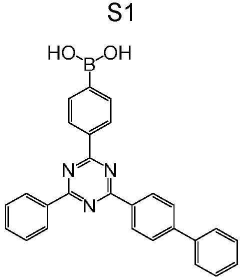 1987895-22-6 | 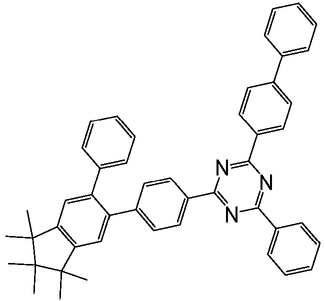 | 76 % |
| A186 | 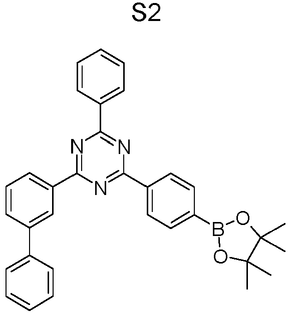 2168567-62-0 | 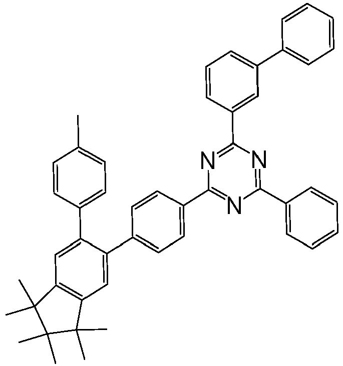 | 74 % |
| A187 | 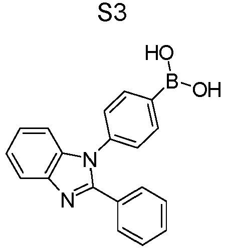 867044-33-5 | 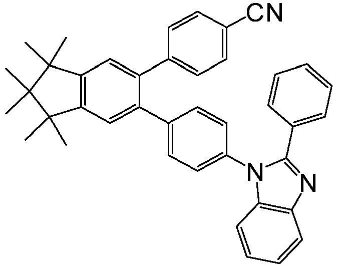 | 75 % |
| A188 | 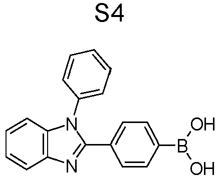 952514-79-3 | 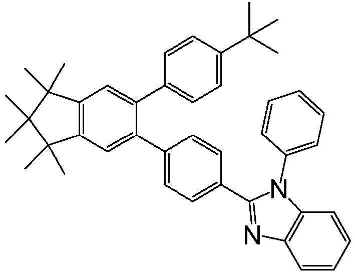 | 77 % |
| A189 | 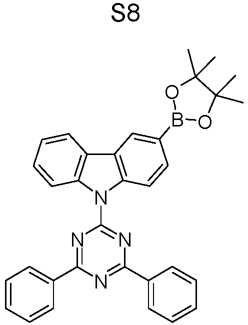 1361094-91-8 | 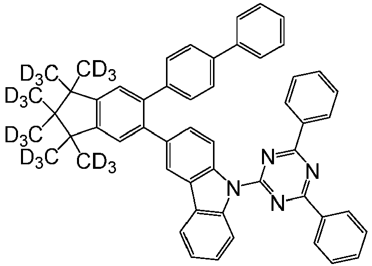 | 75 % |
| A190 | 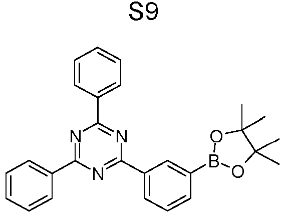 1269508-31-7 | 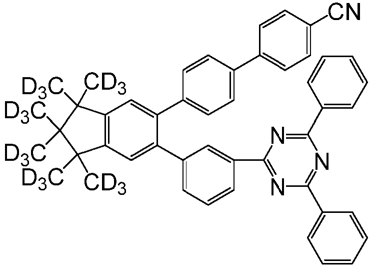 | 78 % |
| A191 | 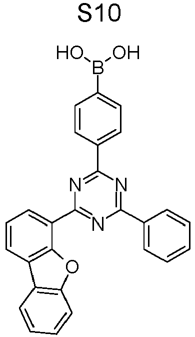 2378846-11-6 | 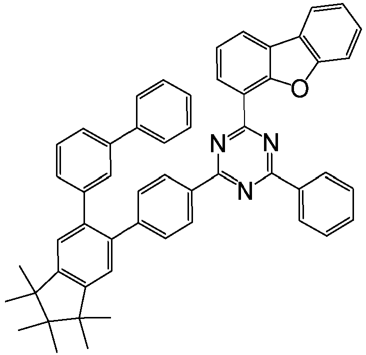 | 72 % |
| A192 | 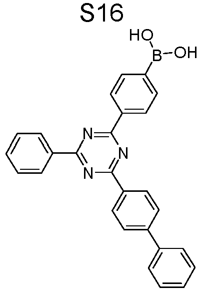 1987895-22-6 | 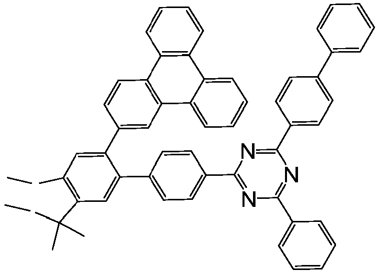 | 79 % |
| A193 | 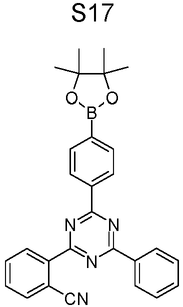 2226916-97-6 | 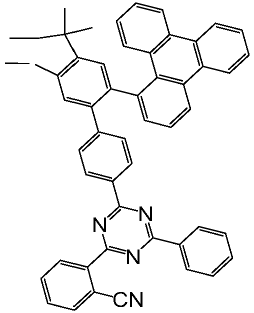 | 75 % |
| A194 | 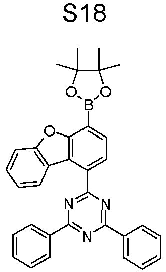 2265924-57-8 | 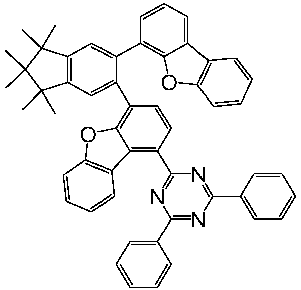 | 75 % |
| A195 | 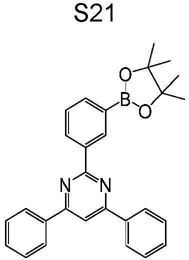 1381862-91-4 | 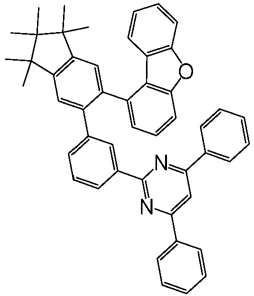 | 70 % |
| A196 | 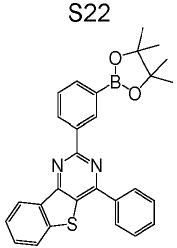 1801325-73-4 | 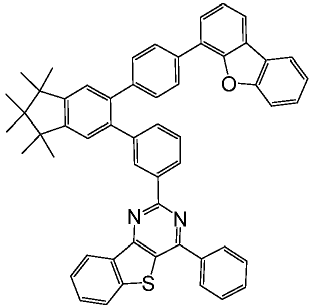 | 63 % |
| A197 | 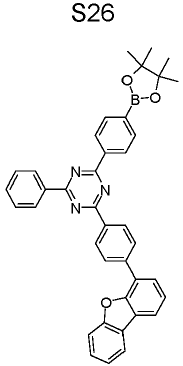 2287210-68-6 | 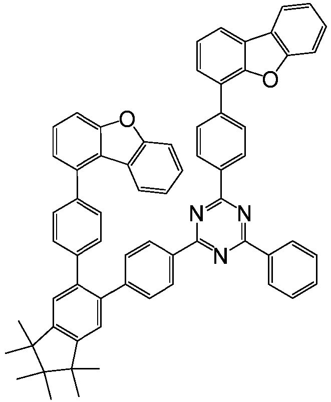 | 71 % |
| A198 | 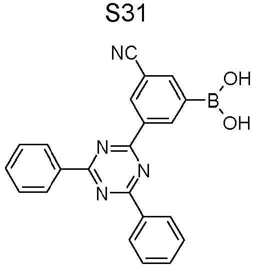 2140928-48-7 | 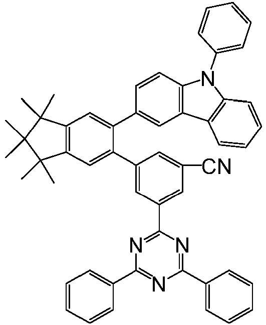 | 69 % |
| A199 | 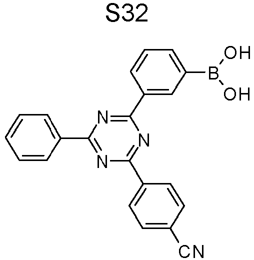 2259756-07-3 | 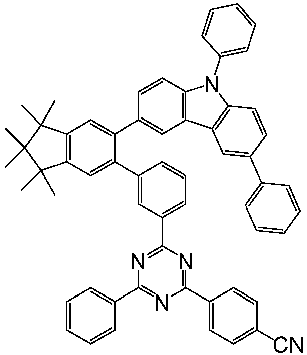 | 69 % |
| A200 | 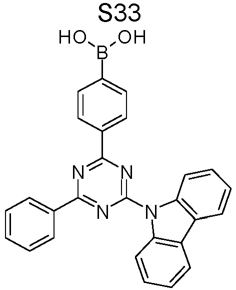 2378846-09-2 | 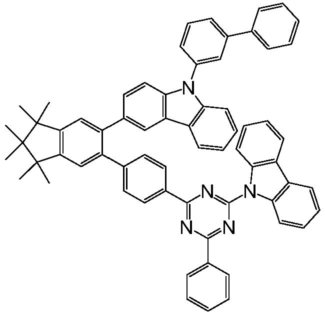 | 61 % |
| A201 | 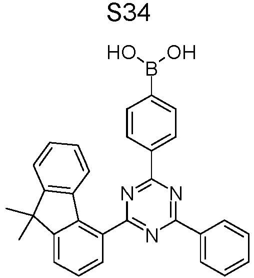 2308565-18-4 | 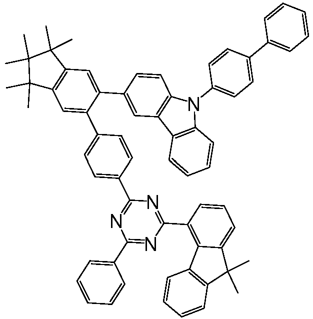 | 75 % |
| A202 | 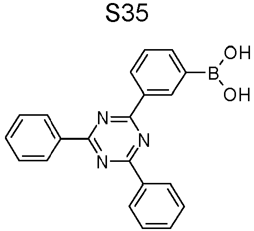 1612243-82-9 | 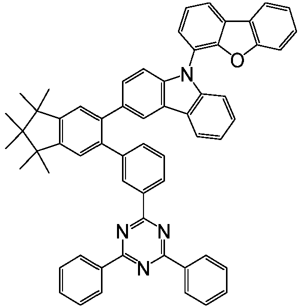 | 76 % |
| A203 | 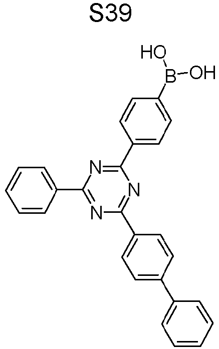 1987895-22-6 | 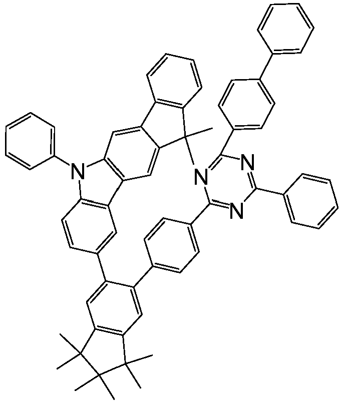 | 67 % |
| A204 | 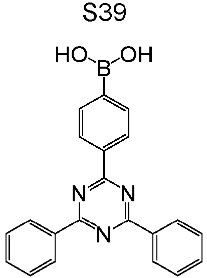 1313018-07-3 | 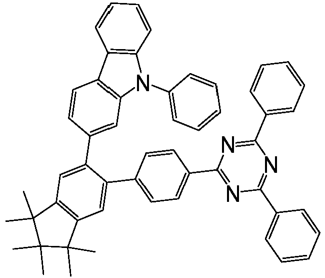 | 73 % |
| A205 | 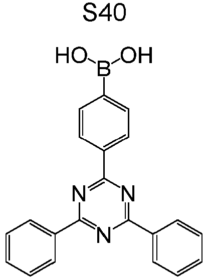 1313018-07-3 | 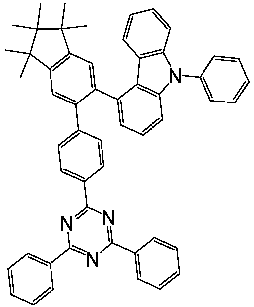 | 74 % |
| A206 | 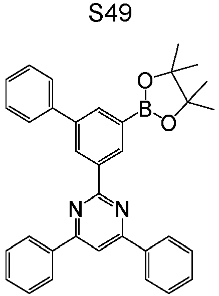 1835206-58-0 | 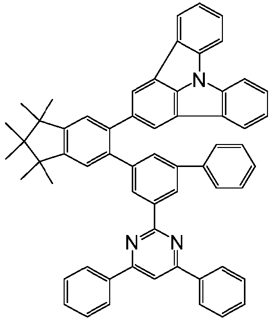 | 73 % |
| A207 | 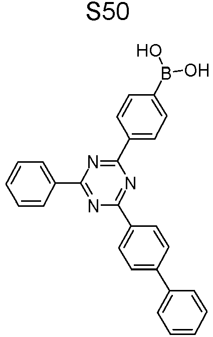 1987895-22-6 | 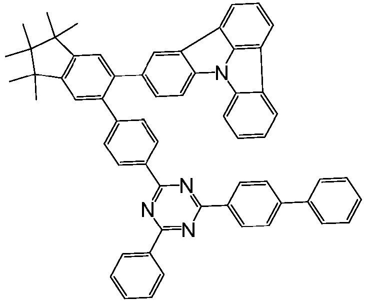 | 79 % |

### Beispiel A300:

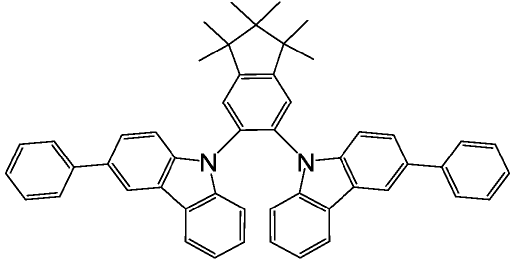

Durchführung analog T. Taisei et al., Chem. Lett., 2019, 48, 1160. Synthese von 3c. Ansatz: 18.0 g (50 mmol), 26.7 g (110 mmol) 3-Phenyl-9H-carbazole [103012-26-6]. Die Reinigung des Rohprodukts erfolgt durch Chromatographie und wiederholte Heißextraktionskristallisation (übliche org. Lösungsmittel bzw. deren Kombinationen, bevorzugt Acetonitril-DCM, 1:3 bis 3:1 vv) und fraktionierte Sublimation bzw. Tempern im Hochvakuum. Ausbeute: 13.7 g (20 mmol) 40 %; Reinheit: ca. 99.9 % ig n. HPLC.

Werden zunächst 25 mmol eines ersten Amins/Carbazols und dann nach 6 h Reaktionszeit 25 mmol eines zweiten Amins/Carbazols zugesetzt, können unsymmetrisch substituierte Verbindungen erhalten werden.

Analog können folgende Verbindungen dargestellt werden:

| Bsp. | Edukte | Produkt | Ausbeute |
|---|---|---|---|
| A301 | 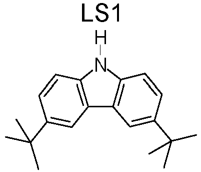 37500-95-1 | 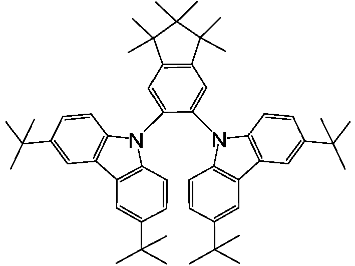 | 42 % |
| A302 | 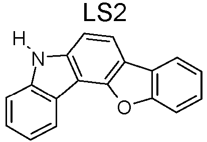 1199616-66-4 | 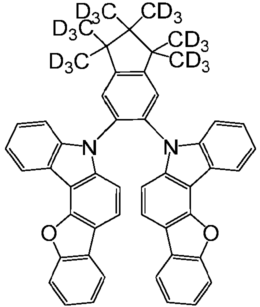 | 38 % |
| A303 | 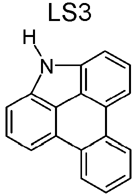 109606-75-9 | 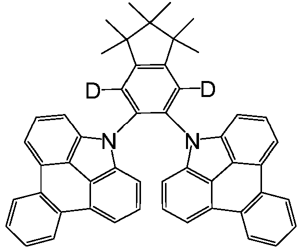 | 41 % |
| | 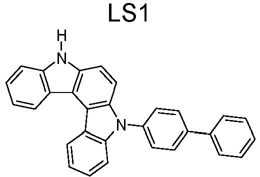 | 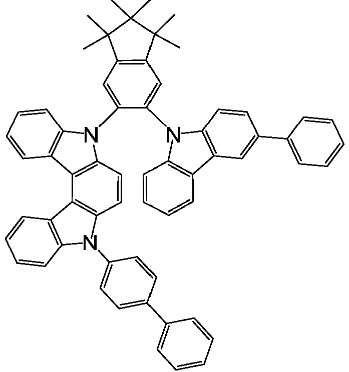 | |
| A304 | 2071630-78-7 | | 26 % |
| | 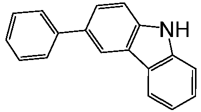 | | |
| | 103012-26-6 | | |
| A305 | 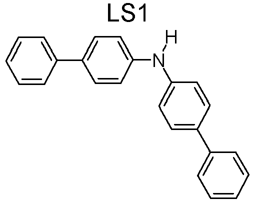 102113-98-4 | 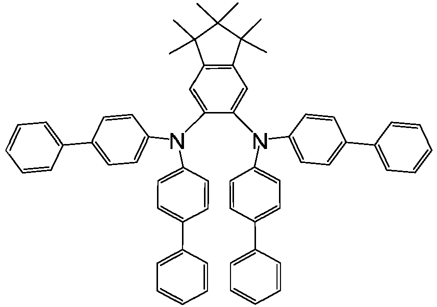 | 31 % |
| A306 | 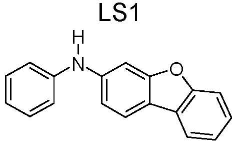 406488-21-9 | 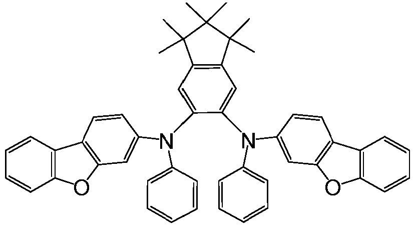 | 29 % |
| A307 | 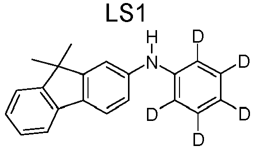 1359833-31-0 | 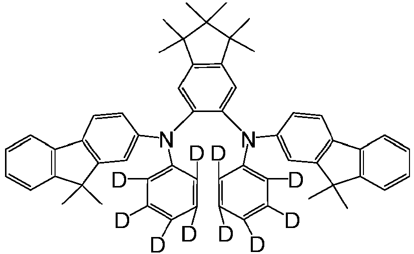 | 33 % |
| | 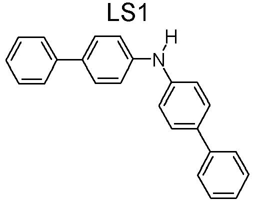 | 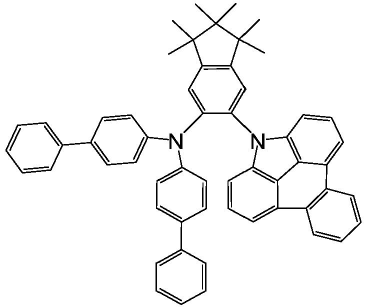 | |
| A308 | 102113-98-4 | | 24 % |
| | 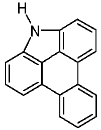 | | |
| | 109606-75-9 | | |
| | 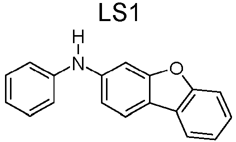 | 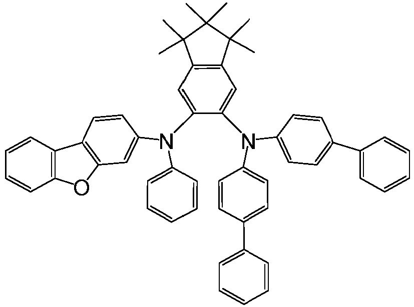 | |
| A309 | 406488-21-9 | | 21 % |
| | 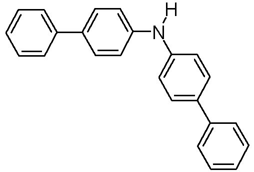 | | |
| | 102113-98-4 | | |

Darüber hinaus werden mit der zur Darstellung von A300 dargelegten Methode folgende Synthone synthetisiert:

| | | | |
|---|---|---|---|
| S100 | 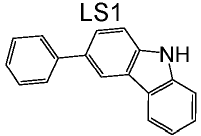 103012-26-6 | 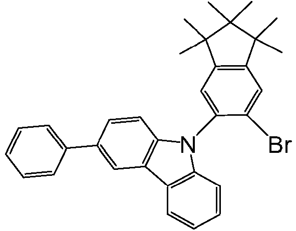 | 57 % |
| | 25 mmol | | |
| S101 | 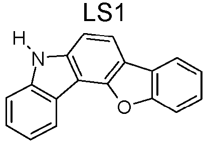 1199616-66-4 | 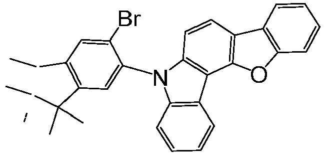 | 54 % |
| | 25 mmol | | |
| S102 | 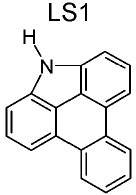 109606-75-9 | 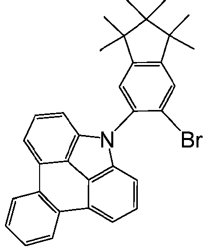 | 64 % |
| S103 | 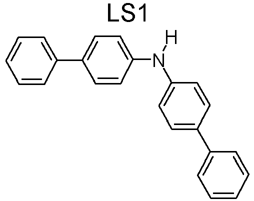 | 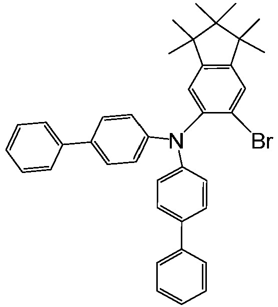 | 46 % |

Die zuvor dargelegten Synthone dienen unter anderem zur Herstellung der erfindungsgemäßen Verbindungen A400 bis A409.

### Beispiel A400:

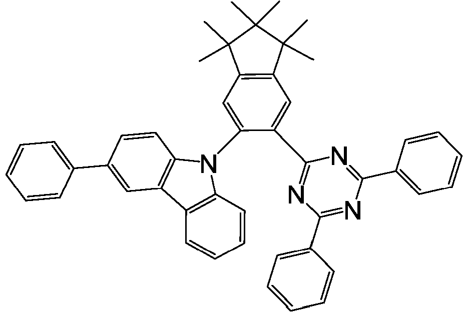

Eine gut gerührte, auf 0 °C gekühlte Lösung von 26.1 g (50 mmol) S100 in 500 ml THF wird tropfenweise mit 42.3 ml (55 mmol) iso-Propylmagnesiumchlorid-Lithiumchlorid-Komplex-Lösung, 1.3 M in THF versetzt. Man rührt 1 h nach und tropft dann eine Lösung von g (60 mmol) 2-Chloro-4,6-diphenyl-1,3,5-triazine [3842-55-5] in 200 ml zu, lässt auf RT erwärmen und rührt 5 h bei 60 °C nach. Man quencht vorsichtig durch Zugabe von 50 ml Methanol, entfernt das Lösungsmittel weitgehend in Vakuum, nimmt den Rückstand in 500 ml Dichlormethan (DCM) auf, wäscht dreimal mit je 200 ml Wasser, einmal mit 200 ml ges. Kochsalzlösung und trocknet über Magnesiumsulfat. Man filtriert vom Trockenmittel ab und engt das Filtrat auf ca. 200 ml im Vakuum ein, wobei das abdestillierte DCM kontinuierlich durch Methanol ersetzt wird. Man saugt vom auskristallisierten Produkt ab, wäscht zweimal mit je 50 ml Methanol und trocknet im Vakuum. Die Reinigung des Rohprodukts erfolgt durch Chromatographie und wiederholte Heißextraktionskristallisation (übliche org. Lösungsmittel bzw. deren Kombinationen, bevorzugt Acetonitril-DCM, 1:3 bis 3:1 vv) und fraktionierte Sublimation bzw. Tempern im Hochvakuum. Ausbeute: 26.5 g (39 mmol) 78 %; Reinheit: ca. 99.9 % ig n. HPLC.

Analog können folgende Verbindungen dargestellt werden:

| Bsp. | Edukte | Produkt | Ausbeute |
|---|---|---|---|
| A401 | 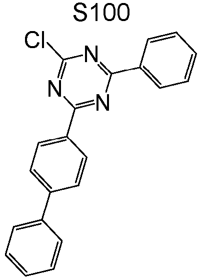 1472062-94-4 | 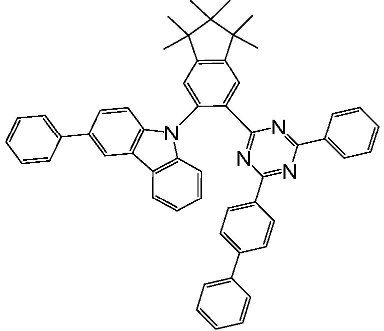 | 73 % |
| A402 | 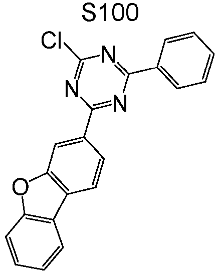 2142681-84-1 | 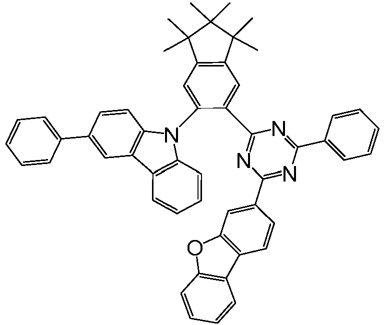 | 75 % |
| A403 | 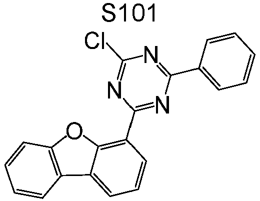 1472729-25-1 | 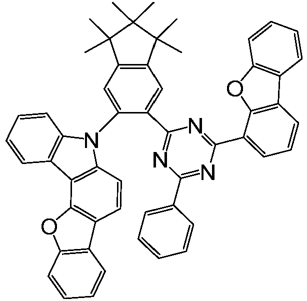 | 71 % |
| A404 | 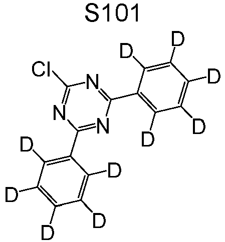 1300115-09-6 | 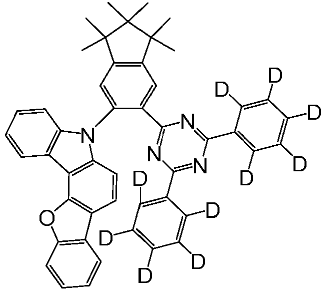 | 77 % |
| A405 | 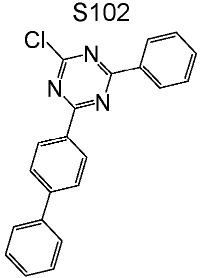 1472062-94-4 | 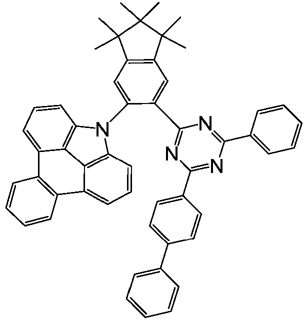 | 78 % |
| A406 | 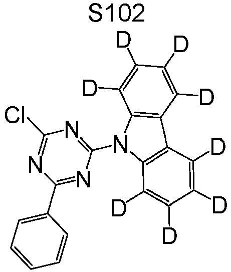 2574571-56-3 | 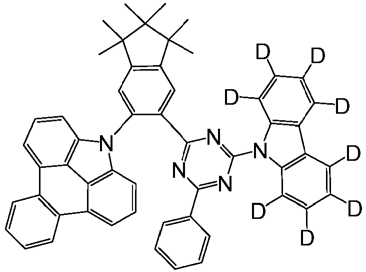 | 76 % |
| A407 | 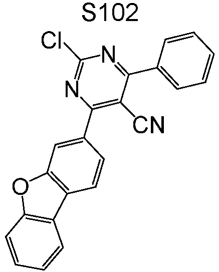 | 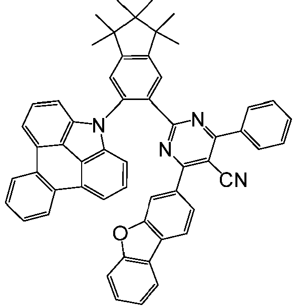 | 64 % |
| A408 | 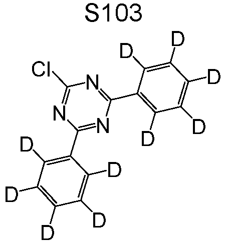 1300115-09-6 | 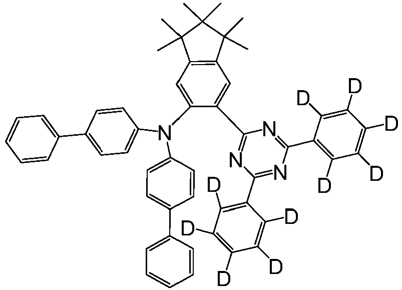 | 63 % |
| A409 | 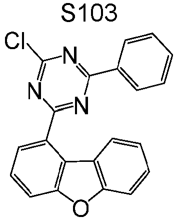 1883265-32-4 | 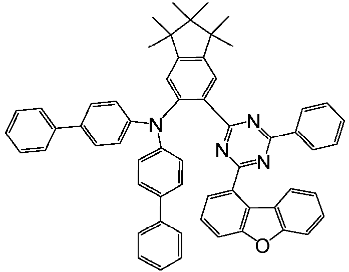 | 61 % |

### Beispiel: Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 25 Minuten mit UV-Ozon vorbehandelt (UV-Ozon Generator PR-100, Firma UVP). Diese beschichteten Glasplättchen bilden die Substrate, auf welche die OLEDs aufgebracht werden.

### 1a) Blaue Fluoreszenz-OLED- Bauteile - BF:

Die erfindungsgemäßen Verbindungen können in der Lochinjektionsschicht (HIL), Lochtransportschicht (HTL), der Elektronenblockierschicht (EBL) und der Elektronentransportschicht (ETL) verwendet werden. Alle Materialien werden in einer Vakuumkammer thermisch aufgedampft. Dabei besteht die Emissionsschicht (EML) immer aus mindestens einem Matrixmaterial (Hostmaterial, Wirtsmaterial) SMB (s. Tabelle 1) und einem emittierenden Dotierstoff (Dotand, Emitter) D, der dem Matrixmaterial bzw. den Matrixmaterialien durch Co-Verdampfung in einem bestimmten Volumenanteil beigemischt wird. Eine Angabe wie SMB:D (97:3%) bedeutet hierbei, dass das Material SMB in einem Volumenanteil von 97% und der Dotand D in einem Anteil von 3% in der Schicht vorliegt. Analog kann auch die Elektronentransportschicht aus einer Mischung zweier Materialien bestehen, s. Tabelle 1. Die zur Herstellung der OLEDs verwendeten Materialien sind in Tabelle 5 gezeigt oder beziehen sich auf die zuvor dargelegten Synthesebeispiele.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Angabe der EQE in (%) und der Spannung in (V) erfolgt bei einer Leuchtdichte von 1000 cd/m². Die Lebensdauer wird bei einer Startleuchtdichte von 10000 cd/m² bestimmt. Die gemessene Zeit, in der die Helligkeit der Referenz auf 80 % der Anfangshelligkeit abgefallen ist, wird zu 100% gesetzt. Die Lebensdauer der OLED-Bauteile enthaltend die erfindungsgemäßen Verbindungen wird in Prozent zur Referenz angegeben.

### Die OLEDs haben folgenden Schichtaufbau:

### Substrat

Lochinjektionsschicht (HIL) aus HTM1 dotiert mit 5 % NDP-9 (kommerziell erhältlich von der Fa. Novaled), 20 nm
Lochtransportschicht (HTL), s. Tabelle 1
Elektronenblockierschicht (EBL), s. Tabelle 1
Emissionsschicht (EML), s. Tabelle 1
Elektronentransportschicht (ETL), s. Tabelle 1
Elektroneninjektionsschicht (EIL) aus ETM2, 1 nm
Kathode aus Aluminium, 100 nm

**Tabelle 1: Aufbau Blaue Fluoreszenz-OLED-Bauteile**

| **Bsp.** | **HTL Dicke** | **EBL Dicke** | **EML Dicke** | **ETL Dicke** |
|---|---|---|---|---|
| **BF-Ref1** | Ref-HTM1 | EBM1 | SMB1:Ref-D1 (95%:5%) | ETM1:ETM2 (50%:50%) |
| | 180 nm | 10 nm | 20 nm | 30 nm |
| **BF-Ref2** | HTM1 | Ref-EBM1 | SMB1:Ref-D1 (95%:5%) | ETM1:ETM2 (50%:50%) |
| | 180 nm | 10 nm | 20 nm | 30 nm |
| **BF-Ref3** | Ref-HTM1 | Ref-EBM1 | SMB1:Ref-D1 (95%:5%) | ETM1:ETM2 (50%:50%) |
| | 180 nm | 10 nm | 20 nm | 30 nm |
| **BF1** | A4 | EBM1 | SMB1:Ref-D1 (95%:5%) | ETM1:ETM2 (50%:50%) |
| | 180 nm | 10 nm | 20 nm | 30 nm |
| **BF2** | HTM1 | A1 | SMB1:Ref-D1 | ETM1:ETM2 |
| | 180 nm | 10 nm | (95%:5%) 20 nm | (50%:50%) 30 nm |
| **BF3** | A4 | A1 | SMB1:Ref-D1 (95%:5%) | ETM1:ETM2 (50%:50%) |
| | 180 nm | 10 nm | 20 nm | 30 nm |
| **BF4** | A7 | EBM1 | SMB1:Ref-D1 (95%:5%) | ETM1:ETM2 (50%:50%) |
| | 180 nm | 10 nm | 20 nm | 30 nm |
| **BF5** | HTM1 | A11 | SMB2:Ref-D1 (95%:5%) | ETM1:ETM2 (50%:50%) |
| | 180 nm | 10 nm | 20 nm | 30 nm |
| **BF6** | A103 | EBM1 | SMB3:Ref-D1 (95%:5%) | ETM1:ETM2 (50%:50%) |
| | 180 nm | 10 nm | 20 nm | 30 nm |
| **BF7** | HTM1 | EBM1 | SMB1:Ref-D1 (95%:5%) | A173:ETM2 (50%:50%) |
| | 180 nm | 10 nm | 20 nm | 30 nm |

**Tabelle 2: Ergebnisse Blaue Fluoreszenz-OLED- Bauteile**

| **Bsp.** | **EQE (%) 1000 cd/m²** | **Spannung (V) 1000 cd/m²** | **LT80 [%] 10000 cd/m²** |
|---|---|---|---|
| **BF-Ref1** | 7.7 | 4.0 | 100 |
| **BF-Ref2** | 7.9 | 4.1 | 100 |
| **BF-Ref3** | 7.5 | 4.0 | 100 |
| **BF1** | 8.2 | 3.8 | 125 |
| **BF2** | 8.4 | 3.9 | 140 |
| **BF3** | 8.5 | 3.8 | 180 |
| **BF4** | 8.3 | 3.7 | 130 |
| **BF5** | 8.6 | 4.0 | 140 |
| **BF6** | 8.5 | 3.9 | 155 |
| **BF7** | 8.3 | 4.1 | 130 |

### 1b) Phosphoreszenz-OLED-Bauteile:

Die erfindungsgemäßen Verbindungen A können in der Lochinjektionsschicht (HIL); der Lochtransportschicht (HTL), der Elektronenblockierschicht (EBL) und in der Emissionsschicht (EML) als Matrixmaterial (Hostmaterial, Wirtsmaterial) M (s. Tabelle 5) bzw. A (s. erfindungsgemäße Materialien) verwendet werden. Hierfür werden alle Materialien in einer Vakuumkammer thermisch aufgedampft. Dabei besteht die Emissionsschicht immer aus mindestens einem bzw. mehreren Matrixmaterialien M und einem phosphoreszierenden Dotierstoff Ir, der dem Matrixmaterial bzw. den Matrixmaterialien durch Co-Verdampfung in einem bestimmten Volumenanteil beigemischt wird. Eine Angabe wie M1:M2:Ir (55%:35%:10%) bedeutet hierbei, dass das Material M1 in einem Volumenanteil von 55%, M2 in einem Volumenanteil von 35% und Ir in einem Volumenanteil von 10% in der Schicht vorliegt. Analog kann auch die Elektronentransportschicht aus einer Mischung zweier Materialien bestehen. Der genaue Aufbau der OLEDs ist Tabelle 3 zu entnehmen. Die zur Herstellung der OLEDs verwendeten Materialien sind in Tabelle 5 gezeigt oder beziehen sich auf die zuvor dargelegten Synthesebeispiele.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Angabe der EQE in (%) und der Spannung in (V) erfolgt bei einer Leuchtdichte von 1000 cd/m². Die Lebensdauer wird bei einer Startleuchtdichte von 1000 cd/m² für Blau und Rot und von 10000 cd/m² für Grün und Gelb bestimmt. Die gemessene Zeit, in der die Helligkeit der Referenz auf 80 % der Anfangshelligkeit abgefallen ist, wird zu 100% gesetzt. Die Lebensdauer der OLED-Bauteile enthaltend die erfindungsgemäßen Verbindungen wird in Prozent zur jeweiligen Referenz angegeben.

### Die OLEDs haben folgenden Schichtaufbau:

### Substrat

Lochinjektionsschicht (HIL) aus HTM1 dotiert mit 5 % NDP-9 (kommerziell erhältlich von der Fa. Novaled), 20 nm
Lochtransportschicht (HTL), s. Tabelle 3
Elektronenblockierschicht (EBL), s. Tabelle 3
Emissionsschicht (EML), s. Tabelle 3
Lochblockierschicht (HBL), s. Tabelle 3
Elektronentransportschicht (ETL), aus ETM1:ETM2 (50%:50%), 30 nm
Elektroneninjektionsschicht (EIL) aus ETM2, 1 nm
Kathode aus Aluminium, 100 nm

**Tabelle 3: Aufbau Phosphoreszenz-OLED-Bauteile**

| **Bsp.** | **HTL Dicke** | **EBL Dicke** | **EML Dicke** | **HBL Dicke** |
|---|---|---|---|---|
| **Blau** | | | | |
| Ref-BP1 | HTM1 | EBM2 | M3:Ref-M1:IrB1 (30%:65%:5%) | HBM2 |
| | 180 nm | 20 nm | 25 nm | 5 nm |
| BP1 | HTM1 | EBM2 | M3:A301:IrB1 (30%:65%:5%) | HBM2 |
| | 180 nm | 20 nm | 25 nm | 5 nm |

| **Grün** | | | | |
|---|---|---|---|---|
| GP-Ref1 | Ref-HTM1 | EBM1 | M1:M2:IrG1 (30%:60%:10%) | HBM1 |
| | 50 nm | 20 nm | 40 nm | 5 nm |
| GP-Ref2 | HTM1 | Ref-EBM1 | M1:M2:IrG1 (30%:60%:10%) | HBM1 |
| | 50 nm | 20 nm | 40 nm | 5 nm |
| GP-Ref3 | HTM1 | EBM1 | M1:Ref-M2:IrG1 (30%:60%:10%) | HBM1 |
| | 50 nm | 20 nm | 40 nm | 5 nm |
| GP1 | A4 | EBM1 | M1:M2:IrG1 (30%:60%:10%) | HBM1 |
| | 50 nm | 20 nm | 40 nm | 5 nm |
| GP2 | HTM1 | A1 | M1:M2:IrG1 (30%:60%:10%) | HBM1 |
| | 50 nm | 20 nm | 40 nm | 5 nm |
| GP3 | HTM1 | A21 | M1:M2:IrG1 (30%:60%:10%) | HBM1 |
| | 50 nm | 20 nm | 40 nm | 5 nm |
| GP4 | HTM1 | A120 | M1:M2:IrG1 (30%:60%:10%) | HBM1 |
| | 50 nm | 20 nm | 40 nm | 5 nm |
| GP5 | HTM1 | A155 | M1:M2:IrG1 (30%:60%:10%) | HBM1 |
| | 50 nm | 20 nm | 40 nm | 5 nm |
| GP6 | A7 | EBM1 | M1:M2:IrG1 (30%:60%:10%) | A185 |
| | 50 nm | 20 nm | 40 nm | 5 nm |
| GP7 | A168 | EBM1 | M1:A143:IrG1 (30%:60%:10%) | HBM1 |
| | 50 nm | 20 nm | 40 nm | 5 nm |
| GP8 | HTM1 | EBM1 | A194:M2:IrG1 | HBM1 |
| | 50 nm | 20 nm | (40%:50%:10%) 40 nm | 5 nm |
| GP9 | HTM1 | EBM1 | A189:IrG1 (84%:16%) | HBM1 |
| | 50 nm | 20 nm | 40 nm | 5 nm |

| **Gelb** | | | | |
|---|---|---|---|---|
| GP-Ref50 | Ref-HTM1 | EBM1 | M1:M2:IrG2 (30%:70%:10%) | HBM1 |
| | 50 nm | 20 nm | 40 nm | 5 nm |
| GP-Ref51 | HTM1 | Ref-EBM1 | M1:M2:IrG2 (30%:70%:10%) | HBM1 |
| | 50 nm | 20 nm | 40 nm | 5 nm |
| GP-Ref52 | HTM1 | EBM1 | M1:Ref-M2:IrG1 (30%:60%:10%) | HBM1 |
| | 50 nm | 20 nm | 40 nm | 5 nm |
| GP50 | A4 | EBM1 | M1:M2:IrG2 (30%:70%:10%) | HBM1 |
| | 50 nm | 20 nm | 40 nm | 5 nm |
| GP51 | HTM1 | A1 | M1:M2:IrG2 (30%:70%:10%) | HBM1 |
| | 50 nm | 20 nm | 40 nm | 5 nm |
| GP52 | A7 | EBM1 | M1:M2:IrG2 (30%:60%:10%) | A172 |
| | 50 nm | 20 nm | 40 nm | 5 nm |
| GP53 | A7 | EBM1 | M1:M2:IrG2 (30%:60%:10%) | A174 |
| | 50 nm | 20 nm | 40 nm | 5 nm |
| GP54 | A7 | EBM1 | M1:M2:IrG2 (30%:60%:10%) | A191 |
| | 50 nm | 20 nm | 40 nm | 5 nm |
| GP55 | A7 | EBM1 | M1:M2:IrG2 (30%:60%:10%) | A193 |
| | 50 nm | 20 nm | 40 nm | 5 nm |
| GP56 | A7 | EBM1 | M1:M2:IrG2 (30%:60%:10%) | A195 |
| | 50 nm | 20 nm | 40 nm | 5 nm |
| GP57 | A169 | EBM1 | M1:A143:IrG2 (30%:60%:10%) | HBM1 |
| | 50 nm | 20 nm | 40 nm | 5 nm |
| GP58 | A169 | EBM1 | M1:A36:IrG2 (30%:60%:10%) | HBM1 |
| | 50 nm | 20 nm | 40 nm | 5 nm |
| GP59 | A169 | EBM1 | M1:A52:IrG2 (30%:60%:10%) | HBM1 |
| | 50 nm | 20 nm | 40 nm | 5 nm |
| GP60 | A169 | EBM1 | M1:A136:IrG2 | HBM1 |
| | 50 nm | 20 nm | (30%:60%:10%) 40 nm | 5 nm |

| **Rot** | | | | |
|---|---|---|---|---|
| RP-Ref1 | Ref-HTM1 | EBM1 | M5:IrR1 (95%:5%) | HBM1 |
| | 50 nm | 20 nm | 35 nm | 10 nm |
| RP-Ref2 | HTM1 | Ref-EBM1 | M5:IrR1 | HBM1 |
| | 50 nm | 20 nm | (95%:5%) 35 nm | 10 nm |
| RP1 | A4 | EBM1 | M5:IrR1 | HBM1 |
| | 50 nm | 20 nm | (95%:5%) 35 nm | 10 nm |
| RP2 | A156 | EBM1 | M5:IrR1 (95%:5%) | HBM1 |
| | 50 nm | 20 nm | 35 nm | 10 nm |
| RP3 | A158 | EBM1 | M5:IrR1 (95%:5%) | HBM1 |
| | 50 nm | 20 nm | 35 nm | 10 nm |
| RP4 | HTM1 | A1 | M5:IrR1 (95%:5%) | HBM1 |
| | 50 nm | 20 nm | 35 nm | 10 nm |
| RP5 | HTM1 | A161 | M5:IrR1 (95%:5%) | HBM1 |
| | 50 nm | 20 nm | 35 nm | 10 nm |
| RP6 | HTM1 | A162 | M5:IrR1 (95%:5%) | HBM1 |
| | 50 nm | 20 nm | 35 nm | 10 nm |
| RP7 | HTM1 | A167 | M5:IrR1 (95%:5%) | HBM1 |
| | 50 nm | 20 nm | 35 nm | 10 nm |
| RP8 | A4 | A1 | A41: IrR1 (95%:5%) | HBM1 |
| | 50 nm | 20 nm | 35 nm | 10 nm |
| RP9 | A4 | A1 | A75:IrR1 (95%:5%) | HBM1 |
| | 50 nm | 20 nm | 35 nm | 10 nm |
| RP10 | A4 | A1 | A77: IrR1 (94%:6%) | HBM1 |
| | 50 nm | 20 nm | 35 nm | 10 nm |
| RP11 | A4 | A1 | A112:IrR1 (95%:5%) | HBM1 |
| | 50 nm | 20 nm | 35 nm | 10 nm |
| RP12 | A4 | A1 | A137:IrR1 | HBM1 |
| | 50 nm | 20 nm | (95%:5%) 35 nm | 10 nm |
| RP13 | A4 | A1 | A401: IrR1 (95%:5%) | HBM1 |
| | 50 nm | 20 nm | 35 nm | 10 nm |
| RP14 | A4 | A1 | A406:IrR1 (95%:5%) | HBM1 |
| | 50 nm | 20 nm | 35 nm | 10 nm |
| RP15 | A4 | A1 | A204:IrR1 (95%:5%) | HBM1 |
| | 50 nm | 20 nm | 35 nm | 10 nm |

**Tabelle 4: Ergebnisse Phosphoreszenz-OLED-Bauteile**

| **Blau** | | | |
|---|---|---|---|
| **Bsp.** | **EQE (%) 1000 cd/m²** | **Spannung (V) 1000 cd/m²** | **LT80 (%) 1000 cd/m²** |
| Ref-BP1 | 21.0 | 4.5 | 100 |
| BP1 | 21.9 | 4.2 | 350 |

| Grün | | | |
|---|---|---|---|
| **Bsp.** | **EQE (%) 1000 cd/m²** | **Spannung (V) 1000 cd/m²** | **LT80 (%) 10000 cd/m²** |
| GP-Ref1 | 22.3 | 3.5 | 100 |
| GP-Ref2 | 22.7 | 3.4 | 100 |
| GP-Ref3 | 22.2 | 3.3 | 100 |
| GP1 | 22.4 | 3.2 | 120 |
| GP2 | 22.7 | 3.1 | 160 |
| GP3 | 22.6 | 3.2 | 150 |
| GP4 | 23.0 | 3.1 | 120 |
| GP5 | 23.2 | 3.1 | 170 |
| GP6 | 23.0 | 3.0 | 115 |
| GP7 | 22.7 | 3.2 | 135 |
| GP8 | 23.1 | 3.3 | 120 |
| GP9 | 23.5 | 3.2 | 70 |

| **Gelb** | | | |
|---|---|---|---|
| GP-Ref50 | 29.0 | 3.3 | 100 |
| GP-Ref51 | 29.6 | 3.1 | 100 |
| GP-Ref52 | 30.1 | 3.2 | 100 |
| GP50 | 30.2 | 3.0 | 135 |
| GP51 | 30.3 | 2.9 | 160 |
| GP52 | 29.8 | 3.0 | 120 |
| GP53 | 30.3 | 3.1 | 110 |
| GP54 | 30.5 | 2.9 | 130 |
| GP55 | 31.0 | 3.0 | 125 |
| GP56 | 30.7 | 2.9 | 120 |
| GP57 | 30.4 | 3.0 | 140 |
| GP58 | 29.8 | 3.0 | 155 |
| GP59 | 30.5 | 2.9 | 135 |
| GP60 | 30.7 | 3.1 | 125 |

| **Rot** | | | |
|---|---|---|---|
| **Bsp.** | **EQE (%) 1000 cd/m²** | **Spannung (V) 1000 cd/m²** | **LT80 (%) 1000 cd/m²** |
| RP-Ref1 | 16.2 | 3.6 | 100 |
| RP-Ref2 | 16.4 | 3.4 | 100 |
| RP1 | 17.0 | 3.3 | 140 |
| RP2 | 16.9 | 3.3 | 130 |
| RP3 | 17.2 | 3.2 | 135 |
| RP4 | 17.1 | 3.2 | 170 |
| RP5 | 17.5 | 3.1 | 190 |
| RP6 | 17.2 | 3.3 | 180 |
| RP7 | 17.1 | 3.2 | 145 |
| RP8 | 17.5 | 3.3 | 160 |
| RP9 | 17.7 | 3.2 | 150 |
| RP10 | 17.4 | 3.1 | 180 |
| RP11 | 17.4 | 3.3 | 155 |
| RP12 | 17.1 | 3.2 | 130 |
| RP13 | 17.6 | 3.2 | 190 |
| RP14 | 17.5 | 3.2 | 160 |
| RP15 | 17.3 | 3.1 | 145 |

**Tabelle 5: Strukturformeln der verwendeten Materialien**

| | |
|---|---|
| 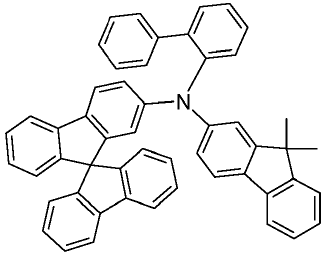 HTM1 | 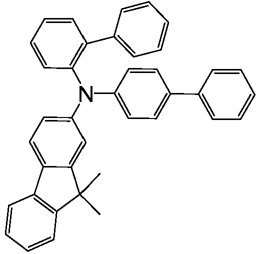 Ref-HTM1 |
| 136463-07-5 | 1549792-41-7 |
| 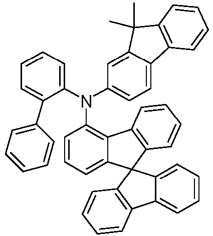 | 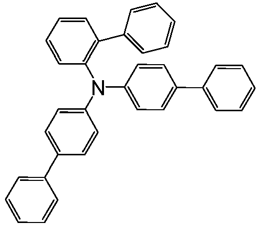 Ref-EBM1 |
| EBM1 | 1443540-42-8 |
| 1450933-44-4 | |
| 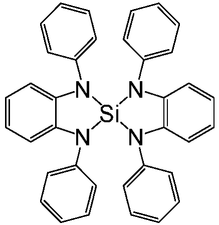 EBM2 | 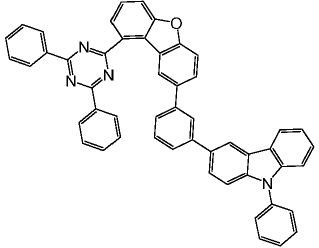 |
| 1206465-62-4 | M1 |
| | 1822310-86-0 |
| 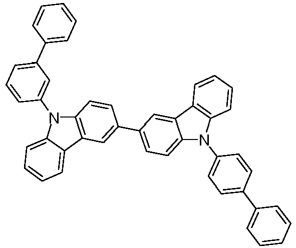 M2 | 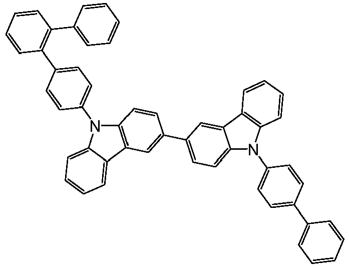 Ref-M2 |
| 1643479-47-3 | 1548581-37-8 |
| 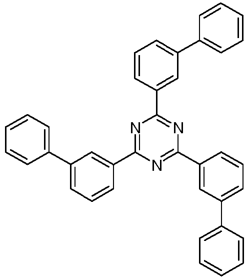 | 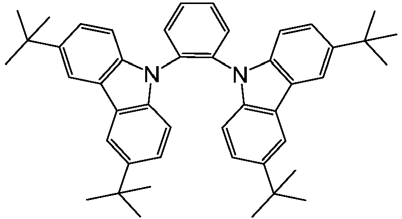 Ref-M1 |
| M3 = HBM2 | 2378004-55-6 |
| 1201800-83-0 | |
| 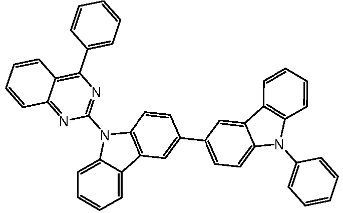 M5 | 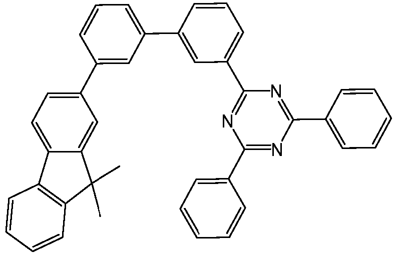 |
| 1398395-92-0 | HBM1 |
| | 1955543-57-3 |
| 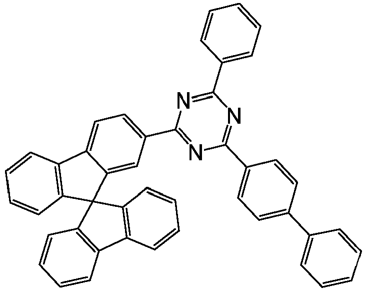 ETM1 | 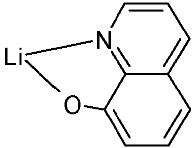 ETM2 |
| | 25387-93-3 |
| 1819335-36-8 | |
| 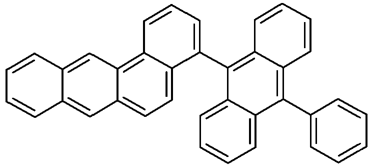 SMB1 | 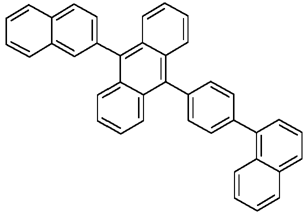 |
| 1087346-88-0 | SMB2 |
| | 667940-34-3 |
| 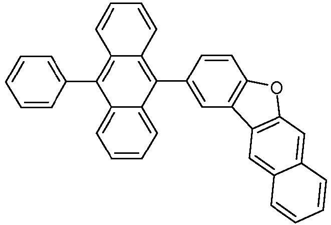 SMB3 | 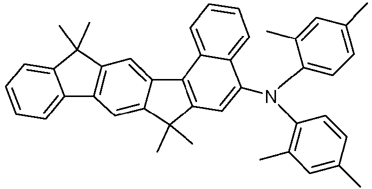 Fluoreszenz-Blau Ref-D1 |
| 1627916-48-6 | 1182175-27-4 |
| Phosphoreszenz-Blau 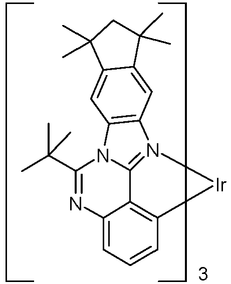 | Phosphoreszenz-Grün 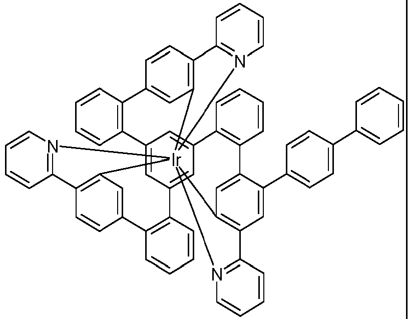 |
| IrB1 | IrG1 |
| 1541114-98-0 | 2245866-06-0 |
| Phosphoreszenz-Gelb | Phosphoreszenz-Tiefrot |
| 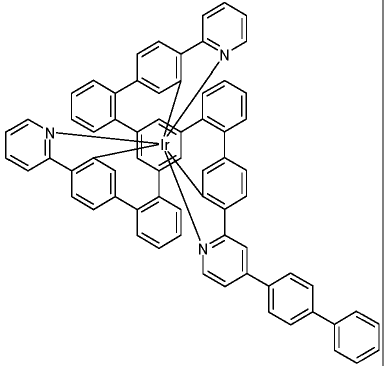 IrG2 | 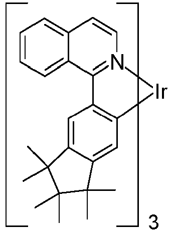 IrR1 |
| | 1562420-79-4 |
| 2245945-28-0 | |

## Patentansprüche

1. Verbindung umfassend mindestens eine Struktur der Formel (I), vorzugsweise Verbindung gemäß der Formel (I), 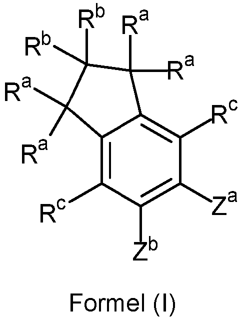 wobei für die Symbole gilt:
Z^{a} steht bei jedem Auftreten gleich oder verschieden für Ar^{a}, N(Ar^{c})₂ oder (Ar)N(Ar^{c})₂;
Z^{b} steht bei jedem Auftreten gleich oder verschieden für Ar^{b}, N(Ar^{d})₂ oder (Ar)N(Ar^{d})₂;
R^{a} ist bei jedem Auftreten gleich oder verschieden eine geradkettige Alkyl-, Alkoxy- oder Thioalkoxygruppe mit 1 bis 10 C-Atomen oder eine verzweigte oder cyclische Alkyl-, Alkoxyoder Thioalkoxygruppe mit 3 bis 10 C-Atomen, die jeweils mit einem oder mehreren Resten R² substituiert sein kann, vorzugsweise eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, dabei können zwei oder mehrere, vorzugsweise benachbarte Substituenten R^{a} miteinander ein Ringsystem bilden;
R^{b} ist bei jedem Auftreten gleich oder verschieden eine geradkettige Alkyl-, Alkoxy- oder Thioalkoxygruppe mit 1 bis 10 C-Atomen oder eine verzweigte oder cyclische Alkyl-, Alkoxyoder Thioalkoxygruppe mit 3 bis 10 C-Atomen, die jeweils mit einem oder mehreren Resten R² substituiert sein kann, vorzugsweise eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, dabei können zwei benachbarte Substituenten R^{b} miteinander ein Ringsystem bilden;
R^{c} ist bei jedem Auftreten gleich oder verschieden H, D, eine geradkettige Alkyl-, Alkoxy- oder Thioalkoxygruppe mit 1 bis 10 C-Atomen, vorzugsweise 1 bis 6 C-Atomen oder eine verzweigte oder cyclische Alkyl-, Alkoxy- oder Thioalkoxygruppe mit 3 bis 10 C-Atomen, die jeweils mit einem oder mehreren Resten R² substituiert sein kann, vorzugsweise H, D, eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, bevorzugt H, D, eine geradkettige Alkylgruppe mit 1 bis 10 C-Atomen, vorzugsweise 1 bis 6 C-Atomen, besonders bevorzugt H, D;
Ar^{a}, Ar^{b}, Ar^{c}, Ar^{d} ist bei jedem Auftreten gleich oder verschieden ein aromatisches oder heteroaromatisches Ringsystem mit 6 bis 60 aromatischen Ringatomen, das mit einem oder mehreren Resten R substituiert sein kann, dabei können zwei Reste Ar^{c}, Ar^{d}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein, vorzugsweise steht Ar^{a}, Ar^{b}, Ar^{c}, Ar^{d} bei jedem Auftreten gleich oder verschieden für eine Aryl- oder Heteroarylgruppe mit 6 bis 40 aromatischen Ringatomen, die mit einem oder mehreren Resten R substituiert sein kann, dabei können zwei Reste Ar, Ar^{c}, Ar^{a}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein;
Ar ist bei jedem Auftreten gleich oder verschieden ein verbindendes aromatisches oder heteroaromatisches Ringsystem mit 5 bis 60 aromatischen Ringatomen, das mit einem oder mehreren Resten R substituiert sein kann, dabei ein Rest Ar mit einem oder beiden der Reste Ar^{c}, Ar^{d}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein, vorzugsweise steht Ar bei jedem Auftreten gleich oder verschieden für eine Arylen- oder Heteroarylengruppe mit 5 bis 40 aromatischen Ringatomen, die mit einem oder mehreren Resten R substituiert sein kann, dabei ein Rest Ar mit einem oder beiden der Reste Ar^{c}, Ar^{a}, welche an dasselbe N-Atom binden, auch durch eine Einfachbindung oder eine Brücke, ausgewählt aus B(R), C(R)₂, Si(R)₂, C=O, C=NR, C=C(R)₂, RC=CR, O, S, S=O, SO₂, N(R), P(R), P(=O)R und einer ortho-verknüpften Phenylengruppe, die mit einem oder mehreren Resten R substituiert sein kann, vorzugsweise ausgewählt aus C(R)₂, O, N(R) und einer ortho-verknüpften Phenylengruppe, die mit einem oder mehreren Resten R substituiert sein kann, miteinander verbrückt sein;
R ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxyoder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R auch miteinander oder einer weiteren Gruppe ein Ringsystem bilden;
Ar' ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verbrückt sein;
R¹ ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxyoder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch - R²C=CR²⁻, -C≡C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxyoder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden, dabei können einer oder mehrere Reste R¹ mit einem weiteren Teil der Verbindung ein Ringsystem bilden;
Ar" ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden
wobei die Gruppen Z^{a} und Z^{b} kein Ringsystem bilden.

2. Verbindung nach Anspruch 1, umfassend mindestens eine Struktur der Formeln (I-1) bis (I-6), vorzugsweise Verbindung gemäß einer der Formeln (I-1) bis (I-6), 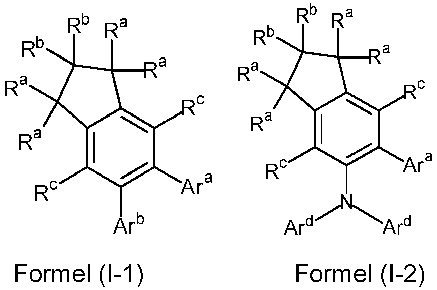 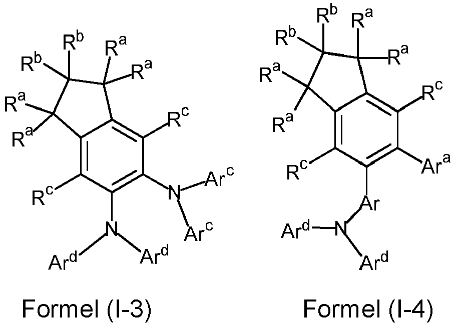 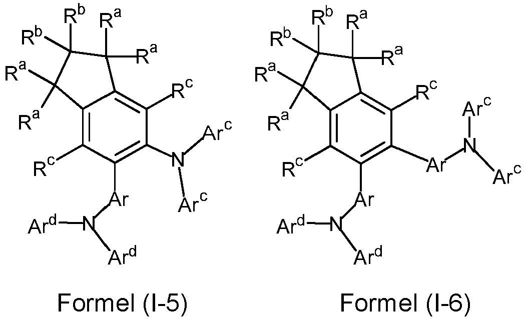 wobei die Symbole Ar, Ar^{a}, Ar^{b}, Ar^{c}, Ar^{d}, R^{a}, R^{b} und R^{c} die in Anspruch 1 genannten Bedeutungen aufweisen.

3. Verbindung nach Anspruch 1 oder 2, **dadurch gekennzeichnet, dass** die Gruppe Ar^{a}, Ar^{b}, Ar^{c} und/oder Ar^{d} gleich oder verschieden bei jedem Auftreten ausgewählt ist aus Strukturen der Formeln (Ar^{a}-1) bis (Ar^{a}-29), 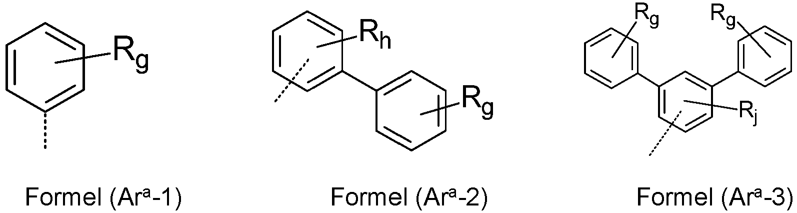 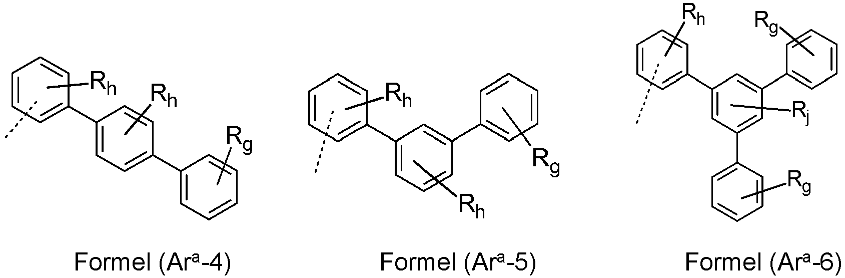 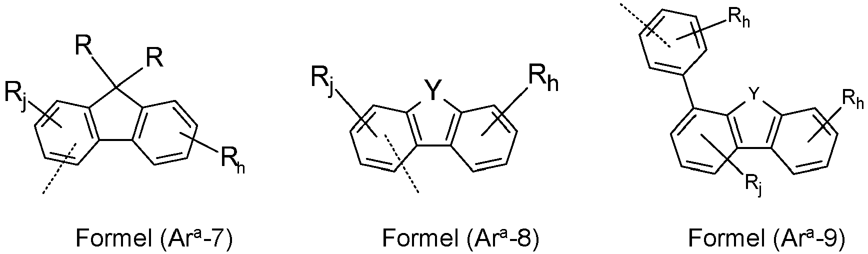 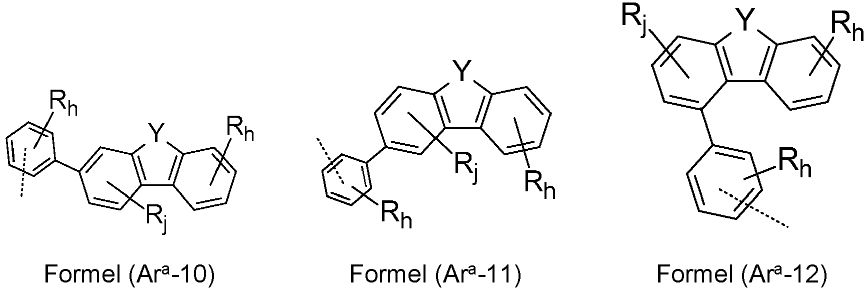 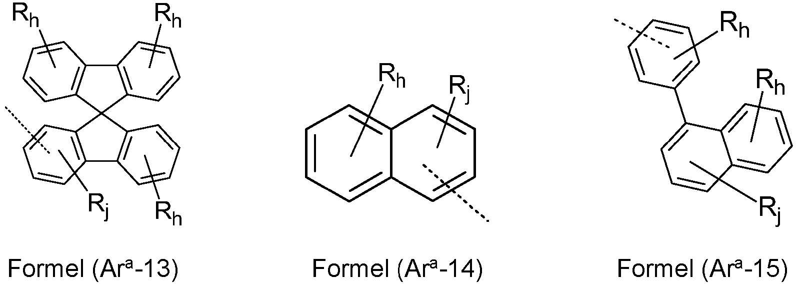 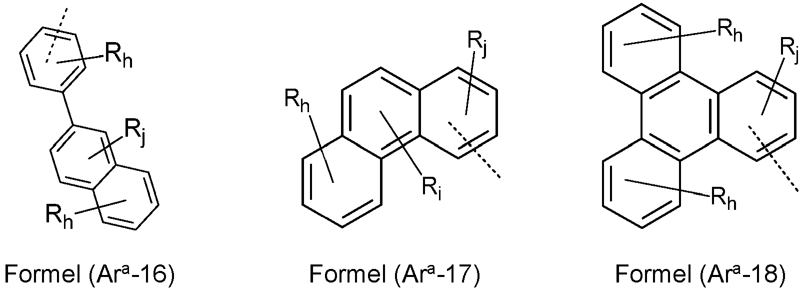 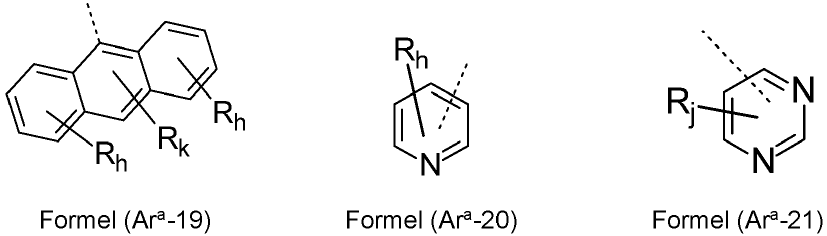 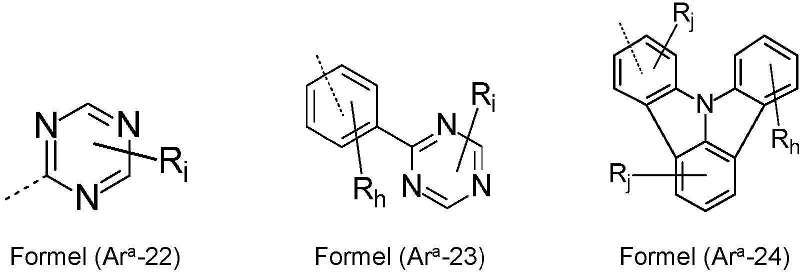 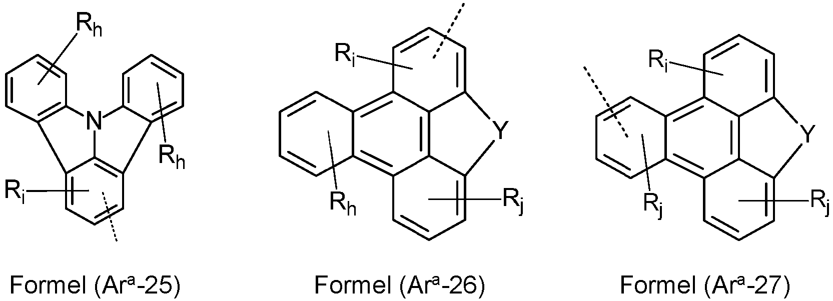 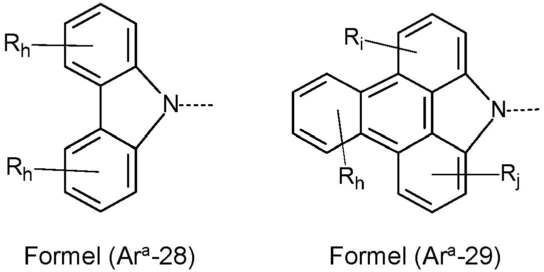 wobei für die verwendeten Symbole gilt:
Y ist O, S oder NR, vorzugsweise O oder NR;
k ist bei jedem Auftreten unabhängig 0 oder 1;
i ist bei jedem Auftreten unabhängig 0, 1 oder 2;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;
R weist die zuvor, insbesondere für Anspruch 1 genannte Bedeutung auf und die gestrichelte Bindung markiert die Anbindungsposition.

4. Verbindung nach einem oder mehreren der Ansprüche 1 bis 3, **dadurch gekennzeichnet, dass** die Gruppe Ar^{a} und/oder Ar^{b} für eine Struktur der Formel -Ar^{e}-Q steht, wobei Ar^{e} bei jedem Auftreten gleich oder verschieden ein verbindendes aromatisches oder heteroaromatisches Ringsystem mit 5 bis 40 aromatischen Ringatomen ist, das mit einem oder mehreren Resten R substituiert sein kann, vorzugsweise steht Ar^{e} bei jedem Auftreten gleich oder verschieden für eine Arylen- oder Heteroarylengruppe mit 5 bis 30 aromatischen Ringatomen, die mit einem oder mehreren Resten R substituiert sein kann, vorzugsweise ausgewählt ist aus Phenylen, Biphenylen, Terphenylen, Quaterphenylen, Fluorenylen, Spirobifluorenylen, Naphthylen, Indolylen, Benzofuranylen, Benzothiophenylen, Carbazolylen, Dibenzofuranylen, Dibenzothiophenylen, Indenocarbazolylen, Indolocarbazolylen, Pyridinylen, Pyrimidinylen, Pyrazinylen, Pyridazinylen, Triazinylen, Chinolinylen, Isochinolinylen, Chinazolinylen, Chinoxalinylen, Phenanthrenylen oder Triphenylenylen, welche jeweils mit einem oder mehreren Resten R substituiert sein können, vorzugsweise Phenylen, Biphenylen, Fluorenylen, Dibenzofuranylen, Triphenylenylen, Carbazolylen, Indolocarbazolylen; und Q für eine Elektronentransportgruppe, vorzugsweise für eine Pyridin-, Pyrimidin-, Pyrazin-, Pyridazin-, Triazin-, Chinazolin-, Chinoxalin-, Chinolin-, Isochinolin-, Imidazolund/oder Benzimidazol-Gruppe steht, die mit einem oder mehreren Resten R substituiert sein kann.

5. Verbindung nach einem oder mehreren der Ansprüche 1 bis 4, **dadurch gekennzeichnet, dass** die Gruppe Ar und/oder Ar^{e} gleich oder verschieden bei jedem Auftreten ausgewählt ist aus Strukturen der Formeln (Ar^{e}-1) bis (Ar^{e}- 9), 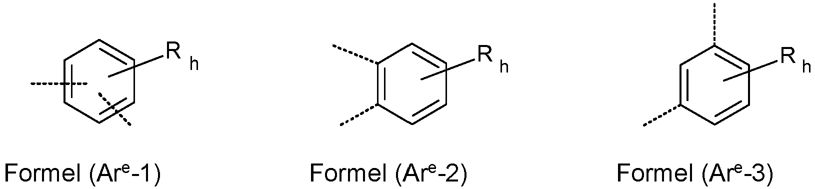 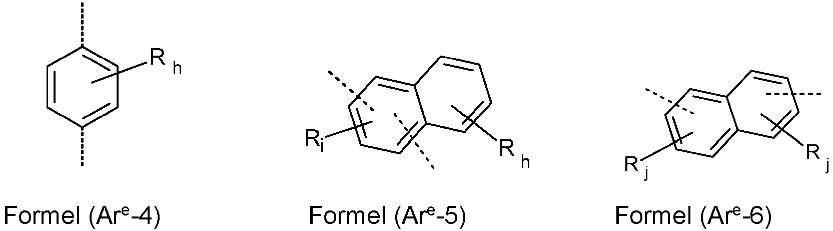 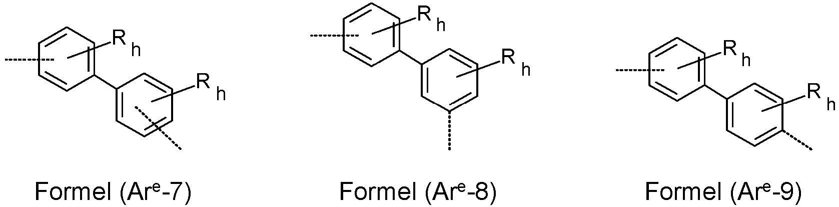 wobei für die verwendeten Symbole gilt:
j ist bei jedem Auftreten unabhängig 0, 1, 2 oder 3;
h ist bei jedem Auftreten unabhängig 0, 1, 2, 3 oder 4;
R weist die zuvor, insbesondere für Anspruch 1 genannte Bedeutung auf und die gestrichelten Bindungen markieren die Anbindungspositionen.

6. Verbindung nach einem oder mehreren der Ansprüche 1 bis 3 und 5, **dadurch gekennzeichnet, dass** die Verbindung mindestens eine Struktur gemäß Formel (I-2) umfasst, wobei die Gruppe Ar^{a} ausgewählt ist aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2) und (Ar^{a}-7) bis (Ar^{a}-18) und/oder die Verbindung mindestens eine Struktur gemäß Formel (I-4) umfasst, wobei die Gruppe Ar ausgewählt ist aus Strukturen der Formeln (Ar^{e}-1) bis (Ar^{e}-4), die Gruppe Ar^{a}, ausgewählt ist aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist, und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18).

7. Verbindung nach einem oder mehreren der Ansprüche 1 bis 3 und 5, **dadurch gekennzeichnet, dass** die Verbindung mindestens eine Struktur gemäß Formel (I-3) umfasst, wobei die Gruppen Ar^{c} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, vorzugsweise sind die Gruppen Ar^{c} bei jedem Auftreten gleich oder verschieden ausgewählt aus Strukturen der Formeln (Ar^{a}-1), (Ar^{a}-2), (Ar^{a}-7) bis (Ar^{a}-18), und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18) und/oder die Verbindung mindestens eine Struktur gemäß Formel (I-5) und/oder Formel (I-6) umfasst, wobei die Gruppe Ar bei jedem Auftreten gleich oder verschieden ausgewählt ist aus Strukturen der Formeln (Ar^{e}-1) bis (Ar^{e}-4), die Gruppen Ar^{c} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, vorzugsweise sind die Gruppen Ar^{c} bei jedem Auftreten gleich oder verschieden ausgewählt aus Strukturen der Formeln (Ar^{a}-1), (Ar^{a}-2), (Ar^{a}-7) bis (Ar^{a}-18), und die Gruppen Ar^{d} bei jedem Auftreten gleich oder verschieden ausgewählt sind aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18).

8. Verbindung nach Anspruch 4, **dadurch gekennzeichnet, dass** die Verbindung mindestens eine Struktur gemäß Formel (I-2) umfasst, wobei die Gruppe Ar^{a} für eine Struktur der Formel Ar^{e}-Q steht, wobei die Symbole Ar^{e} und Q die in Anspruch 5 genannte Bedeutung aufweisen und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18) und/oder die Verbindung mindestens eine Struktur gemäß Formel (I-4) umfasst, wobei die Gruppe Ar ausgewählt ist aus Strukturen der Formeln (Ar^{e}-1) bis (Ar^{e}-4), die Gruppe Ar^{a} für eine Struktur der Formel Ar^{e}-Q steht, wobei die Symbole Ar^{e} und Q die in Anspruch 5 genannte Bedeutung aufweisen und die Gruppen Ar^{d} bei jedem Auftreten gleich oder verschieden ausgewählt sind aus Strukturen der Formeln (Ar^{a}-1) bis (Ar^{a}-23), wobei in Strukturen der Formeln (Ar^{a}-8) bis (Ar^{a}-12) die Gruppe Y vorzugsweise O oder NR ist, wobei die Gruppen Ar^{d} bei jedem Auftreten gleich oder verschieden vorzugsweise ausgewählt sind aus Strukturen der Formeln (Ar^{a}-1), (Ar^{a}-2), (Ar^{a}-7) bis (Ar^{a}-18).

9. Verbindung nach einem oder mehreren der Ansprüche 1 bis 5, **dadurch gekennzeichnet, dass** die Verbindung mindestens eine Struktur gemäß Formel (I-1) und/oder Formel (I-2) umfasst, wobei die Gruppe Ar^{a} eine Elektronentransportgruppe umfasst oder für eine Elektronentransportgruppe steht, wobei Gruppe Ar^{a} vorzugsweise eine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe umfasst oder für eine der genannten Gruppen steht, wobei diese durch einen oder mehrere Reste R substituiert sein können, wobei Triazin-Gruppen besonders bevorzugt sind.

10. Verbindung nach Anspruch 9, **dadurch gekennzeichnet, dass** die Verbindung mindestens eine Struktur gemäß Formel (I-1) umfasst, wobei die Gruppe Ar^{b} eine Elektronentransportgruppe umfasst oder für eine Elektronentransportgruppe steht, wobei Gruppe Ar^{a} vorzugsweise eine Pyridin-, Pyrimidin-, Pyrazin-, Pyridazin-, Triazin-, Chinazolin-, Chinoxalin-, Chinolin-, Isochinolin-, Imidazol- und/oder Benzimidazol-Gruppe umfasst oder für eine der genannten Gruppen steht, wobei diese durch einen oder mehrere Reste R substituiert sein können, wobei Triazin-Gruppen besonders bevorzugt sind.

11. Verbindung nach Anspruch 9, **dadurch gekennzeichnet, dass** die Verbindung mindestens eine Struktur gemäß Formel (I-1) umfasst, wobei die Gruppe Ar^{b} ausgewählt ist aus Strukturen der Formeln (Ar^{a}-1) bis (Ar^{a}-18), wobei in Strukturen der Formeln (Ar^{a}-8) bis (Ar^{a}-12) die Gruppe Y vorzugsweise O oder NR ist; wobei die Gruppe Ar^{a} vorzugsweise für eine Struktur der Formel Ar^{e}-Q steht, wobei die Symbole Ar^{e} und Q die in Anspruch 5 genannte Bedeutung aufweisen.

12. Verbindung nach einem oder mehreren der Ansprüche 1 bis 11, umfassend mindestens eine Struktur der Formeln (II-1) bis (II-154), vorzugsweise Verbindung gemäß einer der Formeln (II-1) bis (II-154), 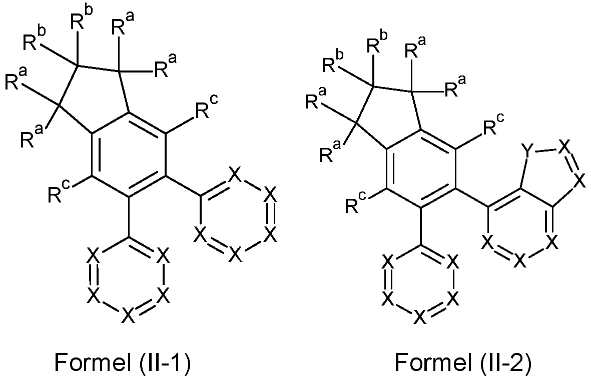 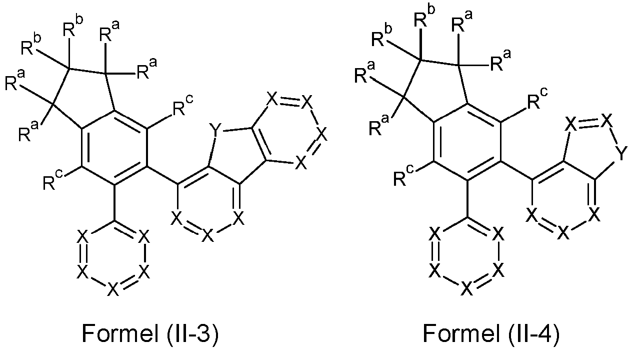 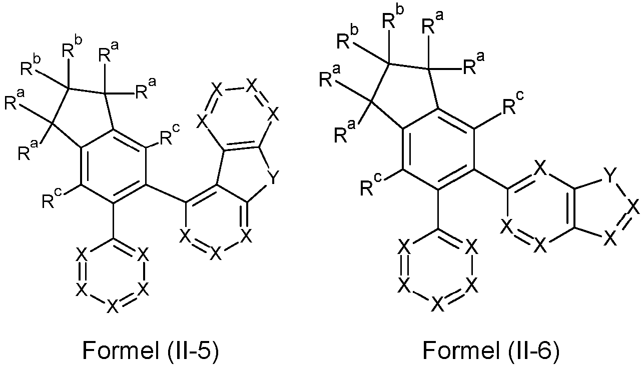 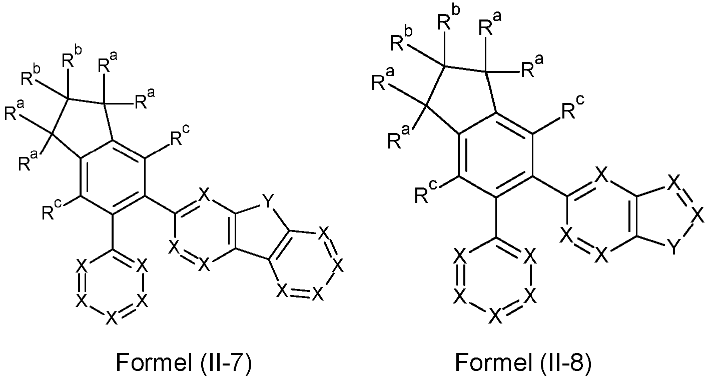 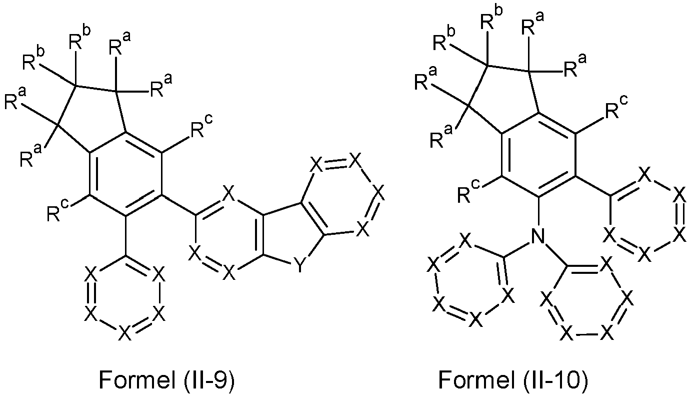 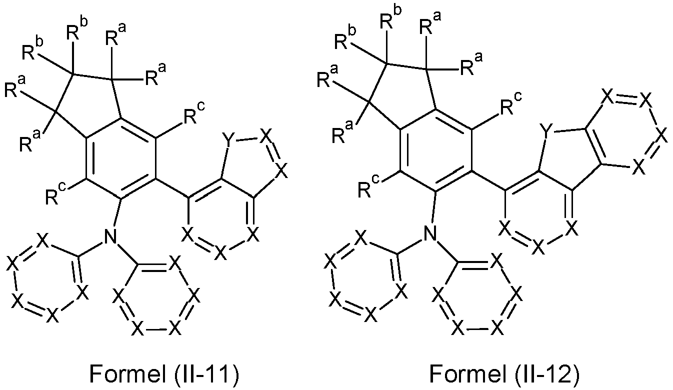 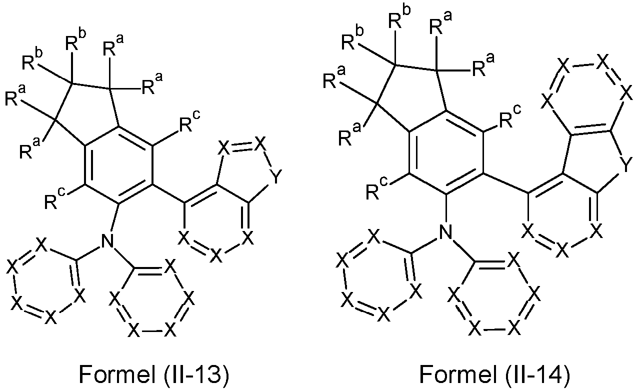 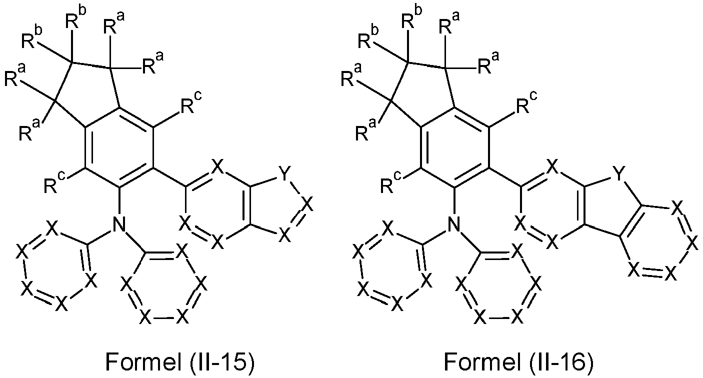 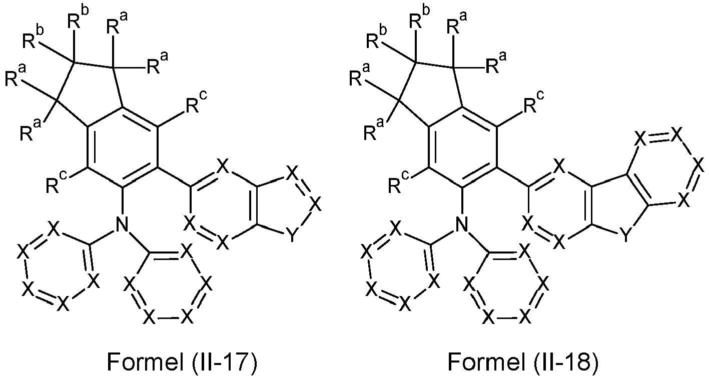 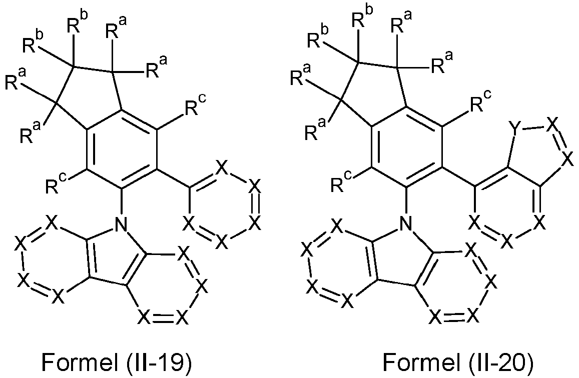 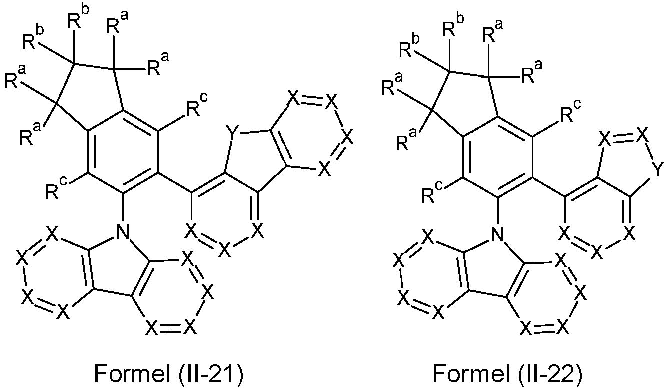 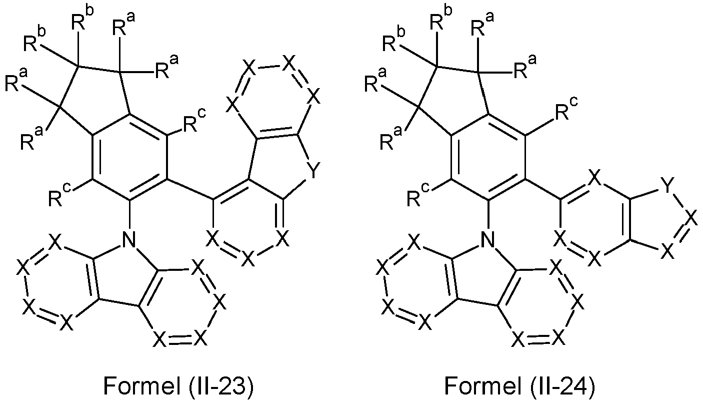 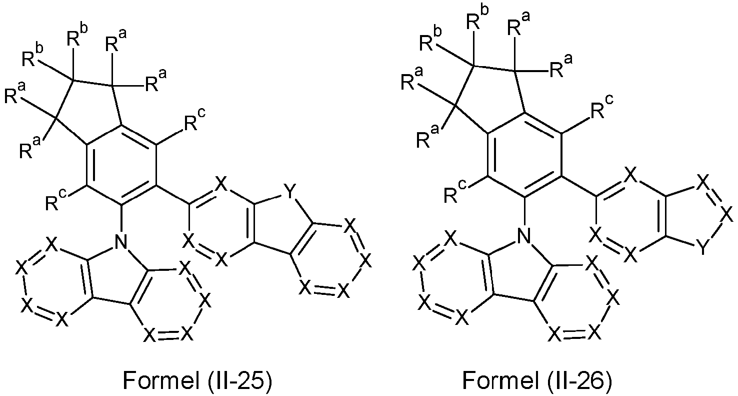 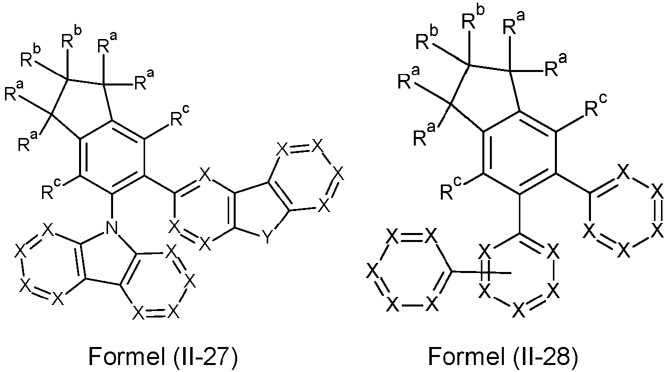 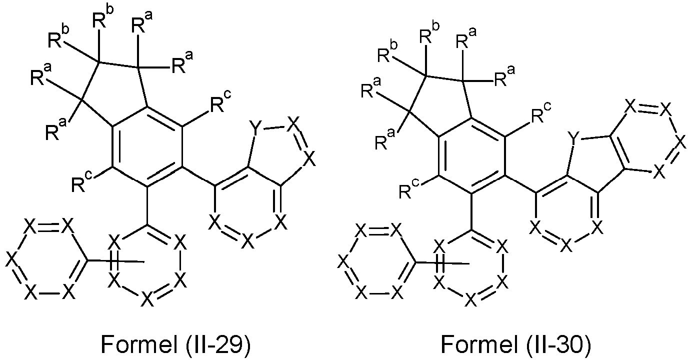 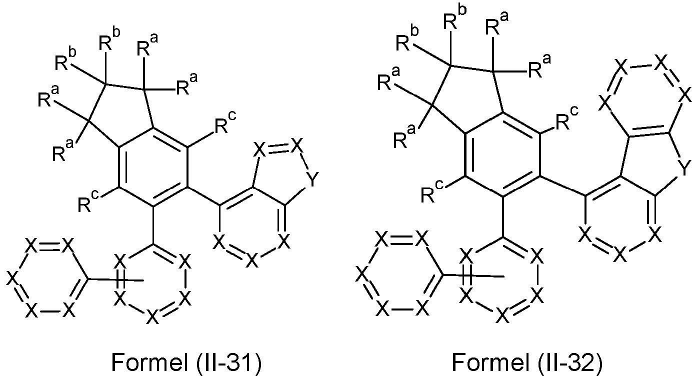 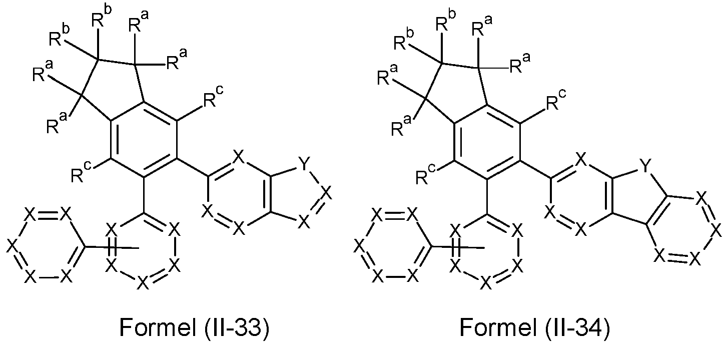 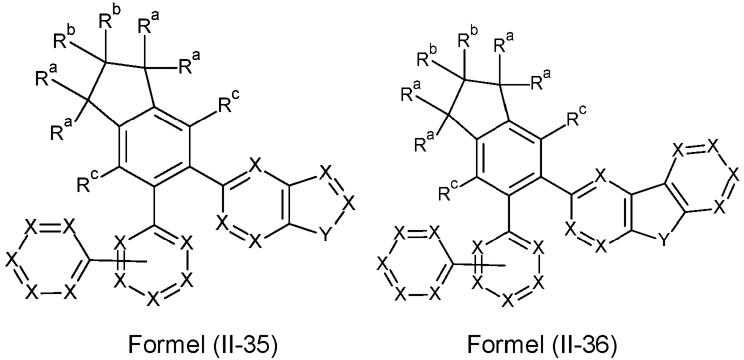 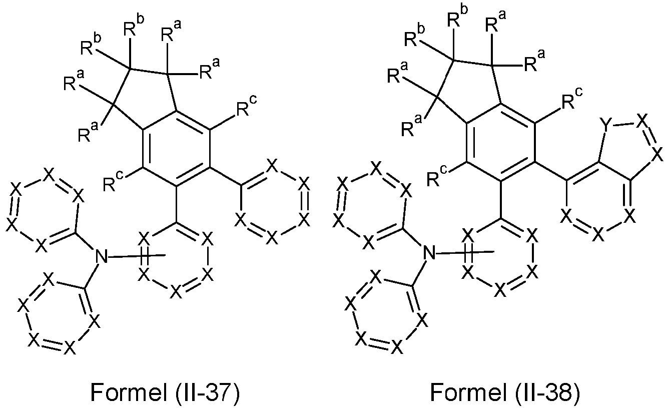 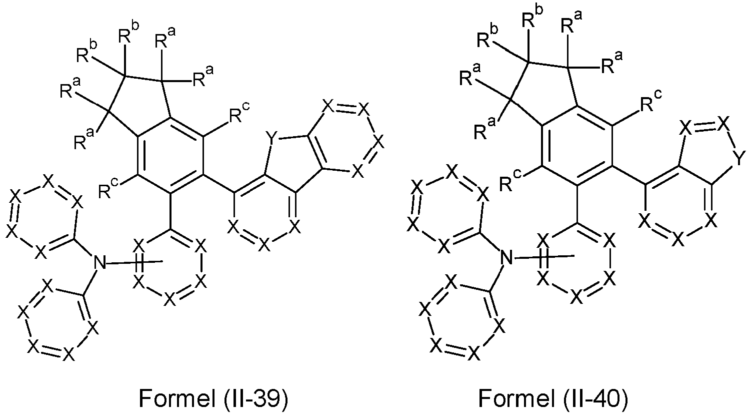 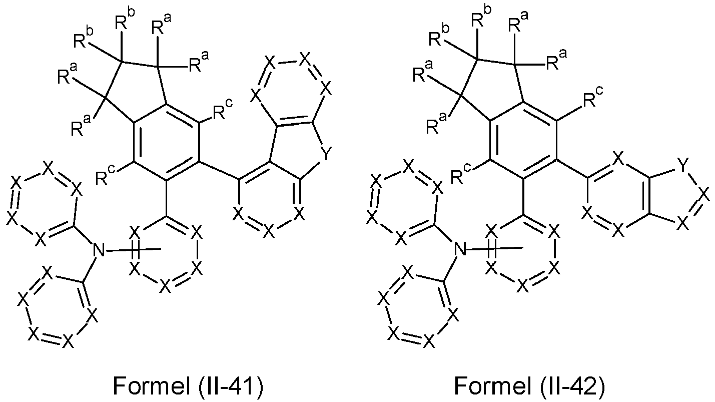 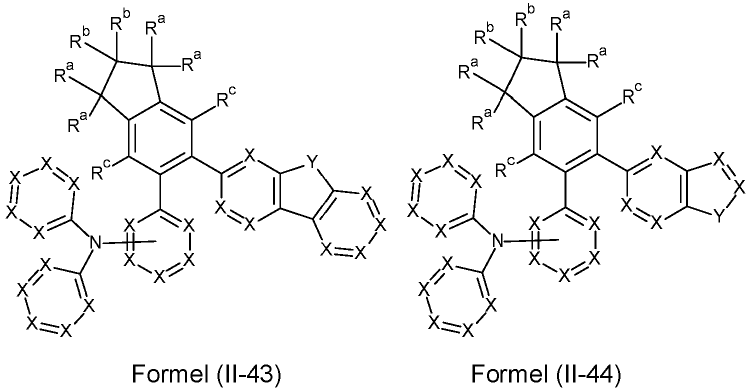 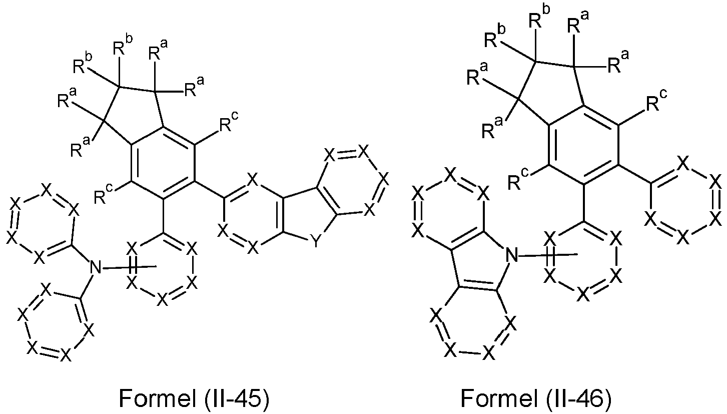 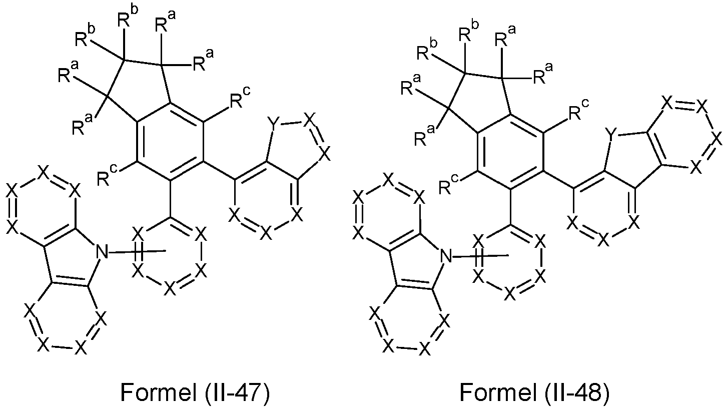 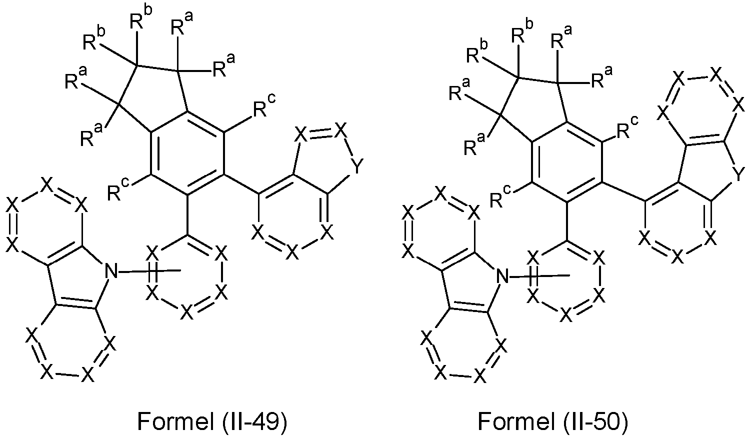 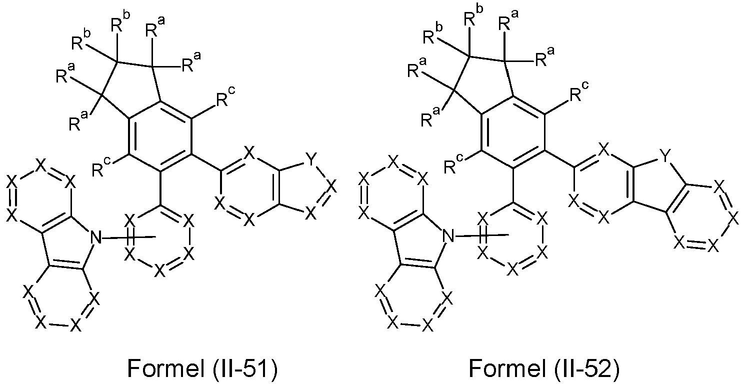 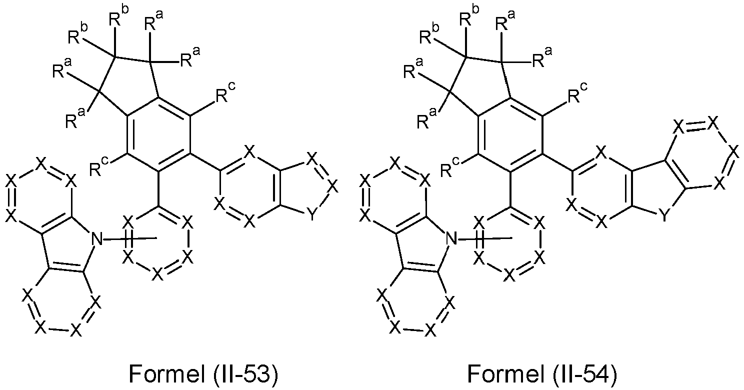 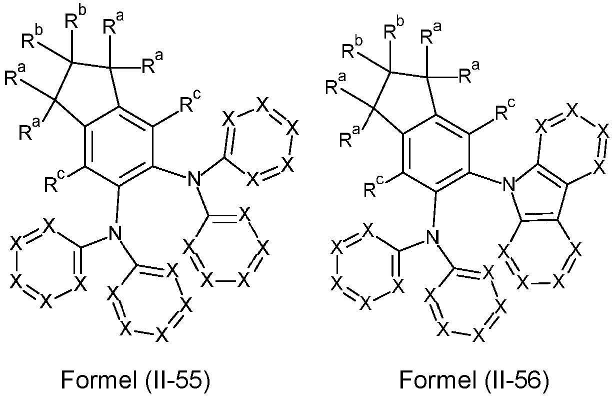 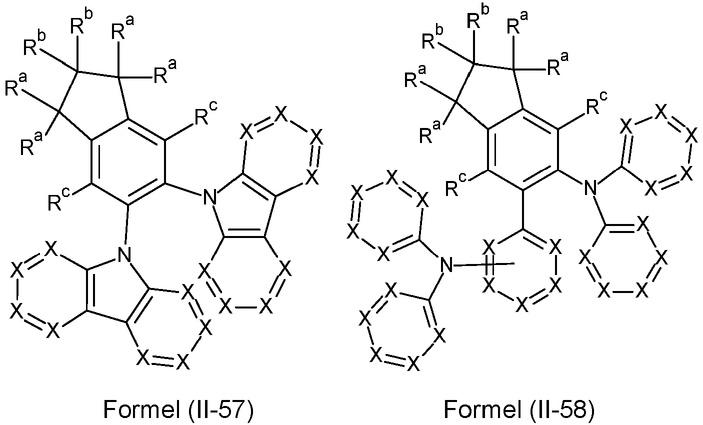 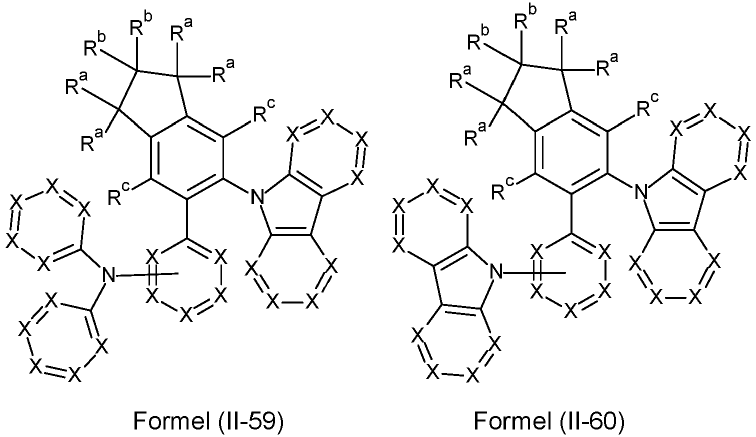 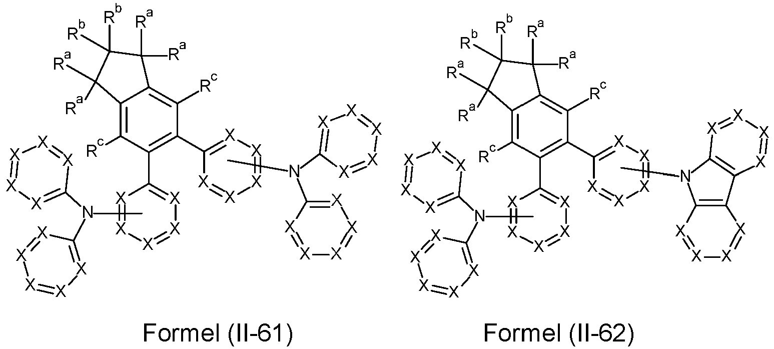 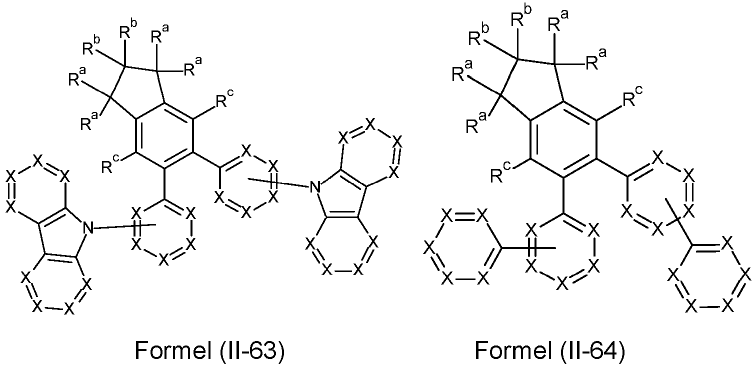 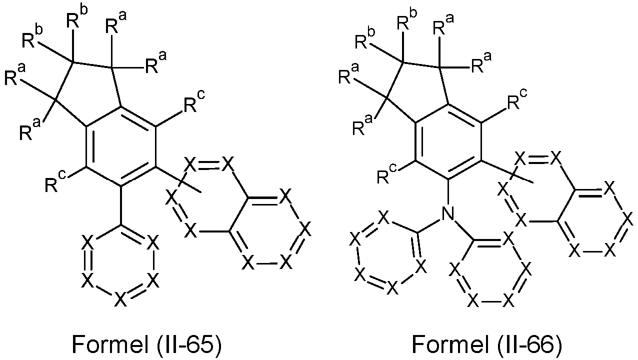 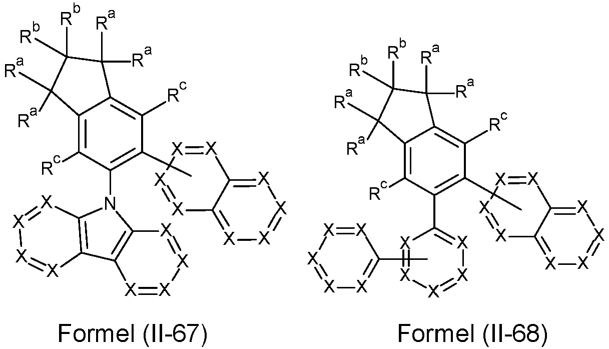 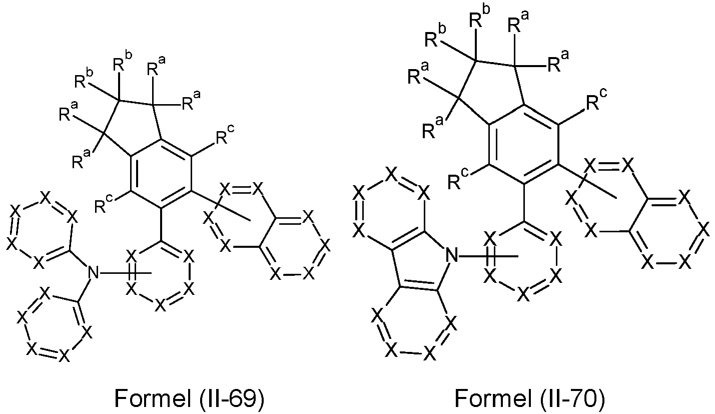 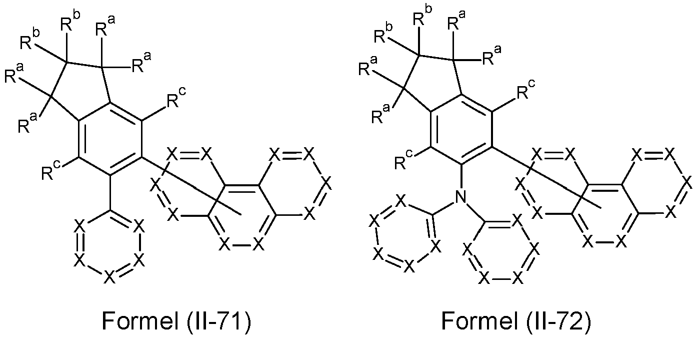 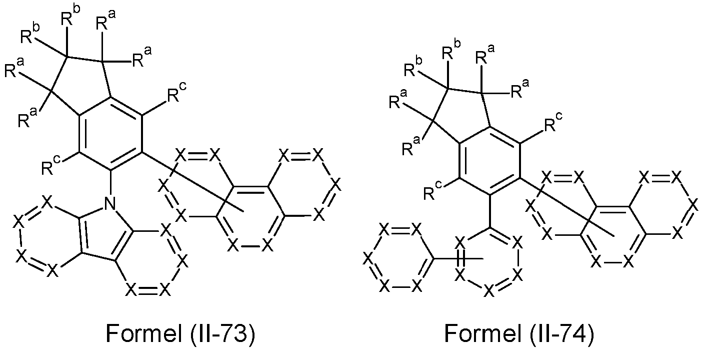 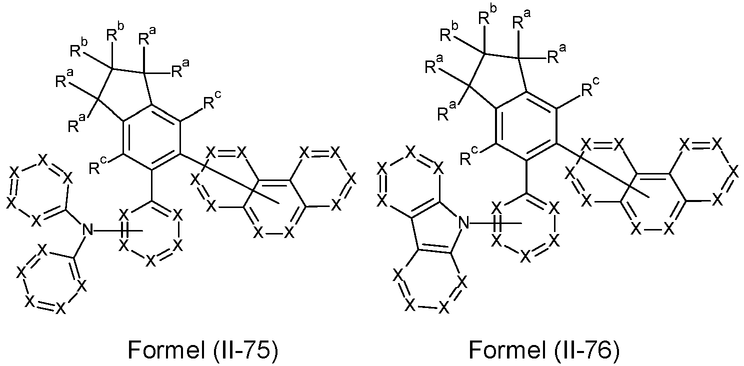 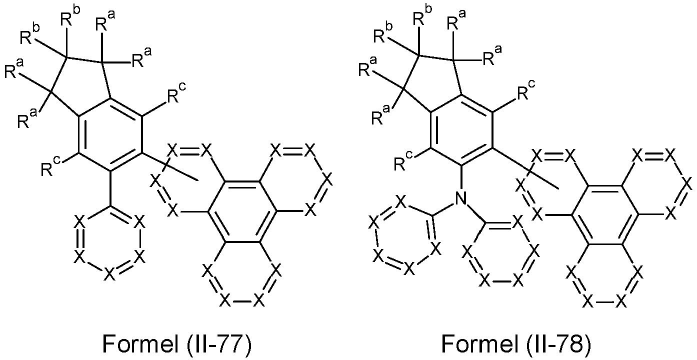 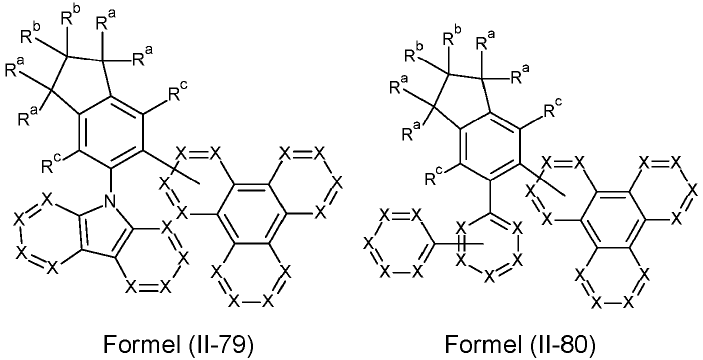 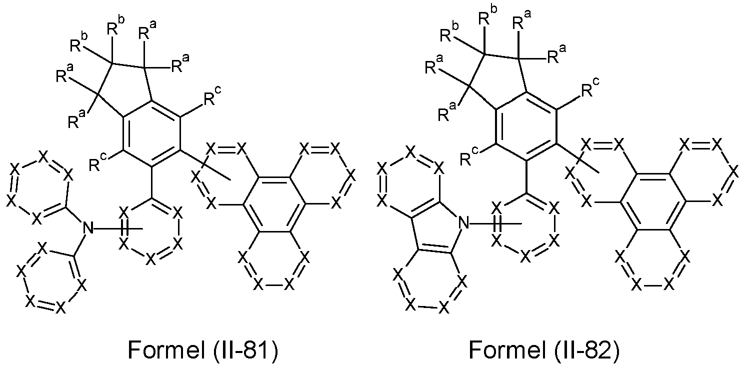 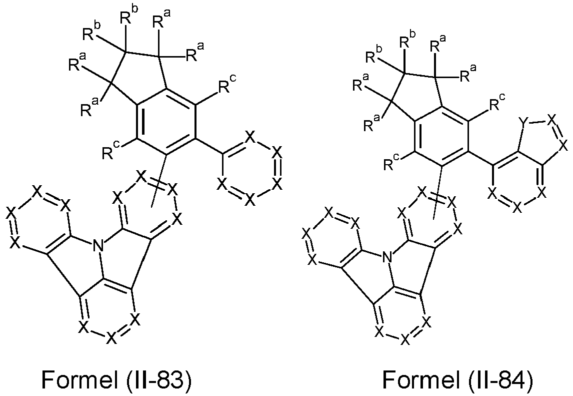 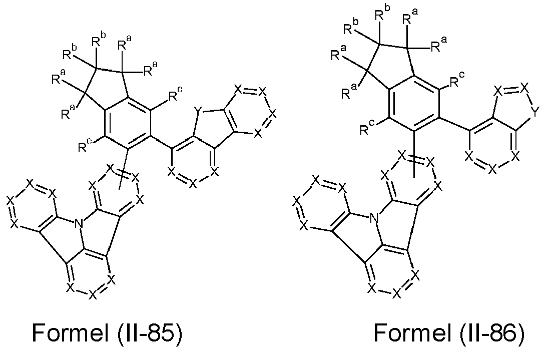 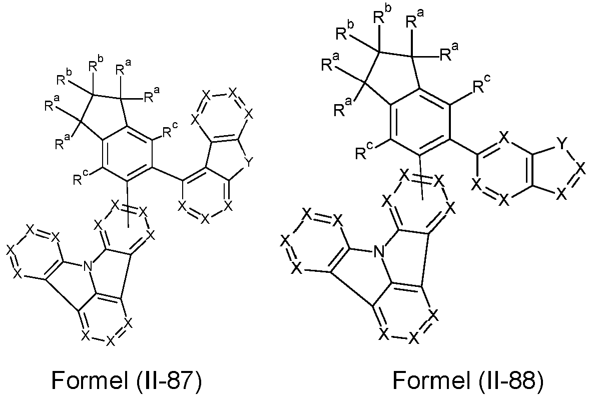 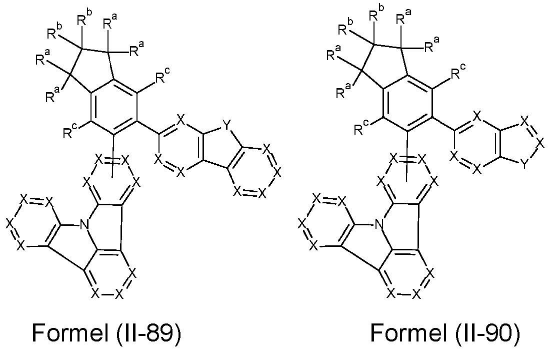 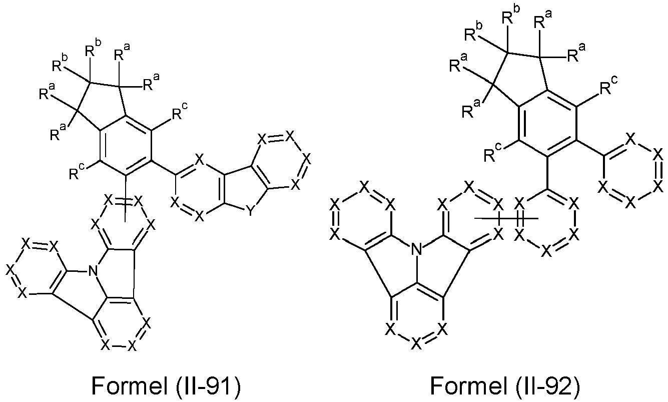 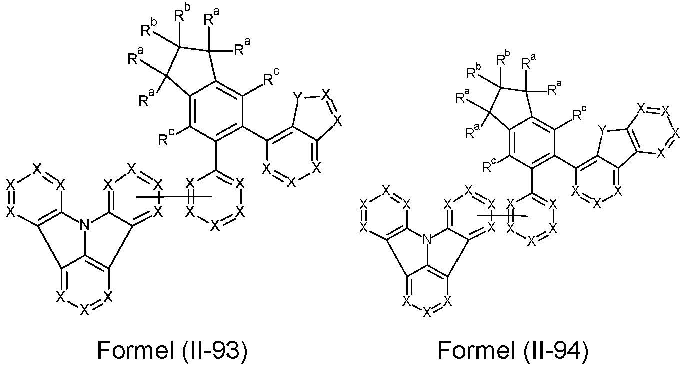 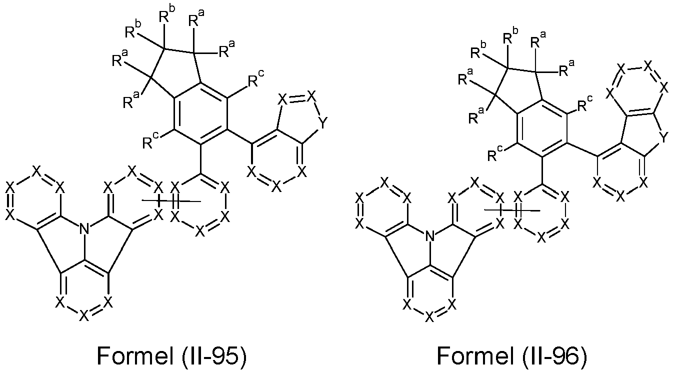 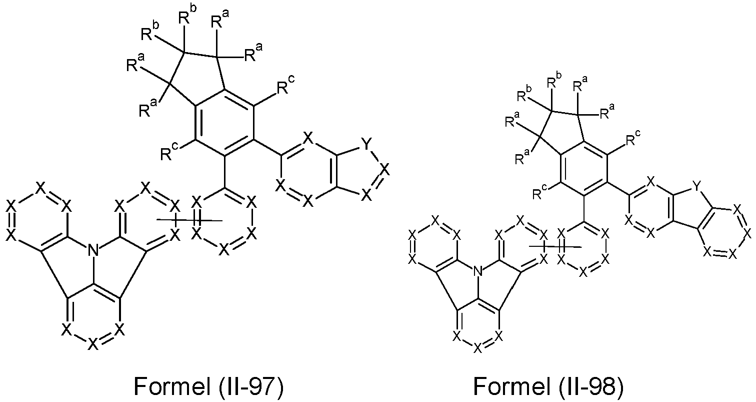 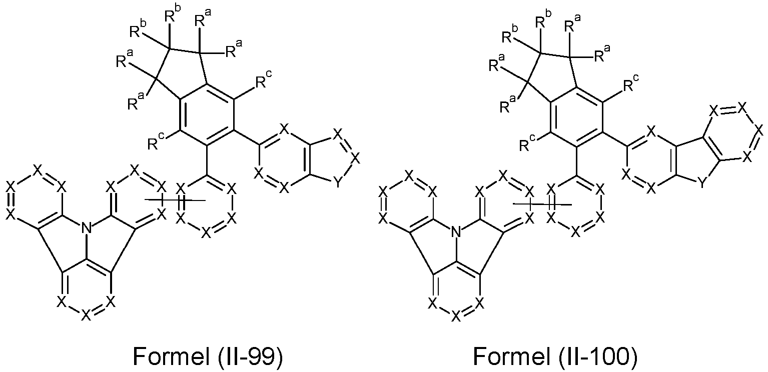 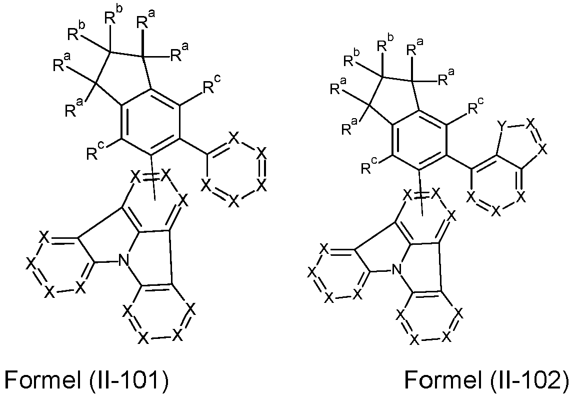 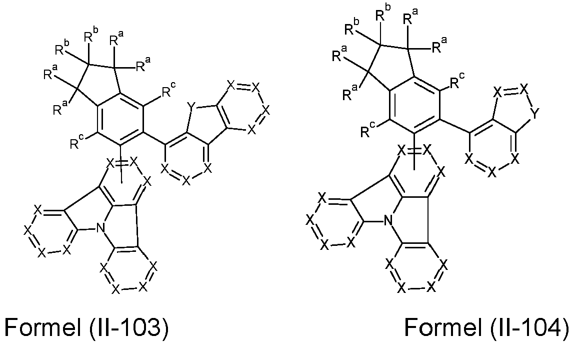 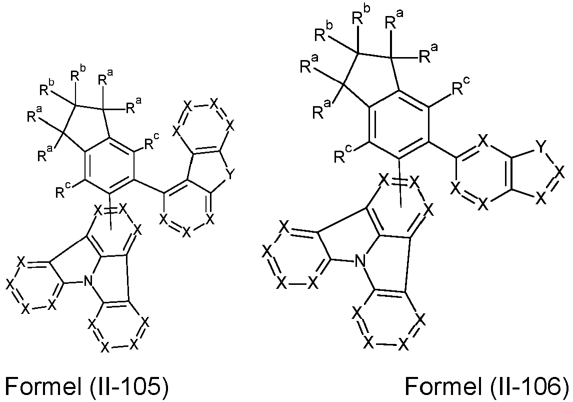 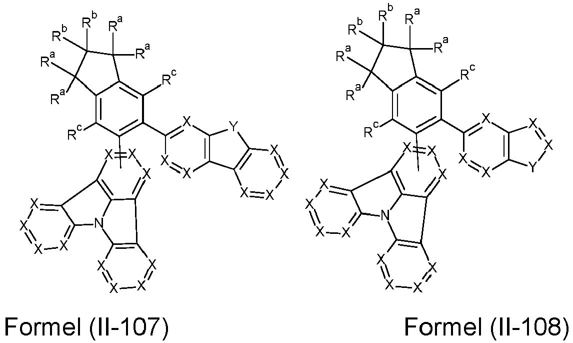 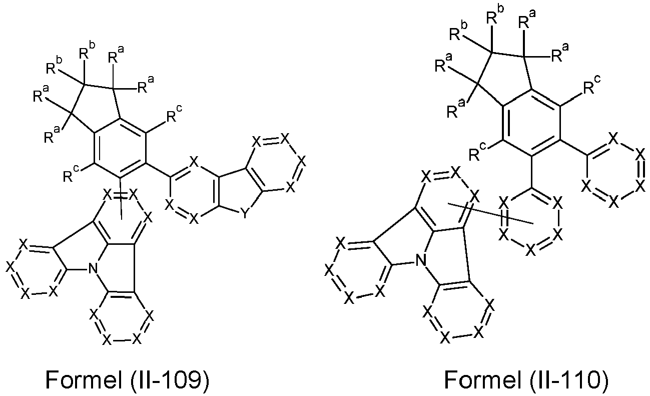 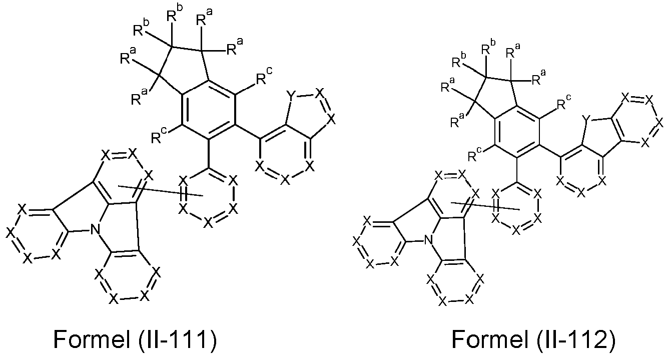 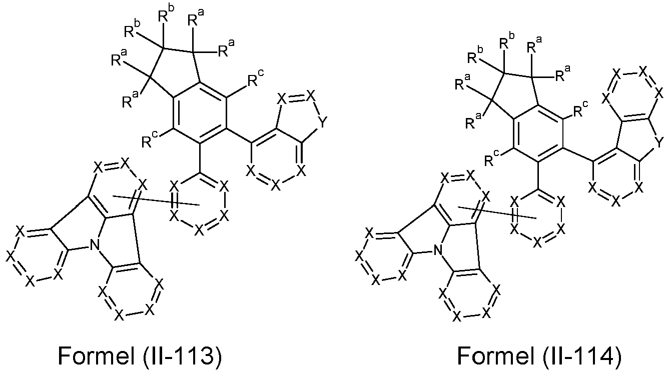 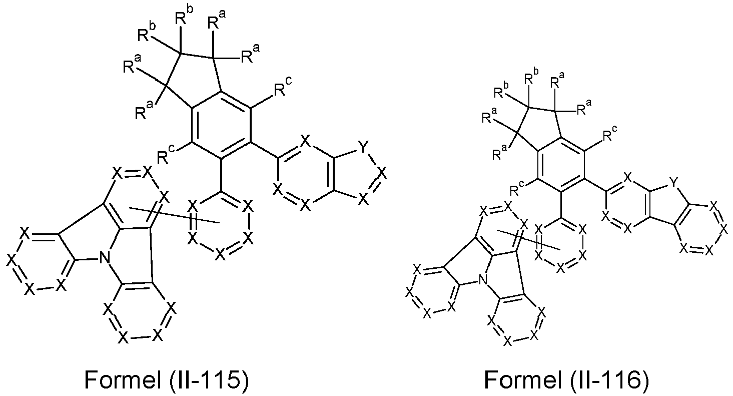 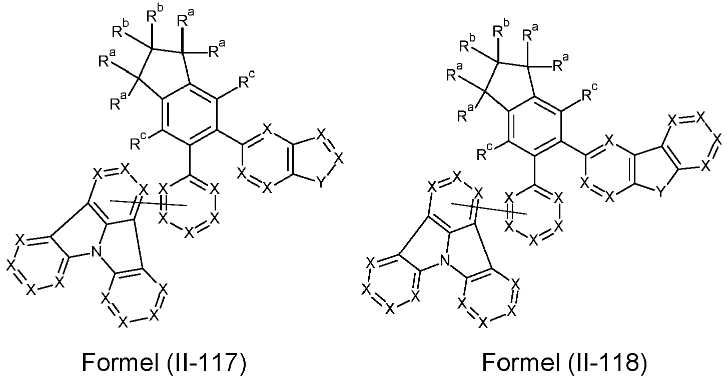 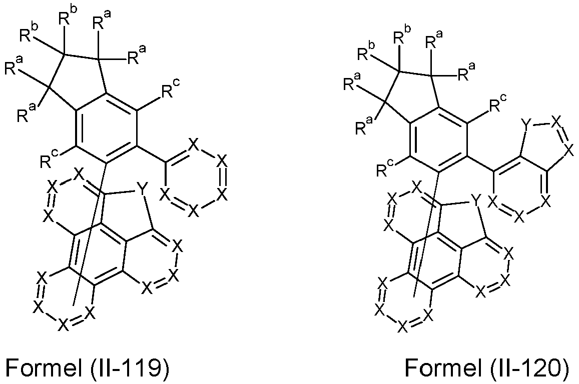 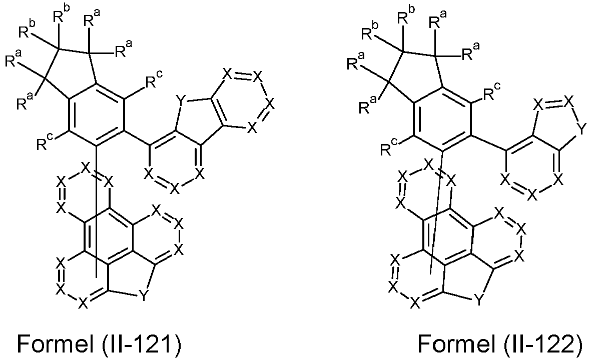 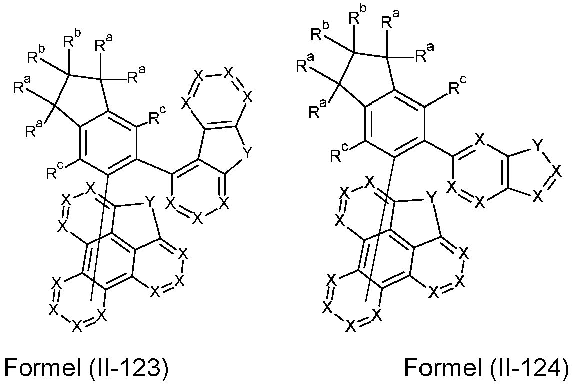 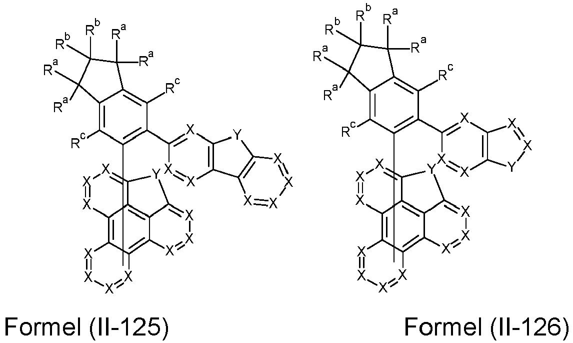 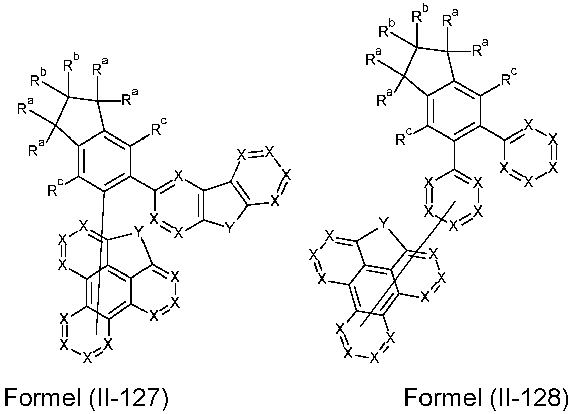 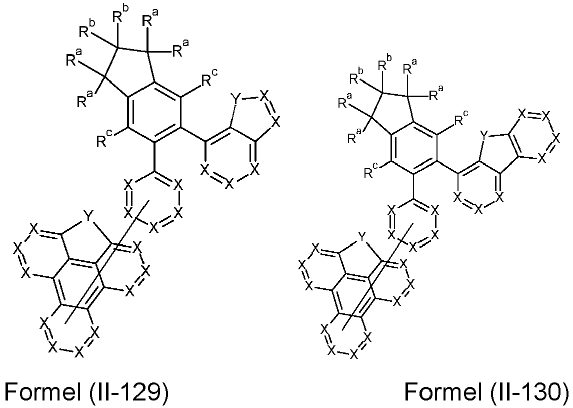 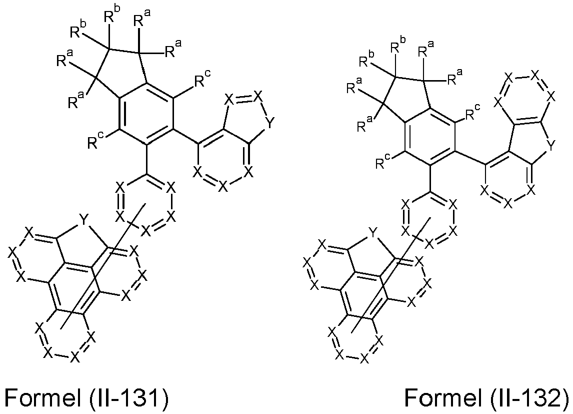 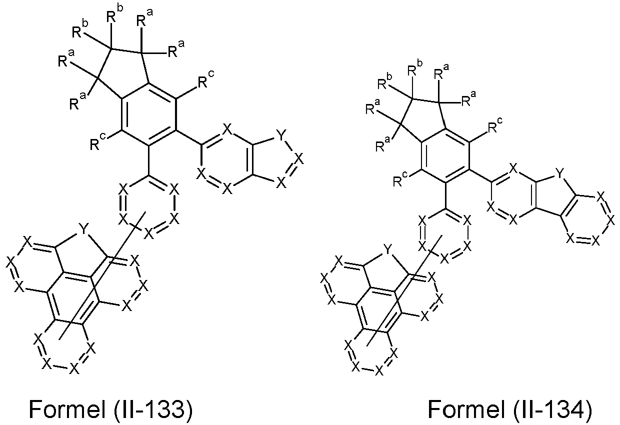 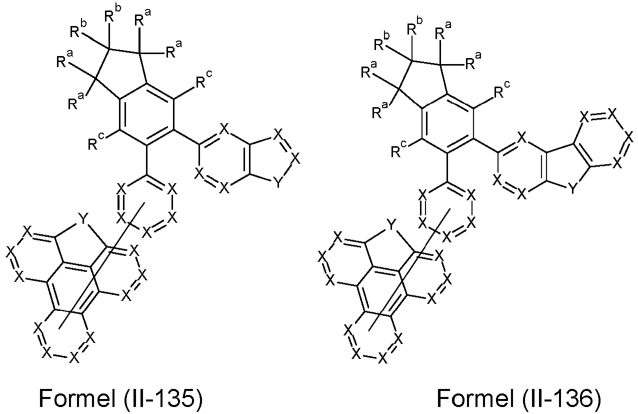 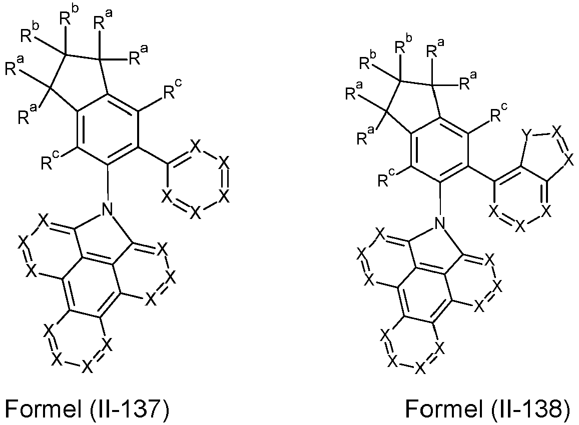 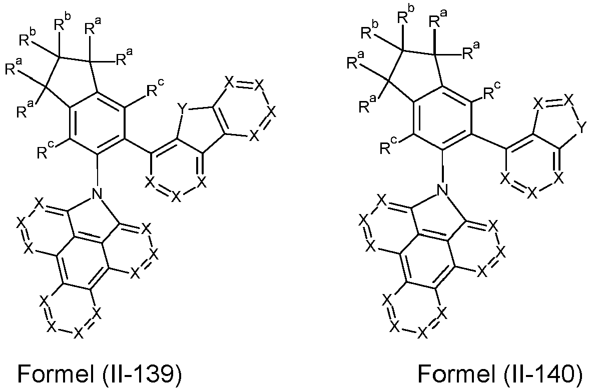 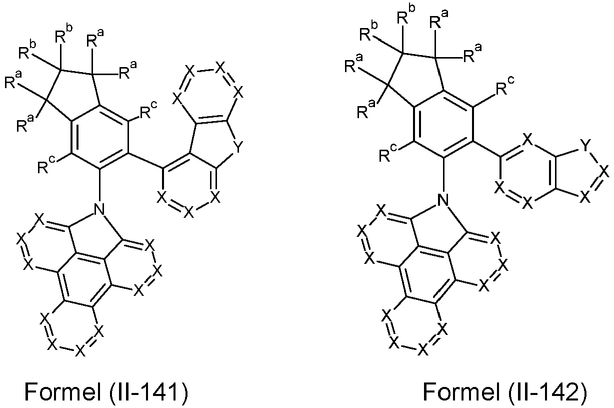 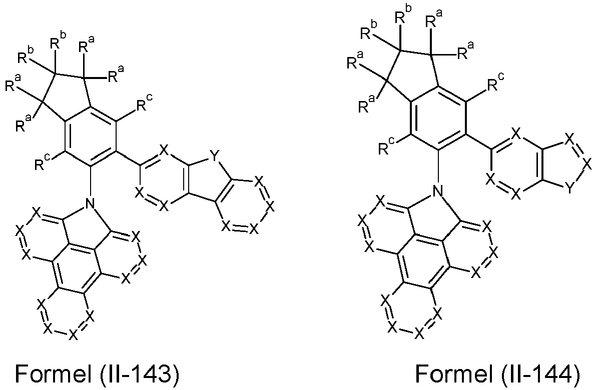 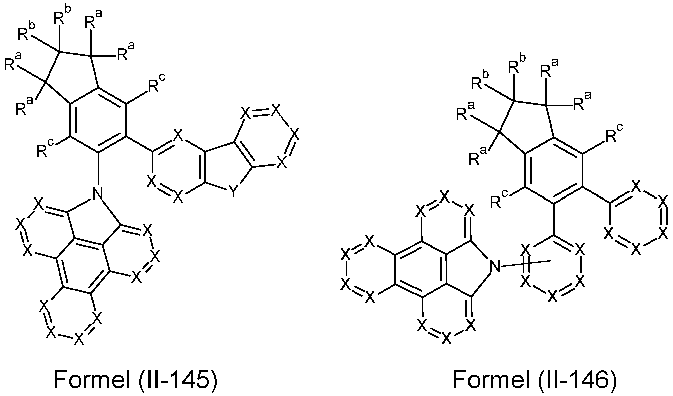 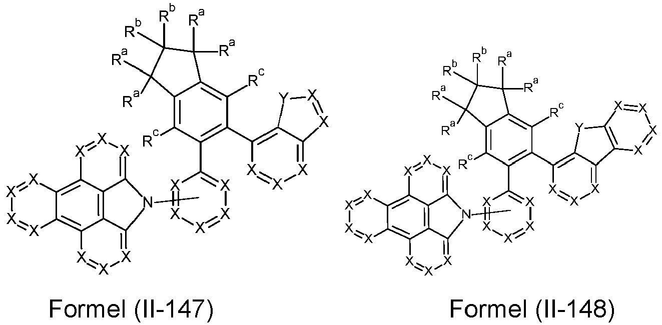 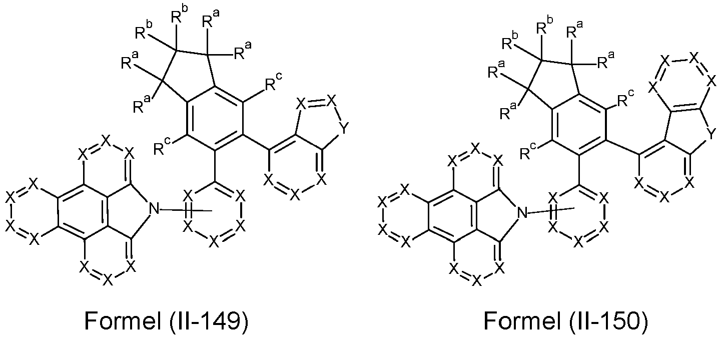 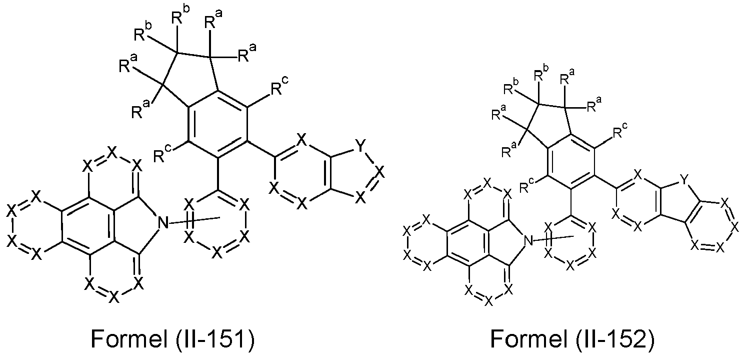 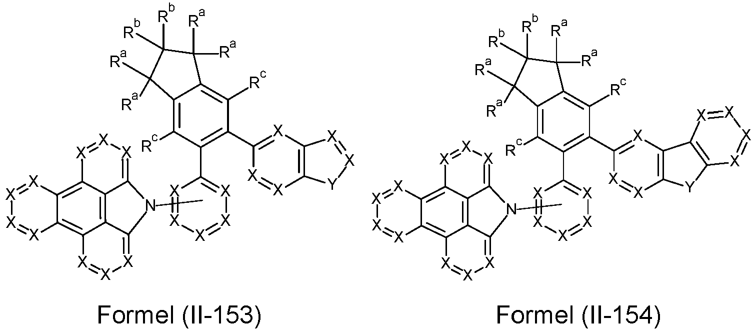 wobei die Symbole R, R^{a}, R^{b} und R^{c} die in Anspruch 1 genannten Bedeutungen aufweisen und für die weiteren Symbole gilt:
X steht bei jedem Auftreten gleich oder verschieden für N, CR oder C, für den Fall, dass eine Gruppe an die Struktur bindet; und
Y steht für O, S, NR oder C(R)₂, vorzugsweise O, NR oder C(R)₂.

13. Verbindung nach einem oder mehreren der Ansprüche 1 bis 12, umfassend mindestens eine Struktur der Formeln (III-1) bis (III-60), vorzugsweise Verbindung gemäß einer der Formeln (III-1) bis (III-60), 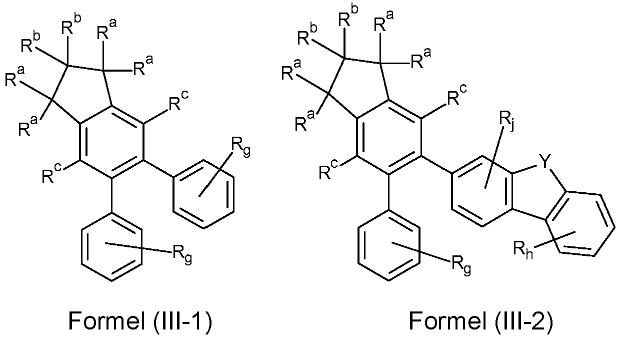 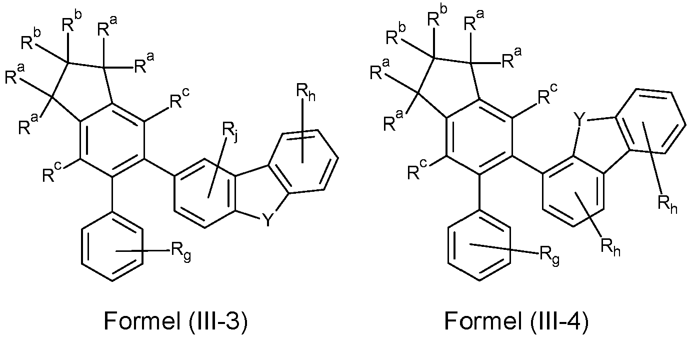 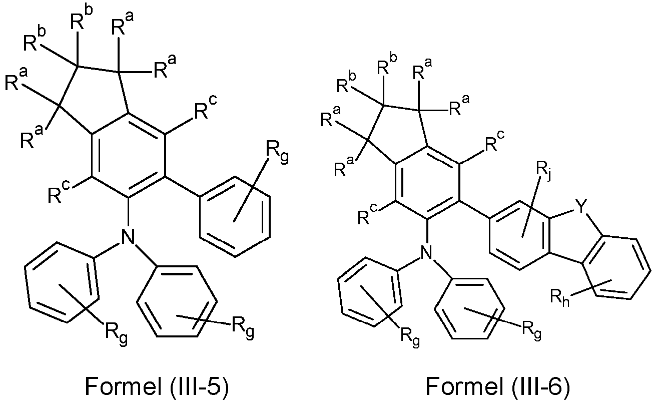 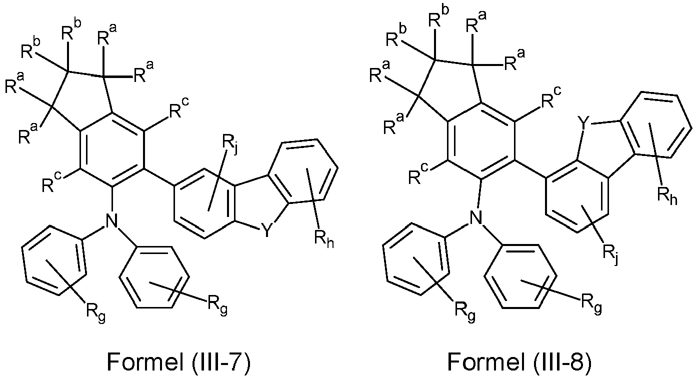 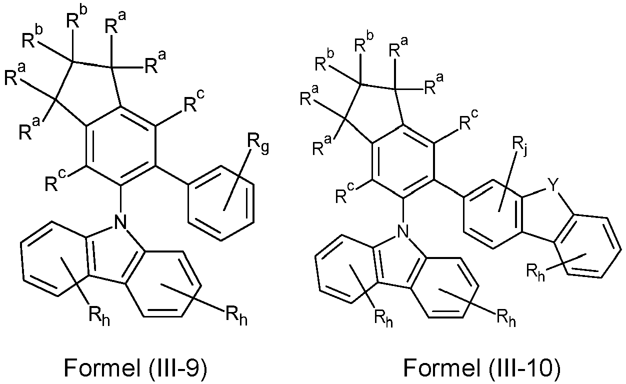 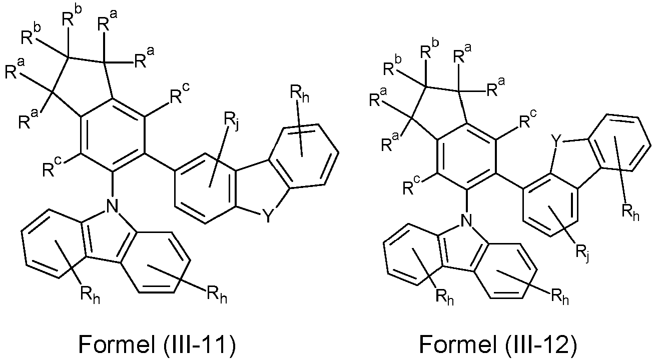 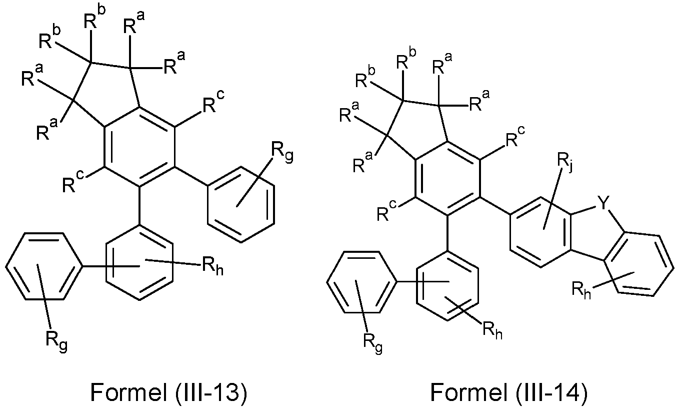 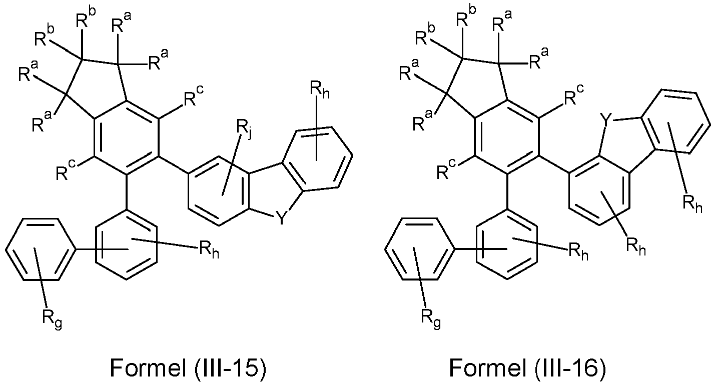 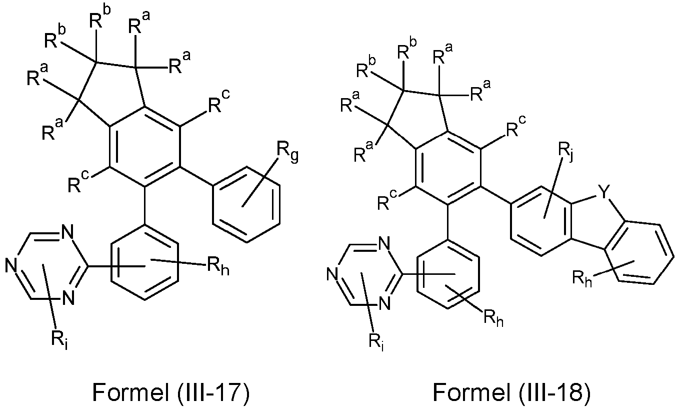 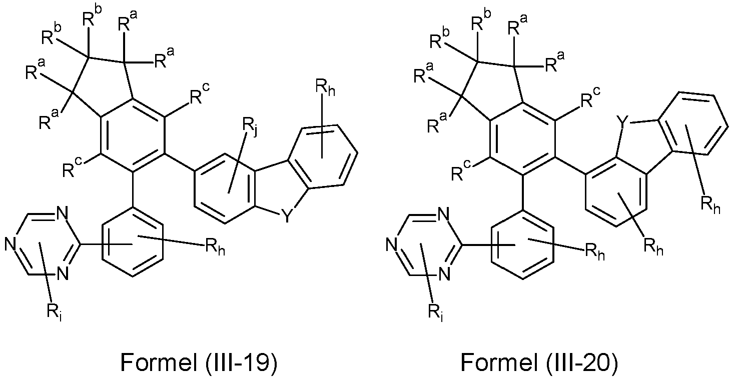 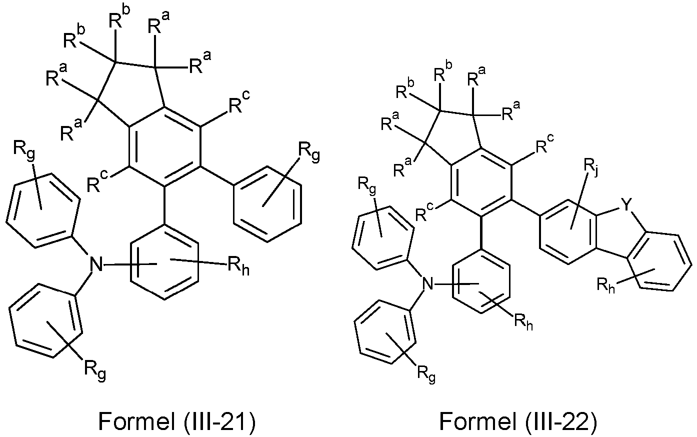 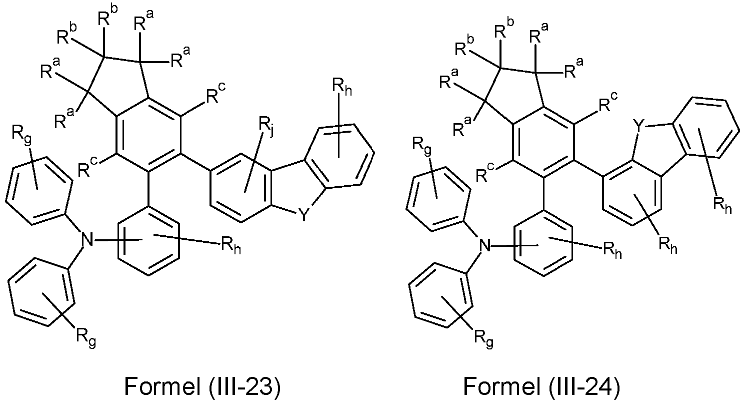 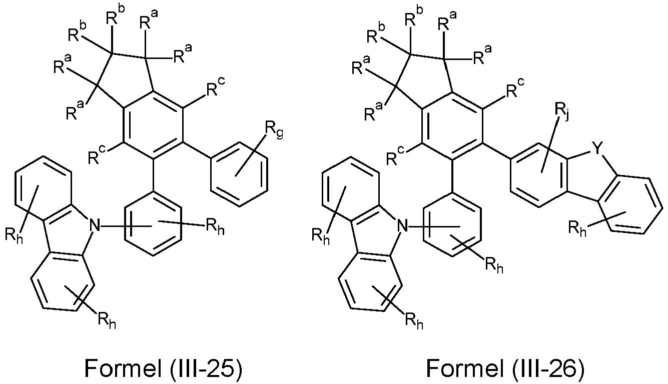 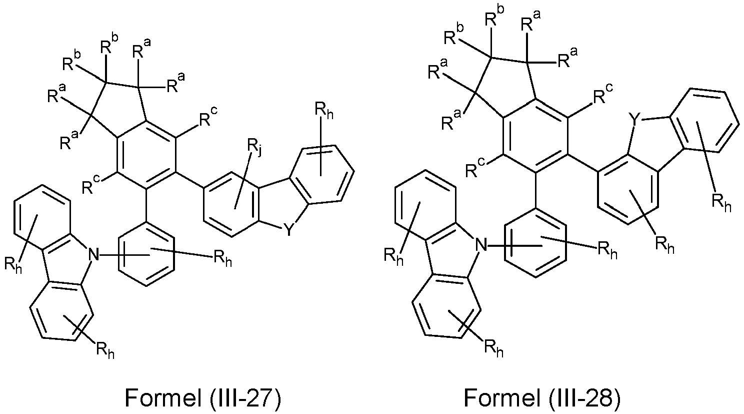 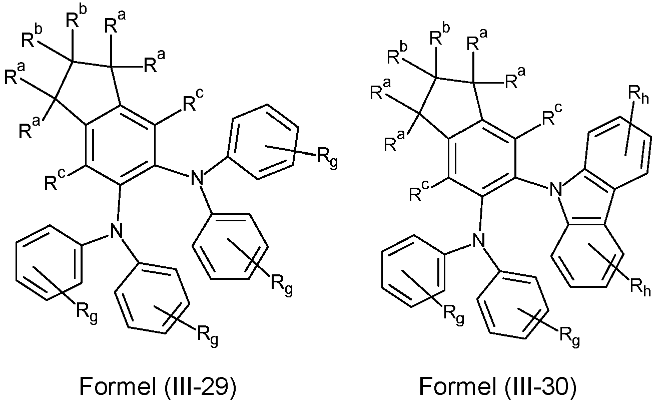 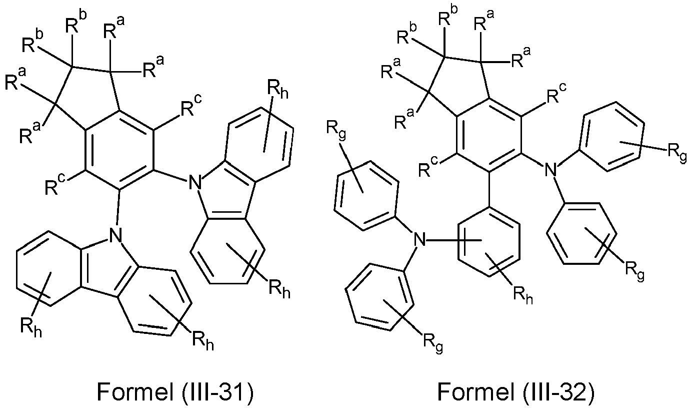 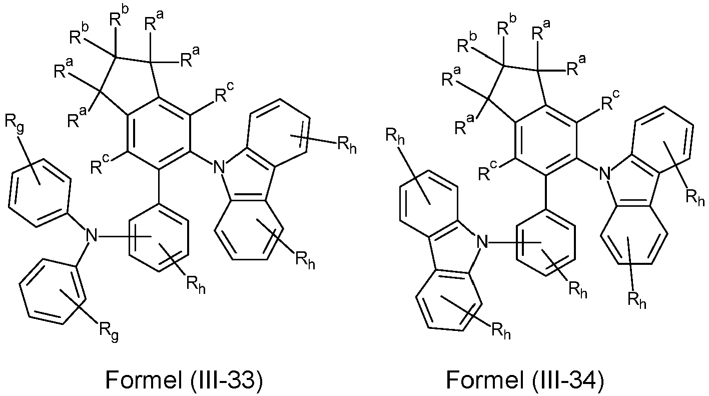 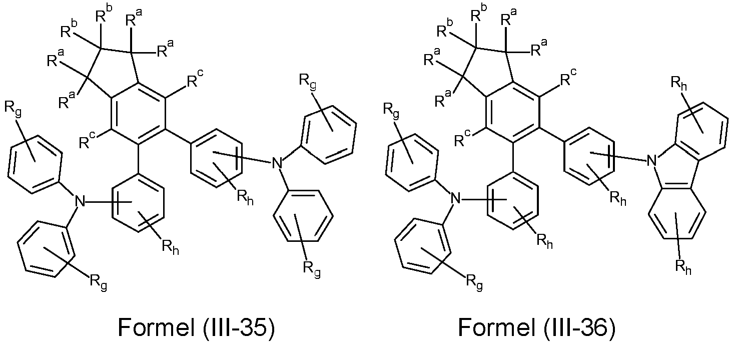 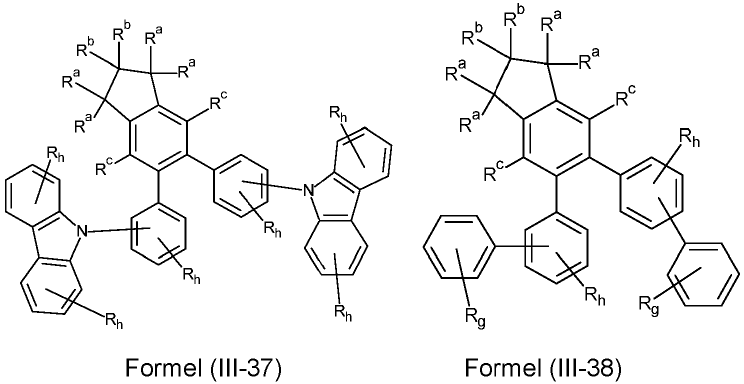 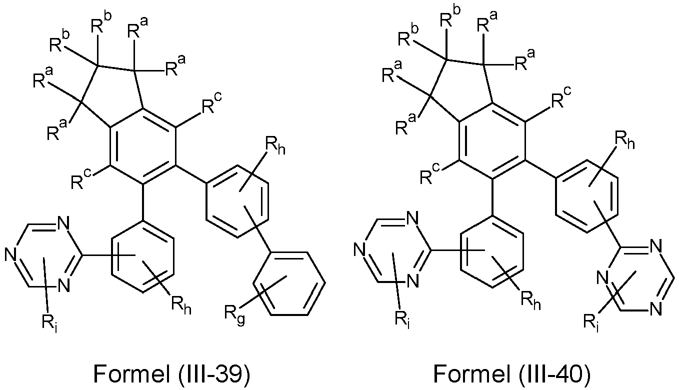 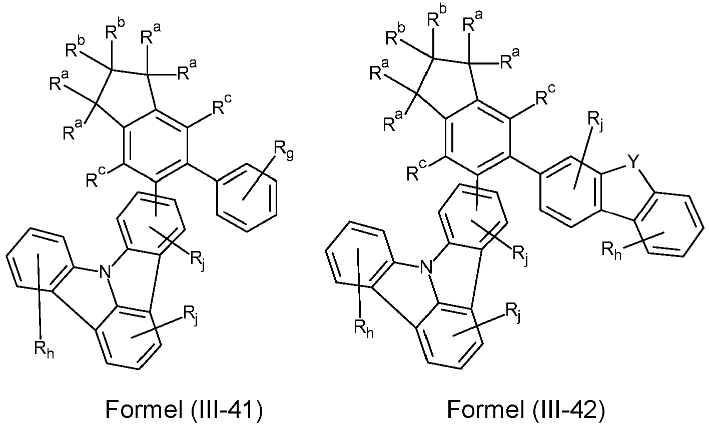 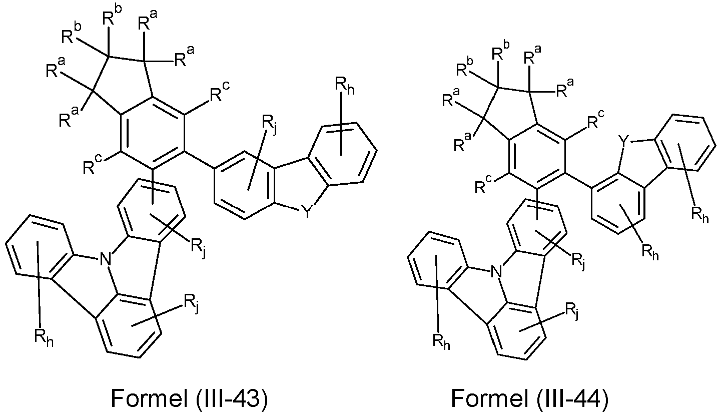 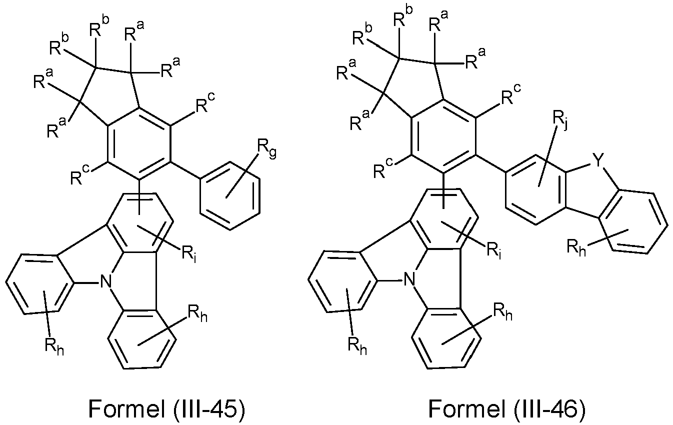 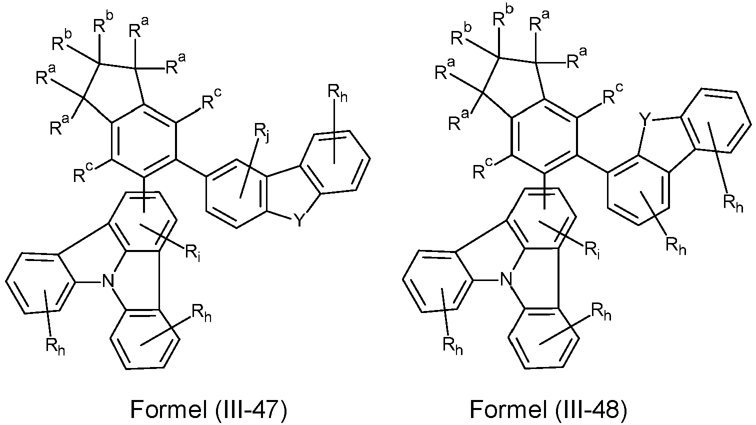 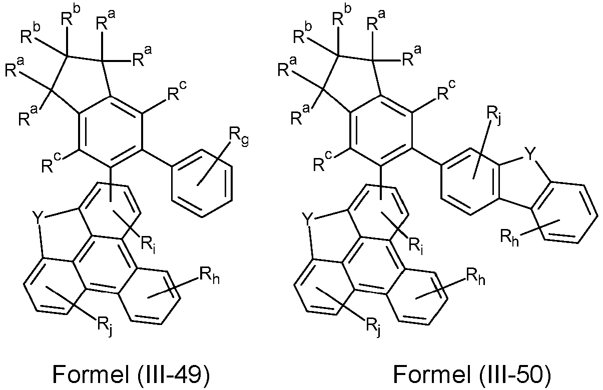 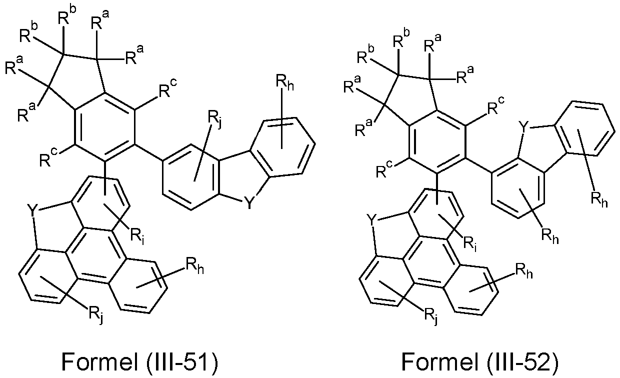 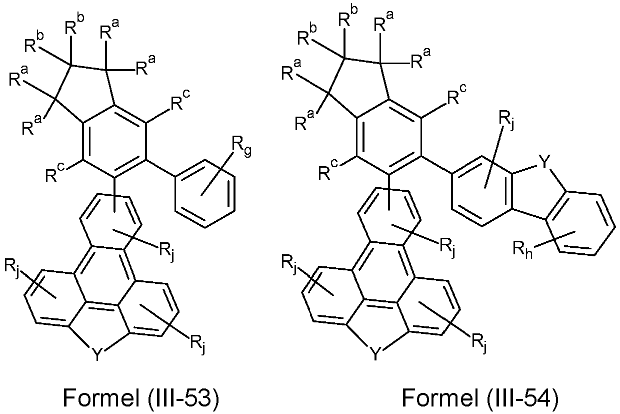 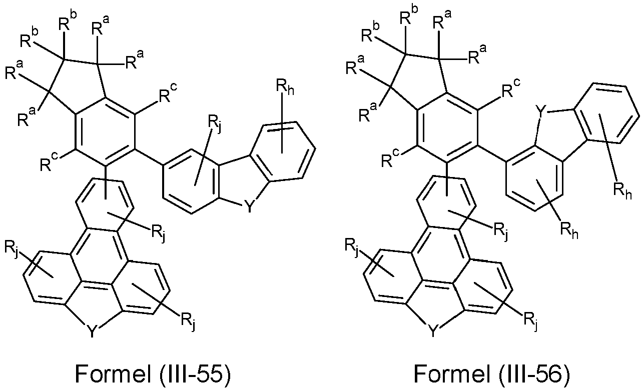 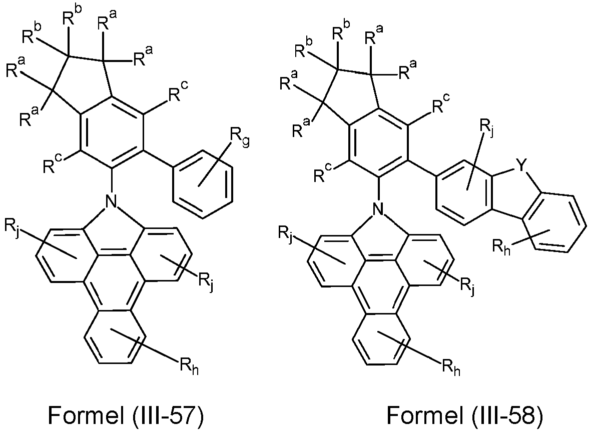 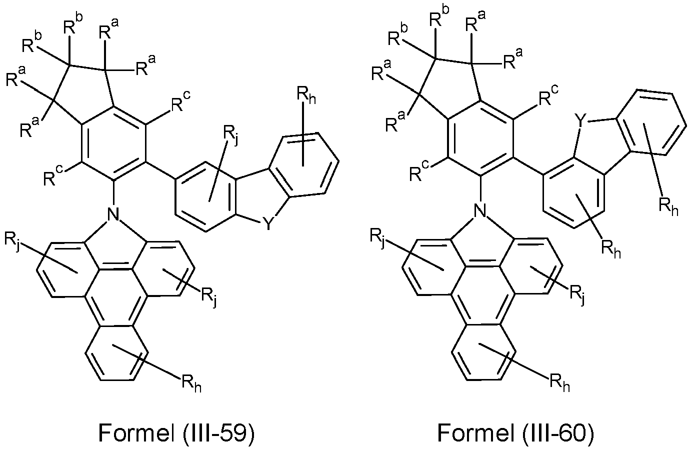 wobei die Symbole R, R^{a}, R^{b} und R^{c} die in Anspruch 1 genannten Bedeutungen aufweisen und für die verwendeten Symbole gilt:
Y ist O, S, NR oder C(R)₂, vorzugsweise O, NR oder C(R)₂;
i ist bei jedem Auftreten unabhängig 0, 1 oder 2;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5.

14. Oligomer, Polymer oder Dendrimer enthaltend eine oder mehrere Verbindungen nach einem der Ansprüche 1 bis 13, wobei statt eines Wasserstoffatoms oder eines Substituenten eine oder mehrere Bindungen der Verbindungen zum Polymer, Oligomer oder Dendrimer vorhanden sind.

15. Formulierung, enthaltend mindestens eine Verbindung nach einem oder mehreren der Ansprüche 1 bis 13 oder ein Oligomer, Polymer oder Dendrimer nach Anspruch 14 und mindestens eine weitere Verbindung, wobei die weitere Verbindung bevorzugt ausgewählt ist aus einem oder mehreren Lösemitteln.

16. Zusammensetzung, enthaltend mindestens eine Verbindung nach einem oder mehreren der Ansprüche 1 bis 13 oder ein Oligomer, Polymer oder Dendrimer nach Anspruch 14 und mindestens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien, vorzugsweise Hostmaterialien.

17. Verfahren zur Herstellung einer Verbindung nach einem oder mehreren der Ansprüche 1 bis 13, **dadurch gekennzeichnet, dass** eine Phenylverbindung, an die eine Cyclopentylgruppe kondensiert ist, synthetisiert wird und mindestens ein aromatischer oder heteroaromatischer Rest eingeführt wird, vorzugsweise mittels einer nukleophilen aromatischen Substitutionsreaktion oder einer Kupplungsreaktion.

18. Verwendung einer Verbindung nach einem oder mehreren der Ansprüche 1 bis 13 oder ein Oligomer, Polymer oder Dendrimer nach Anspruch 14 in einer elektronischen Vorrichtung, vorzugsweise als Hostmaterial, Elektronentransportmaterial, Elektroneninjektionsmaterial, Lochleitermaterial, Lochinjektionsmaterial, Elektronenblockiermaterial, Lochblockiermaterial.

19. Elektronische Vorrichtung enthaltend mindestens eine Verbindung nach einem oder mehreren der Ansprüche 1 bis 13 oder ein Oligomer, Polymer oder Dendrimer nach Anspruch 14.

## Claims

1. Compound comprising at least one structure of the formula (I), preferably compound of the formula (I), 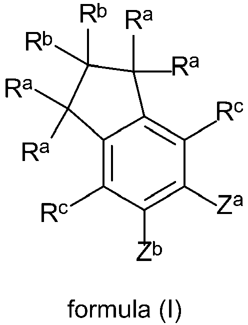 where the following applies to the symbols:
Z^{a} stands on each occurrence, identically or differently, for Ar^{a}, N(Ar^{c})₂ or (Ar)N(Ar^{c})₂;
Z^{b} stands on each occurrence, identically or differently, for Ar^{b}, N(Ar^{d})₂ or (Ar)N(Ar^{d})₂;
R^{a} is on each occurrence, identically or differently, a straight-chain alkyl, alkoxy or thioalkoxy group having 1 to 10 C atoms or a branched or cyclic alkyl, alkoxy or thioalkoxy group having 3 to 10 C atoms, which may in each case be substituted by one or more radicals R², preferably a straight-chain alkyl group having 1 to 10 C atoms or a branched or cyclic alkyl group having 3 to 10 C atoms, which may in each case be substituted by one or more radicals R², two or more, preferably adjacent substituents R^{a} may form a ring system with one another;
R^{b} is on each occurrence, identically or differently, a straight-chain alkyl, alkoxy or thioalkoxy group having 1 to 10 C atoms or a branched or cyclic alkyl, alkoxy or thioalkoxy group having 3 to 10 C atoms, which may in each case be substituted by one or more radicals R², preferably a straight-chain alkyl group having 1 to 10 C atoms or a branched or cyclic alkyl group having 3 to 10 C atoms, which may in each case be substituted by one or more radicals R², two adjacent substituents R^{b} may form a ring system with one another;
R^{c} is on each occurrence, identically or differently, H, D, a straight-chain alkyl, alkoxy or thioalkoxy group having 1 to 10 C atoms, preferably 1 to 6 C atoms, or a branched or cyclic alkyl, alkoxy or thioalkoxy group having 3 to 10 C atoms, which may in each case be substituted by one or more radicals R², preferably H, D, a straight-chain alkyl group having 1 to 10 C atoms or a branched or cyclic alkyl group having 3 to 10 C atoms, which may in each case be substituted by one or more radicals R², with preference H, D, a straight-chain alkyl group having 1 to 10 C atoms, preferably 1 to 6 C atoms, particularly preferably H, D;
Ar^{a}, Ar^{b}, Ar^{c}, Ar^{d} are on each occurrence, identically or differently, an aromatic or heteroaromatic ring system having 6 to 60 aromatic ring atoms, which may be substituted by one or more radicals R, two radicals Ar^{c}, Ar^{d} that are bonded to the same N atom may also be bridged to one another by a single bond or a bridge selected from B(R), C(R)₂, Si(R)₂, C=O, C=NR, C=C(R)₂, RC=CR, O, S, S=O, SO₂, N(R), P(R), P(=O)R and an ortho-linked phenylene group, which may be substituted by one or more radicals R, preferably selected from C(R)₂, O, N(R) and an ortho-linked phenylene group, which may be substituted by one or more radicals R, preferably Ar^{a}, Ar^{b}, Ar^{c}, Ar^{d} stand on each occurrence, identically or differently, for an aryl or heteroaryl group having 6 to 40 aromatic ring atoms, which may be substituted by one or more radicals R, two radicals Ar, Ar^{c}, Ar^{d} that are bonded to the same N atom may also be bridged to one another by a single bond or a bridge selected from B(R), C(R)₂, Si(R)₂, C=O, C=NR, C=C(R)₂, RC=CR, O, S, S=O, SO₂, N(R), P(R), P(=O)R and an ortho-linked phenylene group, which may be substituted by one or more radicals R, preferably selected from C(R)₂, O, N(R) and an ortho-linked phenylene group, which may be substituted by one or more radicals R;
Ar is on each occurrence, identically or differently, a connecting aromatic or heteroaromatic ring system having 5 to 60 aromatic ring atoms, which may be substituted by one or more radicals R, one radical Ar may also be bridged to one or both of the radicals Ar^{c}, Ar^{d} that are bonded to the same N atom by a single bond or a bridge selected from B(R), C(R)₂, Si(R)₂, C=O, C=NR, C=C(R)₂, RC=CR, O, S, S=O, SO₂, N(R), P(R), P(=O)R and an ortho-linked phenylene group, which may be substituted by one or more radicals R, preferably selected from C(R)₂, O, N(R) and an ortho-linked phenylene group, which may be substituted by one or more radicals R, preferably Ar stands on each occurrence, identically or differently, for an arylene or heteroarylene group having 5 to 40 aromatic ring atoms, which may be substituted by one or more radicals R, one radical Ar may also be bridged to one or both of the radicals Ar^{c}, Ar^{d} that are bonded to the same N atom by a single bond or a bridge selected from B(R), C(R)₂, Si(R)₂, C=O, C=NR, C=C(R)₂, RC=CR, O, S, S=O, SO₂, N(R), P(R), P(=O)R and an ortho-linked phenylene group, which may be substituted by one or more radicals R, preferably selected from C(R)₂, O, N(R) and an ortho-linked phenylene group, which may be substituted by one or more radicals R;
R is on each occurrence, identically or differently, H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, a straight-chain alkyl, alkoxy or thioalkoxy group having 1 to 40 C atoms or an alkenyl or alkynyl group having 2 to 40 C atoms or a branched or cyclic alkyl, alkoxy or thioalkoxy group having 3 to 20 C atoms, where the alkyl, alkoxy, thioalkoxy, alkenyl or alkynyl group may in each case be substituted by one or more radicals R¹, where one or more non-adjacent CH₂ groups may be replaced by R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO or SO₂, or an aromatic or heteroaromatic ring system having 5 to 60 aromatic ring atoms, which may in each case be substituted by one or more radicals R¹, or an aryloxy or heteroaryloxy group having 5 to 60 aromatic ring atoms, which may be substituted by one or more radicals R¹; two radicals R may also form a ring system with one another or a further group;
Ar' is on each occurrence, identically or differently, an aromatic or heteroaromatic ring system having 5 to 60 aromatic ring atoms, which may be substituted by one or more radicals R¹, two radicals Ar' that are bonded to the same C atom, Si atom, N atom, P atom or B atom may also be bridged to one another by a single bond or a bridge selected from B(R¹), C(R¹)₂, Si(R¹)₂, C=O, C=NR¹, C=C(R¹)₂, O, S, S=O, SO₂, N(R¹), P(R¹) and P(=O)R¹;
R¹ is on each occurrence, identically or differently, H, D, F, Cl, Br, I, CN, NO₂, N(Ar")₂, N(R²)₂, C(=O)Ar", C(=O)R², P(=O)(Ar")2, P(Ar')₂, B(Ar")₂, B(R²)₂, C(Ar")₃, C(R²)₃, Si(Ar")₃, Si(R²)₃, a straight-chain alkyl, alkoxy or thioalkoxy group having 1 to 40 C atoms or a branched or cyclic alkyl, alkoxy or thioalkoxy group having 3 to 40 C atoms or an alkenyl group having 2 to 40 C atoms, which may in each case be substituted by one or more radicals R², where one or more non-adjacent CH₂ groups may be replaced by -R²C=CR²-, -C≡C-, Si(R²)₂, C=O, C=S, C=Se, C=NR², -C(=O)O-, -C(=O)NR²-, NR², P(=O)(R²), -O-, -S-, SO or SO₂ and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or an aralkyl or heteroaralkyl group having 5 to 60 aromatic ring atoms, which may be substituted by one or more radicals R², or a combination of these systems; two or more, preferably adjacent radicals R¹ may form a ring system with one another, one or more radicals R¹ may form a ring system with a further part of the compound;
Ar" is on each occurrence, identically or differently, an aromatic or heteroaromatic ring system having 5 to 30 aromatic ring atoms, which may be substituted by one or more radicals R², two radicals Ar" that are bonded to the same C atom, Si atom, N atom, P atom or B atom may also be bridged to one another by a single bond or a bridge selected from B(R²), C(R²)₂, Si(R²)₂, C=O, C=NR², C=C(R²)₂, O, S, S=O, SO₂, N(R²), P(R²) and P(=O)R²;
R² is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, Cl, Br, I or CN and which may be substituted by one or more alkyl groups, each having 1 to 4 carbon atoms, two or more, preferably adjacent substituents R² may form a ring system with one another;
where the groups Z^{a} and Z^{b} do not form a ring system.

2. Compound according to Claim 1, comprising at least one structure of the formulae (1-1) to (I-6), preferably compound of one of the formulae (I-1) to (I-6), 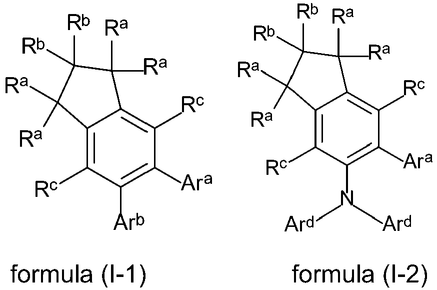 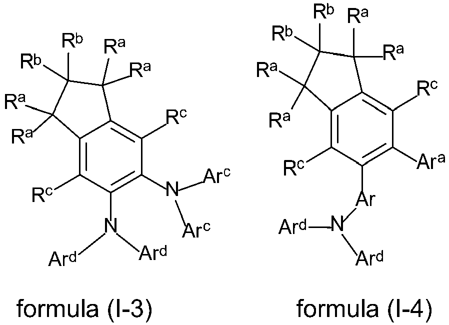 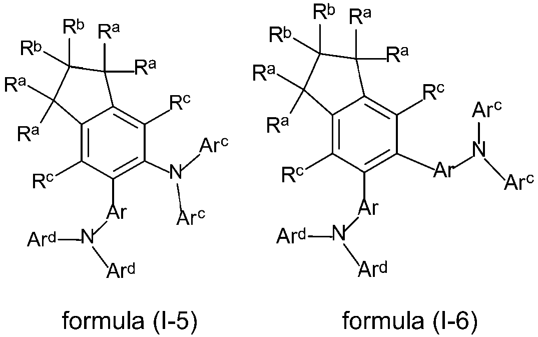 where the symbols Ar, Ar^{a}, Ar^{b}, Ar^{c}, Ar^{d}, R^{a}, R^{b} and R^{c} have the meanings given in Claim 1.

3. Compound according to Claim 1 or 2, **characterised in that** the group Ar^{a}, Ar^{b}, Ar^{c} and/or Ar^{d} is selected, identically or differently on each occurrence, from structures of the formulae (Ar^{a}-1) to (Ar^{a}-29), 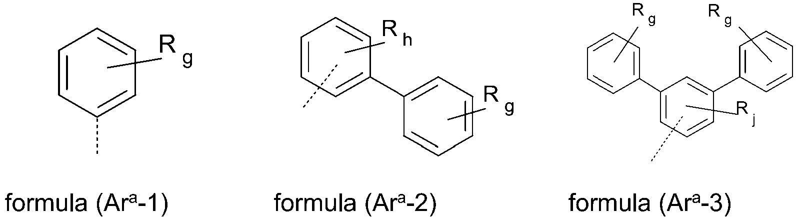 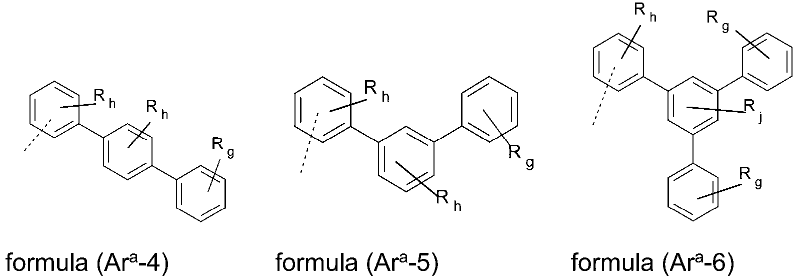 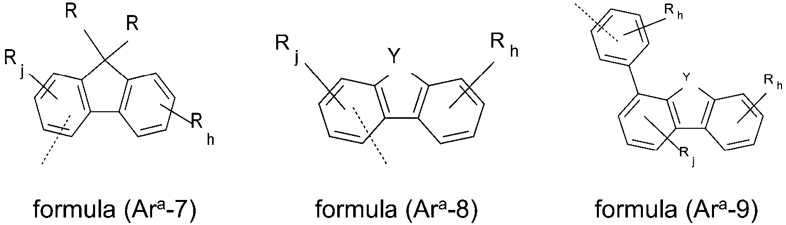 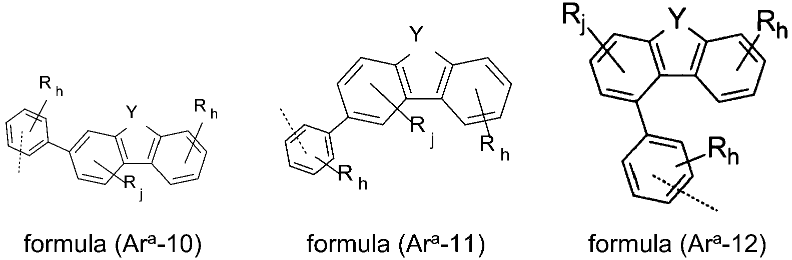 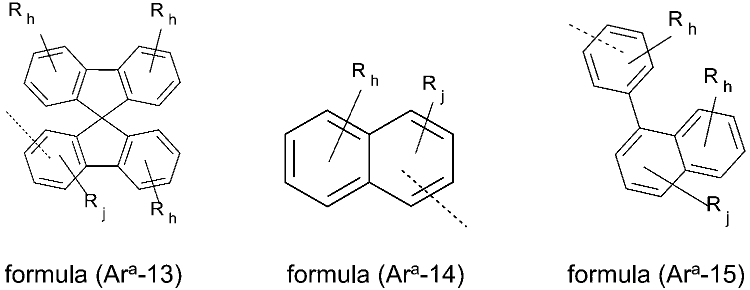 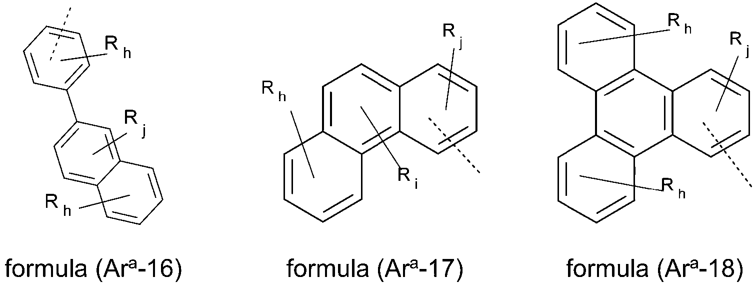 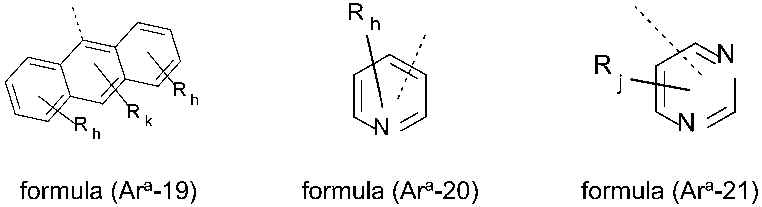 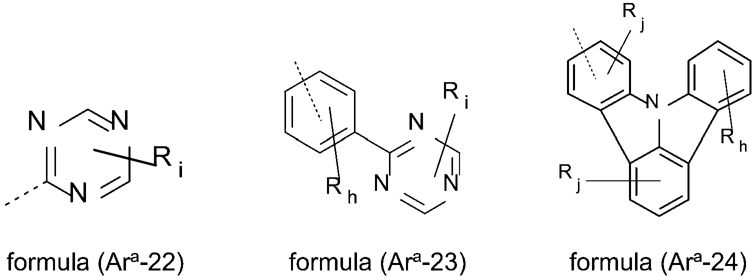 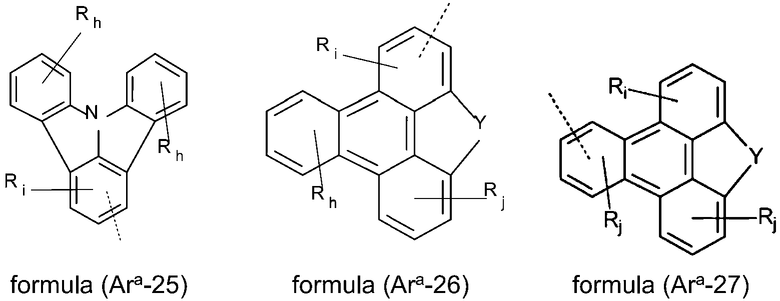 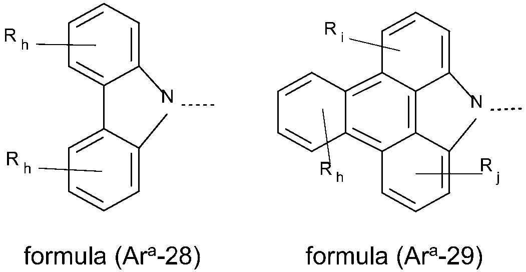 where the following applies to the symbols used:
Y is O, S or NR, preferably O or NR;
k is on each occurrence, independently, 0 or 1;
i is on each occurrence, independently, 0, 1 or 2;
j is on each occurrence, independently, 0, 1, 2 or 3;
h is on each occurrence, independently, 0, 1, 2, 3 or 4;
g is on each occurrence, independently, 0, 1, 2, 3, 4 or 5;
R has the meaning given above, in particular for Claim 1, and the dashed bond marks the bonding position.

4. Compound according to one or more of Claims 1 to 3, **characterised in that** the group Ar^{a} and/or Ar^{b} stands for a structure of the formula -Ar^{e}-Q, where Ar^{e} is on each occurrence, identically or differently, a connecting aromatic or heteroaromatic ring system having 5 to 40 aromatic ring atoms, which may be substituted by one or more radicals R, preferably Ar^{e} stands on each occurrence, identically or differently, for an arylene or heteroarylene group having 5 to 30 aromatic ring atoms, which may be substituted by one or more radicals R, is preferably selected from phenylene, biphenylene, terphenylene, quarterphenylene, fluorenylene, spirobifluorenylene, naphthylene, indolylene, benzofuranylene, benzothiophenylene, carbazolylene, dibenzofuranylene, dibenzothiophenylene, indenocarbazolylene, indolocarbazolylene, pyridinylene, pyrimidinylene, pyrazinylene, pyridazinylene, triazinylene, quinolinylene, isoquinolinylene, quinazolinylene, quinoxalinylene, phenanthrenylene or triphenylenylene, which may in each case be substituted by one or more radicals R, preferably phenylene, biphenylene, fluorenylene, dibenzofuranylene, triphenylenylene, carbazolylene or indolocarbazolylene; and Q stands for an electron-transport group, preferably for a pyridine, pyrimidine, pyrazine, pyridazine, triazine, quinazoline, quinoxaline, quinoline, isoquinoline, imidazole and/or benzimidazole group, which may be substituted by one or more radicals R.

5. Compound according to one or more of Claims 1 to 4, **characterised in that** the group Ar and/or Ar^{e} is selected, identically or differently on each occurrence, from structures of the formulae (Ar^{e}-1) to (Ar^{e}-9), 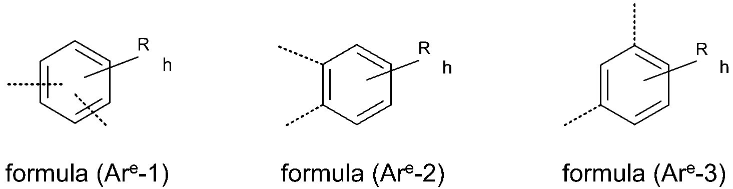 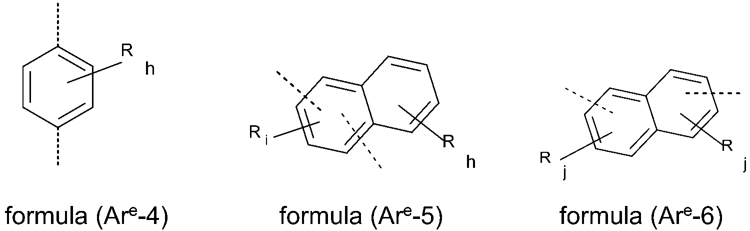 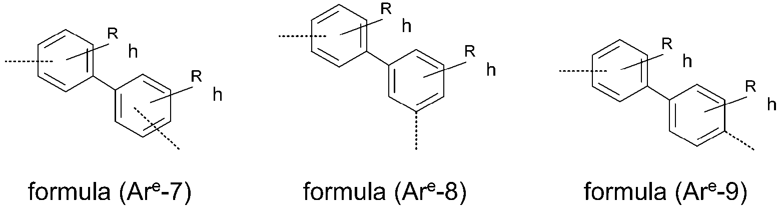 where the following applies to the symbols used:
j is on each occurrence, independently, 0, 1, 2 or 3;
h is on each occurrence, independently, 0, 1, 2, 3 or 4;
R has the meaning given above, in particular for Claim 1, and the dashed bonds mark the bonding positions.

6. Compound according to one or more of Claims 1 to 3 and 5, **characterised in that** the compound comprises at least one structure of formula (I-2), where the group Ar^{a} is selected from structures of the formulae (Ar^{a}-1) to (Ar^{a}-18), where, in structures of the formulae (Ar^{a}-8) to (Ar^{a}-12), the group Y is preferably O or NR, and the groups Ar^{d} are selected on each occurrence, identically or differently, from structures of the formulae (Ar^{a}-1) to (Ar^{a}-23), where, in structures of the formulae (Ar^{a}-8) to (Ar^{a}-12), the group Y is preferably O or NR, where the groups Ar^{d} are preferably selected on each occurrence, identically or differently, from structures of the formulae (Ar^{a}-1), (Ar^{a}-2) and (Ar^{a}-7) to (Ar^{a}-18), and/or the compound comprises at least one structure of formula (I-4), where the group Ar is selected from structures of the formulae (Ar^{e}-1) to (Ar^{e}-4), the group Ar^{a} is selected from structures of the formulae (Ar^{a}-1) to (Ar²-18), where, in structures of the formulae (Ar^{a}-8) to (Ar^{a}-12), the group Y is preferably O or NR, and the groups Ar^{d} are selected on each occurrence, identically or differently, from structures of the formulae (Ar²-1) to (Ar^{a}-23), where, in structures of the formulae (Ar^{a}-8) to (Ar^{a}-12), the group Y is preferably O or NR, where the groups Ar^{d} are preferably selected on each occurrence, identically or differently, from structures of the formulae (Ar^{a}-1), (Ar^{a}-2), (Ar^{a}-7) to (Ar^{a}-18).

7. Compound according to one or more of Claims 1 to 3 and 5, **characterised in that** the compound comprises at least one structure of formula (I-3), where the groups Ar^{c} are selected on each occurrence, identically or differently, from structures of the formulae (Ar^{a}-1) to (Ar^{a}-23), where, in structures of the formulae (Ar^{a}-8) to (Ar^{a}-12), the group Y is preferably O or NR, the groups Ar^{c} are preferably selected on each occurrence, identically or differently, from structures of the formulae (Ar^{a}-1), (Ar^{a}-2), (Ar^{a}-7) to (Ar^{a}-18); and the groups Ar^{d} are selected on each occurrence, identically or differently, from structures of the formulae (Ar^{a}-1) to (Ar^{a}-23), where, in structures of the formulae (Ar^{a}-8) to (Ar^{a}-12), the group Y is preferably O or NR, where the groups Ar^{d} are preferably selected on each occurrence, identically or differently, from structures of the formulae (Ar^{a}-1), (Ar^{a}-2), (Ar^{a}-7) to (Ar^{a}-18), and/or the compound comprises at least one structure of formula (I-5) and/or formula (I-6), where the group Ar is selected on each occurrence, identically or differently, from structures of the formulae (Ar^{e}-1) to (Ar^{e}-4), the groups Ar^{c} are selected on each occurrence, identically or differently, from structures of the formulae (Ar^{a}-1) to (Ar^{a}-23), where, in structures of the formulae (Ar^{a}-8) to (Ar^{a}-12), the group Y is preferably O or NR, the groups Ar^{c} are preferably selected on each occurrence, identically or differently, from structures of the formulae (Ar^{a}-1), (Ar^{a}-2), (Ar^{a}-7) to (Ar^{a}-18); and the groups Ar^{d} are selected on each occurrence, identically or differently, from structures of the formulae (Ar^{a}-1) to (Ar^{a}-18), where, in structures of the formulae (Ar^{a}-8) to (Ar²-12), the group Y is preferably O or NR, where the groups Ar^{d} are preferably selected on each occurrence, identically or differently, from structures of the formulae (Ar^{a}-1), (Ar^{a}-2), (Ar^{a}-7) to (Ar^{a}-18).

8. Compound according to Claim 4, **characterised in that** the compound comprises at least one structure of formula (I-2), where the group Ar^{a} stands for a structure of the formula Ar^{e}-Q, where the symbols Ar^{e} and Q have the meaning given in Claim 5 and the groups Ar^{d} are selected on each occurrence, identically or differently, from structures of the formulae (Ar²-1) to (Ar^{a}-23), where, in structures of the formulae (Ar^{a}-8) to (Ar^{a}-12), the group Y is preferably O or NR, where the groups Ar^{d} are preferably selected on each occurrence, identically or differently, from structures of the formulae (Ar^{a}-1), (Ar^{a}-2), (Ar^{a}-7) to (Ar²-18), and/or the compound comprises at least one structure of formula (I-4), where the group Ar is selected from structures of the formulae (Ar^{e}-1) to (Ar^{e}-4), the group Ar^{a} stands for a structure of the formula Ar^{e}-Q, where the symbols Ar^{e} and Q have the meaning given in Claim 5 and the groups Ar^{d} are selected on each occurrence, identically or differently, from structures of the formulae (Ar²-1) to (Ar^{a}-23), where, in structures of the formulae (Ar^{a}-8) to (Ar²-12), the group Y is preferably O or NR, where the groups Ar^{d} are preferably selected on each occurrence, identically or differently, from structures of the formulae (Ar^{a}-1), (Ar^{a}-2), (Ar^{a}-7) to (Ar^{a}-18).

9. Compound according to one or more of Claims 1 to 5, **characterised in that** the compound comprises at least one structure of formula (1-1) and/or formula (I-2), where the group Ar^{a} comprises an electron-transport group or stands for an electron-transport group, where group Ar^{a} preferably comprises a pyridine, pyrimidine, pyrazine, pyridazine, triazine, quinazoline, quinoxaline, quinoline, isoquinoline, imidazole and/or benzimidazole group or stands for one of the said groups, where these may be substituted by one or more radicals R, where triazine groups are particularly preferred.

10. Compound according to Claim 9, **characterised in that** the compound comprises at least one structure of formula (I-1), where the group Ar^{b} comprises an electron-transport group or stands for an electron-transport group, where group Ar^{a} preferably comprises a pyridine, pyrimidine, pyrazine, pyridazine, triazine, quinazoline, quinoxaline, quinoline, isoquinoline, imidazole and/or benzimidazole group or stands for one of the said groups, where these may be substituted by one or more radicals R, where triazine groups are particularly preferred.

11. Compound according to Claim 9, **characterised in that** the compound comprises at least one structure of formula (I-1), where the group Ar^{b} is selected from structures of the formulae (Ar^{a}-1) to (Ar²-18), where, in structures of the formulae (Ar^{a}-8) to (Ar²-12), the group Y is preferably O or NR; where the group Ar^{a} preferably stands for a structure of the formula Ar^{e}-Q, where the symbols Ar^{e} and Q have the meaning given in Claim 5.

12. Compound according to one or more of Claims 1 to 11, comprising at least one structure of the formulae (II-1) to (II-154), preferably compound of one of the formulae (II-1) to (II-154), 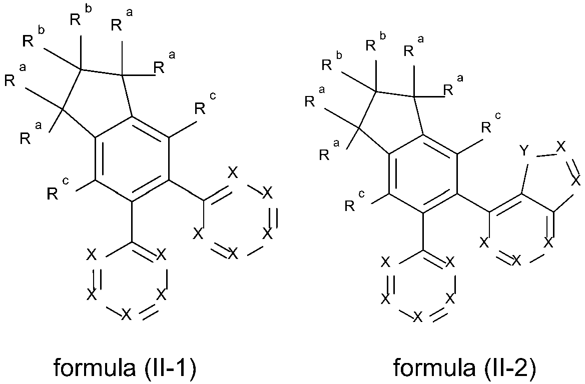 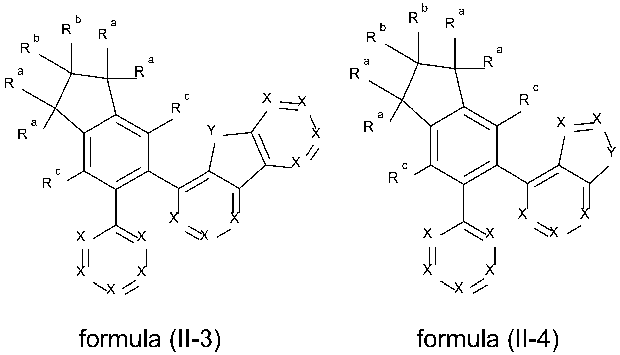 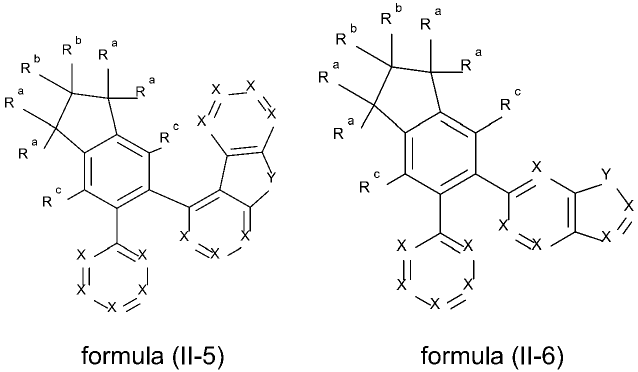 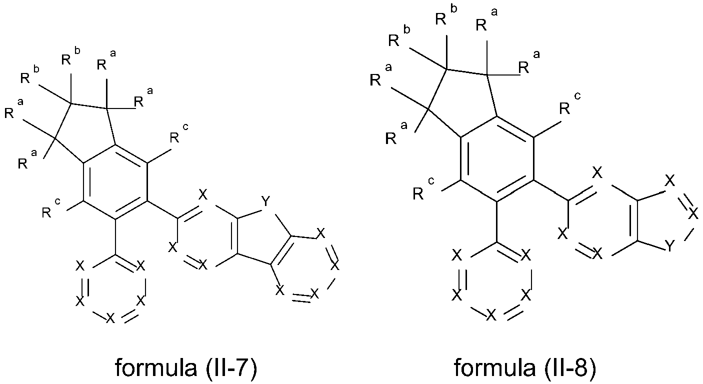 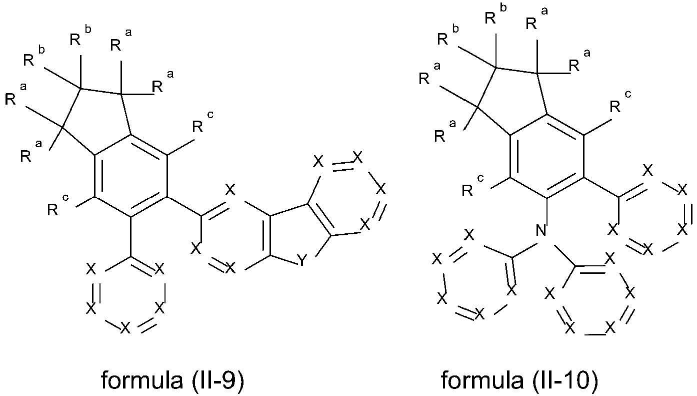 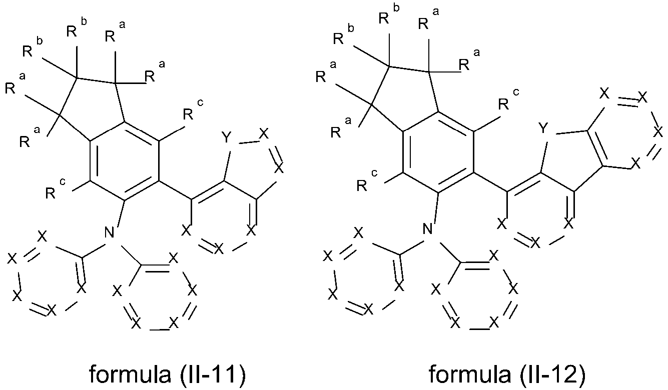 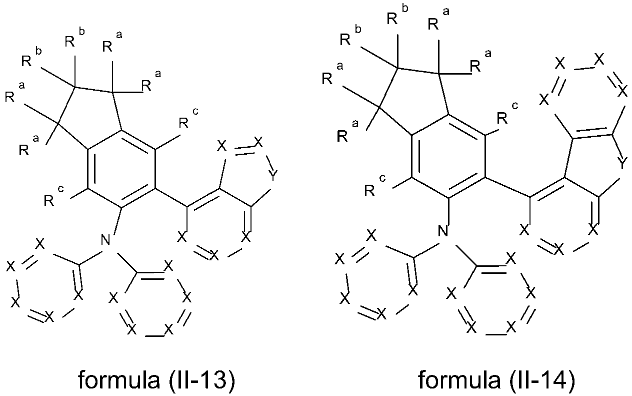 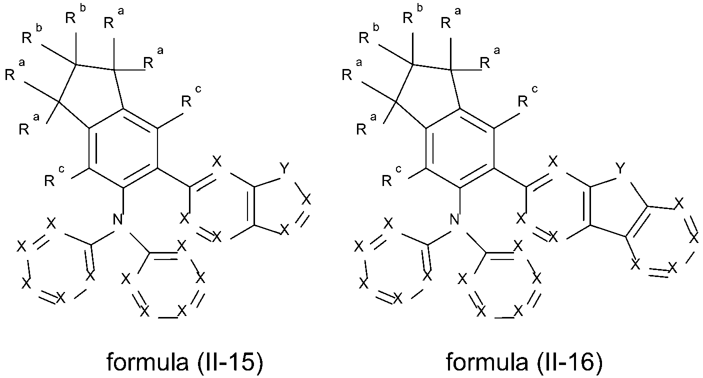 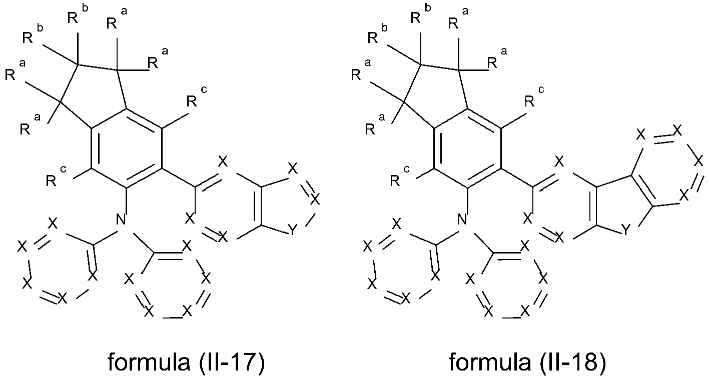 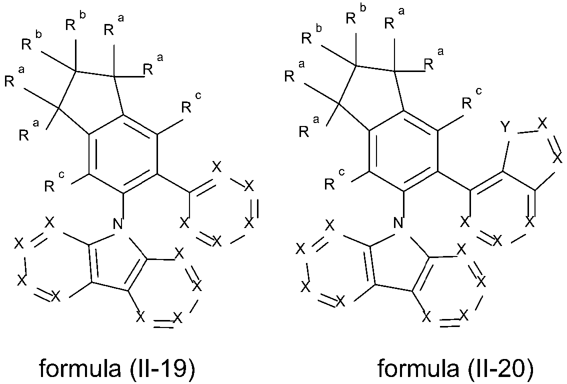 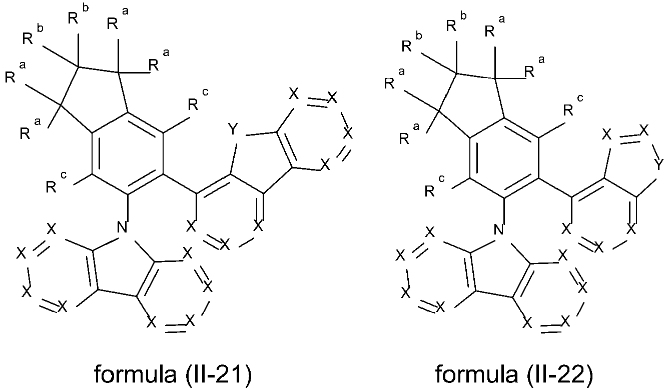 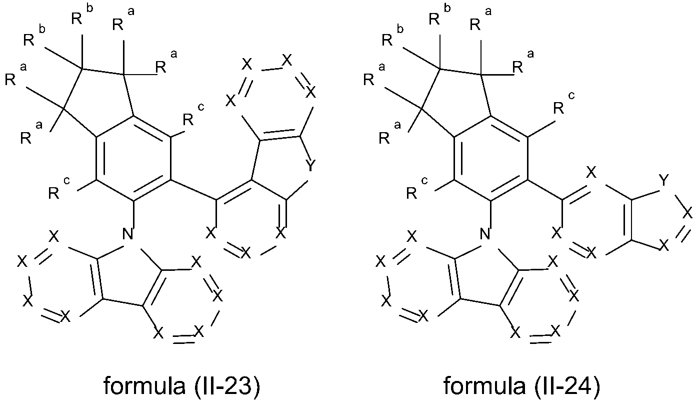 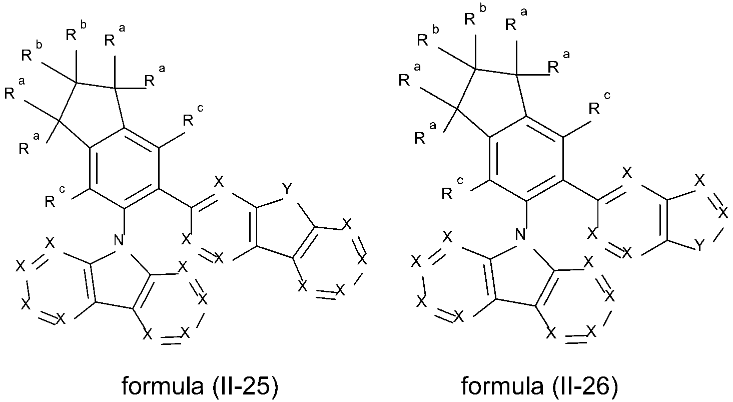 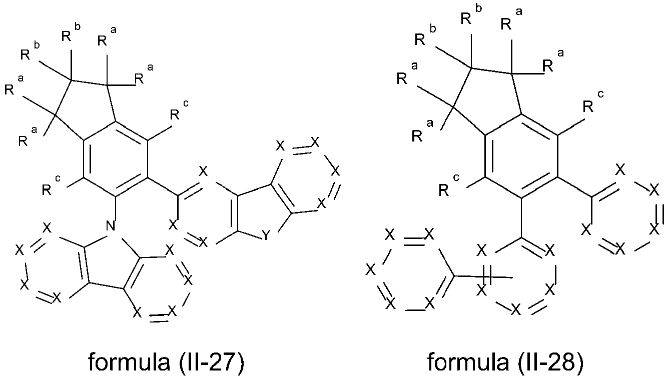 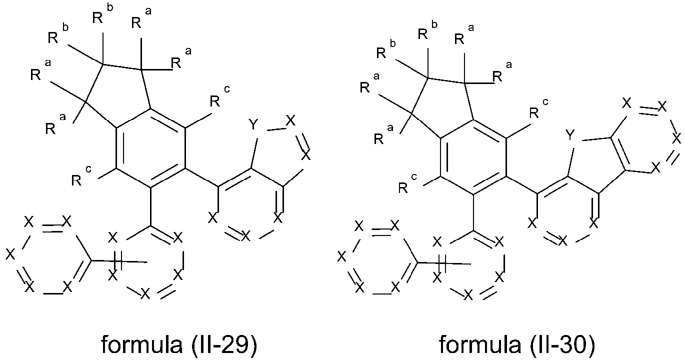 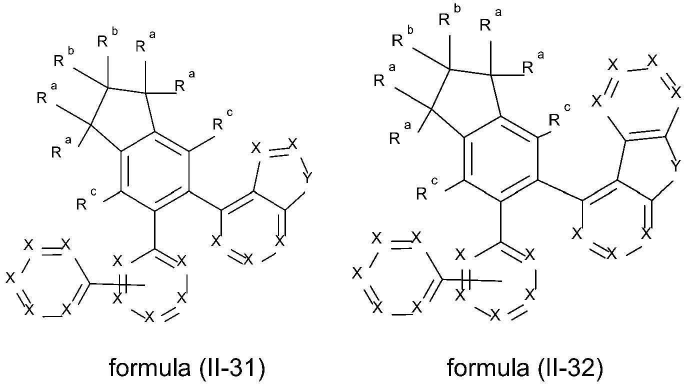 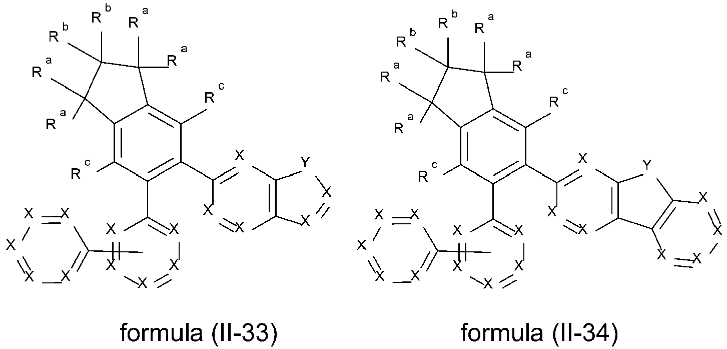 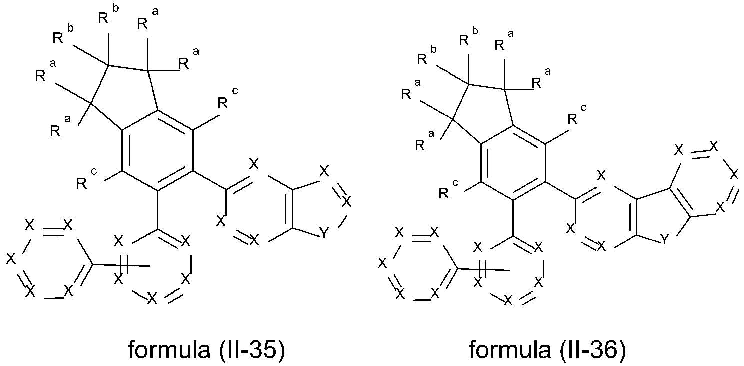 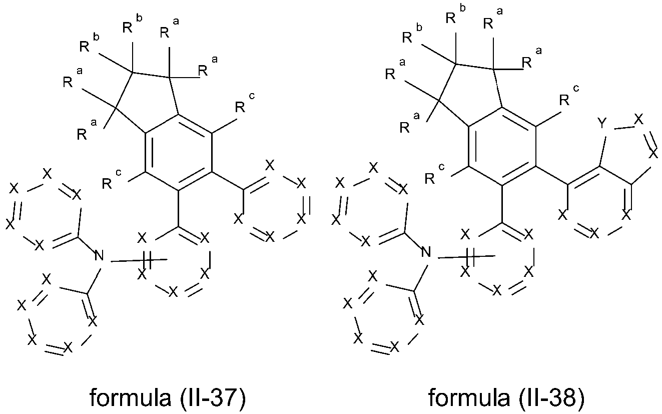 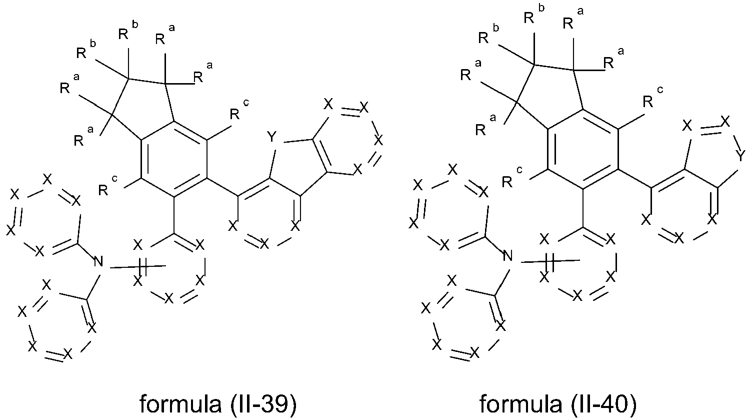 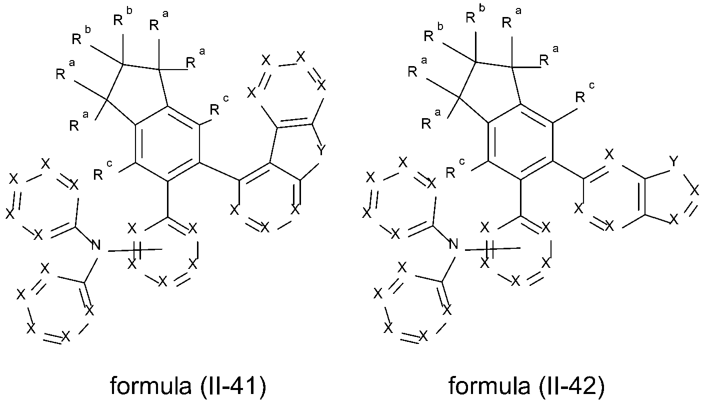 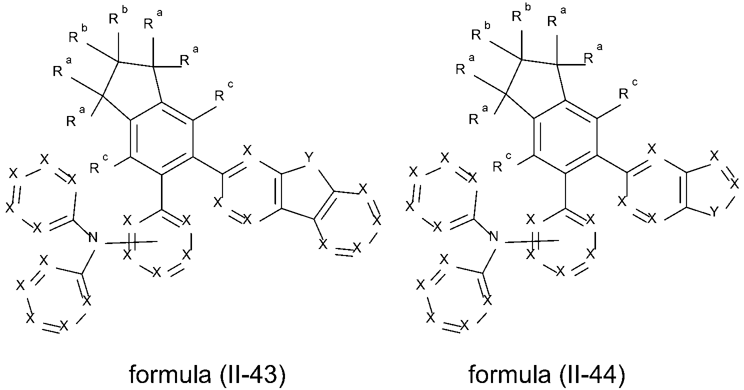 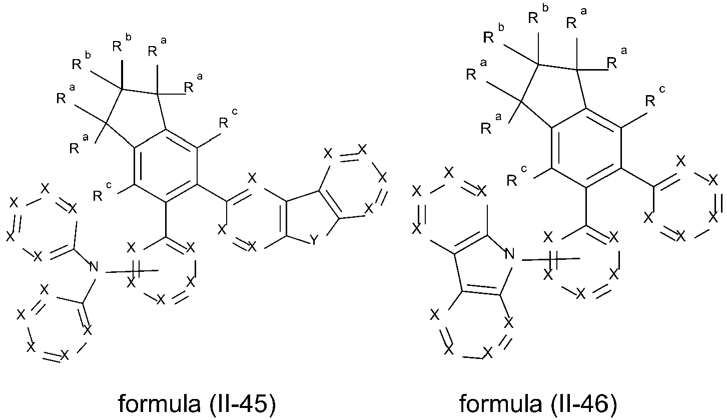 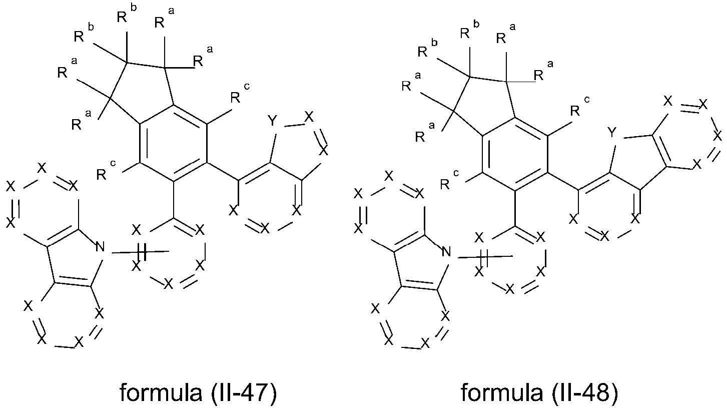 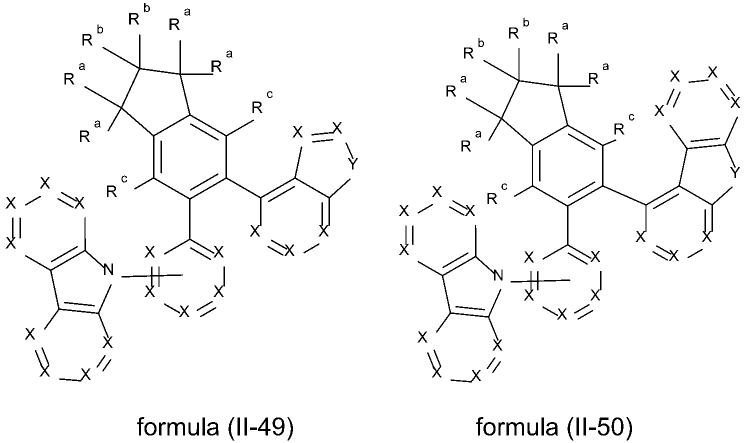 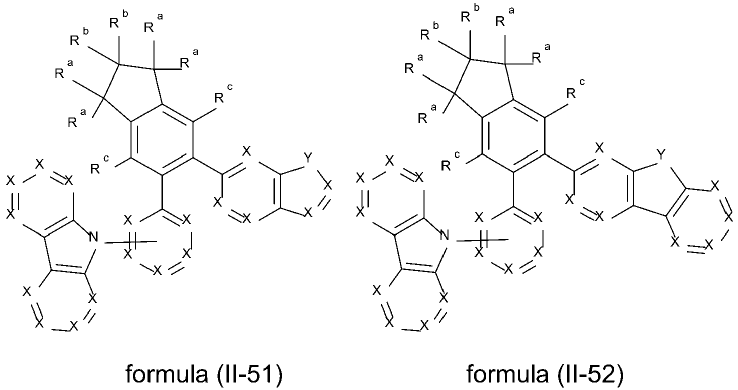 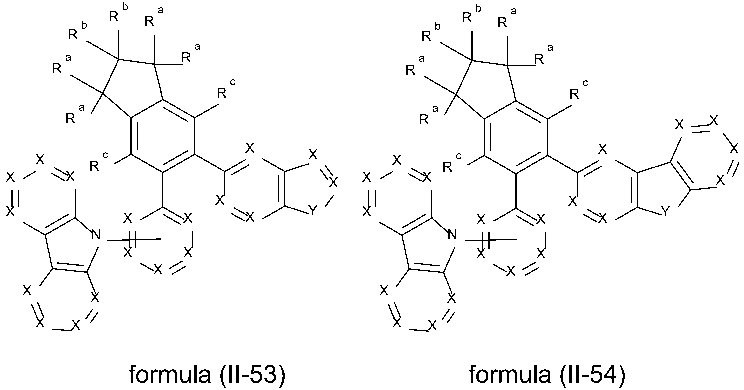 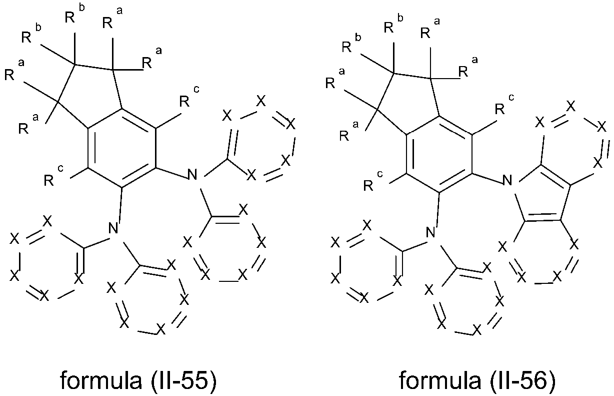 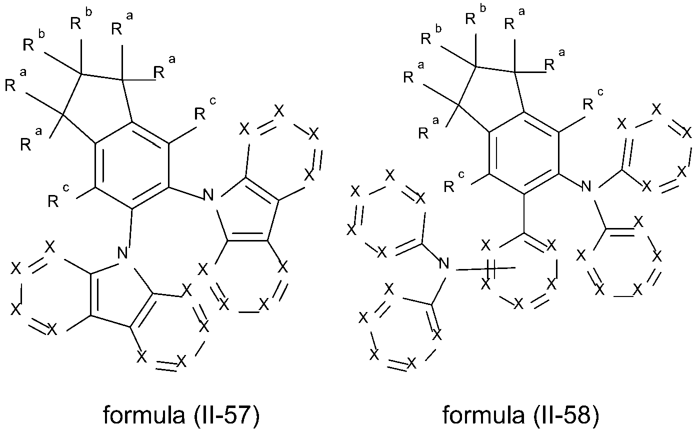 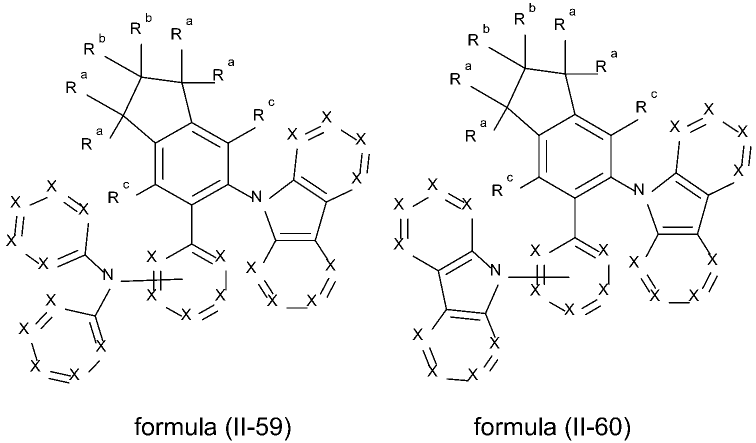 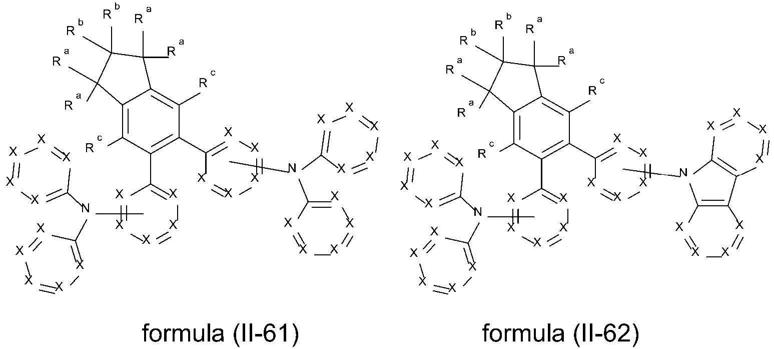 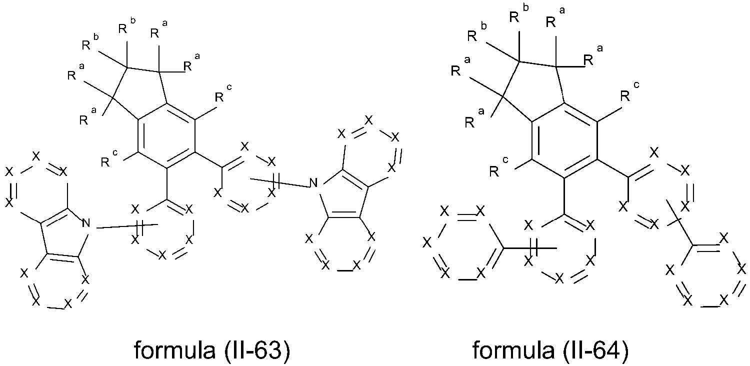 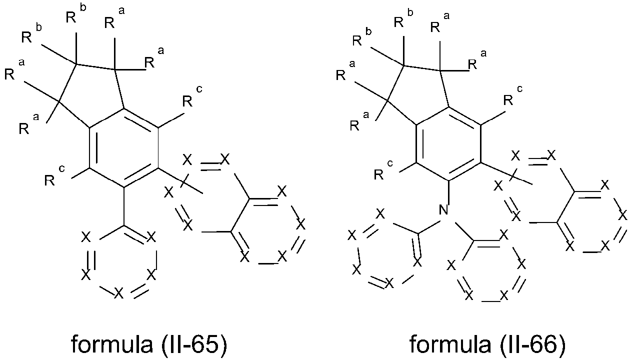 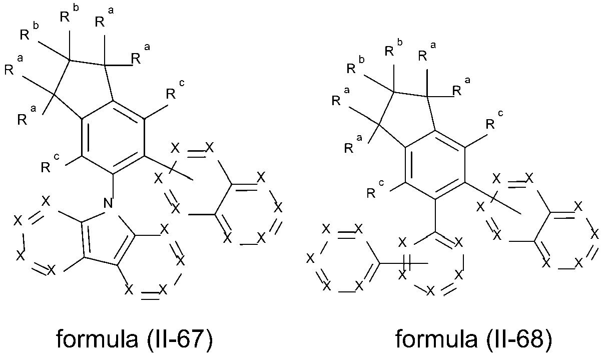 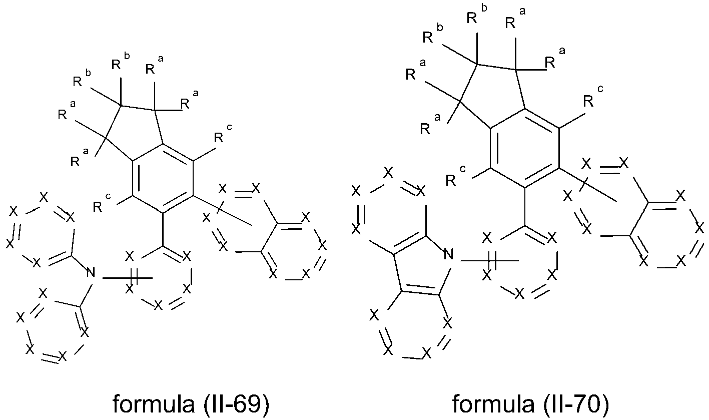 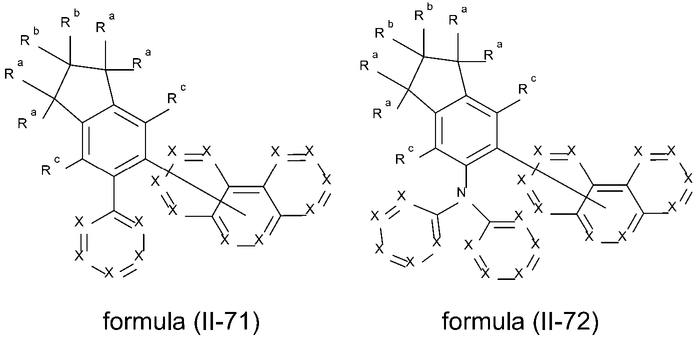 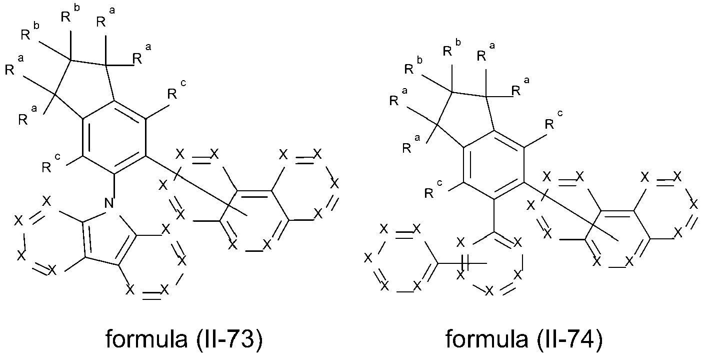 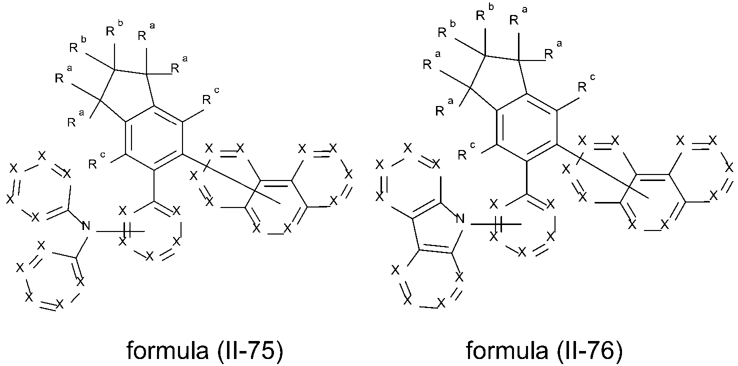 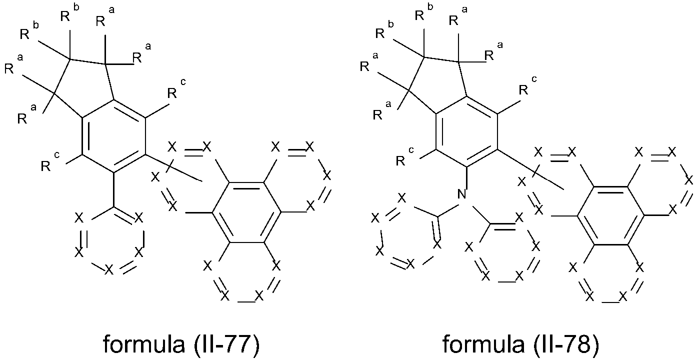 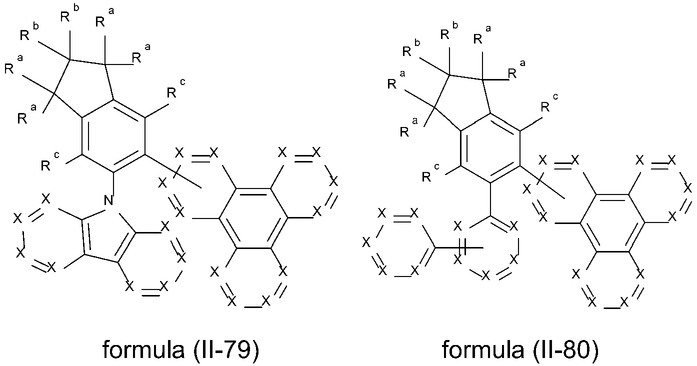 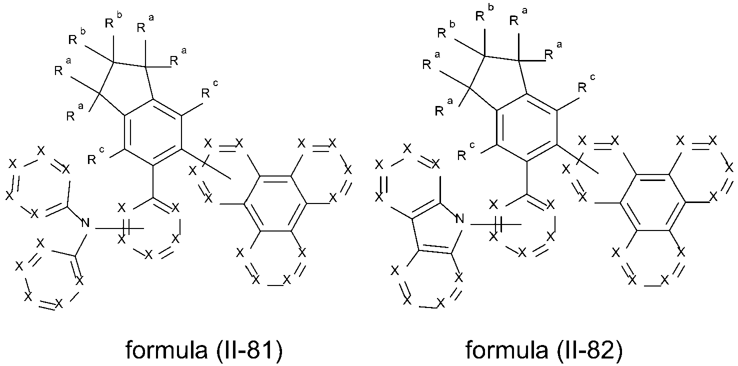 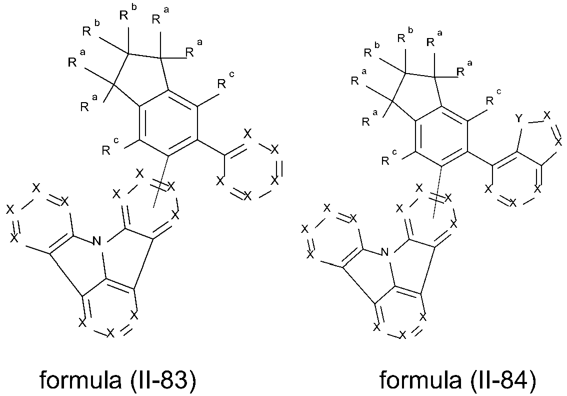 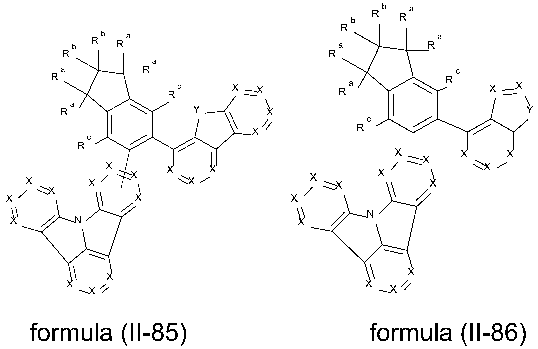 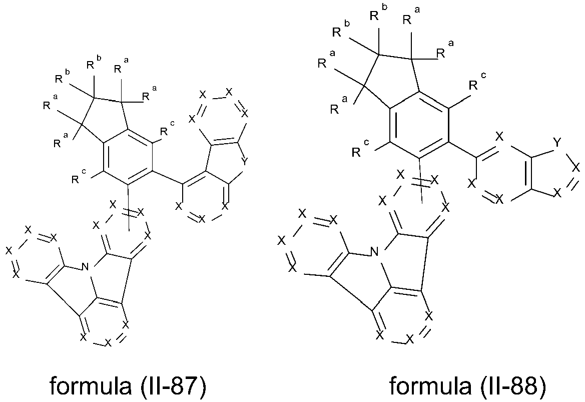 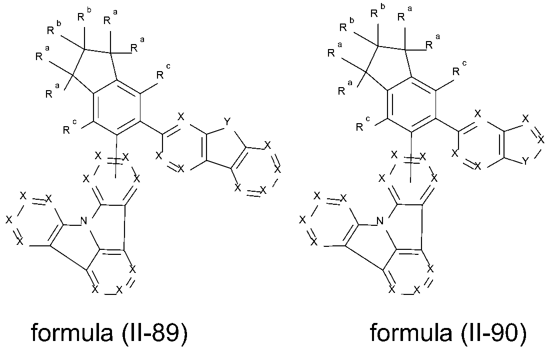 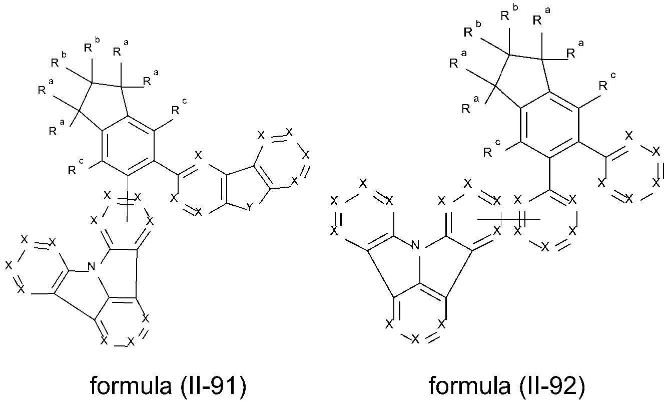 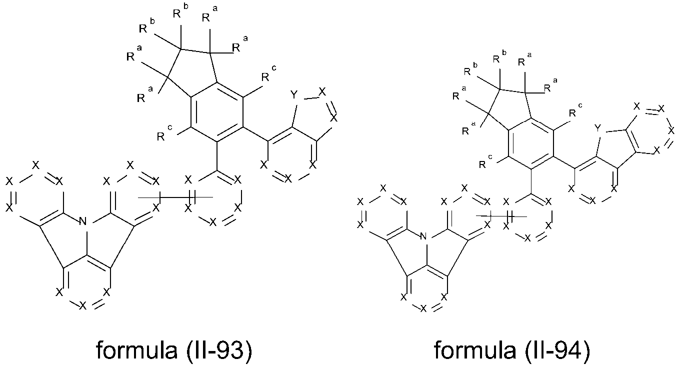 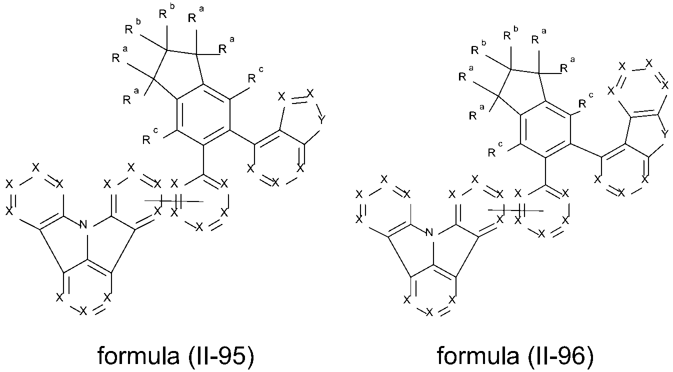 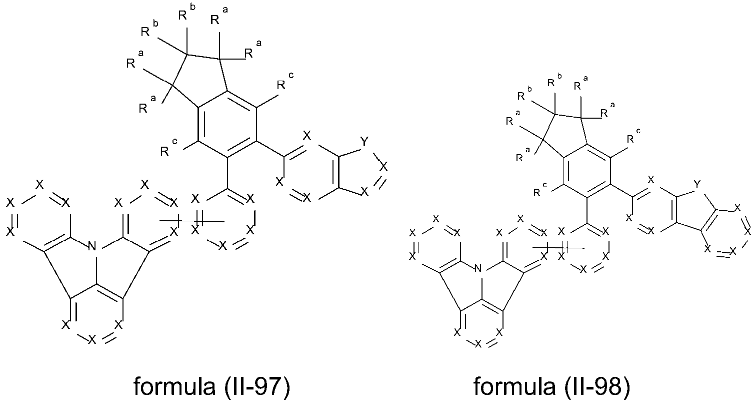 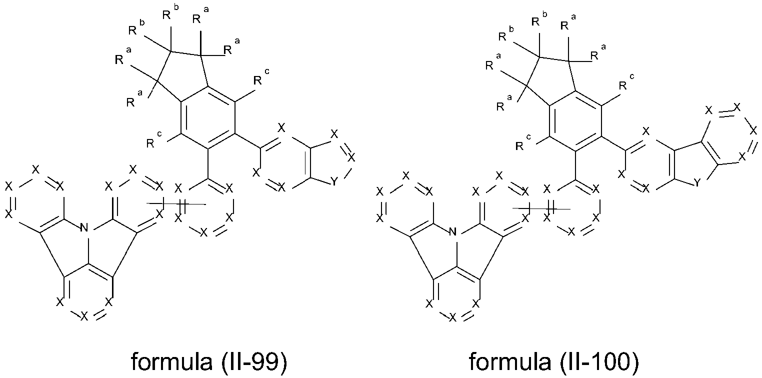 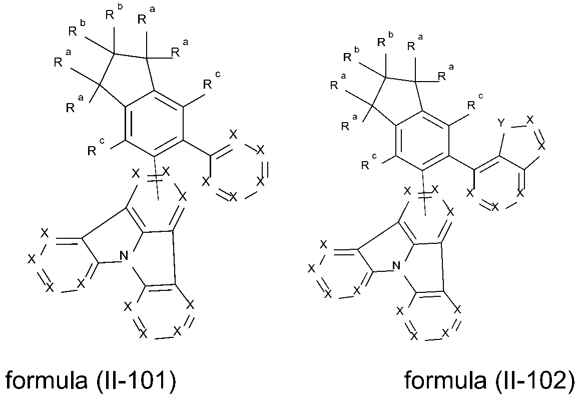 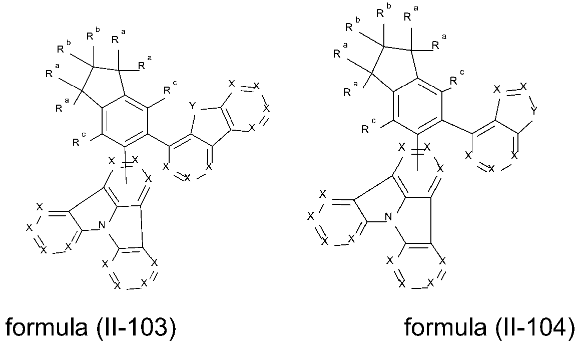 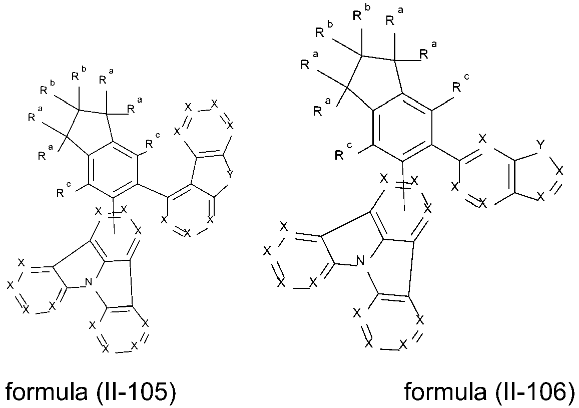 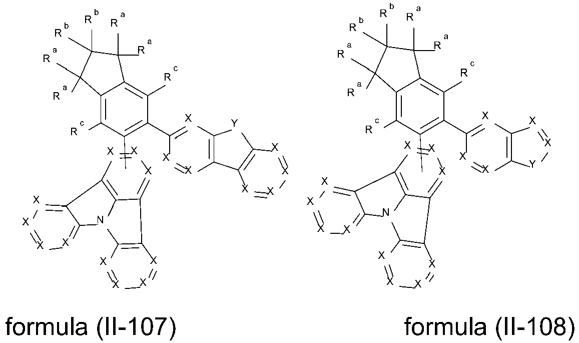 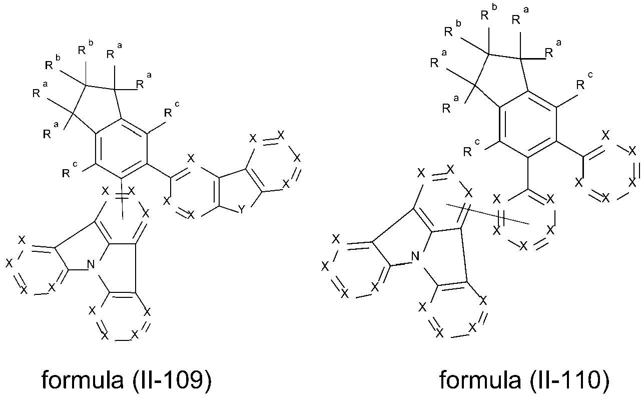 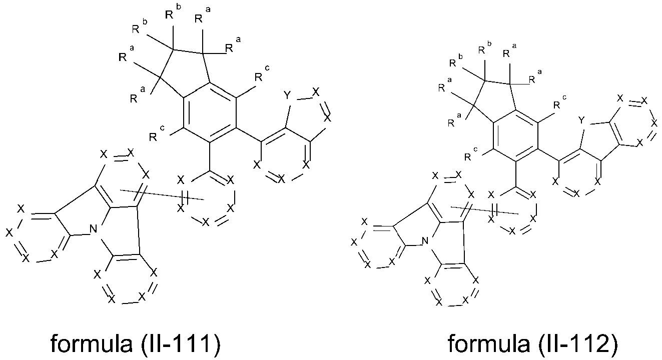 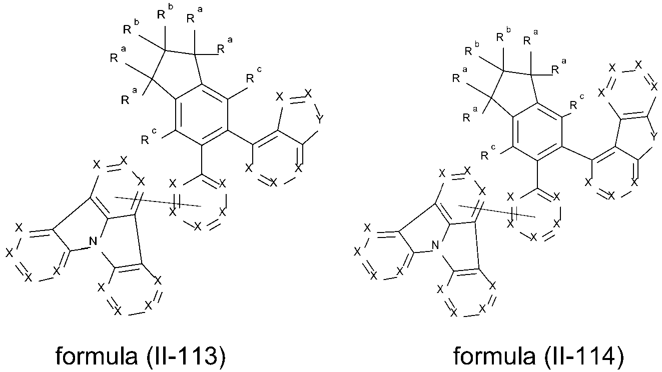 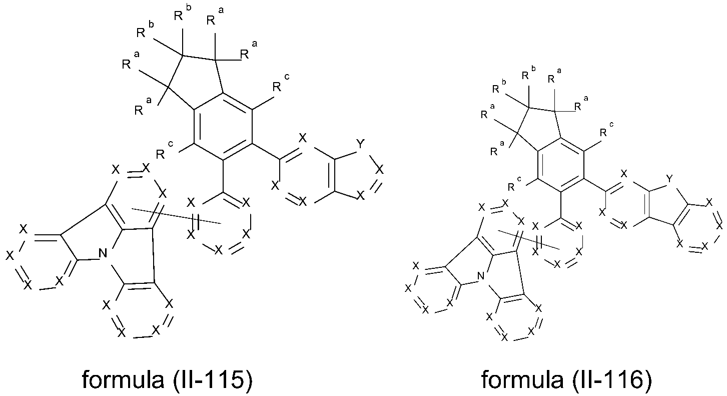 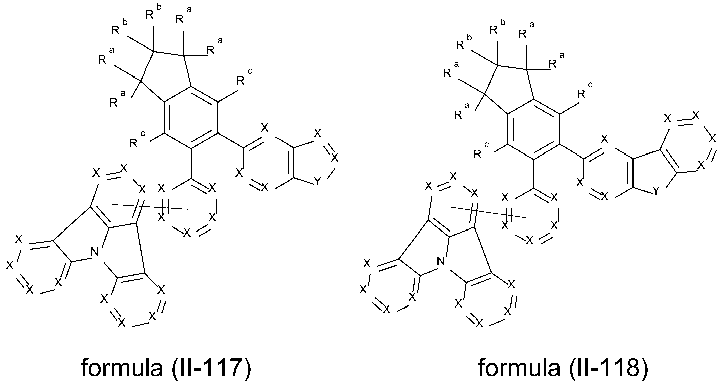 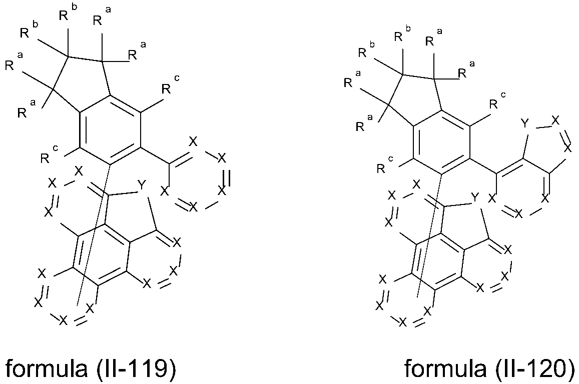 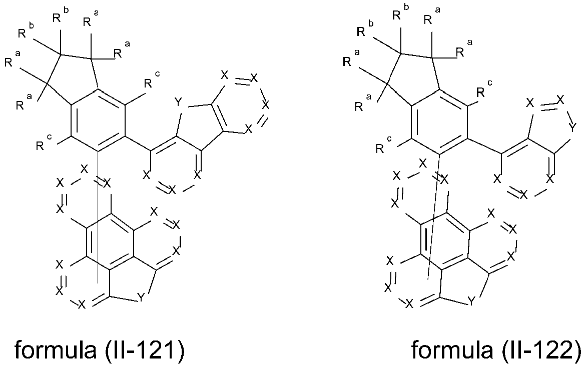 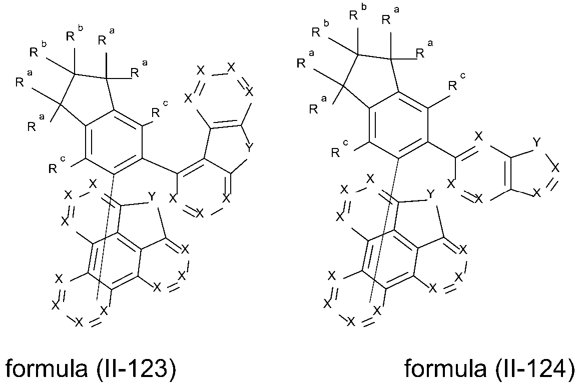 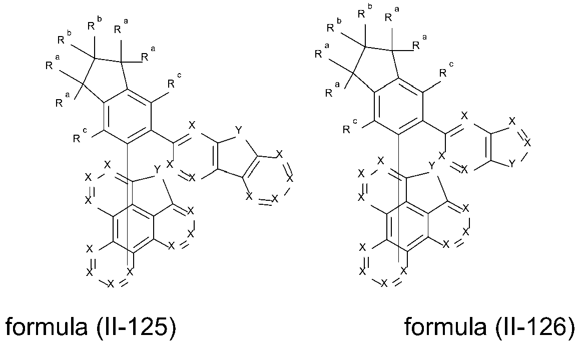 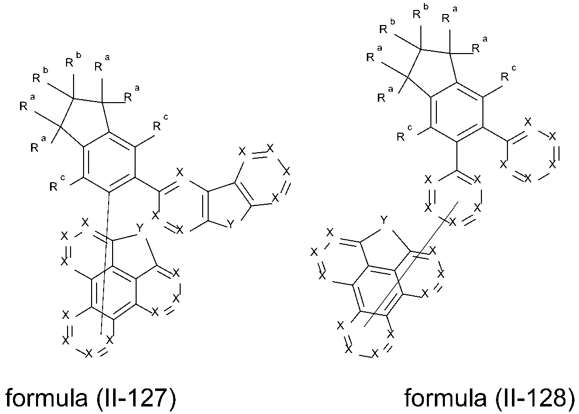 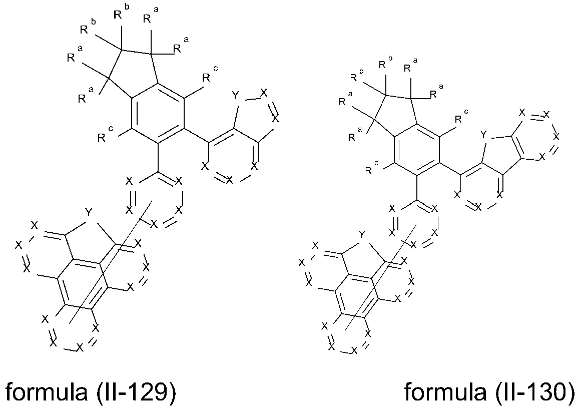 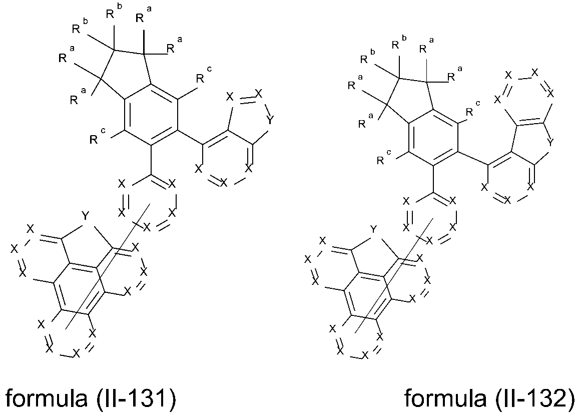 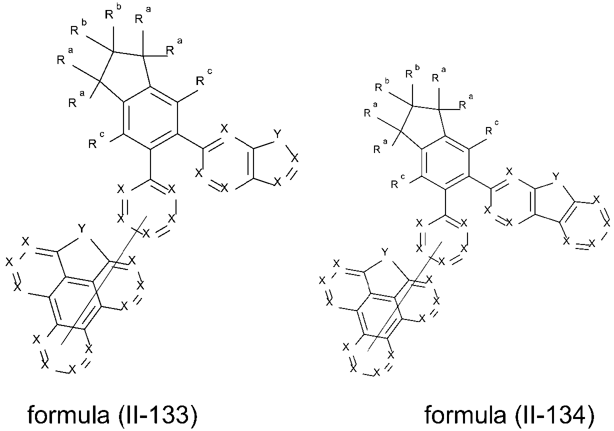 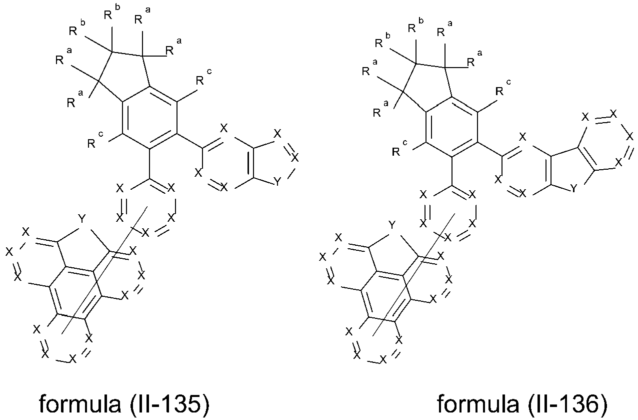 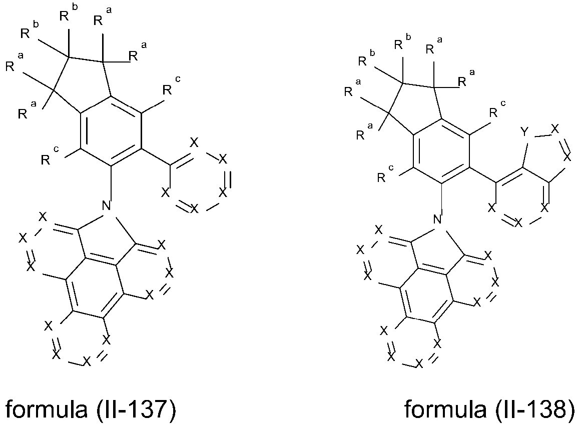 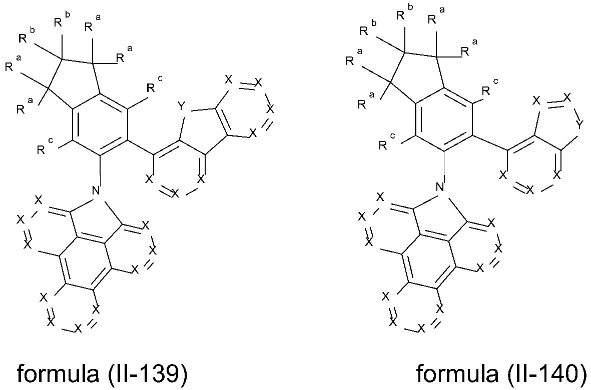 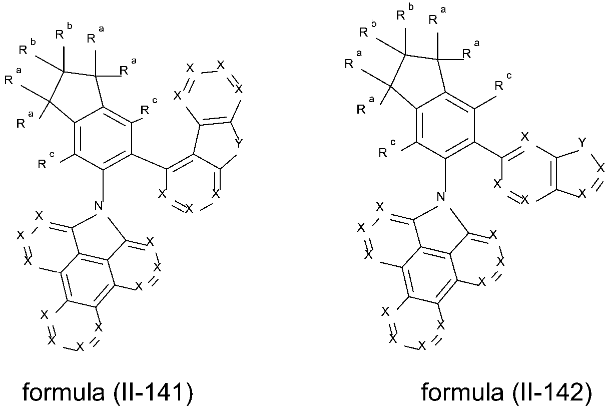 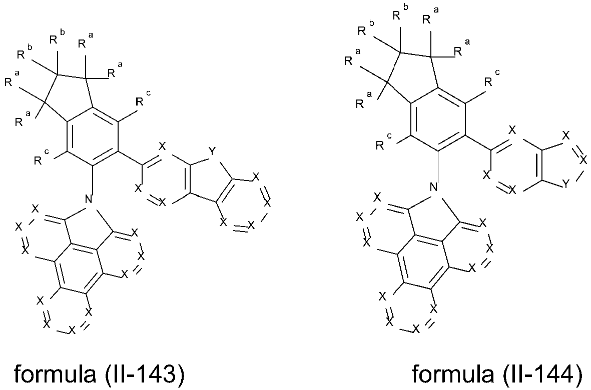 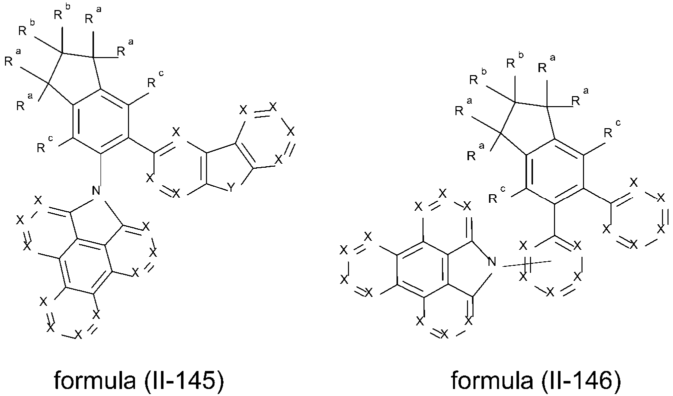 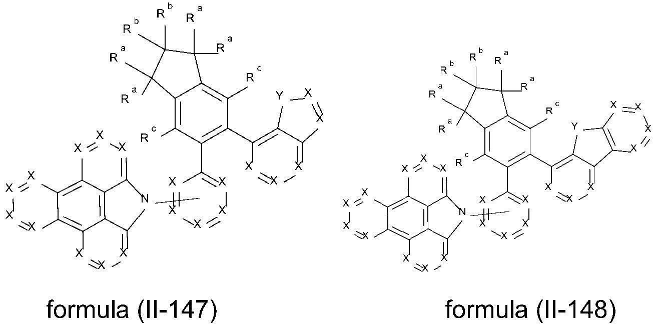 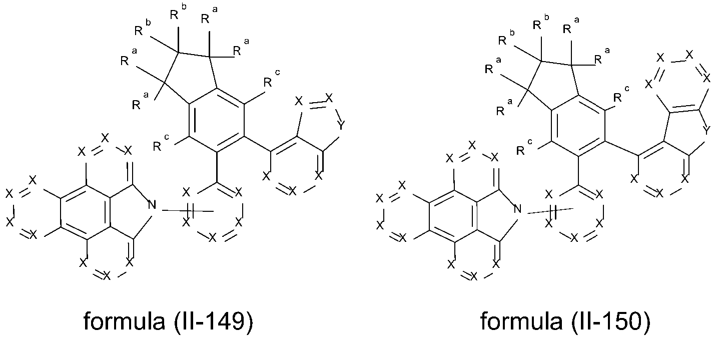 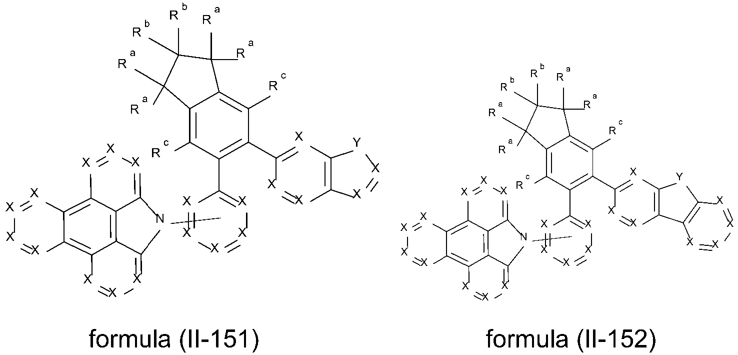 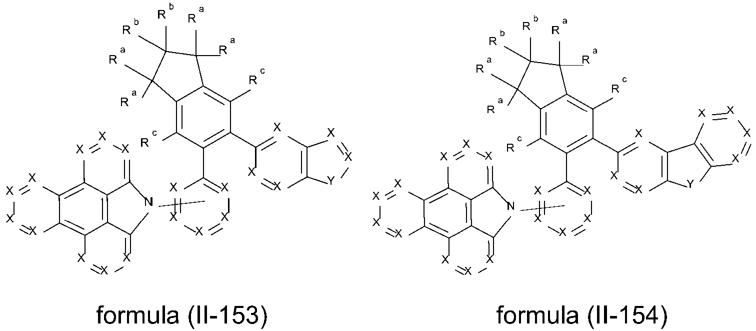 where the symbols R, R^{a}, R^{b} and R^{c} have the meanings given in Claim 1 and the following applies to the other symbols:
X stands on each occurrence, identically or differently, for N, CR or C in the case where a group is bonded to the structure; and
Y stands for O, S, NR or C(R)₂, preferably O, NR or C(R)₂.

13. Compound according to one or more of Claims 1 to 12, comprising at least one structure of the formulae (III-1) to (III-60), preferably compound of one of the formulae (III-1) to (III-60), 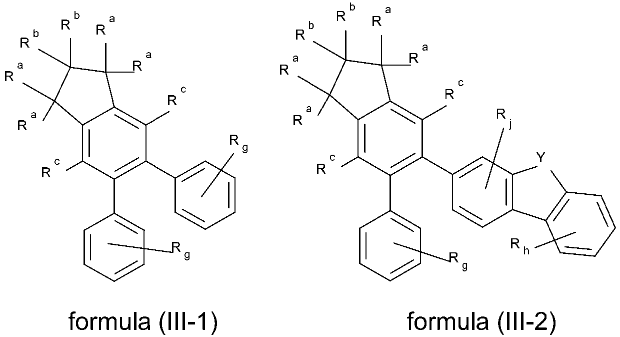 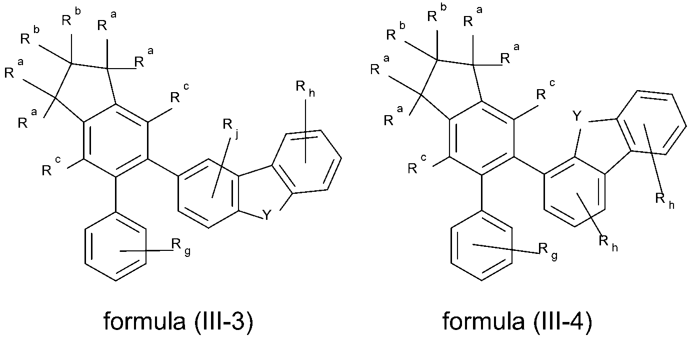 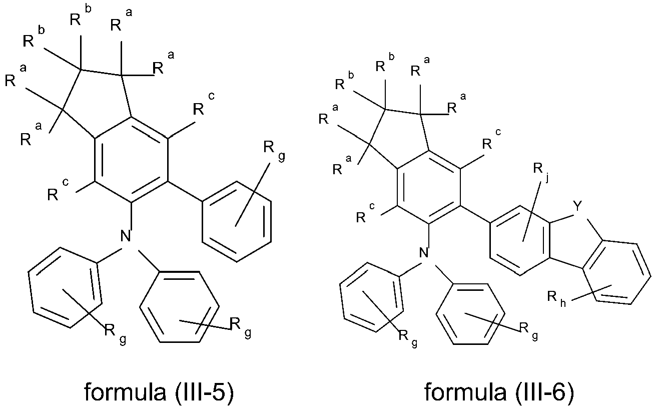 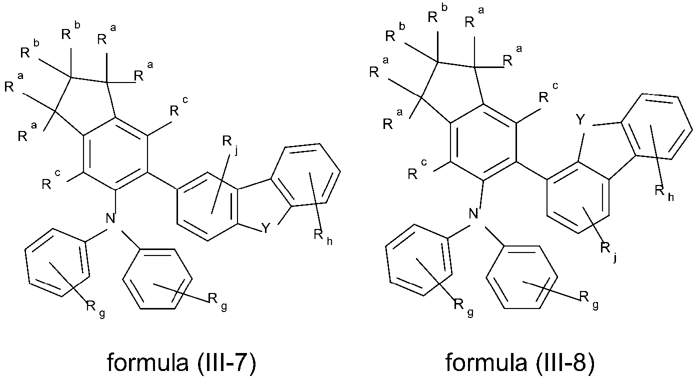 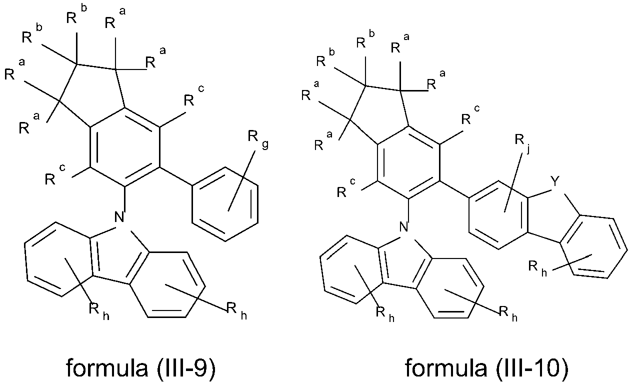 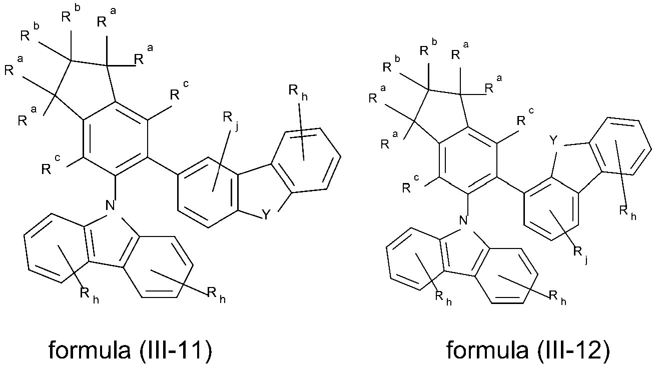 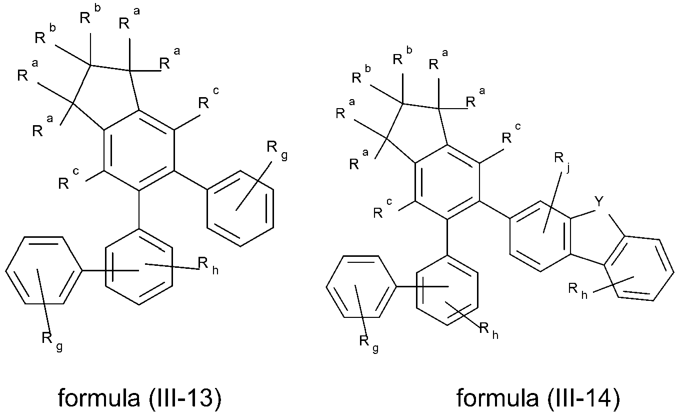 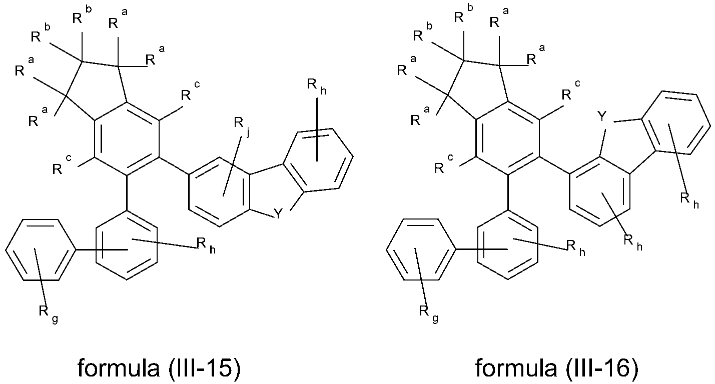 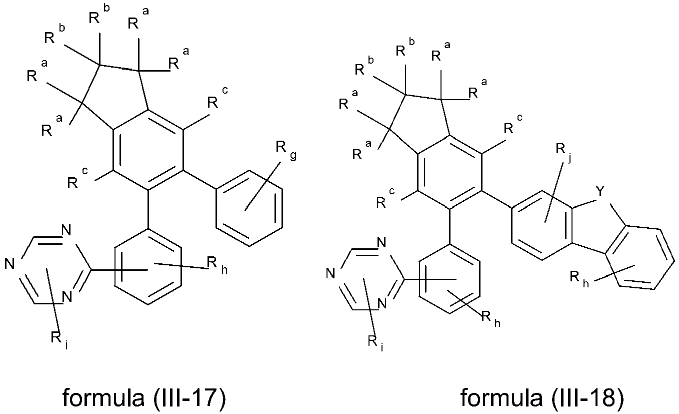 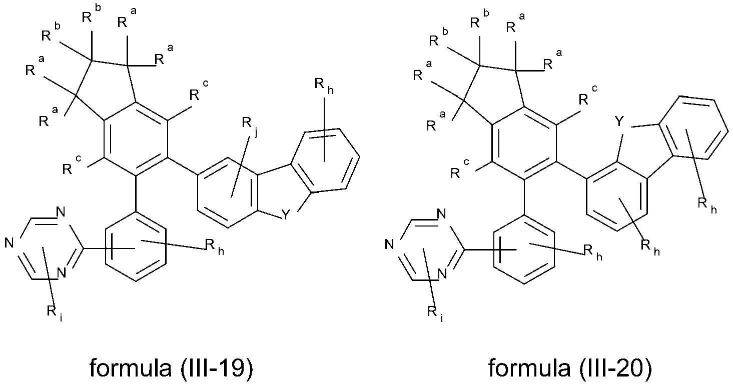 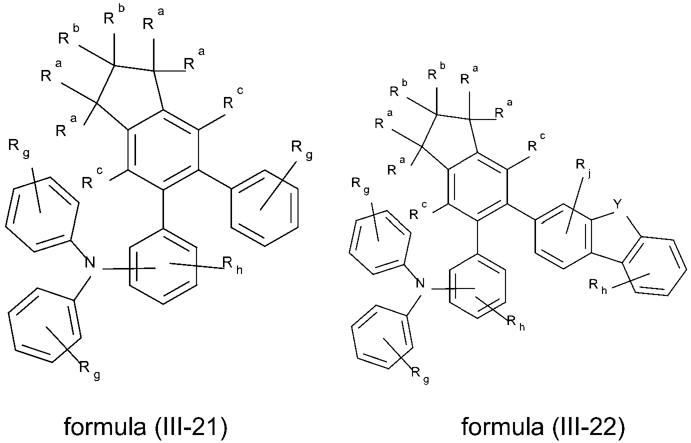 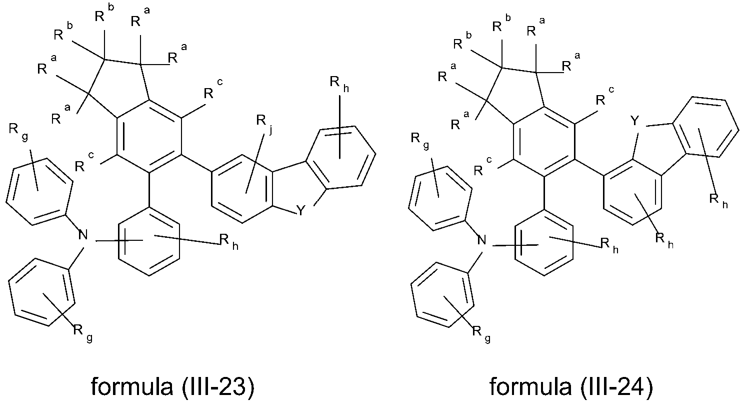 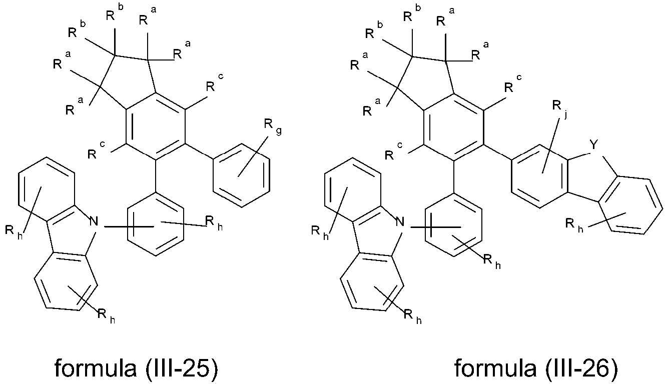 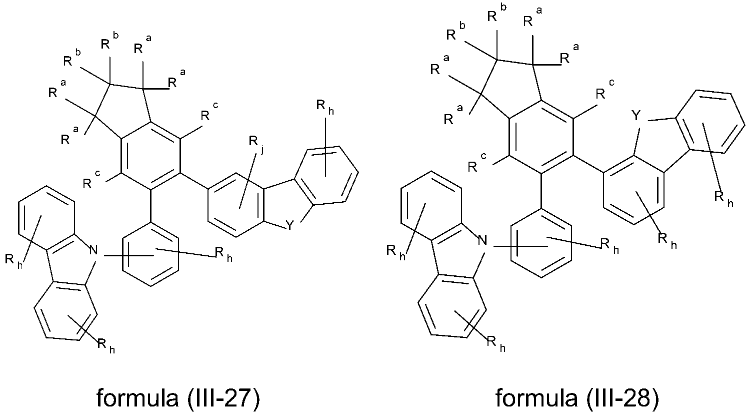 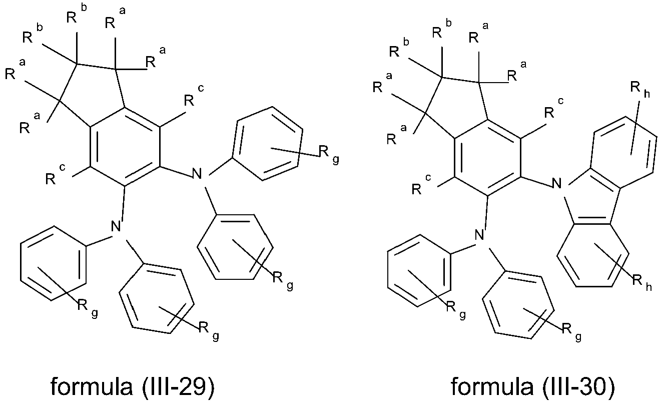 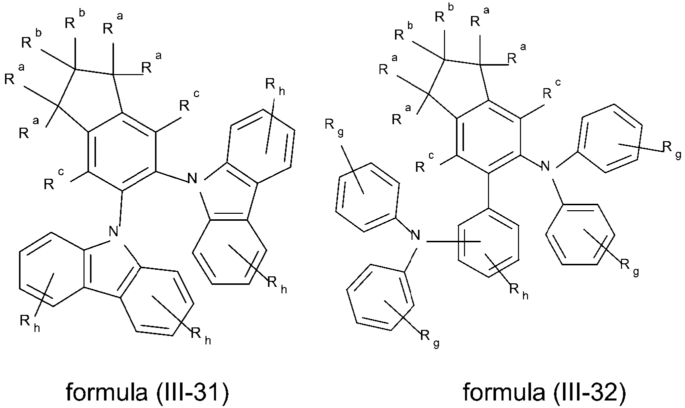 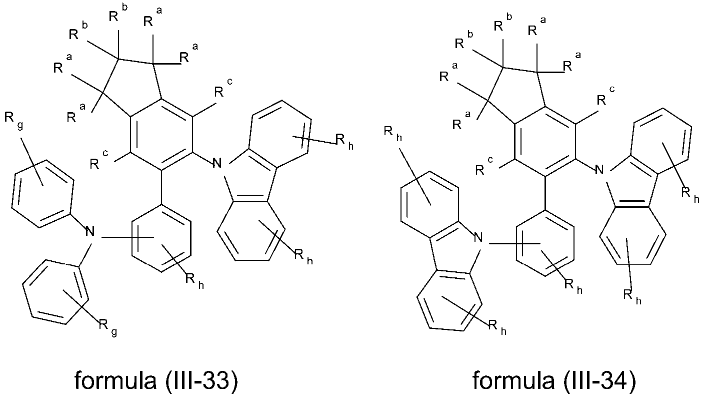 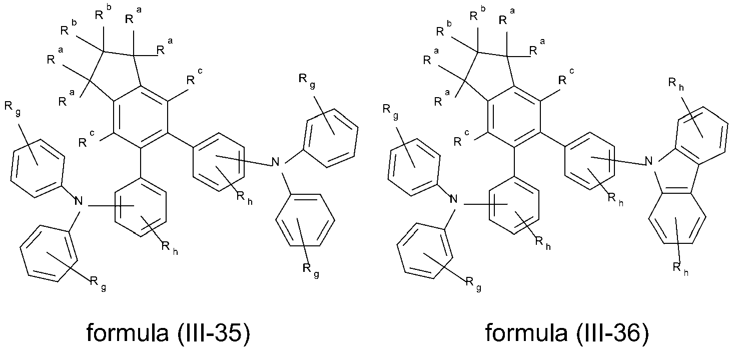 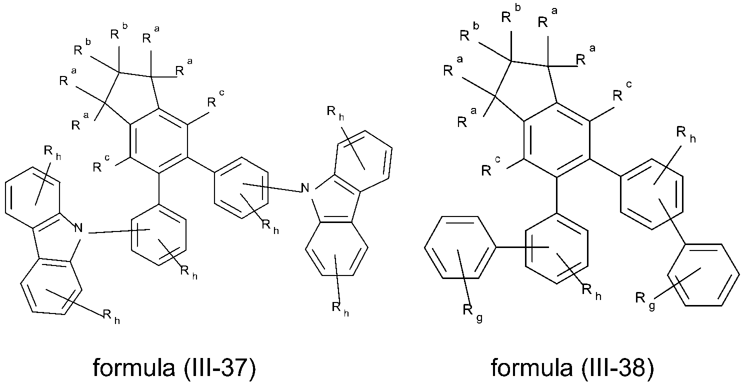 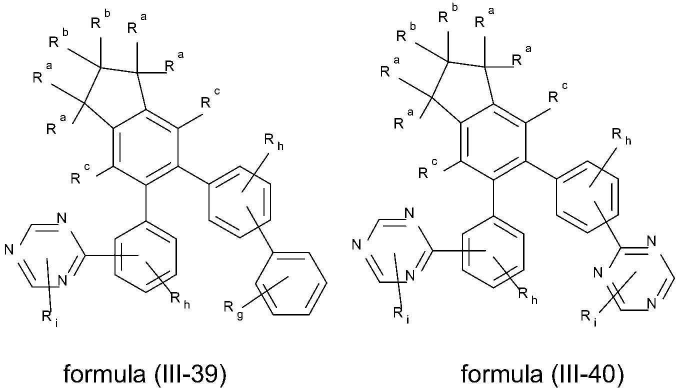 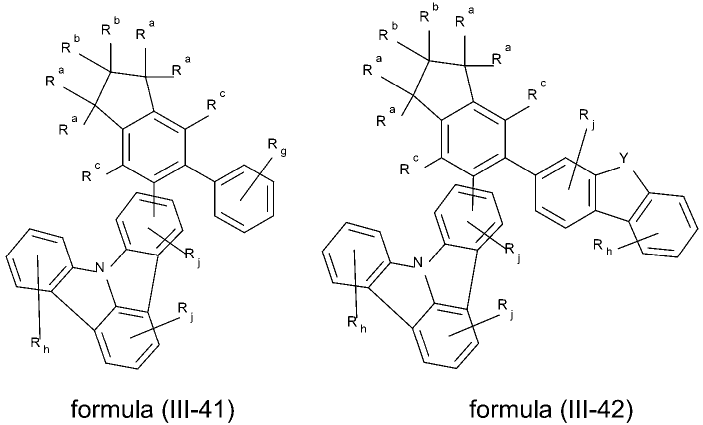 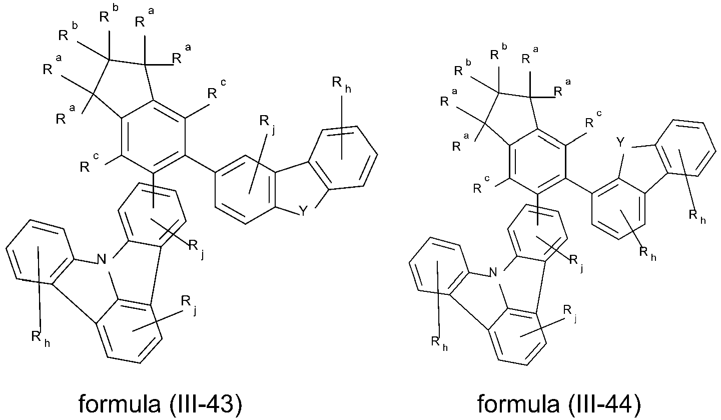 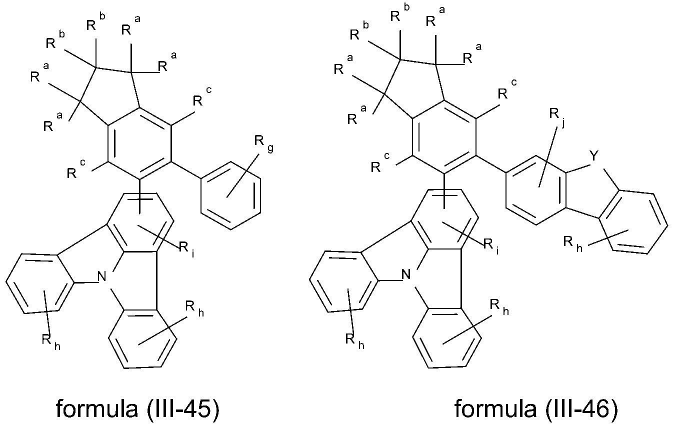 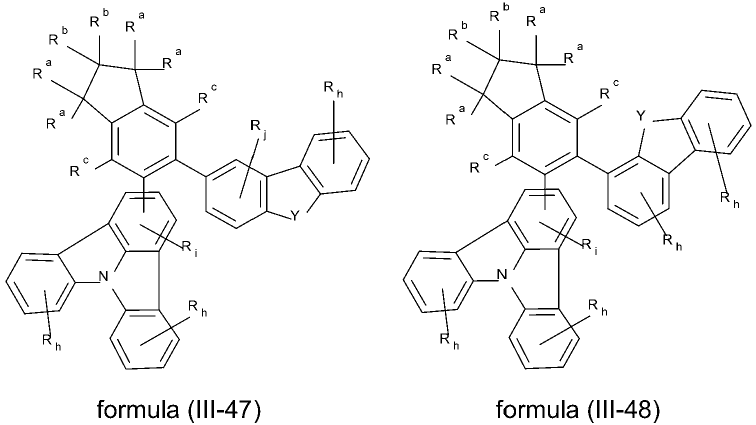 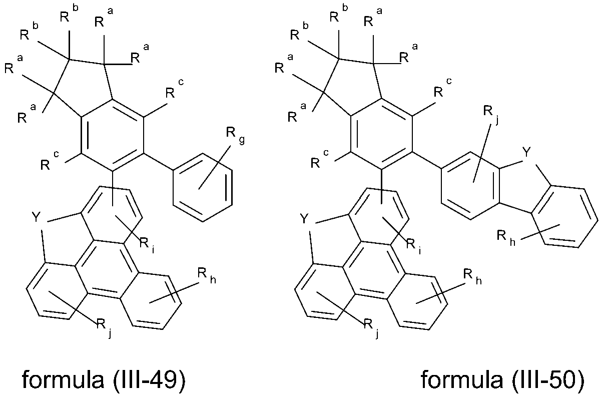 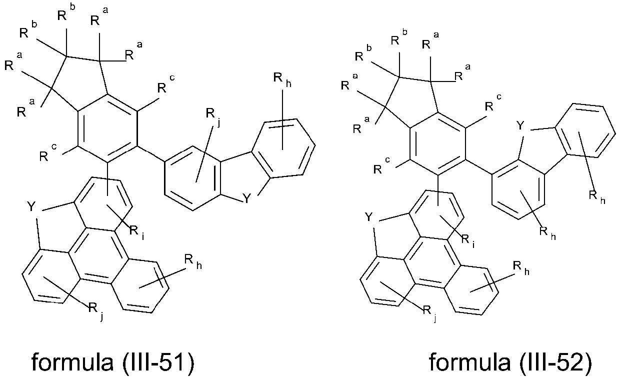 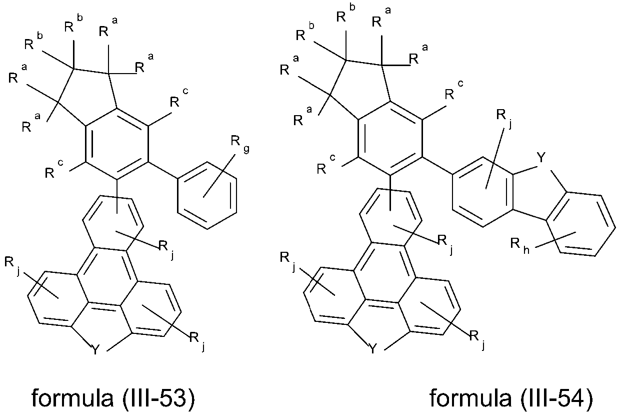 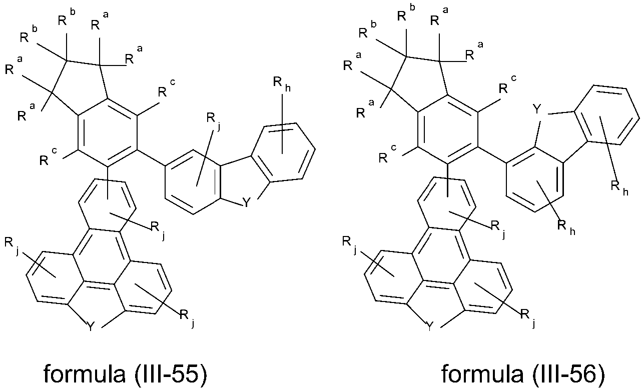 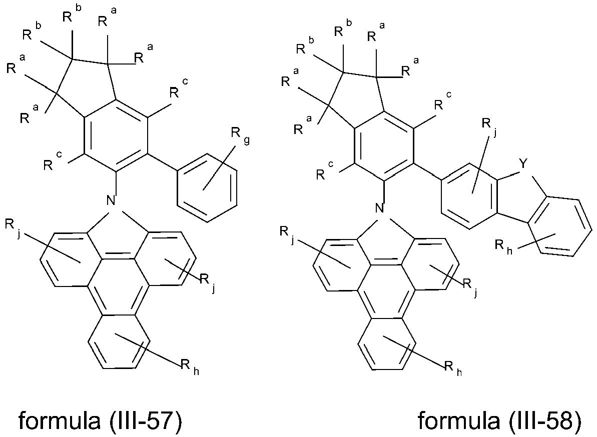 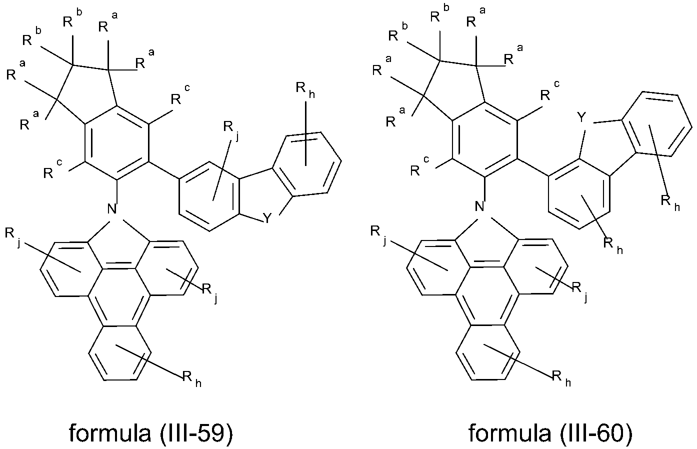 where the symbols R, R^{a}, R^{b} and R^{c} have the meanings given in Claim 1 and the following applies to the symbols used:
Y is O, S, NR or C(R)₂, preferably O, NR or C(R)₂;
i is on each occurrence, independently, 0, 1 or 2;
j is on each occurrence, independently, 0, 1, 2 or 3;
h is on each occurrence, independently, 0, 1, 2, 3 or 4;
g is on each occurrence, independently, 0, 1, 2, 3, 4 or 5.

14. Oligomer, polymer or dendrimer containing one or more compounds according to one of Claims 1 to 13, in which, instead of a hydrogen atom or a substituent, one or more bonds are present from the compounds to the polymer, oligomer or dendrimer.

15. Formulation comprising at least one compound according to one or more of Claims 1 to 13 or an oligomer, polymer or dendrimer according to Claim 14 and at least one further compound, where the further compound is preferably selected from one or more solvents.

16. Composition comprising at least one compound according to one or more of Claims 1 to 13 or an oligomer, polymer or dendrimer according to Claim 14 and at least one further compound selected from the group consisting of fluorescent emitters, phosphorescent emitters, emitters which exhibit TADF, host materials, electron-transport materials, electron-injection materials, hole-conductor materials, hole-injection materials, electron-blocking materials and hole-blocking materials, preferably host materials.

17. Process for the preparation of a compound according to one or more of Claims 1 to 13, **characterised in that** a phenyl compound onto which a cyclopentyl group is condensed is synthesised and at least one aromatic or heteroaromatic radical is introduced, preferably by means of a nucleophilic aromatic substitution reaction or a coupling reaction.

18. Use of a compound according to one or more of Claims 1 to 13 or an oligomer, polymer or dendrimer according to Claim 14 in an electronic device, preferably as host material, electron-transport material, electron-injection material, hole-conductor material, hole-injection material, electron-blocking material, hole-blocking material.

19. Electronic device containing at least one compound according to one or more of Claims 1 to 13 or an oligomer, polymer or dendrimer according to Claim 14.

## Revendications

1. Composé comprenant au moins une structure de formule (I), préférablement un composé de formule (I), 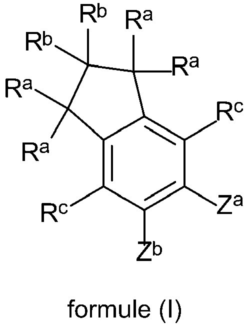 dans laquelle ce qui suit s'applique aux symboles :
Z^{a} représente à chaque occurrence, de manière identique ou différente, Ar^{a}, N(Ar^{c})₂ ou (Ar)N(Ar^{c})₂ ;
Z^{b} représente à chaque occurrence, de manière identique ou différente, Ar^{b}, N(Ar^{d})₂ ou (Ar)N(Ar^{d})₂ ;
R^{a} est à chaque occurrence, de manière identique ou différente, un groupement alkyle, alcoxy ou thioalcoxy à chaîne linéaire ayant de 1 à 10 atomes de C ou un groupement alkyle, alcoxy ou thioalcoxy ramifié ou cyclique ayant de 3 à 10 atomes de C, qui peut dans chaque cas être substitué par un ou plusieurs radicaux R², préférablement un groupement alkyle à chaîne linéaire ayant de 1 à 10 atomes de C ou un groupement alkyle ramifié ou cyclique ayant de 3 à 10 atomes de C, qui peut dans chaque cas être substitué par un ou plusieurs radicaux R², deux, ou plus, substituants R^{a}, préférablement adjacents, peuvent former un noyau les uns avec les autres ;
R^{b} est à chaque occurrence, de manière identique ou différente, un groupement alkyle, alcoxy ou thioalcoxy à chaîne linéaire ayant de 1 à 10 atomes de C ou un groupement alkyle, alcoxy ou thioalcoxy ramifié ou cyclique ayant de 3 à 10 atomes de C, qui peut dans chaque cas être substitué par un ou plusieurs radicaux R², préférablement un groupement alkyle à chaîne linéaire ayant de 1 à 10 atomes de C ou un groupement alkyle ramifié ou cyclique ayant de 3 à 10 atomes de C, qui peut dans chaque cas être substitué par un ou plusieurs radicaux R², deux substituants R^{b} adjacents peuvent former un noyau l'un avec l'autre ;
R^{c} est à chaque occurrence, de manière identique ou différente, H, D, un groupement alkyle, alcoxy ou thioalcoxy à chaîne linéaire ayant de 1 à 10 atomes de C, préférablement de 1 à 6 atomes de C, ou un groupement alkyle, alcoxy ou thioalcoxy ramifié ou cyclique ayant de 3 à 10 atomes de C, qui peut dans chaque cas être substitué par un ou plusieurs radicaux R², préférablement H, D, un groupement alkyle à chaîne linéaire ayant de 1 à 10 atomes de C ou un groupement alkyle ramifié ou cyclique ayant de 3 à 10 atomes de C, qui peut dans chaque cas être substitué par un ou plusieurs radicaux R², de préférence H, D, un groupement alkyle à chaîne linéaire ayant de 1 à 10 atomes de C, préférablement de 1 à 6 atomes de C, particulièrement préférablement H, D ;
Ar^{a}, Ar^{b}, Ar^{c}, Ar^{d} sont à chaque occurrence, de manière identique ou différente, un noyau aromatique ou hétéroaromatique ayant de 6 à 60 atomes de cycle aromatique, qui peut être substitué par un ou plusieurs radicaux R, deux radicaux Ar^{c}, Ar^{d} qui sont liés au même atome de N peuvent également être pontés l'un avec l'autre par une liaison simple ou un pont choisi parmi B(R), C(R)₂, Si(R)₂, C=O, C=NR, C=C(R)₂, RC=CR, O, S, S=O, SO₂, N(R), P(R), P(=O)R et un groupement phénylène à liaison ortho, qui peut être substitué par un ou plusieurs radicaux R, préférablement choisi parmi C(R)₂, O, N(R) et un groupement phénylène à liaison ortho, qui peut être substitué par un ou plusieurs radicaux R, préférablement Ar^{a}, Ar^{b}, Ar^{c}, Ar^{d} représentent à chaque occurrence, de manière identique ou différente, un groupement aryle ou hétéroaryle ayant de 6 à 40 atomes de cycle aromatique, qui peut être substitué par un ou plusieurs radicaux R, deux radicaux Ar, Ar^{c}, Ar^{d} qui sont liés au même atome de N peuvent également être pontés l'un avec l'autre par une liaison simple ou un pont choisi parmi B(R), C(R)₂, Si(R)₂, C=O, C=NR, C=C(R)₂, RC=CR, O, S, S=O, SO₂, N(R), P(R), P(=O)R et un groupement phénylène à liaison ortho, qui peut être substitué par un ou plusieurs radicaux R, préférablement choisi parmi C(R)₂, O, N(R) et un groupement phénylène à liaison ortho, qui peut être substitué par un ou plusieurs radicaux R ;
Ar est à chaque occurrence, de manière identique ou différente, un noyau aromatique ou hétéroaromatique de connexion ayant de 5 à 60 atomes de cycle aromatique, qui peut être substitué par un ou plusieurs radicaux R, un radical Ar peut également être ponté à un, ou les deux, parmi les radicaux Ar^{c}, Ar^{d} qui sont liés au même atome de N par une liaison simple ou un pont choisi parmi B(R), C(R)₂, Si(R)₂, C=O, C=NR, C=C(R)₂, RC=CR, O, S, S=O, SO₂, N(R), P(R), P(=O)R et un groupement phénylène à liaison ortho, qui peut être substitué par un ou plusieurs radicaux R, préférablement choisi parmi C(R)₂, O, N(R) et un groupement phénylène à liaison ortho, qui peut être substitué par un ou plusieurs radicaux R, préférablement Ar représente à chaque occurrence, de manière identique ou différente, un groupement arylène ou hétéroarylène ayant de 5 à 40 atomes de cycle aromatique, qui peut être substitué par un ou plusieurs radicaux R, un radical Ar peut également être ponté à un, ou les deux, parmi les radicaux Ar^{c}, Ar^{d} qui sont liés au même atome de N par une liaison simple ou un pont choisi parmi B(R), C(R)₂, Si(R)₂, C=O, C=NR, C=C(R)₂, RC=CR, O, S, S=O, SO₂, N(R), P(R), P(=O)R et un groupement phénylène à liaison ortho, qui peut être substitué par un ou plusieurs radicaux R, préférablement choisi parmi C(R)₂, O, N(R) et un groupement phénylène à liaison ortho, qui peut être substitué par un ou plusieurs radicaux R ;
R est à chaque occurrence, de manière identique ou différente, H, D, OH, F, CI, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, un groupement alkyle, alcoxy ou thioalcoxy à chaîne linéaire ayant de 1 à 40 atomes de C ou un groupement alcényle ou alcynyle ayant de 2 à 40 atomes de C ou un groupement alkyle, alcoxy ou thioalcoxy ramifié ou cyclique ayant de 3 à 20 atomes de C, où le groupement alkyle, alcoxy, thioalcoxy, alcényle ou alcynyle peut dans chaque cas être substitué par un ou plusieurs radicaux R¹, où un ou plusieurs groupements CH₂ non adjacents peuvent être remplacés par R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO ou SO₂, ou un noyau aromatique ou hétéroaromatique ayant de 5 à 60 atomes de cycle aromatique, qui peut dans chaque cas être substitué par un ou plusieurs radicaux R¹, ou un groupement aryloxy ou hétéroaryloxy ayant de 5 à 60 atomes de cycle aromatique, qui peut être substitué par un ou plusieurs radicaux R¹ ; deux radicaux R peuvent également former un noyau l'un avec l'autre ou un autre groupement ;
Ar' est à chaque occurrence, de manière identique ou différente, un noyau aromatique ou hétéroaromatique ayant de 5 à 60 atomes de cycle aromatique, qui peut être substitué par un ou plusieurs radicaux R¹, deux radicaux Ar' qui sont liés au même atome de C, atome de Si, atome de N, atome de P ou atome de B peuvent également être pontés l'un avec l'autre par une liaison simple ou un pont choisi parmi B(R¹), C(R¹)₂, Si(R¹)₂, C=O, C=NR¹, C=C(R¹)₂, O, S, S=O, SO₂, N(R¹), P(R¹) et P(=O)R¹ ;
R¹ est à chaque occurrence, de manière identique ou différente, H, D, F, Cl, Br, I, CN, NO₂, N(Ar")2, N(R²)2, C(=O)Ar", C(=O)R², P(=O)(Ar")2, P(Ar")₂, B(Ar")₂, B(R²)₂, C(Ar")₃, C(R²)₃, Si(Ar")₃, Si(R²)₃, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C ou un groupement alcényle ayant de 2 à 40 atomes de C, qui peut dans chaque cas être substitué par un ou plusieurs radicaux R², où un ou plusieurs groupements CH₂ non adjacents peuvent être remplacés par -R²C=CR²-, -C≡C-, Si(R²)₂, C=O, C=S, C=Se, C=NR², -C(=O)O-, -C(=O)NR²-, NR², P(=O)(R²), -O-, -S-, SO ou SO₂ et où un ou plusieurs atomes de H peuvent être remplacés par D, F, CI, Br, I, CN ou NO₂, ou un noyau aromatique ou hétéroaromatique ayant de 5 à 60 atomes de cycle aromatique, qui peut dans chaque cas être substitué par un ou plusieurs radicaux R², ou un groupement aryloxy ou hétéroaryloxy ayant de 5 à 60 atomes de cycle aromatique, qui peut être substitué par un ou plusieurs radicaux R², ou un groupement aralkyle ou hétéroaralkyle ayant de 5 à 60 atomes de cycle aromatique, qui peut être substitué par un ou plusieurs radicaux R², ou une combinaison de ces systèmes ; deux, ou plus, radicaux R¹, préférablement adjacents, peuvent former un noyau les uns avec les autres, un ou plusieurs radicaux R¹ peuvent former un noyau avec une autre partie du composé ;
Ar" est à chaque occurrence, de manière identique ou différente, un noyau aromatique ou hétéroaromatique ayant de 5 à 30 atomes de cycle aromatique, qui peut être substitué par un ou plusieurs radicaux R², deux radicaux Ar" qui sont liés au même atome de C, atome de Si, atome de N, atome de P ou atome de B peuvent également être pontés l'un avec l'autre par une liaison simple ou un pont choisi parmi B(R²), C(R²)₂, Si(R²)₂, C=O, C=NR², C=C(R²)₂, O, S, S=O, SO₂, N(R²), P(R²) et P(=O)R² ;
R² est choisi à chaque occurrence, de manière identique ou différente, dans le groupe constitué par H, D, F, CN, un radical hydrocarboné aliphatique ayant de 1 à 20 atomes de C ou un noyau aromatique ou hétéroaromatique ayant de 5 à 30 atomes de cycle aromatique, où un ou plusieurs atomes de H peuvent être remplacés par D, F, CI, Br, I ou CN et qui peut être substitué par un ou plusieurs groupements alkyle, ayant chacun de 1 à 4 atomes de carbone, deux, ou plus, substituants R², préférablement adjacents, peuvent former un noyau les uns avec les autres ;
où les groupements Z^{a} et Z^{b} ne forment pas de noyau.

2. Composé selon la revendication 1, comprenant au moins une structure de formules (I-1) à (I-6), préférablement un composé répondant à l'une des formules (I-1) à (I-6), 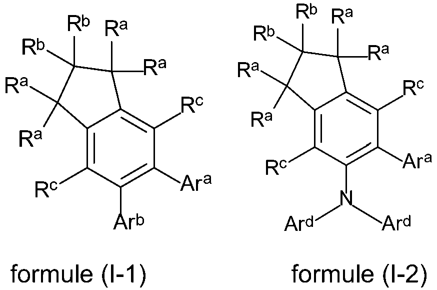 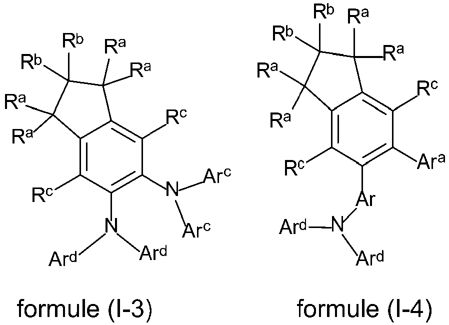 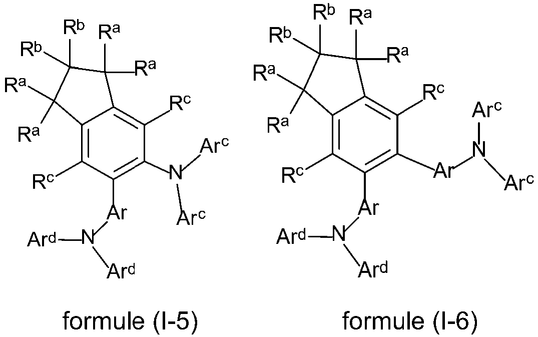 dans lesquelles les symboles Ar, Ar^{a}, Ar^{b}, Ar^{c}, Ar^{d}, R^{a}, R^{b} et R^{c} revêtent les significations données selon la revendication 1.

3. Composé selon la revendication 1 ou 2, **caractérisé en ce que** le groupement Ar^{a}, Ar^{b}, Ar^{c} et/ou Ar^{d} est choisi, de manière identique ou différente à chaque occurrence, parmi les structures de formules (Ar^{a}-1) à (Ar^{a}-29), 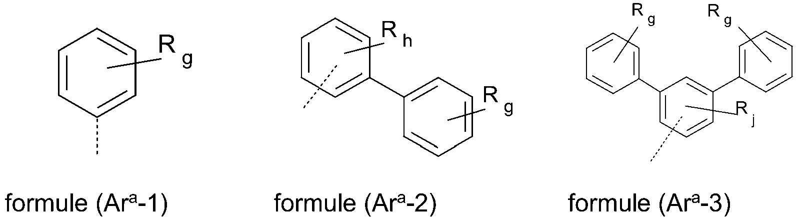 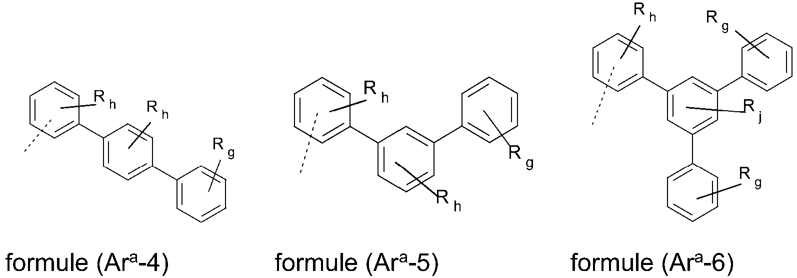 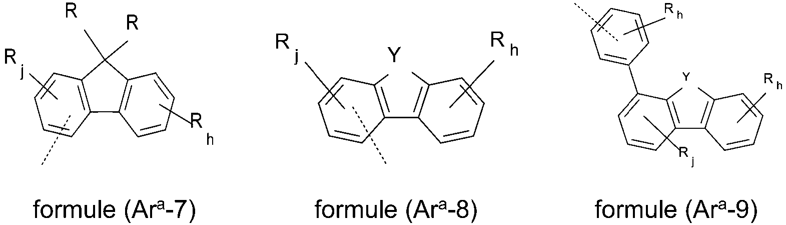 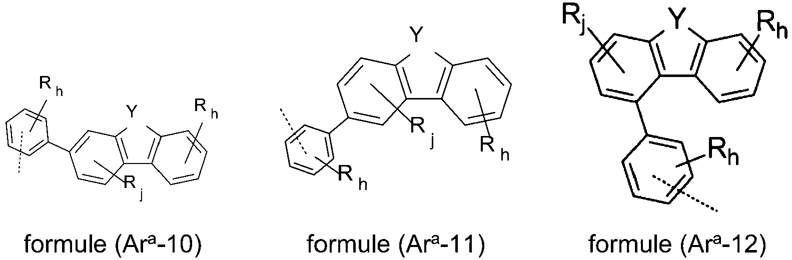 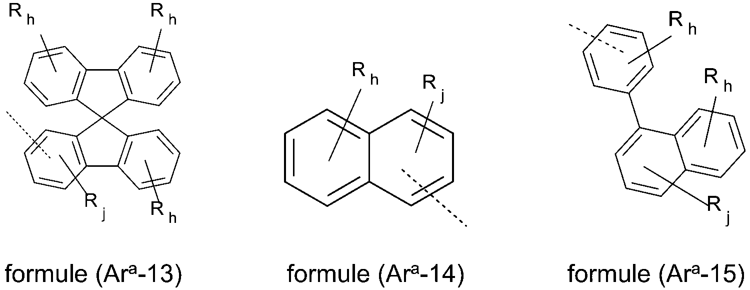 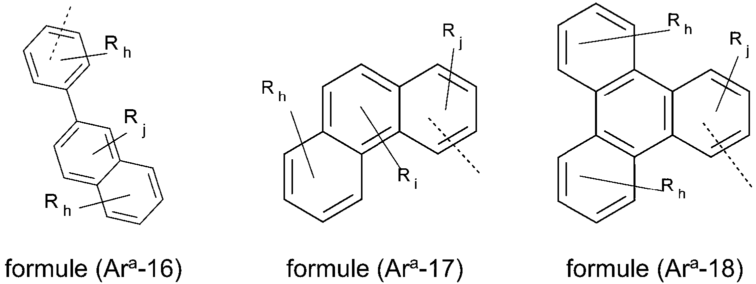 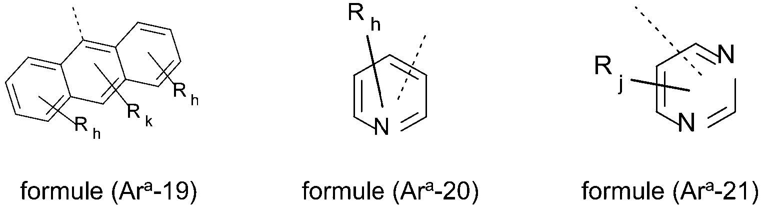 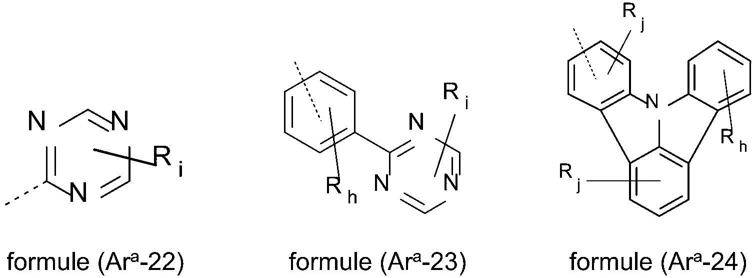 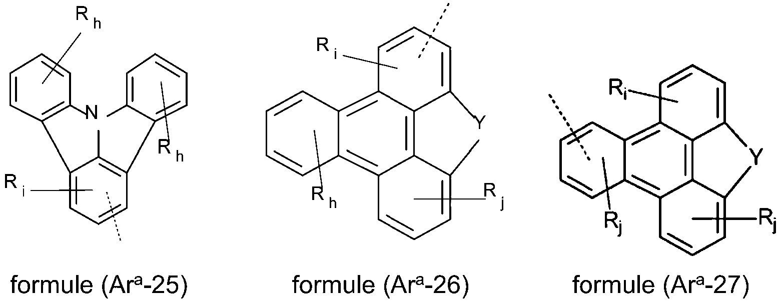 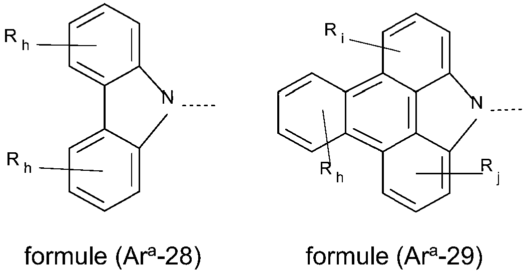 dans lesquelles ce qui suit s'applique aux symboles utilisés :
Y est O, S ou NR, préférablement O ou NR ;
k est à chaque occurrence, indépendamment, 0 ou 1 ;
i est à chaque occurrence, indépendamment, 0, 1 ou 2 ;
j est à chaque occurrence, indépendamment, 0, 1, 2 ou 3 ;
h est à chaque occurrence, indépendamment, 0, 1, 2, 3 ou 4 ;
g est à chaque occurrence, indépendamment, 0, 1, 2, 3, 4 ou 5 ;
R revêt la signification donnée ci-dessus, en particulier selon la revendication 1, et la liaison en pointillés marque la position de liaison.

4. Composé selon l'une ou plusieurs parmi les revendications 1 à 3, **caractérisé en ce que** le groupement Ar^{a} et/ou Ar^{b} représente une structure de formule -Ar^{e}-Q, où Ar^{e} est à chaque occurrence, de manière identique ou différente, un noyau aromatique ou hétéroaromatique de connexion ayant de 5 à 40 atomes de cycle aromatique, qui peut être substitué par un ou plusieurs radicaux R, préférablement Ar^{e} représente à chaque occurrence, de manière identique ou différente, un groupement arylène ou hétéroarylène ayant de 5 à 30 atomes de cycle aromatique, qui peut être substitué par un ou plusieurs radicaux R, est préférablement choisi parmi phénylène, biphénylène, terphénylène, quarterphénylène, fluorénylène, spirobifluorénylène, naphtylène, indolylène, benzofuranylène, benzothiophénylène, carbazolylène, dibenzofuranylène, dibenzothiophénylène, indénocarbazolylène, indolocarbazolylène, pyridinylène, pyrimidinylène, pyrazinylène, pyridazinylène, triazinylène, quinoléinylène, isoquinoléinylène, quinazolinylène, quinoxalinylène, phénanthrénylène ou triphénylénylène, qui peut dans chaque cas être substitué par un ou plusieurs radicaux R, préférablement phénylène, biphénylène, fluorénylène, dibenzofuranylène, triphénylénylène, carbazolylène ou indolocarbazolylène ; et Q représente un groupement de transport d'électrons, préférablement un groupement pyridine, pyrimidine, pyrazine, pyridazine, triazine, quinazoline, quinoxaline, quinoléine, isoquinoléine, imidazole et/ou benzimidazole, qui peut être substitué par un ou plusieurs radicaux R.

5. Composé selon l'une ou plusieurs parmi les revendications 1 à 4, **caractérisé en ce que** le groupement Ar et/ou Ar^{e} est choisi, de manière identique ou différente à chaque occurrence, parmi les structures de formules (Ar^{e}-1) à (Ar^{e}-9), 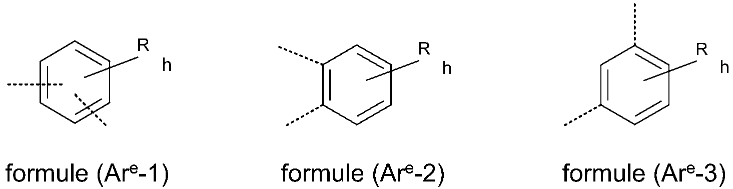 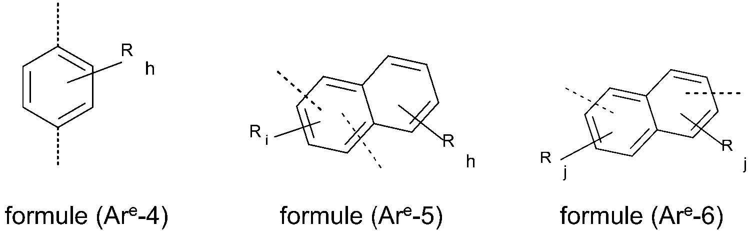 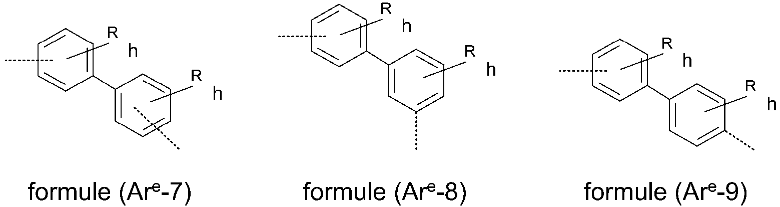 dans lesquelles ce qui suit s'applique aux symboles utilisés :
j est à chaque occurrence, indépendamment, 0, 1, 2 ou 3 ;
h est à chaque occurrence, indépendamment, 0, 1, 2, 3 ou 4 ;
R revêt la signification donnée ci-dessus, en particulier selon la revendication 1, et les liaisons en pointillés marquent la position de liaisons.

6. Composé selon l'une ou plusieurs parmi les revendications 1 à 3 et 5, **caractérisé en ce que** le composé comprend au moins une structure de formule (I-2), où le groupement Ar^{a} est choisi parmi les structures de formules (Ar^{a}-1) à (Ar^{a}-18), où, dans les structures de formules (Ar^{a}-8) à (Ar^{a}-12), le groupement Y est préférablement O ou NR, et les groupements Ar^{d} sont choisis à chaque occurrence, de manière identique ou différente, parmi les structures de formules (Ar^{a}-1) à (Ar^{a}-23), où, dans les structures de formules (Ar^{a}-8) à (Ar^{a}-12), le groupement Y est préférablement O ou NR, où les groupements Ar^{d} sont préférablement choisis à chaque occurrence, de manière identique ou différente, parmi les structures de formules (Ar^{a}-1), (Ar^{a}-2) et (Ar^{a}-7) à (Ar^{a}-18), et/ou le composé comprend au moins une structure de formule (I-4), où le groupement Ar est choisi parmi les structures de formules (Ar^{e}-1) à (Ar^{e}-4), le groupement Ar^{a} est choisi parmi les structures de formules (Ar^{a}-1) à (Ar^{a}-18), où, dans les structures de formules (Ar^{a}-8) à (Ar^{a}-12), le groupement Y est préférablement O ou NR, et les groupements Ar^{d} sont choisis à chaque occurrence, de manière identique ou différente, parmi les structures de formules (Ar^{a}-1) à (Ar^{a}-23), où, dans les structures de formules (Ar^{a}-8) à (Ar^{a}-12), le groupement Y est préférablement O ou NR, où les groupements Ar^{d} sont préférablement choisis à chaque occurrence, de manière identique ou différente, parmi les structures de formules (Ar^{a}-1), (Ar^{a}-2), (Ar^{a}-7) à (Ar^{a}-18).

7. Composé selon l'une ou plusieurs parmi les revendications 1 à 3 et 5, **caractérisé en ce que** le composé comprend au moins une structure de formule (I-3), où les groupements Ar^{c} sont choisis à chaque occurrence, de manière identique ou différente, parmi les structures de formules (Ar^{a}-1) à (Ar^{a}-23), où, dans les structures de formules (Ar^{a}-8) à (Ar^{a}-12), le groupement Y est préférablement O ou NR, les groupements Ar^{c} sont préférablement choisis à chaque occurrence, de manière identique ou différente, parmi les structures de formules (Ar^{a}-1), (Ar^{a}-2), (Ar^{a}-7) à (Ar^{a}-18) ; et les groupements Ar^{d} sont choisis à chaque occurrence, de manière identique ou différente, parmi les structures de formules (Ar^{a}-1) à (Ar^{a}-23), où, dans les structures de formules (Ar^{a}-8) à (Ar^{a}-12), le groupement Y est préférablement O ou NR, où les groupements Ar^{d} sont préférablement choisis à chaque occurrence, de manière identique ou différente, parmi les structures de formules (Ar^{a}-1), (Ar^{a}-2), (Ar^{a}-7) à (Ar^{a}-18), et/ou le composé comprend au moins une structure de formule (I-5) et/ou de formule (I-6), où le groupement Ar est choisi à chaque occurrence, de manière identique ou différente, parmi les structures de formules (Ar^{e}-1) à (Ar^{e}-4), les groupements Ar^{c} sont choisis à chaque occurrence, de manière identique ou différente, parmi les structures de formules (Ar^{a}-1) à (Ar^{a}-23), où, dans les structures de formules (Ar^{a}-8) à (Ar^{a}-12), le groupement Y est préférablement O ou NR, les groupements Ar^{c} sont préférablement choisis à chaque occurrence, de manière identique ou différente, parmi les structures de formules (Ar^{a}-1), (Ar^{a}-2), (Ar^{a}-7) à (Ar^{a}-18) ; et les groupements Ar^{d} sont choisis à chaque occurrence, de manière identique ou différente, parmi les structures de formules (Ar^{a}-1) à (Ar²-18), où, dans les structures de formules (Ar^{a}-8) à (Ar^{a}-12), le groupement Y est préférablement O ou NR, où les groupements Ar^{d} sont préférablement choisis à chaque occurrence, de manière identique ou différente, parmi les structures de formules (Ar^{a}-1), (Ar^{a}-2), (Ar^{a}-7) à (Ar^{a}-18).

8. Composé selon la revendication 4, **caractérisé en ce que** le composé comprend au moins une structure de formule (I-2), où le groupement Ar^{a} représente une structure de formule Ar^{e}-Q, où les symboles Ar^{e} et Q revêtent la signification donnée selon la revendication 5 et les groupements Ar^{d} sont choisis à chaque occurrence, de manière identique ou différente, parmi les structures de formules (Ar^{a}-1) à (Ar^{a}-23), où, dans les structures de formules (Ar^{a}-8) à (Ar^{a}-12), le groupement Y est préférablement O ou NR, où les groupements Ar^{d} sont préférablement choisis à chaque occurrence, de manière identique ou différente, parmi les structures de formules (Ar^{a}-1), (Ar^{a}-2), (Ar^{a}-7) à (Ar²-18), et/ou le composé comprend au moins une structure de formule (I-4), où le groupement Ar est choisi parmi les structures de formules (Ar^{e}-1) à (Ar^{e}-4), le groupement Ar^{a} représente une structure de formule Ar^{e}-Q, où les symboles Ar^{e} et Q revêtent la signification donnée selon la revendication 5 et les groupements Ar^{d} sont choisis à chaque occurrence, de manière identique ou différente, parmi les structures de formules (Ar^{a}-1) à (Ar^{a}-23), où, dans les structures de formules (Ar^{a}-8) à (Ar^{a}-12), le groupement Y est préférablement O ou NR, où les groupements Ar^{d} sont préférablement choisis à chaque occurrence, de manière identique ou différente, parmi les structures de formules (Ar^{a}-1), (Ar^{a}-2), (Ar^{a}-7) à (Ar^{a}-18).

9. Composé selon l'une ou plusieurs parmi les revendications 1 à 5, **caractérisé en ce que** le composé comprend au moins une structure de formule (I-1) et/ou de formule (I-2), où le groupement Ar^{a} comprend un groupement de transport d'électrons ou représente un groupement de transport d'électrons, où le groupement Ar^{a} comprend préférablement un groupement pyridine, pyrimidine, pyrazine, pyridazine, triazine, quinazoline, quinoxaline, quinoléine, isoquinoléine, imidazole et/ou benzimidazole ou représente l'un desdits groupements, où ceux-ci peuvent être substitués par un ou plusieurs radicaux R, les groupements triazine étant particulièrement préférés.

10. Composé selon la revendication 9, **caractérisé en ce que** le composé comprend au moins une structure de formule (I-1), où le groupement Ar^{b} comprend un groupement de transport d'électrons ou représente un groupement de transport d'électrons, où le groupement Ar^{a} comprend préférablement un groupement pyridine, pyrimidine, pyrazine, pyridazine, triazine, quinazoline, quinoxaline, quinoléine, isoquinoléine, imidazole et/ou benzimidazole ou représente l'un desdits groupements, où ceux-ci peuvent être substitués par un ou plusieurs radicaux R, les groupements triazine étant particulièrement préférés.

11. Composé selon la revendication 9, **caractérisé en ce que** le composé comprend au moins une structure de formule (I-1), où le groupement Ar^{b} est choisi parmi les structures de formules (Ar^{a}-1) à (Ar^{a}-18), où, dans les structures de formules (Ar^{a}-8) à (Ar^{a}-12), le groupement Y est préférablement O ou NR ; où le groupement Ar^{a} préférablement représente une structure de formule Ar^{e}-Q, où les symboles Ar^{e} et Q revétent la signification donnée selon la revendication 5.

12. Composé selon l'une ou plusieurs parmi les revendications 1 à 11, comprenant au moins une structure de formules (II-1) à (11-154), préférablement un composé répondant à l'une des formules (II-1) à (II-154), 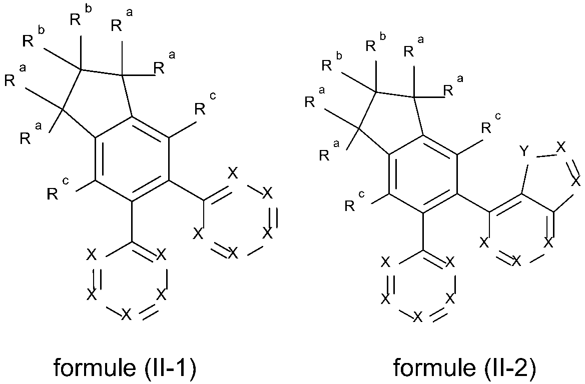 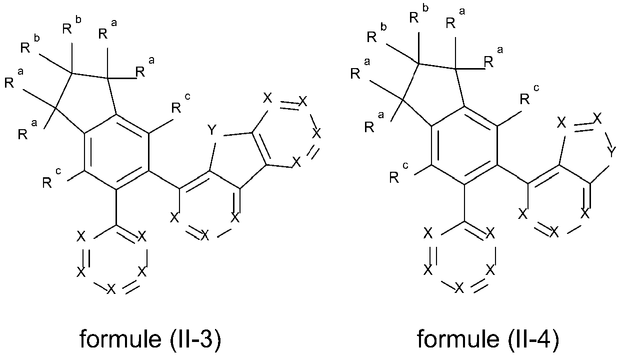 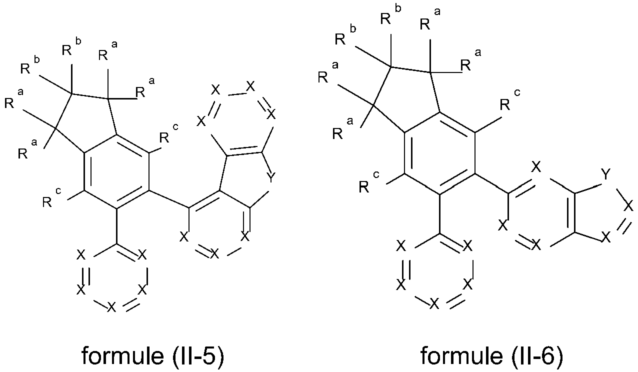 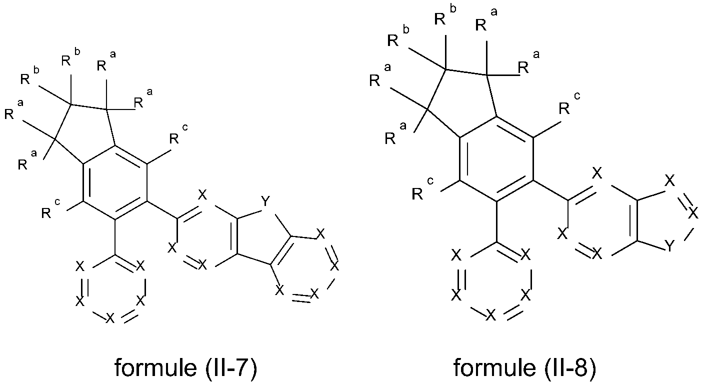 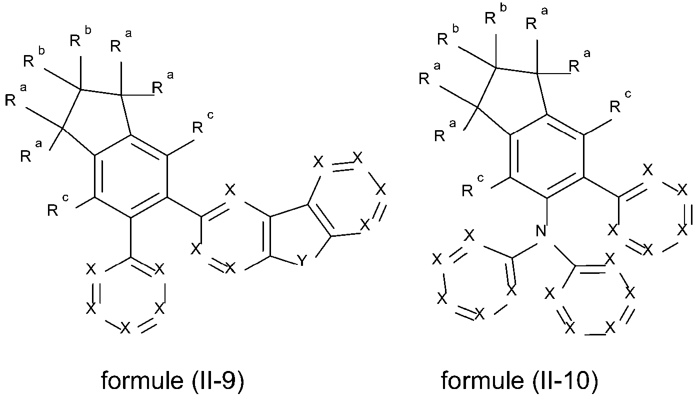 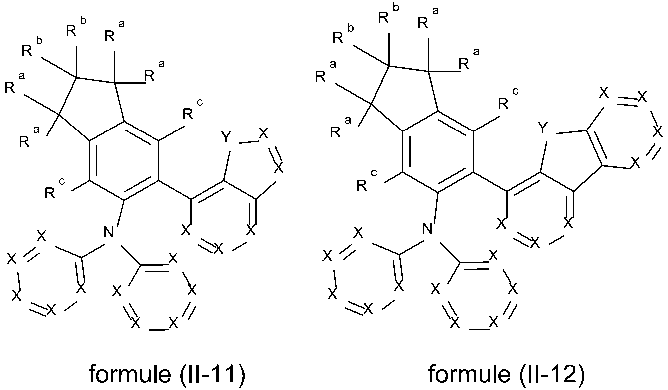 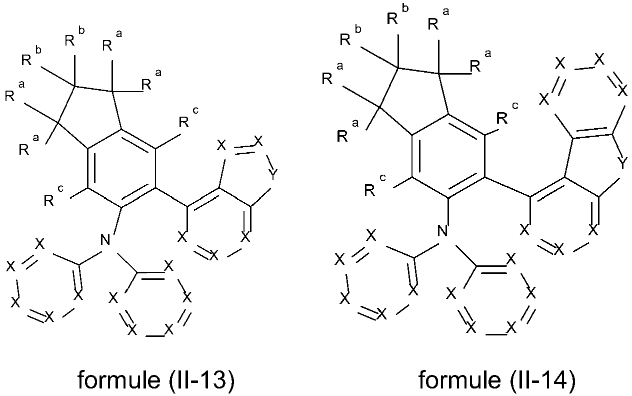 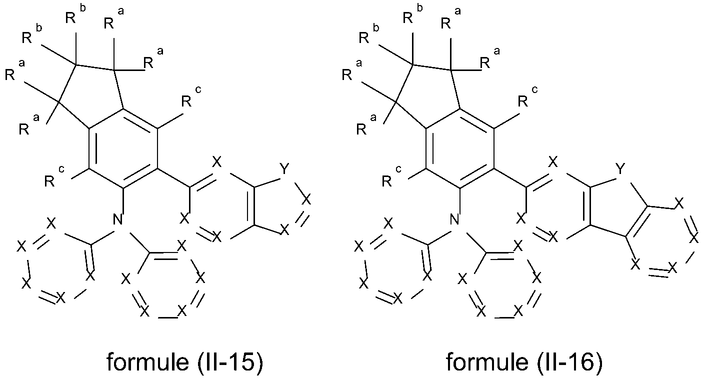 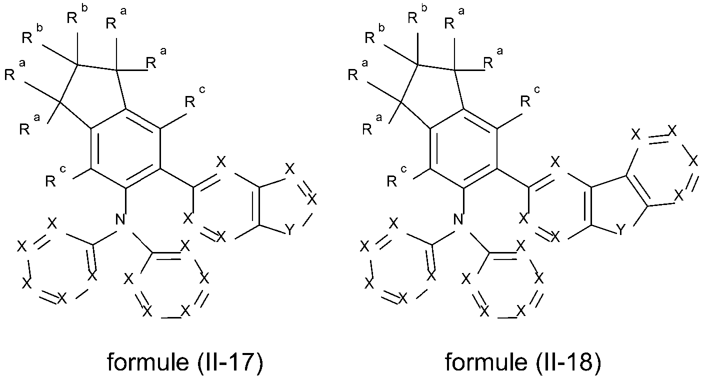 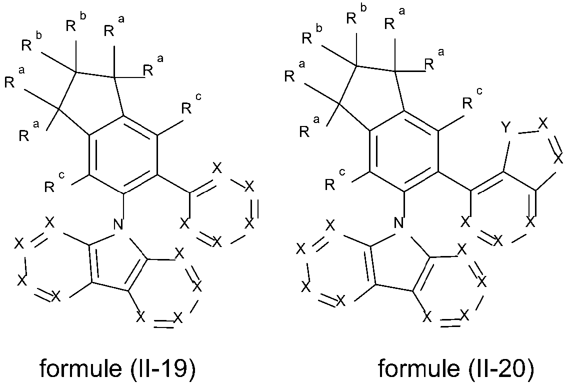 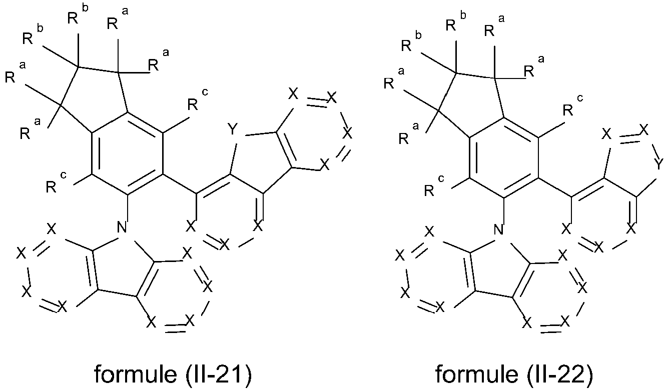 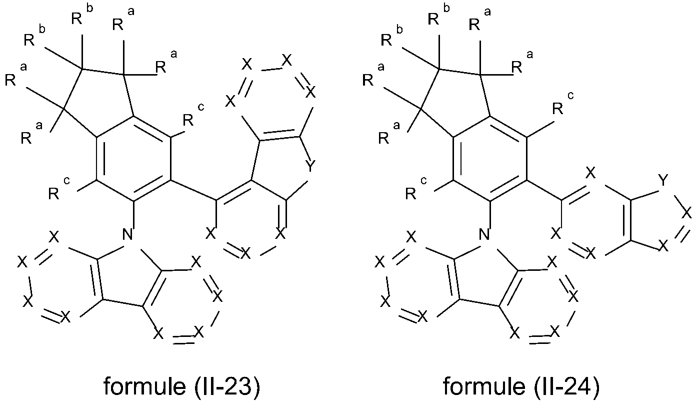 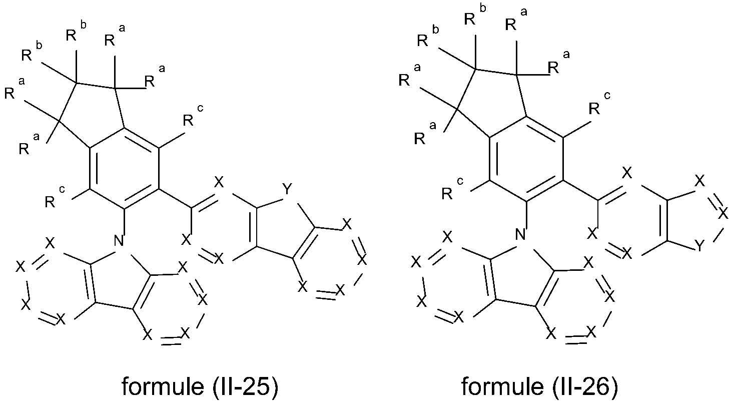 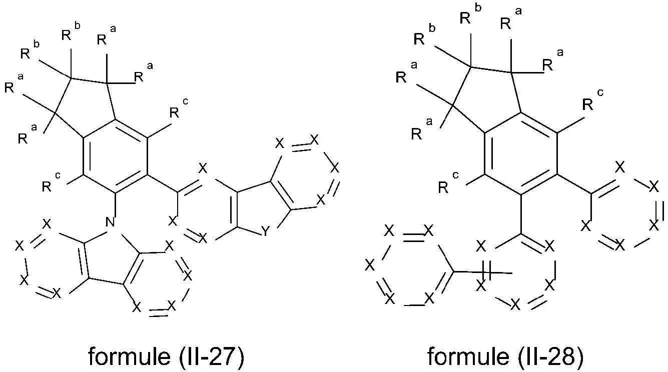 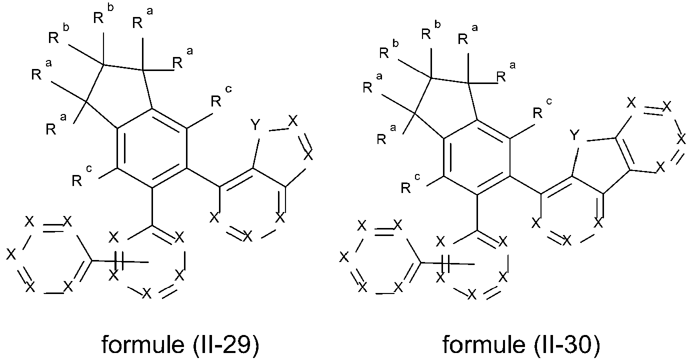 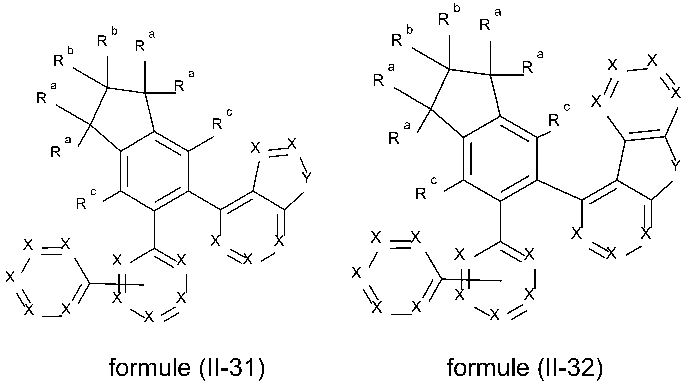 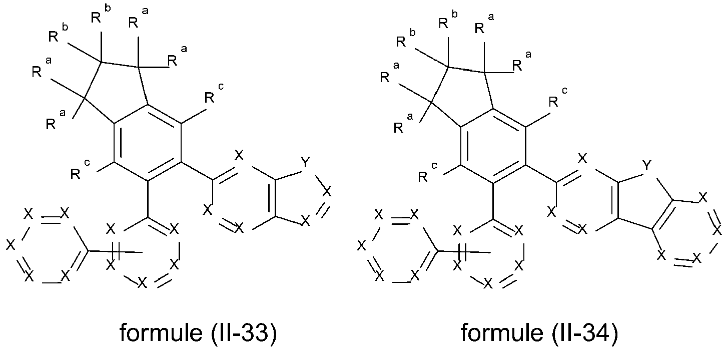 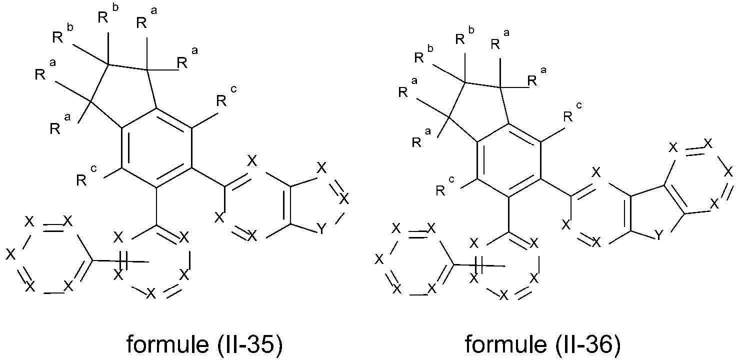 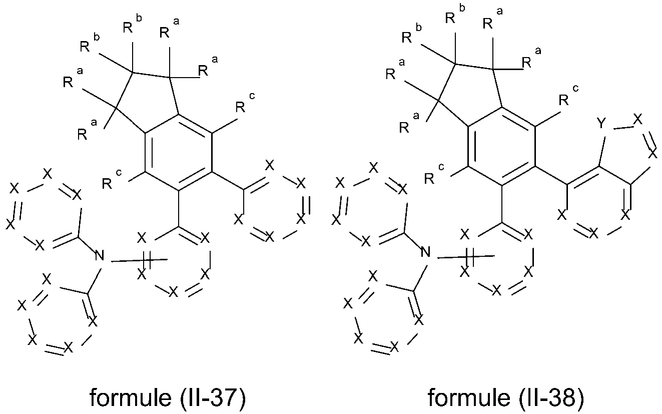 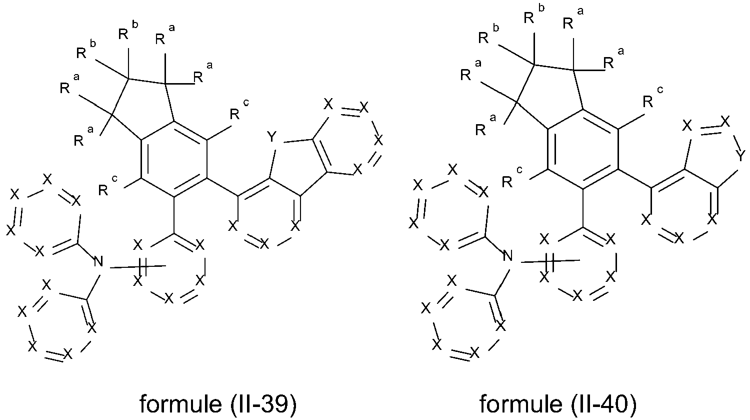 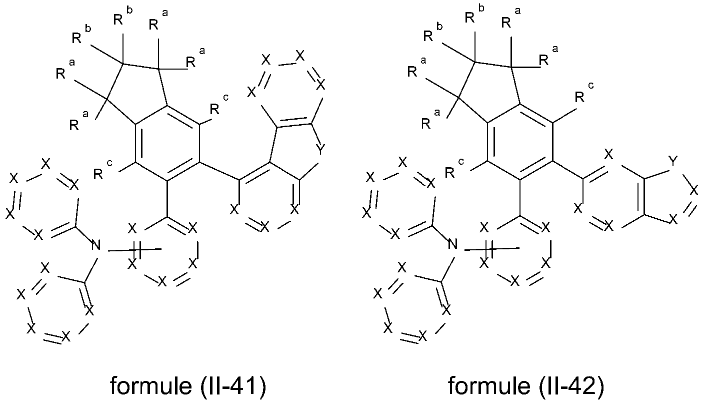 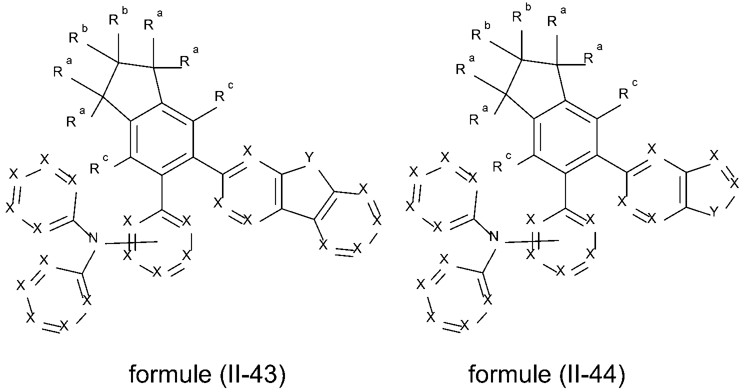 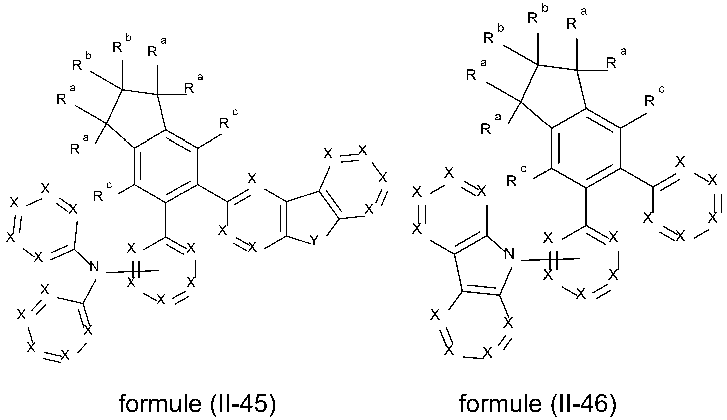 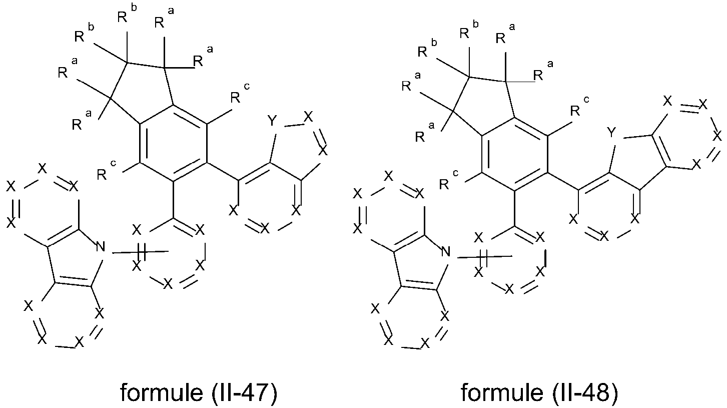 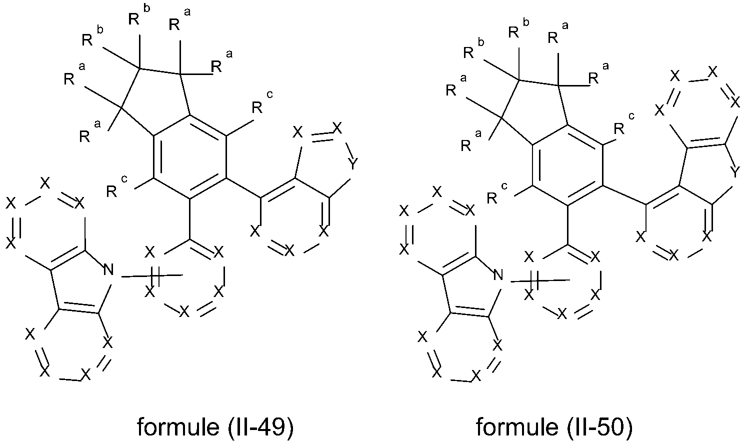 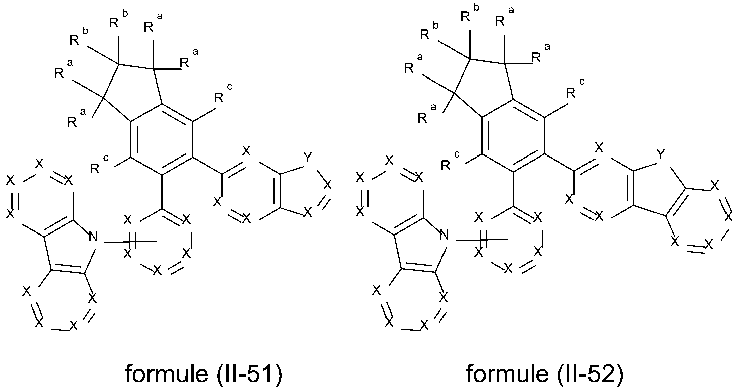 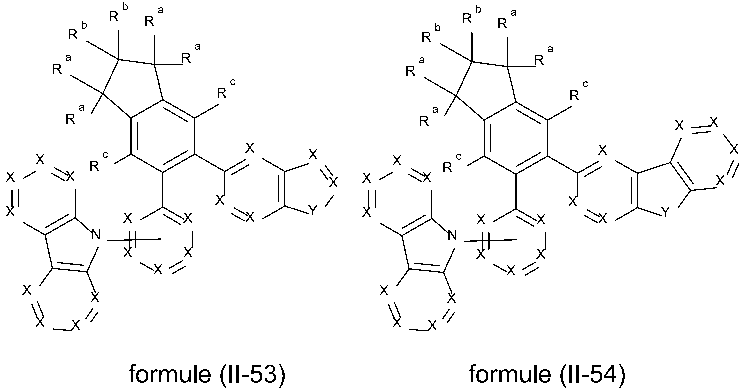 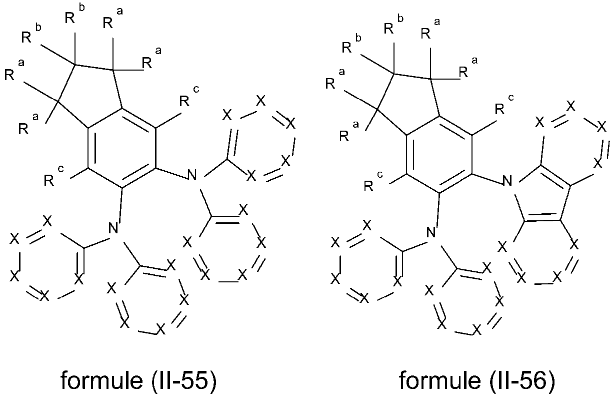 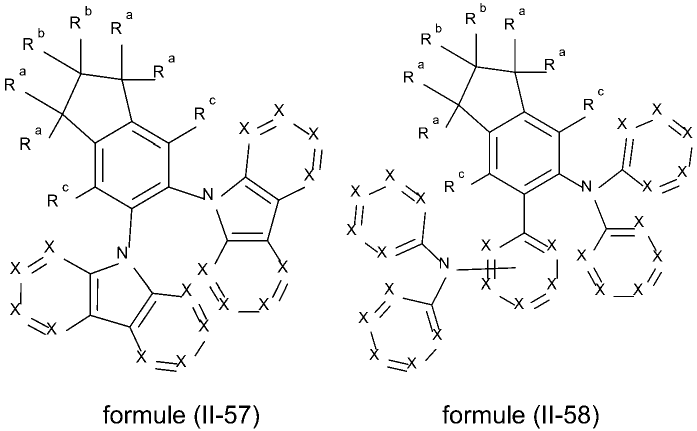 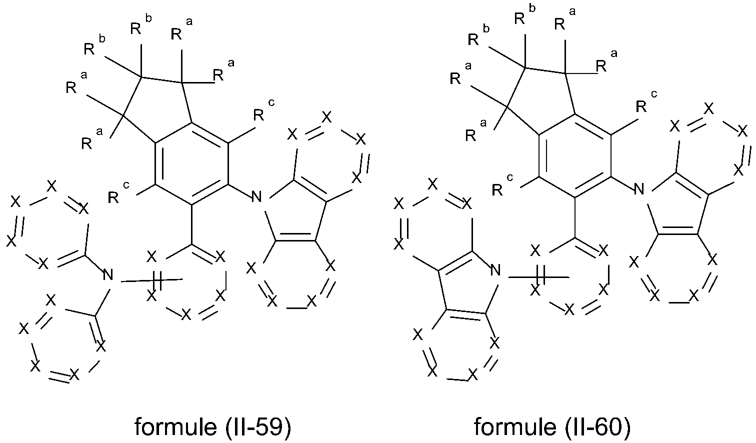 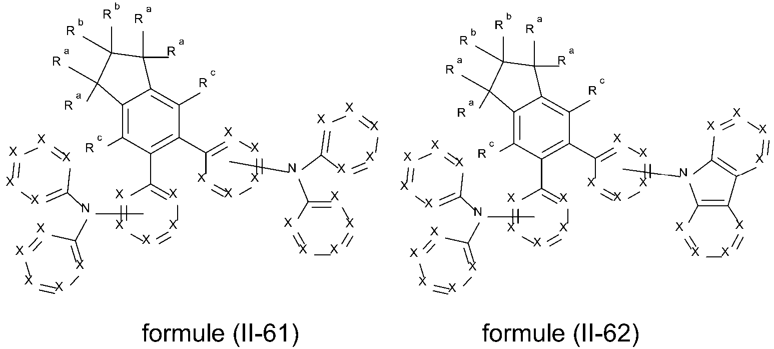 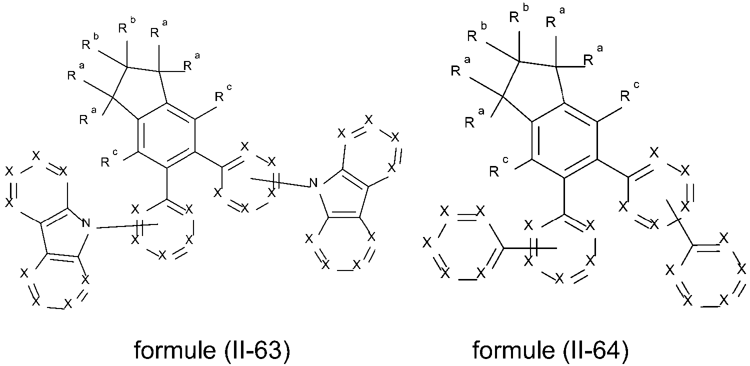 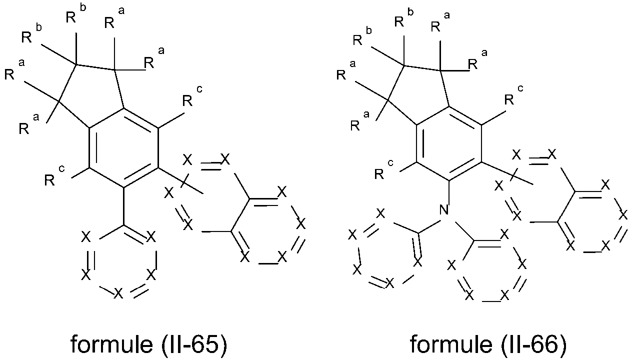 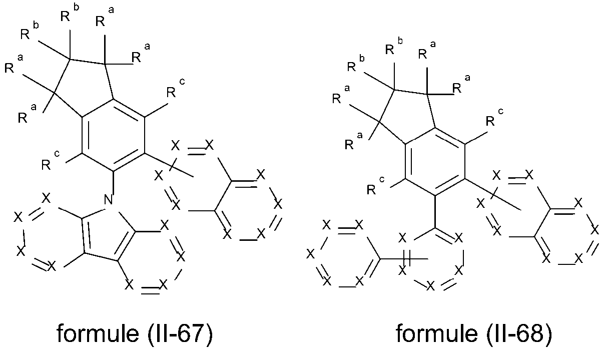 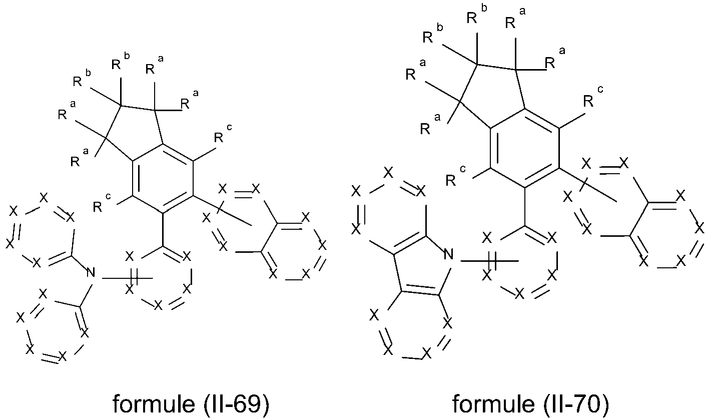 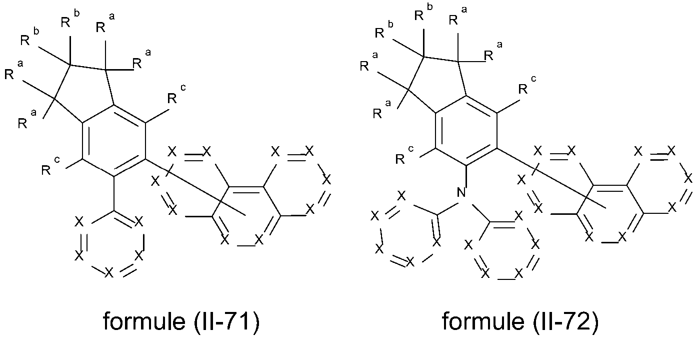 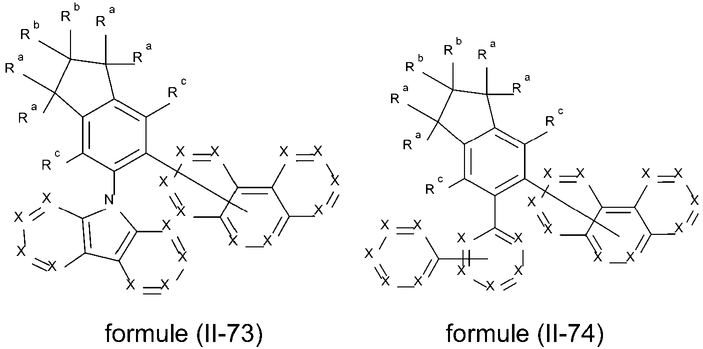 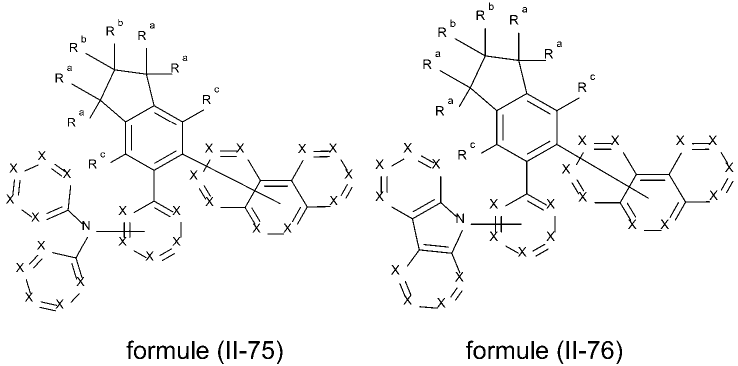 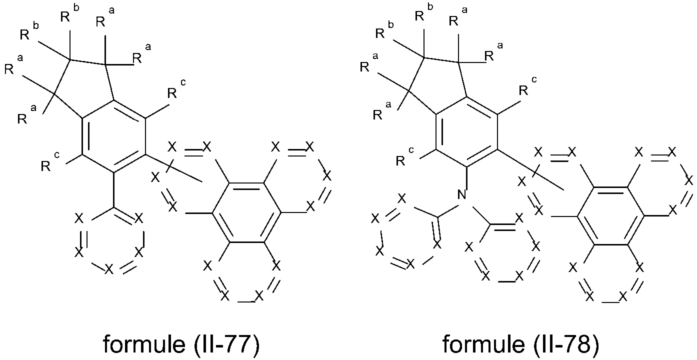 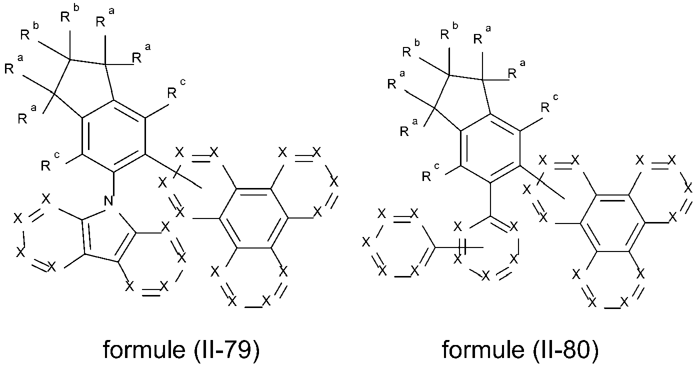 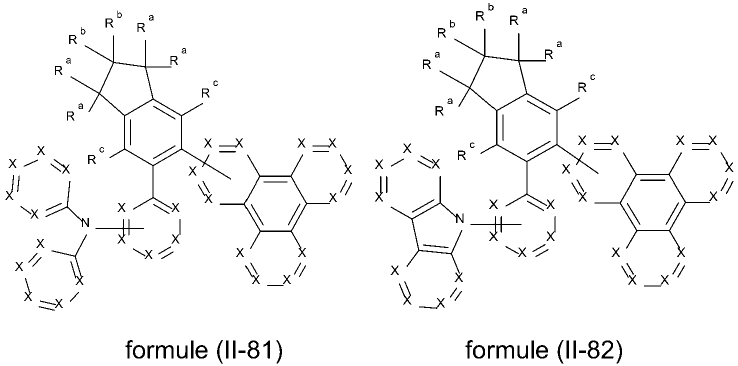 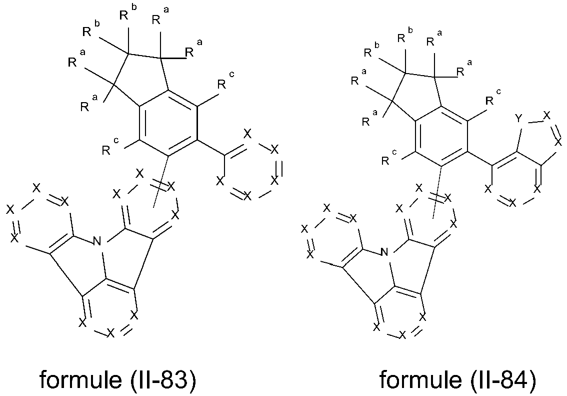 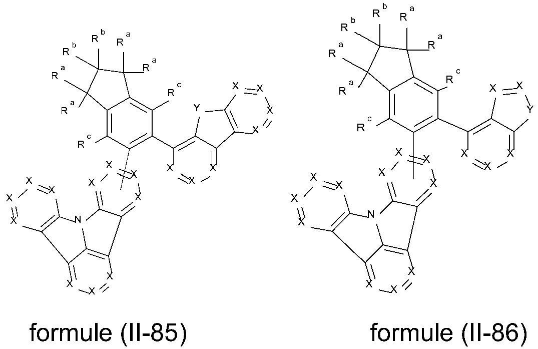 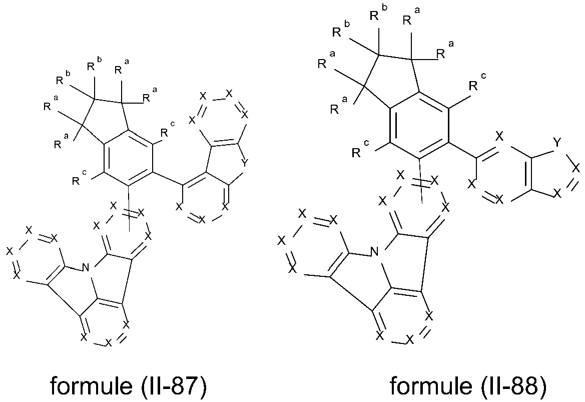 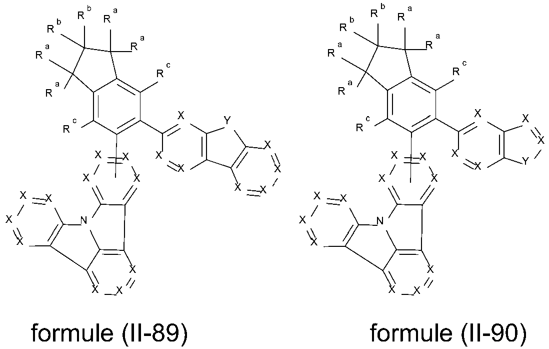 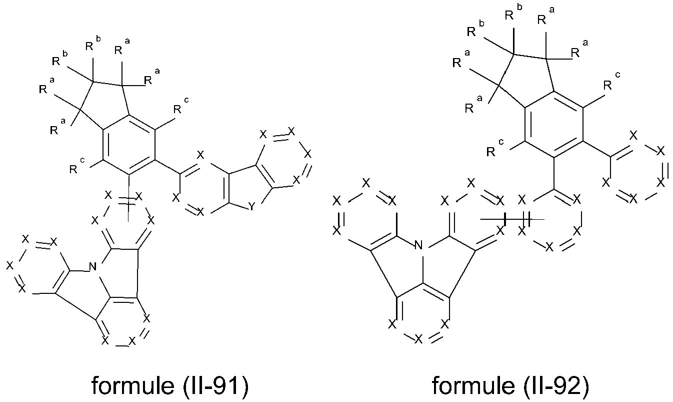 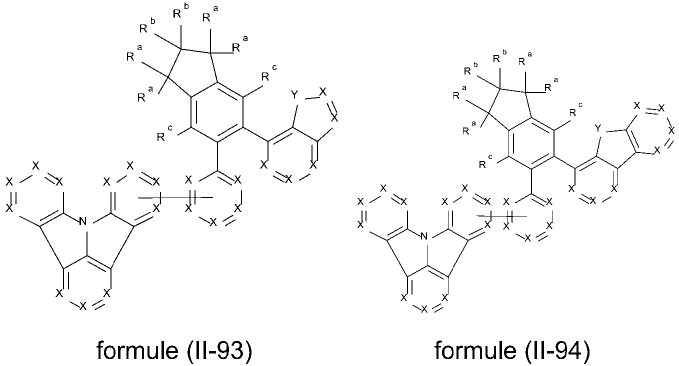 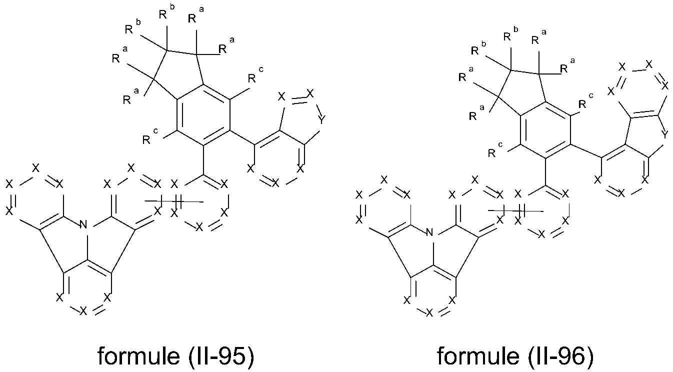 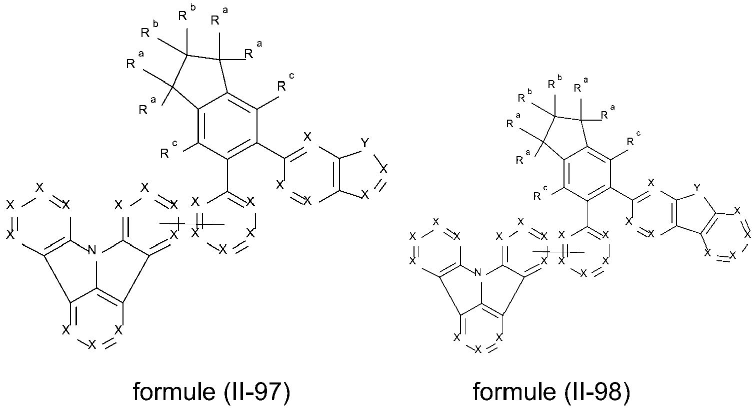 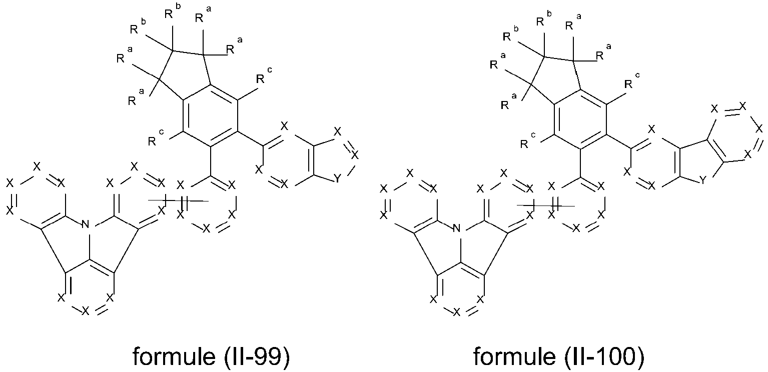 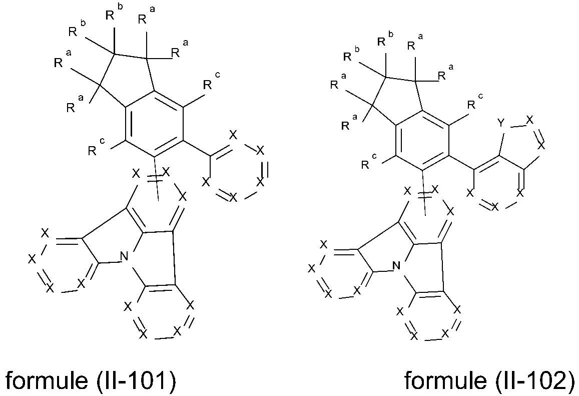 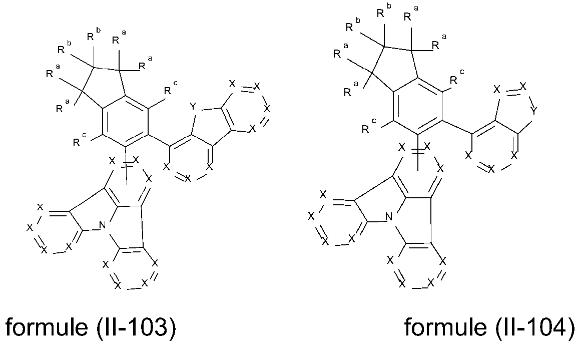 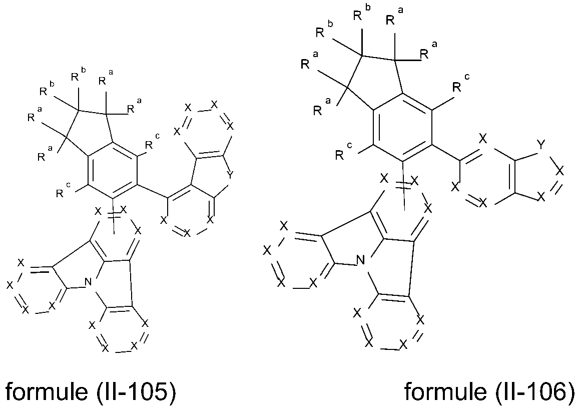 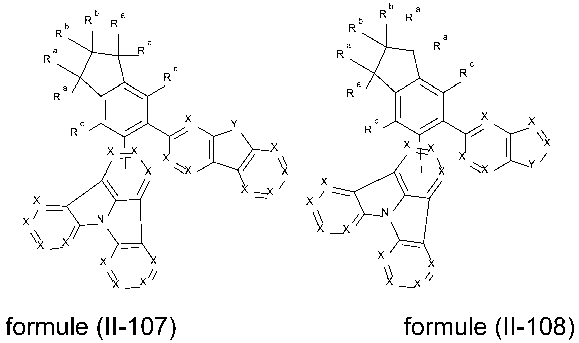 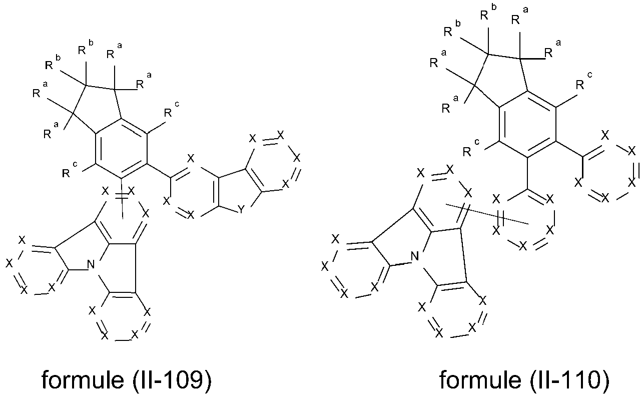 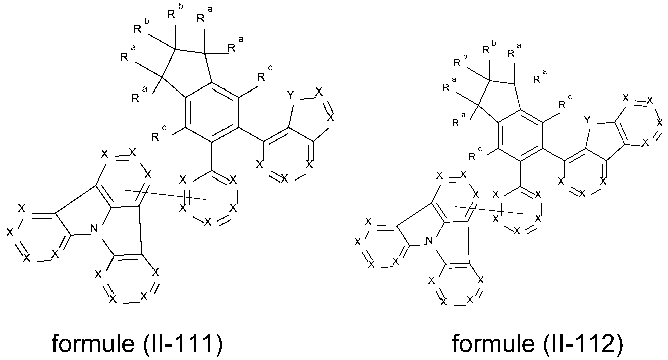 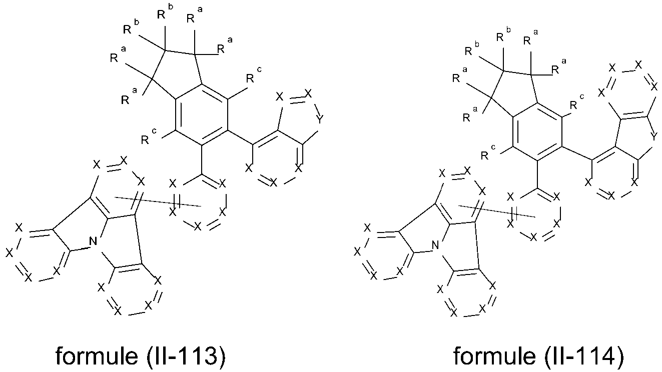 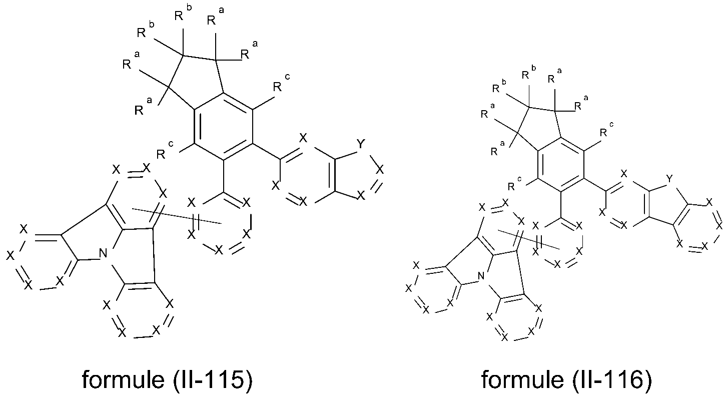 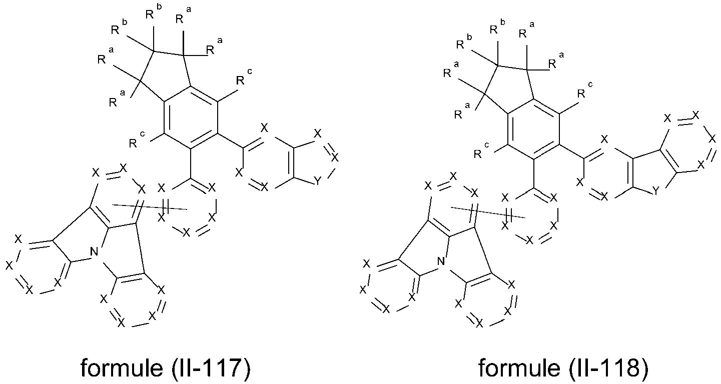 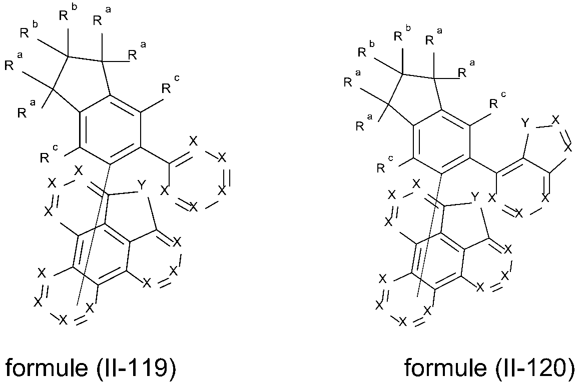 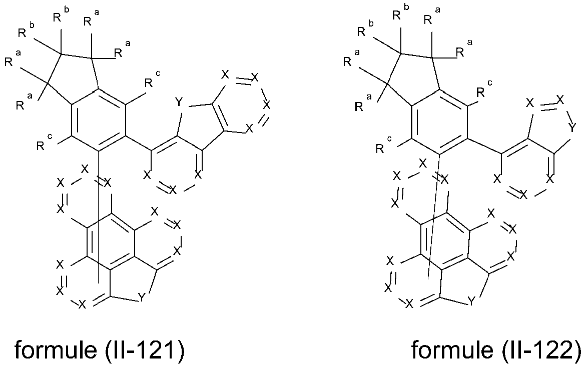 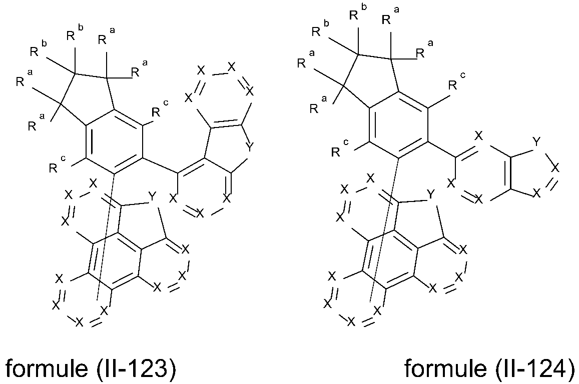 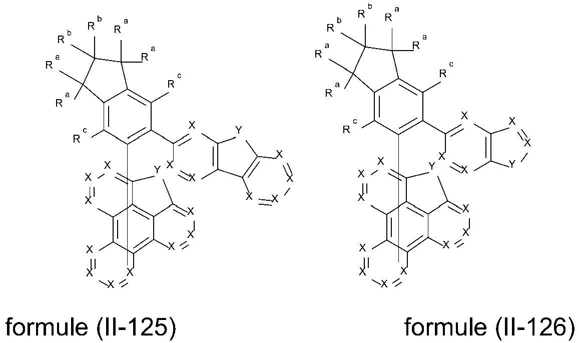 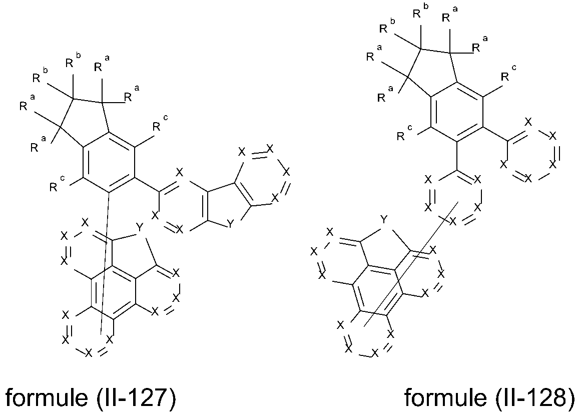 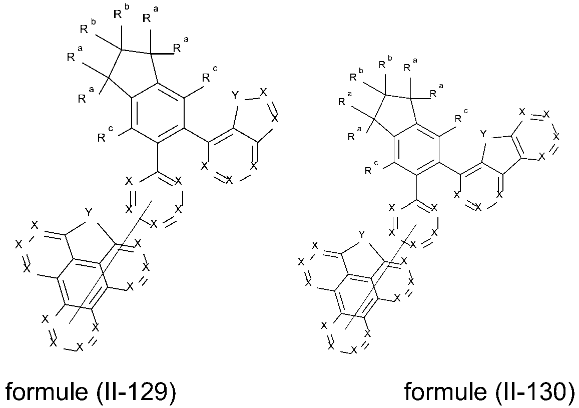 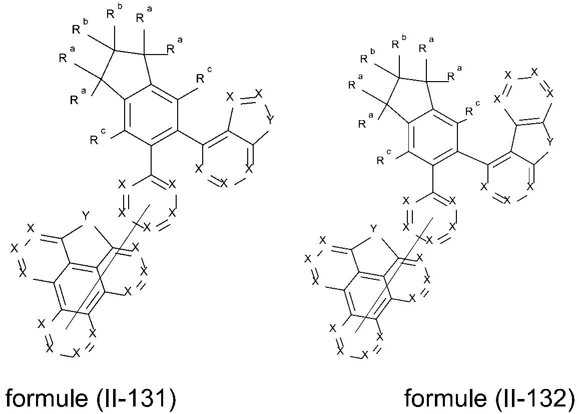 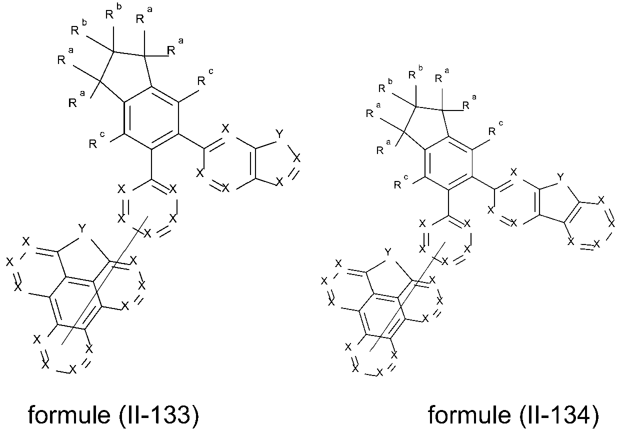 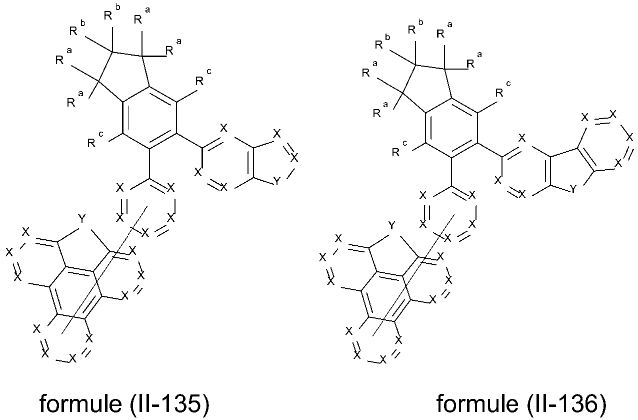 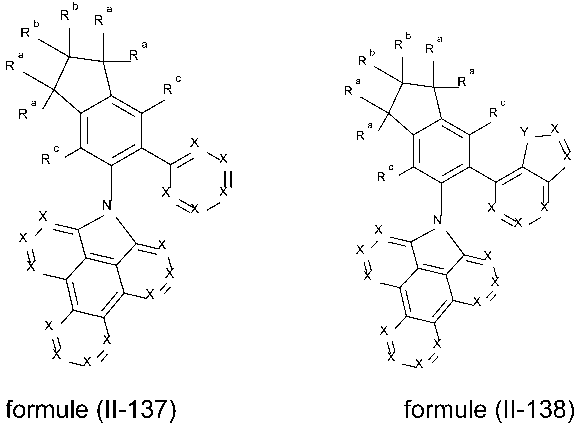 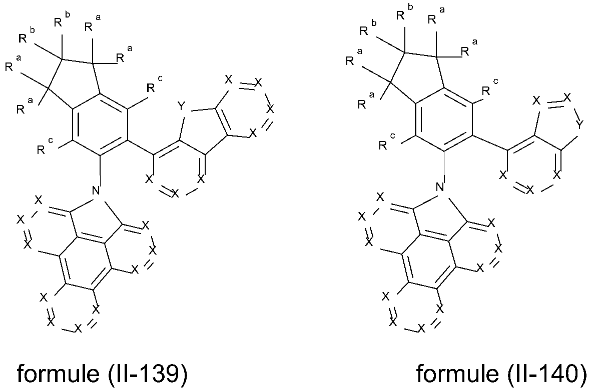 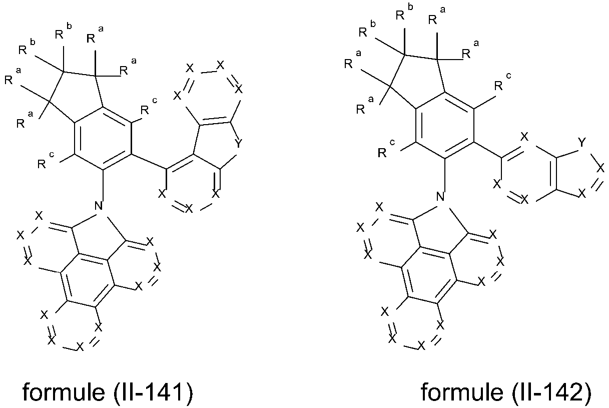 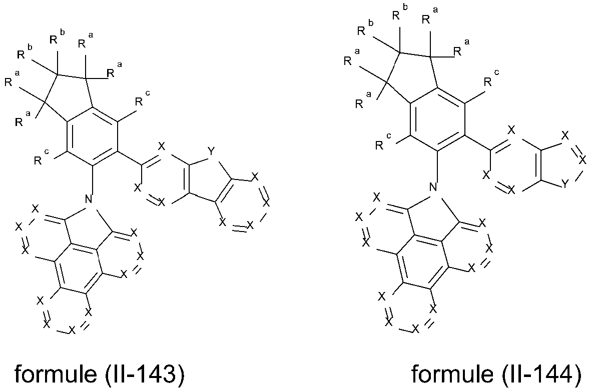 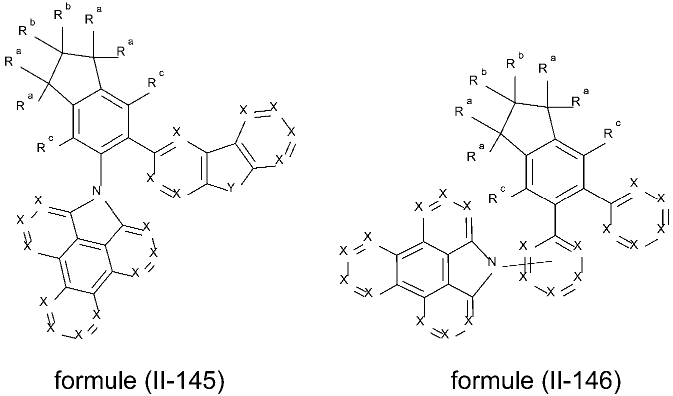 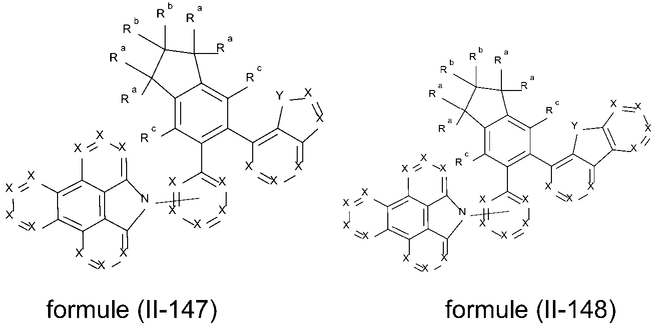 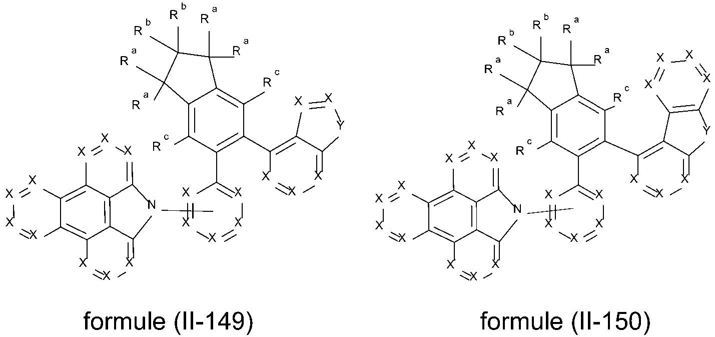 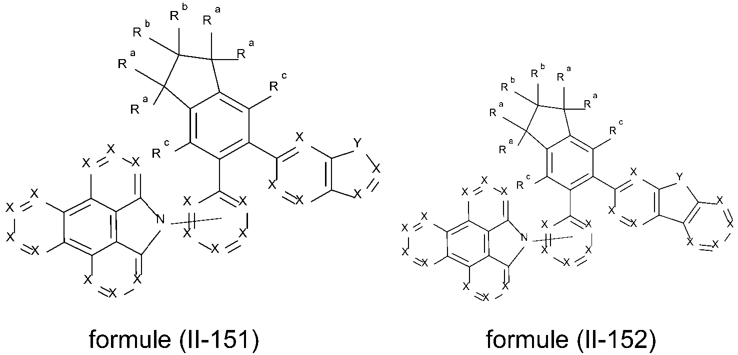 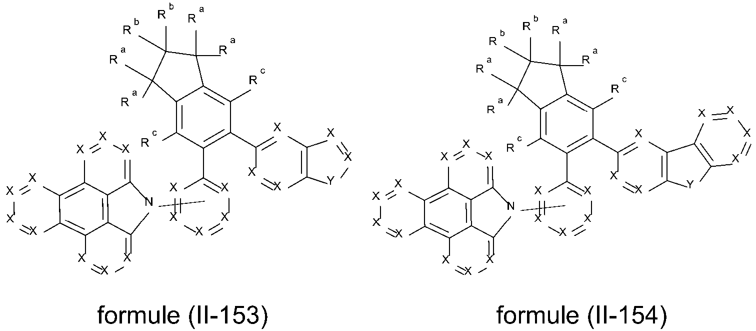 dans lesquelles les symboles R, R^{a}, R^{b} et R^{c} revêtent les significations données selon la revendication 1 et ce qui suit s'applique aux autres symboles :
X représente à chaque occurrence, de manière identique ou différente, N, CR ou C dans le cas où un groupement est lié à la structure ; et
Y représente O, S, NR ou C(R)₂, préférablement O, NR ou C(R)₂.

13. Composé selon l'une ou plusieurs parmi les revendications 1 à 12, comprenant au moins une structure de formules (III-1) à (III-60), préférablement un composé répondant à l'une des formules (III-1) à (III-60), 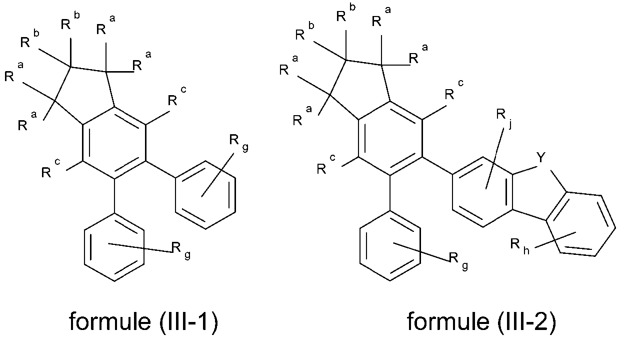 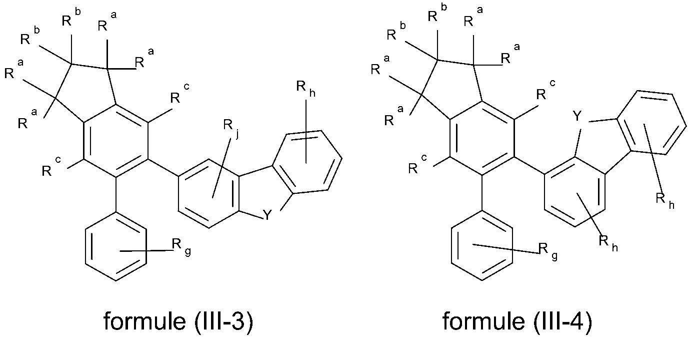 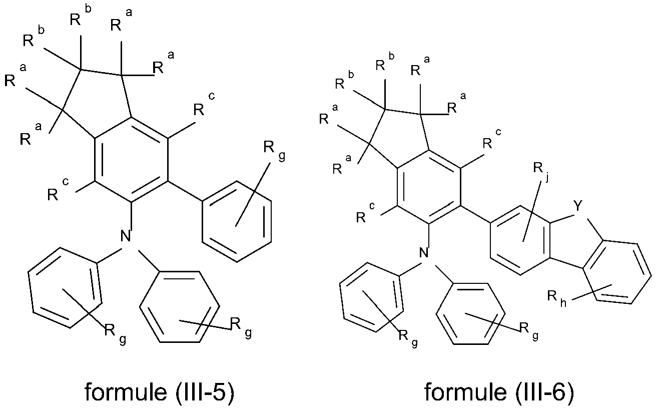 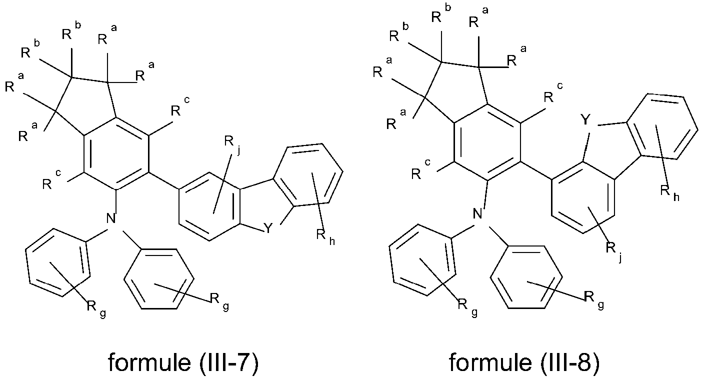 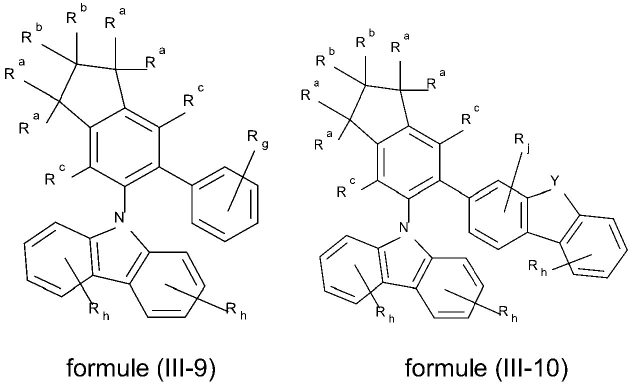 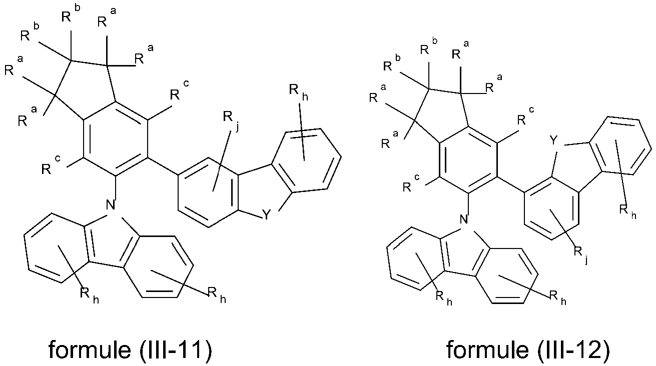 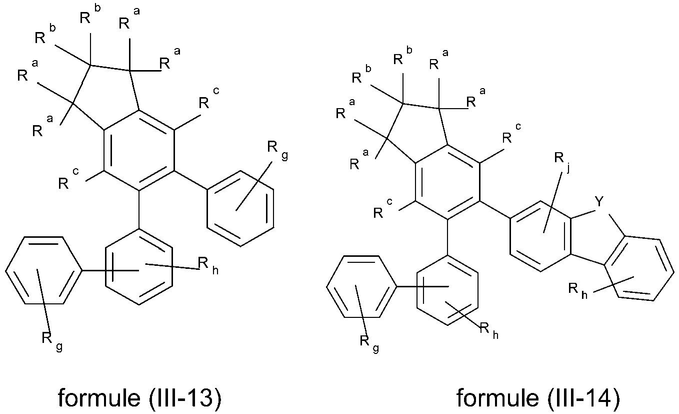 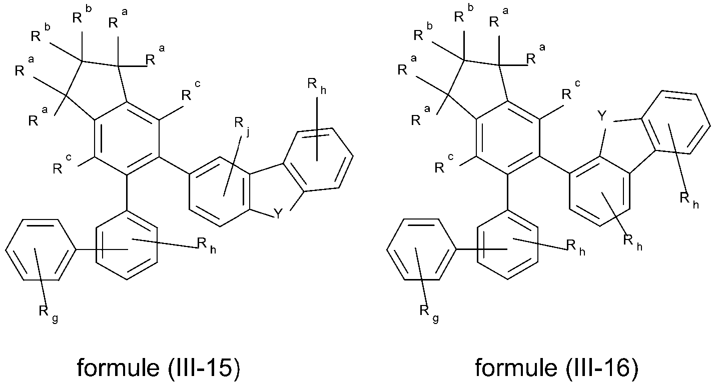 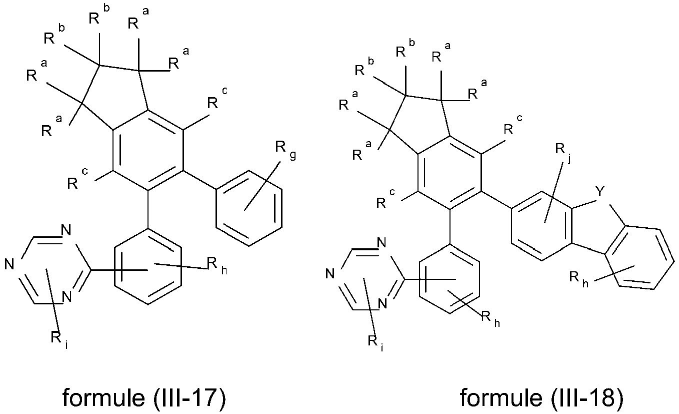 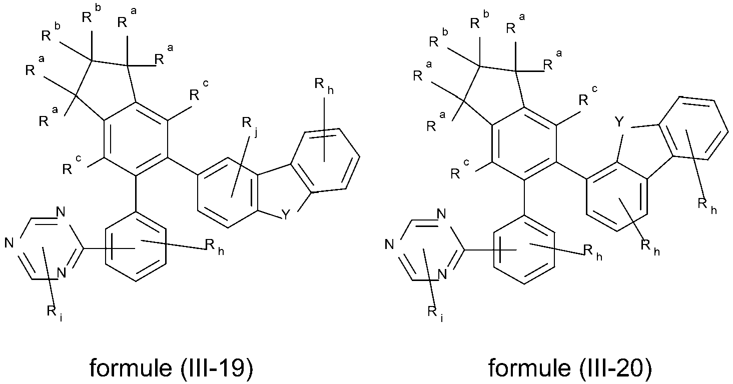 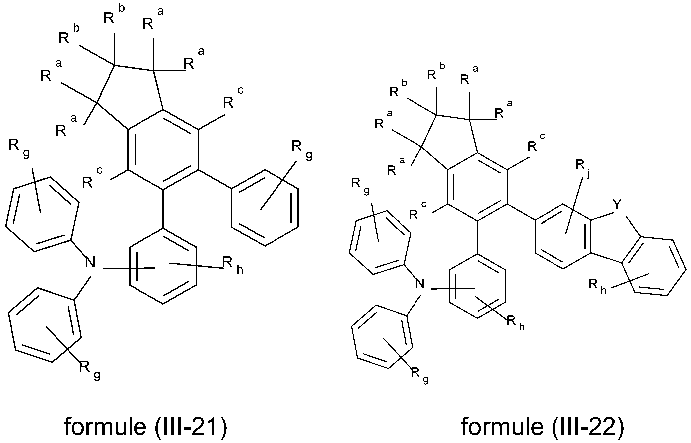 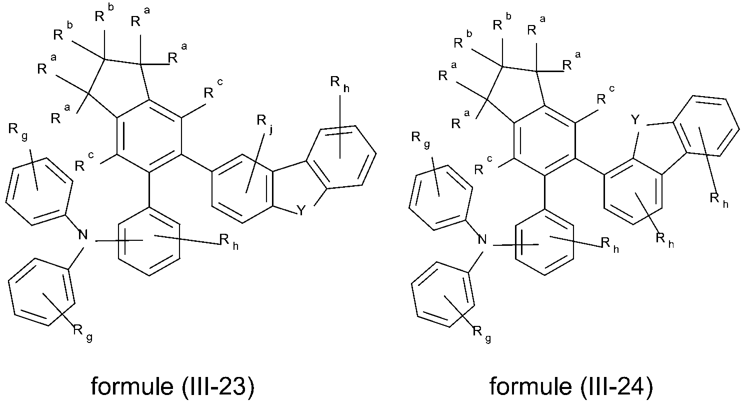 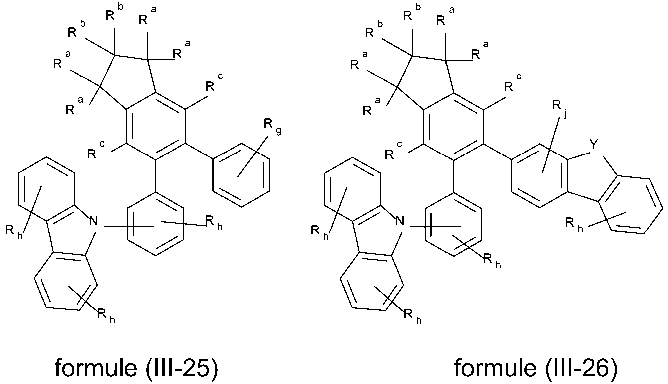 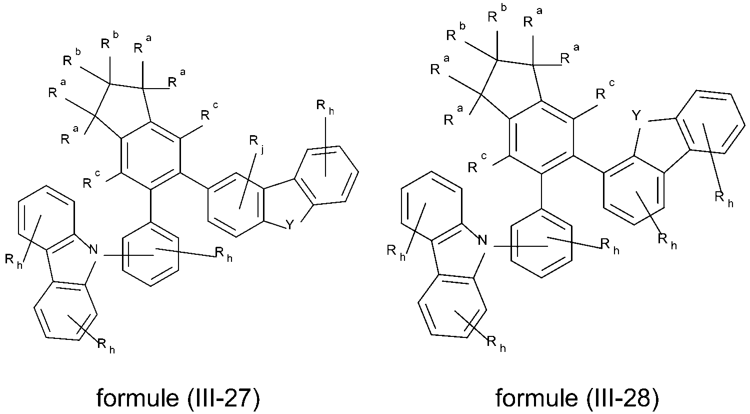 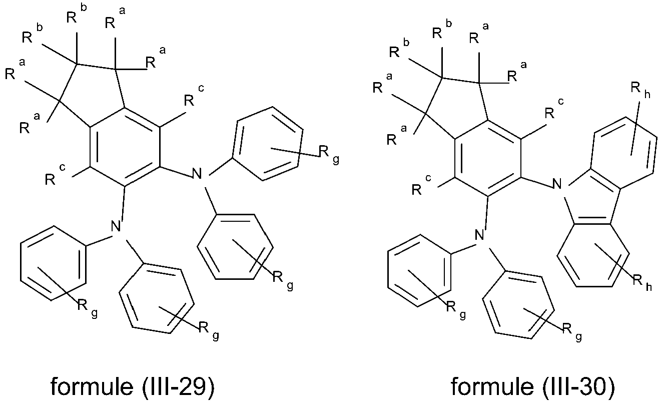 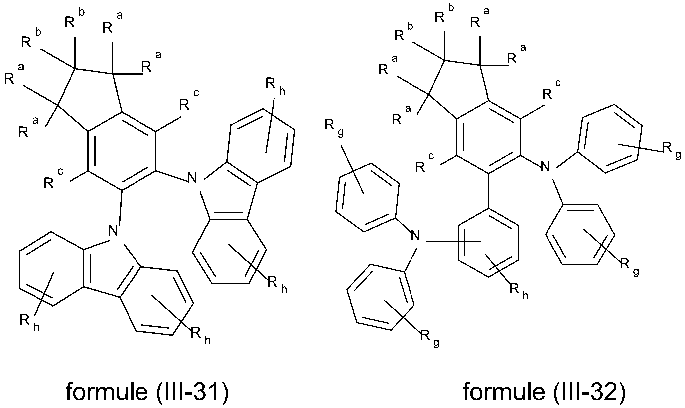 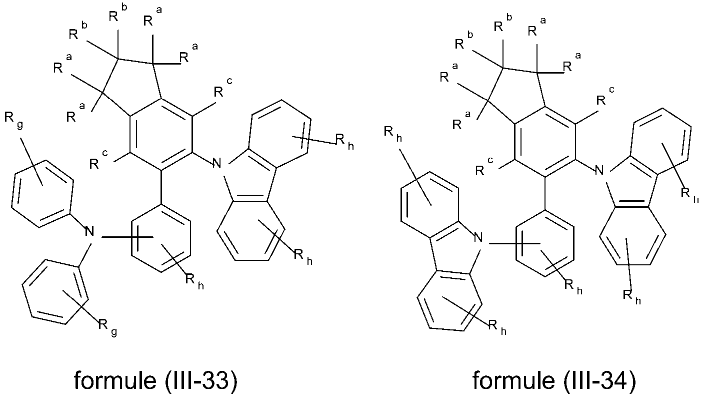 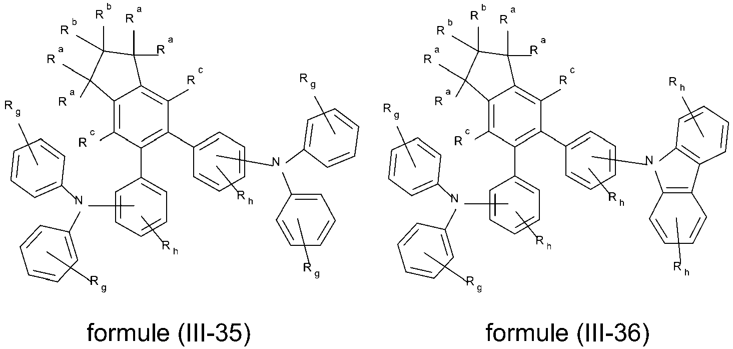 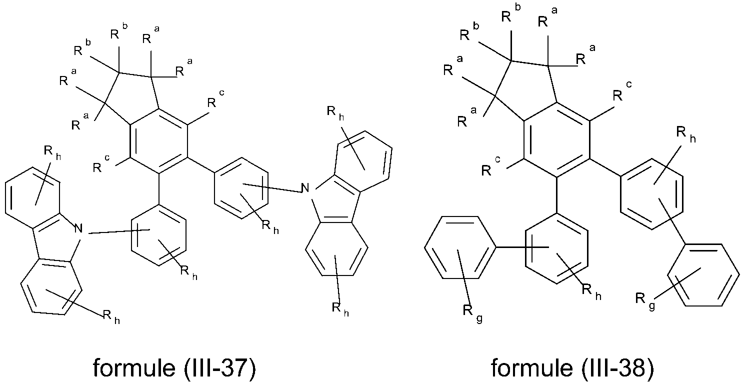 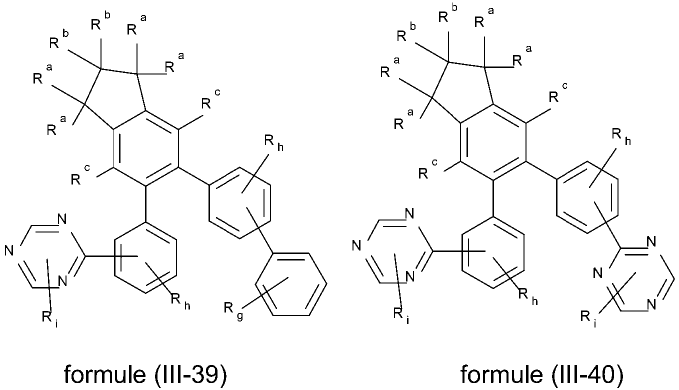 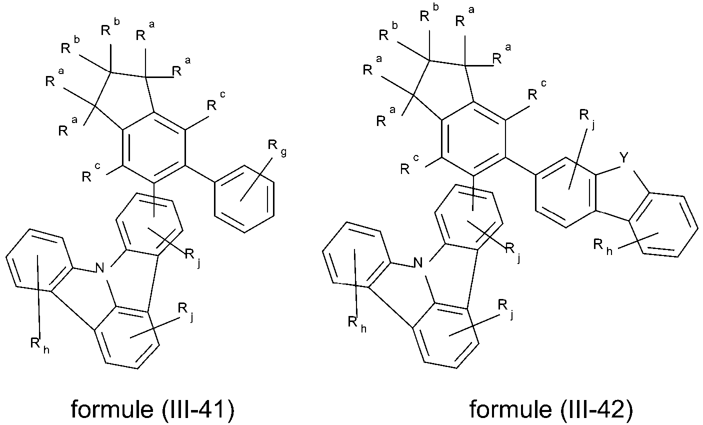 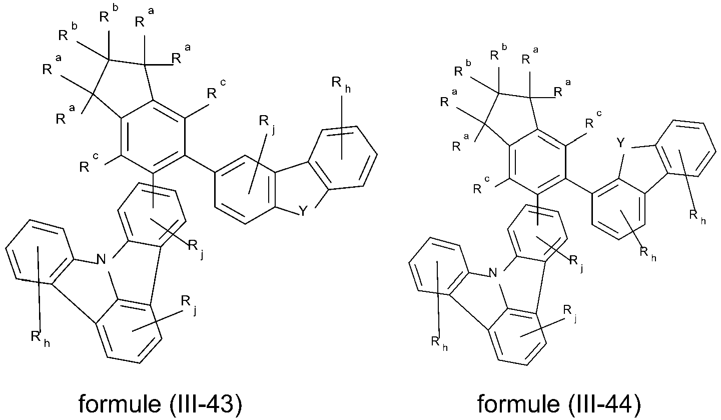 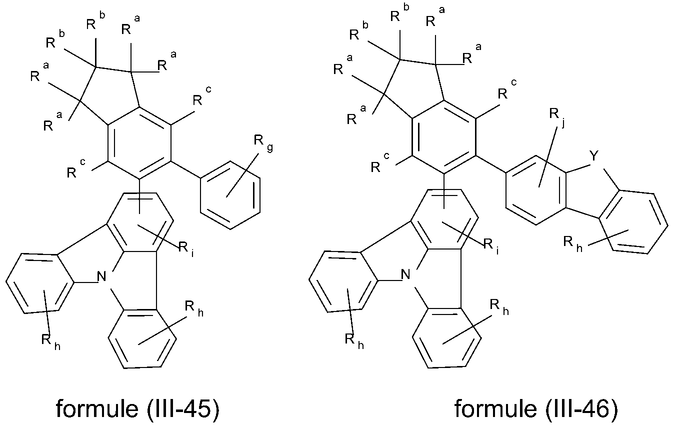 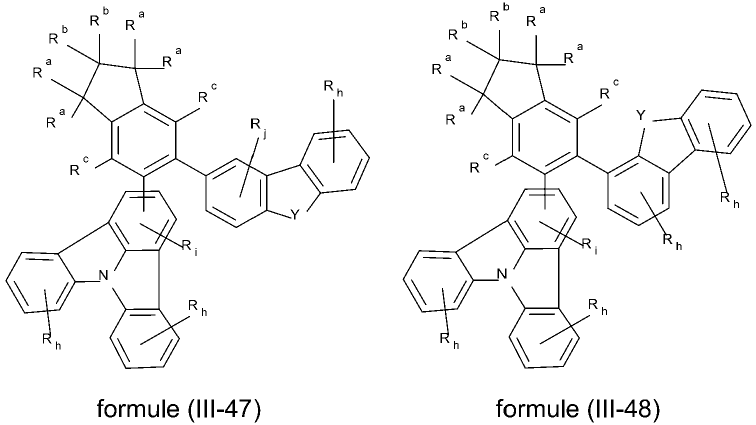 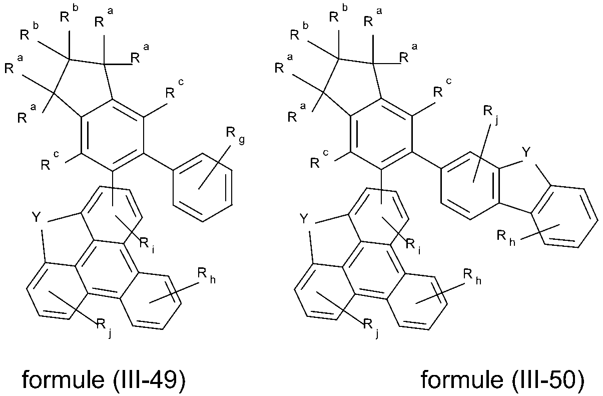 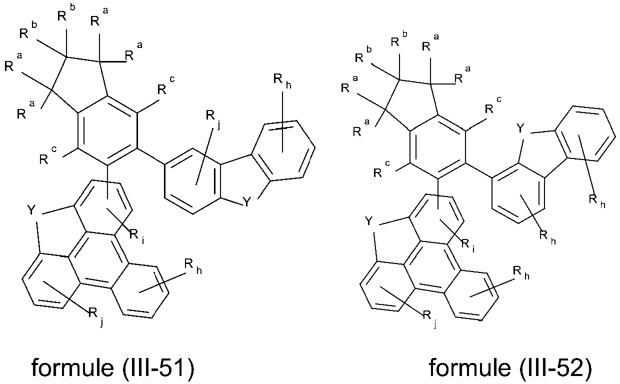 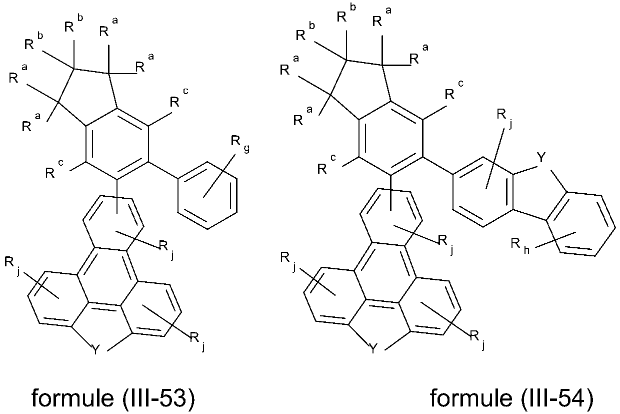 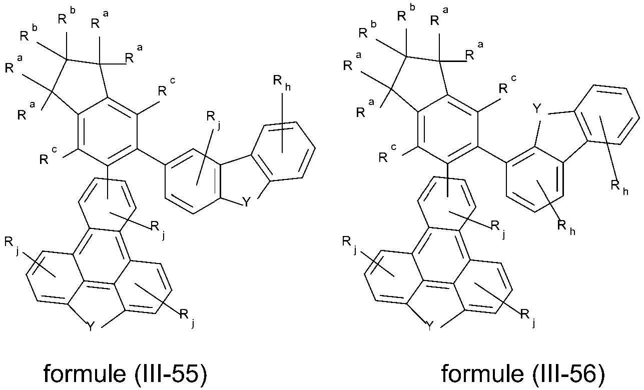 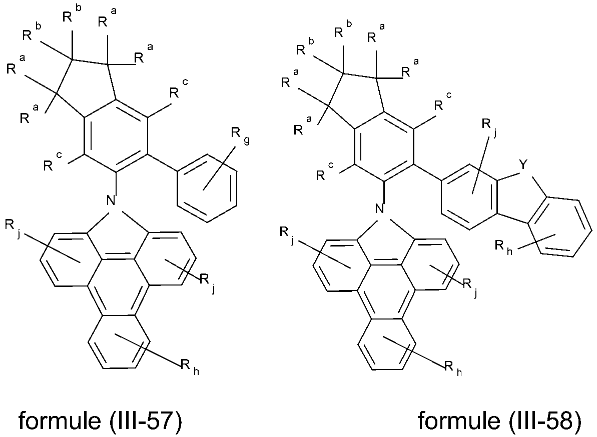 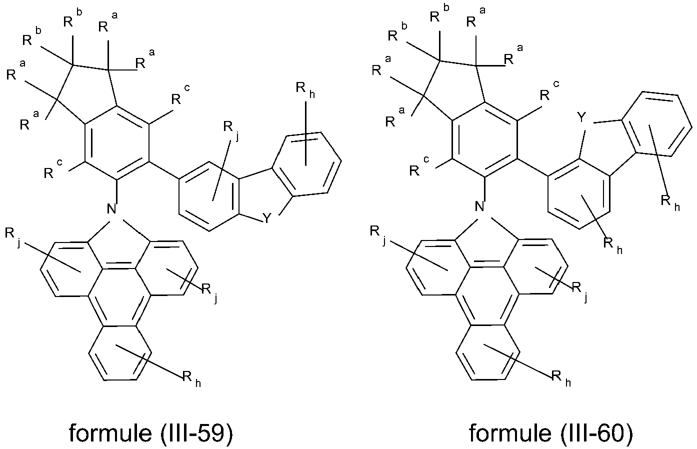 dans lesquelles les symboles R, R^{a}, R^{b} et R^{c} revêtent les significations données selon la revendication 1 et ce qui suit s'applique aux symboles utilisés :
Y est O, S, NR ou C(R)₂, préférablement O, NR ou C(R)₂ ;
i est à chaque occurrence, indépendamment, 0, 1 ou 2 ;
j est à chaque occurrence, indépendamment, 0, 1, 2 ou 3 ;
h est à chaque occurrence, indépendamment, 0, 1, 2, 3 ou 4 ;
g est à chaque occurrence, indépendamment, 0, 1, 2, 3, 4 ou 5.

14. Oligomère, polymère ou dendrimère contenant un ou plusieurs composés selon l'une des revendications 1 à 13, où, au lieu d'un atome d'hydrogène ou d'un substituant, une ou plusieurs liaisons sont présentes entre les composés et le polymère, l'oligomère ou le dendrimère.

15. Formulation comprenant au moins un composé selon l'une ou plusieurs parmi les revendications 1 à 13 ou un oligomère, polymère ou dendrimère selon la revendication 14 et au moins un composé supplémentaire, où le composé supplémentaire est préférablement choisi parmi un ou plusieurs solvants.

16. Composition comprenant au moins un composé selon l'une ou plusieurs parmi les revendications 1 à 13 ou un oligomère, polymère ou dendrimère selon la revendication 14 et au moins un composé supplémentaire choisi dans le groupe constitué par les émetteurs fluorescents, les émetteurs phosphorescents, les émetteurs présentant une TADF, les matériaux hôtes, les matériaux de transport d'électrons, les matériaux d'injection d'électrons, les matériaux conducteurs de trous, les matériaux d'injection de trous, les matériaux de blocage d'électrons et les matériaux de blocage de trous, préférablement les matériaux hôtes.

17. Procédé de préparation d'un composé selon l'une ou plusieurs parmi les revendications 1 à 13, **caractérisé en ce qu'**un composé phényle, sur lequel est condensé un groupement cyclopentyle, est synthétisé et au moins un radical aromatique ou hétéroaromatique est introduit, préférablement au moyen d'une réaction de substitution aromatique nucléophile ou d'une réaction de couplage.

18. Utilisation d'un composé selon l'une ou plusieurs parmi les revendications 1 à 13 ou d'un oligomère, polymère ou dendrimère selon la revendication 14, dans un dispositif électronique, préférablement comme matériau hôte, matériau de transport d'électrons, matériau d'injection d'électrons, matériau conducteur de trous, matériau d'injection de trous, matériau de blocage d'électrons, matériau de blocage de trous.

19. Dispositif électronique contenant au moins un composé selon l'une ou plusieurs parmi les revendications 1 à 13 ou un oligomère, polymère ou dendrimère selon la revendication 14.
